(19) Europäisches Patentamt
European Patent Office
Office européen des brevets

(11) EP 3 870 236 B1

(12) **EUROPEAN PATENT SPECIFICATION**

(45) Date of publication and mention of the grant of the patent:
**09.08.2023 Bulletin 2023/32**

(21) Application number: **19801109.0**

(22) Date of filing: **22.10.2019**

(51) International Patent Classification (IPC):
***A61K 47/69*** *(2017.01)*

(52) Cooperative Patent Classification (CPC):
(C-Sets available)
**A61K 31/5377; A61K 9/1075; A61K 45/06; A61K 47/34; A61P 29/00; C08F 220/34** (Cont.)

(86) International application number:
**PCT/IB2019/058992**

(87) International publication number:
**WO 2020/084471 (30.04.2020 Gazette 2020/18)**

(54) **NANOPARTICLE ENCAPSULATION TO TARGET G PROTEIN-COUPLED RECEPTORS IN ENDOSOMES**

NANOPARTIKELVERKAPSELUNG ZUR ABZIELUNG AUF PROTEINGEKOPPELTE REZEPTOREN IN ENDOSOMEN

ENCAPSULATION DE NANOPARTICULES POUR CIBLER DES RÉCEPTEURS COUPLÉS À DES PROTÉINES G DANS DES ENDOSOMES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2018 AU 2018903997**

(43) Date of publication of application:
**01.09.2021 Bulletin 2021/35**

(73) Proprietor: **Takeda Pharmaceutical Company Limited**
**Osaka-shi, Osaka 541-0045 (JP)**

(72) Inventors:
- **BUNNETT, Nigel**
  **Clayton, Victoria 3800 (AU)**
- **DAVIS, Thomas**
  **Clayton, Victoria 3800 (AU)**
- **WHITTAKER, Michael**
  **Clayton, Victoria 3800 (AU)**
- **VELDHUIS, Nicholas**
  **Clayton, Victoria 3800 (AU)**

(74) Representative: **Fish & Richardson P.C.**
**Highlight Business Towers**
**Mies-van-der-Rohe-Straße 8**
**80807 München (DE)**

(56) References cited:
**WO-A1-2016/025747 WO-A1-2017/143397**

- **WEIWEI GAO ET AL: "pH-Responsive Nanoparticles for Drug Delivery", MOLECULAR PHARMACEUTICS, vol. 7, no. 6, 6 December 2010 (2010-12-06), pages 1913-1920, XP055657768, US ISSN: 1543-8384, DOI: 10.1021/mp100253e**
- **KEJIN ZHOU ET AL: "Tunable, Ultrasensitive pH-Responsive Nanoparticles Targeting Specific Endocytic Organelles in Living Cells", ANGEWANDTE CHEMIE INTERNATIONAL EDITION, vol. 50, no. 27, 14 April 2011 (2011-04-14), pages 6109-6114, XP055169265, ISSN: 1433-7851, DOI: 10.1002/anie.201100884**
- **XIN MA ET AL: "Application of Nanoparticles for Targeting G Protein-Coupled Receptors", INTERNATIONAL JOURNAL OF MOLECULAR SCIENCES, vol. 19, no. 7, 10 July 2018 (2018-07-10), page 2006, XP055657820, DOI: 10.3390/ijms19072006**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PASRICHA PANKAJ J ET AL: "Aprepitant Has Mixed Effects on Nausea and Reduces Other Symptoms in Patients With Gastroparesis and Related Disorders", GASTROENTEROLOGY : OFFICIAL PUBLICATION OF THE AMERICAN GASTROENTEROLOGICAL ASSOCIATION, WILLIAMS & WILKINS, US, vol. 154, no. 1, 28 October 2017 (2017-10-28), page 65, XP085311721, ISSN: 0016-5085, DOI: 10.1053/J.GASTRO.2017.08.033**
- **THOMSEN ALEX R B ET AL: "Therapeutic Targeting of Endosomal G-Protein-Coupled Receptors", TRENDS IN PHARMACOLOGICAL SCIENCES, ELSEVIER, HAYWARTH, GB, vol. 39, no. 10, 1 September 2018 (2018-09-01), pages 879-891, XP085481163, ISSN: 0165-6147, DOI: 10.1016/J.TIPS.2018.08.003**


(52) Cooperative Patent Classification (CPC): (Cont.)

C-Sets
**A61K 31/5377, A61K 2300/00**

## Description

### Cross Reference to Related Applications

This application claims the benefit of Australian Provisional Application No. 2018903997 filed on October 22, 2018.

### Field of the invention

The present invention relates to compositions and their use in methods for modulating endosomal GPCR signaling. In particular, the present invention relates to use of polymeric nanoparticles for the targeted delivery of hydrophobic modulators of endosomal GPCRs and their use in the treatment of associated diseases and disorders.

### Background of the invention

The ability of cells to respond to the extracellular environment requires cell surface signaling proteins such as G protein-coupled receptors (GPCRs) and Receptor Tyrosine Kinases (RTKs such as EGF or insulin receptors) to bind extracellular ligands which rapidly initiate intracellular signaling pathways. Once a cellular response has been achieved, these signals can be terminated by coupling to β-arrestin1/2 adaptor proteins, and internalisation *via* clathrin/dynamin-mediated pathways for recruitment to the endosomal membrane network. Classical receptor signaling paradigms hypothesize that internalisation facilitates signal termination by disassembling receptor-bound ligand complexes in early endosomes and sorting receptors into recycling endosomes or toward the degradative lysosomal pathway. This hypothesis has been challenged by evidence of internalised receptors remaining associated with their cognate G proteins that can initiate sustained endosomal-derived signaling processes.

G protein-coupled receptors (GPCRs), the largest family of cell-surface receptors, participate in most pathophysiological processes and are the target of ~30% of therapeutic drugs (Audet, M. & Bouvier, M. Nat Chem Biol 2008, 4, 397-403). Cell-surface GPCRs interact with extracellular ligands and couple to heterotrimeric G proteins, which trigger plasma membrane delimited signals (second messenger formation, growth factor receptor transactivation, ion channel regulation). Ligand removal and receptor association with β-arrestins (βarrs) terminate plasma membrane signals.

Until recently, it was widely assumed that activation of GPCRs, subsequent downstream signaling and signal termination took place exclusively at the plasma membrane. Plasma membrane signaling is terminated within minutes of activation via phosphorylation of the receptor by GPCR kinases (GRKs) that are selective for the active ligand-bound receptor conformation. GRKs phosphorylate C-terminal S/T-rich domains of GPCRs (Sato, P. Y., et al. Physiological reviews 2015, 95, 377-404). Phosphorylated receptors then bind to βarr, which sterically prevents coupling between receptor and G-protein, thus terminating agonist-mediated G-protein activation. βarrs further promote the transfer of ligand-bound receptor from the cell surface to early endosomes via dynamin- and clathrin-dependent endocytosis. Once endocytosed, the ligand and phosphate groups are removed from the GPCR and the receptor is either rapidly redistributed to the cell membrane or it is transported to a lysosome for degradation.

Recently, however, it has been discovered that a diverse range of GPCRs do not always follow the conventional paradigm. Studies have found that following ligand binding and activation of the receptor, some cell surface GPCRs internalise and redistribute into early endosomes where heterotrimeric G protein signaling is maintained for an extended period of time. Accordingly, rather than merely acting as a conduit for GPCR trafficking to recycling or degradatory pathways, endosomes can be a vital site of signal transduction (Murphy, J. E. et al. Proc Natl Acad Sci USA 2009, 106, 17615-17622). By recruiting GPCRs and mitogen-activated protein kinases to endosomes, βarrs can mediate endosomal GPCR signaling (Murphy, J. E. et al. Proc Natl Acad Sci USA 2009, 106, 17615-17622; DeFea, K. A. et al. Proc Natl Acad Sci USA 2000, 97, 11086-11091; DeFea, K. A. et al. J Cell Biol 2000, 148, 1267-1281).

βarrs recruit diverse signaling proteins to activated receptors at plasma and endosomal membranes and are essential mediators of signaling. The MAPK cascades [ERK, c-Jun amino-terminal kinase (JNK), p38] are the most thoroughly characterized βarr-dependent signaling pathways. The first evidence that βarrs are active participants in signaling was the observation that dominant negative mutants of βarr inhibited $\beta_2$AR-induced activation of ERK1/2 (Daaka Y, et al. J Biol Chem 1998, 273, 685-688). Subsequently, βarrs were found to couple $\beta_2$AR to c-Src and mediate ERK1/2 activation (Lutterall L. M. et al. Science 1999, 283, 655-661). βarrs similarly participate in ERK1/2 signaling by other GPCRs, including neurokinin-1 receptor (NKiR), protease-activated receptor 2 ($PAR_2$), angiotensin II type 1A receptor ($AT_{1A}R$), and vasopressin V2 receptor ($V_2R$). These observations led to the view that βarrs are scaffolds that couple activated GPCRs with MAPK signaling complexes. βarrs thereby mediate a second wave of GPCR signaling that is distinct from G protein-dependent signaling at the plasma membrane.

Thomsen ARB et al. describe in Trends in Pharmacological Sciences, vol. 39, no. 10, 1 September 2018, on pages 879-891 a therapeutic targeting of endosomal G-protein-coupled receptors.

Weiwei G et al. describe in Molecular Pharmaceutics, vol. 7, no. 6, 6 December 2010, on pages 1913-1920 pH-responsive nanoparticles for drug delivery.

Kejin Z et al. describe in Angewandte Chemie International Edition, vol. 50, no. 27, 14 April 2011, on pages 6109-6114 tunable, ultrasensitive pH-responsive nanoparticles targeting endocytic organelles in living cells.

WO 2016/025747 A1 describes non-linear copolymers as intracellular delivery vehicles. Xin M et al. describe in International Journal of Molecular Sciences, vol. 19, no. 7, 10 July 2018 (2018-07-10), on page 2006 an application of nanoparticles for targeting G-protein-coupled receptors.

Accordingly, modulating endocytosed GPCRs may advantageously provide novel compositions for use in methods of treating the vast number of diseases and disorders linked to this large family of receptors.

## Summary of the invention

The present invention is defined by the independent claims. The dependent claims depict additional embodiments of the invention.

New compositions and methods are provided to deliver modulators of GPCRs to the endosomal lumen, where they are able to interact with the endocytosed receptor.

Accordingly, in one aspect the present invention provides an aqueous liquid comprising polymeric nanoparticles of copolymer chains assembled to form a core / shell structure, the copolymer chains having:

(i) an acid-responsive hydrophobic polymer block that forms the core of the nanoparticles, wherein the acid-responsive hydrophobic polymer block comprises tertiary amine functional groups that are protonated in an acidic environment; and

(ii) a hydrophilic polymer block that forms the shell of the nanoparticles and is solvated by the aqueous liquid,

wherein the nanoparticles contain within their core a hydrophobic modulator of endosomal GPCR signaling, or a pharmaceutically acceptable salt thereof.

In one embodiment, the polymeric nanoparticles disclosed herein are micelles.

In some embodiments, the tertiary amine functional groups are protonated at about pH = 6.1.

In some embodiments, the acid-responsive hydrophobic polymer block comprises a homopolymer or copolymer of 2-(diisopropylamino)ethyl methacrylate (DiPAEMA).

In some embodiments, the acid-responsive hydrophobic polymer block comprises a copolymer of 2-(diisopropylamino)ethyl methacrylate (DiPAEMA) and a polyalkyleneoxide methacrylate.

In some embodiments, the hydrophilic polymer block comprises a copolymer of poly(ethylene glycol)methacrylate (PEGMA) and 2-(dimethylamino)ethyl methacrylate (DMAEMA).

In some embodiments, the hydrophobic modulator of endosomal GPCR signaling or pharmaceutically acceptable salt thereof is an inhibitor of an endosomal GPCR.

In some embodiments, the hydrophobic modulator of endosomal GPCR signaling or pharmaceutically acceptable salt thereof is an inhibitor of endosomal NKiR signaling, or pharmaceutically acceptable salt thereof.

In some embodiments, the hydrophobic modulator of endosomal GPCR signaling or pharmaceutically acceptable salt thereof is selected from the group consisting of aprepitant, fosaprepitant, tradipitant, maropitant, HTX-019, netupitant, serlopitant, orvepitant, NAS-911B, ZD-6021, KD-018, DNK-333, NT-432, NK-949, NT-814, EU-C-001, vestipitant, 1144814, SCH-900978, AZD-2738, BL-1833, casopitant, AV-810, KRP-103, 424887, cizolirtine, vofopitant, L-742694, capsazepine, GR-82334, MEN-11149, L-732138, NiK-004, TA-5538, CP-96345, lanepitant, LY-2590443, dapitant, burapitant, befetupitant, CJ-17493, AVE-5883, CGP-49823, CP-122721, CP-99994, SLV-317, TAK-637, L-733060, L-703606, dilopetine, MPC-4505, L-742311, FK-888, WIN-64821, NIP-530, SLV-336, ezlopitant, TKA-457, figopitant, ZD-4794, CP-100263, GR-203040, L-709210, MEN-10930, MEN-11467, LY-306740, FK-355, WIN-67689, WIN-51708, FK-224, BL-1832, CAM-6108, CP-98984, WS-9326A, L-741671, L-737488, L-740141, L-760735, L-161664, YM-49244, Sch-60059, SDZ-NKT-343, S-18523, RPR-111905, S-19752, L-161644, LY-297911, RPR-107880, L-736281, anthrotainin, RP-73467, WIN-64745, WIN-68577, WIN-62577 WIN-66306, RP-67580, CP-0364, L-743986, S-16474, CGP-47899, FR-113680, YM-44778, GR-138676, CGP-73400, CAM-2445, MDL-105172A, L-756867, isbufylline, R-673, SR-48968 and SR-14033, GW679769, CP-0578, and a pharmaceutically acceptable salt thereof.

In some embodiments, the hydrophobic modulator of endosomal GPCR signaling or pharmaceutically acceptable salt thereof is an inhibitor of endosomal CGRP and/or CLR signalling or pharmaceutically acceptable salt thereof.

In some embodiments, the hydrophobic modulator of endosomal GPCR signaling or pharmaceutically acceptable salt thereof is selected from the group consisting of BI 44370, MK-3207, olcegepant, ubrogepant, rimegepant, SB-268262, telcagepant, and a pharmaceutically acceptable salt thereof.

In some embodiments, the aqueous liquid further comprises a second hydrophobic modulator of endosomal GPCR signaling or a pharmaceutically acceptable salt thereof, wherein the second hydrophobic modulator of endosomal

GPCR signalling or pharmaceutically acceptable salt thereof is contained within the core of the polymeric nanoparticles.

In a further aspect of the invention there is provided a pharmaceutical composition comprising the aqueous liquid described above and, optionally, a pharmaceutically acceptable excipient.

Accordingly, in a further aspect of the invention there is provided an aqueous liquid described above for use in a method for the treatment of a disease or disorder mediated by endosomal NKiR signaling, the method comprising administering to a subject in need thereof an effective amount of the aqueous liquid. The disease or disorder mediated by endosomal NKiR signaling is selected from chemotherapy-induced nausea and vomiting (CINV), cyclic vomiting syndrome, postoperative nausea and vomiting, affective and addictive disorders including depression and anxiety, generalised anxiety disorder (GAD), gastrointestinal disorders including inflammatory bowel disease, irritable bowel syndrome, gastroparesis and functional dyspepsia, chronic inflammatory disorders including arthritis, respiratory disorders including COPD and asthma, urogenital disorders, sensory disorders and pain including somatic pain and visceral pain, pruritus, viral and bacterial infections and proliferative disorders (cancer), and combinations thereof. In one embodiment, the method further comprises administering to the subject an effective amount of a second modulator of endosomal GPCR signaling or a pharmaceutically acceptable salt thereof.

In a further aspect of the invention there is provided an aqueous liquid described above for use in a method for the treatment of a disease or disorder mediated by endosomal CGRP receptor signalingand/or CLR signalling, the method comprising administering to a subject in need thereof an effective amount of the aqueous liquid. The disease or disorder mediated by endosomal CGRP receptor and/or CLR signaling is selected from the group consisting of: migraine and symptoms associated with migraine including pain, photophobia, phonophobia, nausea and vomiting, sensory disorders, pain including somatic pain and visceral pain, pain associated with cluster and chronic daily headache, respiratory disorders including COPD and asthma, gastrointestinal disorders including inflammatory bowel disease, irritable bowel syndrome, gastroparesis and functional dyspepsia, and chronic inflammatory disorders including osteoarthritis and rheumatoid arthritis.

Calcitonin gene-related peptide ("CGRP") is known to be expressed throughout the nervous system. The CGRP receptor includes calcitonin receptor-like receptor ("CLR"), a GPCR, and receptor activity modifying protein 1 (RAMP1), a single transmembrane protein that imparts ligand specificity and ensures CLR targeting to the cell surface. Noxious stimuli evoke CGRP release from the terminals of primary sensory neurons in the dorsal horn of the spinal cord and in peripheral tissues. CGRP activates CLR/RAMP1 on spinal neurons to induce nociception and on peripheral arterioles to cause neurogenic inflammation. See Yarwood, et al., (Proc. Natl. Acad. Sci. USA. 114(46):12309-12314.)

In yet another aspect of the invention there is provided a pharmaceutical composition comprising the aqueous liquid according to the invention.

These and other aspects of the present invention will become more apparent to the skilled addressee upon reading the following detailed description in connection with the accompanying examples and claims.

## Brief Description of the Drawings

The invention will herein be described by way of example only with reference to the following non-limiting Figures in which:

Figure 1 illustrates a DIPMA and BMA nanoparticles schematic. A) DIPMA nanoparticles are comprised by a hydrophobic core of P(PEGMA-co-DMAEMA) and a hydrophilic shell of P(DIPMA-co-DEGMA). While **B**) BMA nanoparticles are formed by the same hydrophilic shell but replace the hydrophobic core by BMA. C) illustrates TEM images of DIPMA nanoparticles. The top images show DIPMA-Ap loaded nanoparticles and the bottom images show DIPMA-empty nanoparticles. **D**) illustrates a graphical representation showing pH disassembly of DIPMA and BMA nanoparticles. 200 $\mu$g of DIPMA and BMA nanoparticles loaded with Nile red were added into PBS solutions of different pH ranging from 7.4 to 5.0. Disassembly of 50% of DIPMA-Nr nanoparticles is observed at pH 6.08 $\pm$ 0.064, while BMA-Nr did not show disassembly properties with pH changes. Mean $\pm$ SEM. n = 3.

Figure 2 illustrate a graphical representation of $^1$H-NMR spectra. **A**) Polymerization of the cationic monomer DMAEMA and the hydrophilic monomer PEGMA to form P(PEGMA-co-DMAEMA). **B**) Chain extension of P(PEGMA-co-DMAEMA) by the addition of the pH responsive monomer DIPMA and the charge screening monomer DEGMA to form P(PEGMA-co-DMAEMA)-b-(DIPMA-co-DEGMA). **C**) Chain extension of P(PEGMA-co-DMAEMA), with BMA to form the non pH-responsive copolymer P(PEGMA-co-DMAEMA)-b-(BMA).

Figure 3 illustrates a graphical representation of GPC traces of P(PEGMA-co-DMAEMA) and resultant diblock copolymers P(PEGMA-co-DMAEMA)-b-P(DIPMA-co-DEGMA) and P(PEGMA-co-DMAEMA)-b-P(BMA-co-DEGMA). A shift to higher molecular weight (i.e., shorter retention time) was observed after the chain extension with the monomers DIPMA and DEGMA to form P(PEGMA-co-DMAEMA)-b-P(DIPMA-co-DEGMA) and BMA to

form P(PEGMA-co-DMAEMA)-b-P(BMA-co-DEGMA).

Figure 4 illustrates images showing the uptake of DIPMA nanoparticles labelled with Cy5 on HEK-293 cells transfected with NKiR -GFP and stimulated with 10 nM of SP. DIPMA-Cy5 nanoparticles were incubated 40 min prior 10 nM SP addition. Images were captured **A**) 30 min and **B**) 60 min after SP addition. NKiR-GFP localized on the plasma membrane as well as endosomes is observed. Co-distribution of DIPMA-Cy5 nanoparticles with NKiRGFP positive endosomes is observed at all time points chosen. Scale bar = 1 $\mu$m

Figure 5 illustrates a graphical representation of concentration dose response curves of nuclear ERK stimulated by SP and inhibited by Ap. Nuclear ERK response over time using **A**) different concentrations of SP. **B**) Pre incubating with different concentrations of Ap, followed by 5 nM SP stimulation. **C**) AUC of the of the 65 min time traces for nuclear ERK responses of SP and Ap nuclear. Results are expressed as normalized values by the maximum nuclear ERK response to PDBu 1 $\mu$M. Mean $\pm$ SEM. n = 6.

Figure 6 illustrates a graphical representation of intracellular calcium measurements in HEK-293 and HEK-hNKiR cells stimulated with empty nanoparticles. Baseline was measured for 5 min prior the addition of 10, 3.16 and 1 $\mu$g of nanoparticles **A**) and **B**) correspond to i[Ca+2] measurements in HEK-293 and HEK-hNKiR cells, respectively, stimulated with DIPMA and BMA nanoparticles. **C**) and **D**) show AUC of **A**) and **B**). Data are shown as mean $\pm$ SEM. n = 4.

Figure 7 illustrates the effect of empty nanoparticles of nuclear ERK signaling and cell viability on HEK- hNKiR cells. **A**) Nanoparticles were incubated for 30 min prior measurement of nuclear ERK activation triggered by empty nanoparticles indicating no activation of nuclear ERK. **B**) AUC of nuclear ERK time traces (n=6). **C** and **D**) Effect of nanoparticles on the reducing power of cells assessed using the alamarBlue cell viability reagent (n=4). Data are shown as mean $\pm$ SEM.

Figure 8 illustrates a graphical representation of nuclear ERK measurements in HEK-hNKiR cells stimulated with 10 $\mu$g of empty DIPMA and BMA nanoparticles. Nanoparticles were added 30 min prior baseline reading. **A**) Baseline was measured for 5 min followed by the addition of 5 nM of SP. **B**) Area under the curve of time traces shown in A. Data are shown as mean $\pm$ SEM. n = 2

Figure 9 illustrates a graphical representation of the assessment of the locomotor patterns after administration of DIPMA nanoparticles. Animals were treated with DIPMA-Ap and control nanoparticles and latency to fall was recorded 30-240 min post treatment. Nanoparticles did not produce difference in the latency to fall when compared to vehicle. Mean $\pm$ SEM. n = 3.

Figure 10 illustrates distribution of DIPMA-Cy5 nanoparticles after intrathecal administration. **A**) DIPMA-Cy5 administration showed a localized fluorescence within the site of injection (L3-L4) that starts to decrease 2 h post administration and is lost completely after 24 h. **B**) Quantification of the radiant efficiency of the images shown in **A.** Mean $\pm$ SEM. n = 3. **C**) Locomotor activity of animals treated with nanoparticles assessed using Rotarod (n=3).

Figure 11 illustrates the assessment of the analgesic potential of DIPMA-Ap nanoparticles in a capsaicin evoked acute pain model. Nanoparticles were administered i.t 30 min prior i.pl capsaicin injection and mechanical hyperalgesia was assessed using Von Frey filaments on the **A**) Capsaicin stimulated paw (Ipsilateral) and **B**) Unstimulated paw (Contralateral). Values Data are shown as percentage of basal Von Frey response. Mean $\pm$ SEM. n = 6

Figure 12 illustrates the assessment of the analgesic potential of DIPMA-$A_p$ nanoparticles in a chronic pain model assessed using a Randall-Selitto electronic algesimeter following administration of **A**) 100, 300 nM and 1 $\mu$M aprepitant (n =4 per concentration), **B**) 10, 30 or 50 $\mu$g of BMA nanoparticles loaded with 100, 300 or 500nM of aprepitant (n=6 per each concentration), or **C**) 10, 30 or 50 $\mu$g of acid-responsive DIPMA nanoparticles loaded with 100, 300 or 500nM of aprepitant (n=6 per each concentration).

Figure 13 illustrates graphical representation of mitochondrial function as an indicator of cell viability in cells treated with DEAEMA and DIPMA particles. Cell viability was determined by measuring mitochondrial activity using CellTiter Glo. Cells were treated up to 24 H with DEAEMA (**A**-**D**) or DIPMA (**E**-**H**) nanoparticles containing 0%, 5%, 10%, 20% and 50% of DEGMA at increasing. The cell viability was expressed as a percentage of vehicle controls. Mean $\pm$ S.E.M; n=4-5 individual experiments.

Figure 14 illustrates graphical representation of nuclear membrane permeability as an indicator of cell death in cells treated with DEAEMA and DIPMA particles. Cell death was determined by measuring nuclear membrane permeability using propidium iodide. Cells were treated up to 24 H with DEAEMA (**A**-**D**) or DIPMA (**E**-**H**) nanoparticles containing 0%, 5%, 10%, 20% and 50% of DEGMA at increasing. The cell death was expressed as a percentage of 0.2% Triton X-100 (maximal cell death). Mean ± S.E.M; n=4-5 individual experiments.

Figure 15 illustrates the cell viability assay results of DEAMA and DIPMA nanoparticles at different concentrations after exposures of 12 hrs, 24 hrs, 48 hrs, and 72 hrs.

Figure 16 illustrates the uptake and disassembly of DIPMA nanoparticles in HEK-293 cells. **A)** shows the uptake of DIPMA-Cy5 nanoparticles into cells expressing Rab5-GFP. **B**) shows the colocalization of DIPMA-Cy5 nanoparticles in Rab5-GFP early endosomes and Rab7-GFP late endosomes after 30 min. **C)** shows the colocalization of DIPMA-Cy5 nanoparticles in endosomes containing NKiR-GFP at 30 min after stimulation with SP to induce NKiR endocytosis and addition. **D)-F)** shows the uptake of DIPMA-CO nanoparticles, determined by high content imaging (**D, E**) and confocal microscopy **(F). D**) shows time course of Coumarin 153 cellular fluorescence; **E**) shows integrated response over 30 min. PitStop2 (PS2), Dyngo4a (Dy4) and Bafilomycin A (BFA) inhibited the accumulation of Coumarin 153 cellular fluorescence. A, B, C, F, representative results, n=4-5 experiments; D, E, n=4-5 experiments.

Figure 17 illustrates the uptake and disassembly of DIPMA nanoparticles in HEK-293 cells. **A)** shows the colocalization of DIPMA-Cy5 nanoparticles in Rab5-GFP early endosomes and Rab7-GFP late endosomes after 60 min. **B)** shows the colocalization of DIPMA-Cy5 nanoparticles in endosomes containing NKiR-GFP at 60 min after stimulation with SP to induce NKiR endocytosis. addition. Representative results, n=4-5 experiments.

Figure 18 illustrates the effects of DIPMA-AP and BMA-AP nanoparticles on acute inflammatory and neuropathic pain. **A)** In the capsaicin (CAP)-evoked model of acute nociception in mice, vehicle (Veh), AP or the nanoparticles were administered by intrathecal (i.t.) injection 30 min before intraplantar (i.pl.) injection of capsaicin. In the CFA-evoked model of sustained inflammatory pain in mice, CFA was administered by intraplantar injection; after 48 h, vehicle, AP or the nanoparticles were administered by intrathecal injection. The SNS model of chronic neuropathic pain was studied in rats. Vehicle, AP or the nanoparticles were administered by intrathecal injection 10 days after SNS. Paw withdrawal responses were assessed using Von Frey filaments (VFF) in mice and the Randall-Selitto test in rats. **B**) and **C**) show capsaicin-induced mechanical allodynia in mice. **D)-F)** show CFA-evoked mechanical hyperalgesia in mice. **G)-I)** show SNS-evoked mechanical hyperalgesia in rats. (n) denotes animal numbers. n = 6. ### = ****, # = ***. $P < 0.005$.

Figure 19 illustrates the effects of DIPMA-AP and BMA-AP nanoparticles on neuropahic pain in the SNS model. The SNS model of chronic neuropathic pain was studied in rats. Vehicle, AP, DIPMA-AP, or BMA-AP was administered by intrathecal injection 10 days after SNS. Paw withdrawal responses were assessed using the Randall-Selitto test. **A)** shows the variation of withdrawal threshold over the time course. **B**) is an area under curve (AUC) plot from 0-7 h. (n) denotes animal numbers. n = 6. # # = ****, # = ***. $P < 0.005$.

Figure 20 illustrates the sensitization and activation of pain transmission by DIPMA-AP and BMA-AP nanoparticles. **A)-F)** show C-fiber reflex and wind-up in SNS rats. C-fiber reflexes (**A**-**C**) and wind-up (**D-F**) were measured at 10 d after SNS. Vehicle (Veh), AP or DIPMA-AP was administered by intrathecal injection. **A**) and **D**) show representative recordings comparing AP and DIPMA-AP. **B**) and **E**) illustrate time course of effects. **C**) and **F**) show integrated responses. (n) animal numbers. n = 5. ## = ****, $P<0.05$. **G**)-**I**) illustrate cell-attached patch-clamp recordings of SP-induced excitation of neurons in slices of rat spinal cord. **G**) shows representative traces, n= 6-8 experiments. **H**) compares normalized firing rate. **I**) compares firing time. n=6-8. ** $P<0.05$.

Figure 21 illustrates the antagonism of $NK_1R$ signaling in endosomes of HEK293 cells by DIPMA-AP and BMA-AP nanoparticles. A)-C) illustrate SP-induced activation of nuclear ERK in HEK-$hNK_1R$ cells expressing dynamin wildtype (Dyn WT, **A**) or dynamin K44E (Dyn K44E, **B**)**. C**) shows SP concentration-response curves (n=6). **D**) and **E**) illustrate the lack of effects of BMA-empty and DIPMA- empty on nuclear ERK activity in HEK293 cells over 30 min (n=6-7). **F**) shows the lack of effects BMA-empty and DIPMA- empty on viability of HEK293 cells over 24 and 48 h (n=4). **G**)-**I**) illustrate the effects of free AP, BMA-AP and DIPMA-AP on SP-induced activation of nuclear ERK in HEK-hNKiR cells. Cells were incubated with AP or nanoparticles for 30 min; they were either challenged with SP (no wash, **G**), or were washed, recovered in antagonist-free medium, and then challenged with SP (post-wash, **H**). **I**) is the AUC of 30 min ERK assays. Results are expressed as normalized values by the maximum nuclear ERK response to PDBu 1 μM. (n=6). $P<0.05$.

Figure 22 illustrates SP-induced activation of nuclear ERK in HEK-hNKiR cells. **A**) shows effects of graded concentrations of SP on nuclear ERK activity. **B**) shows SP concentration-response curves. **C**) illustrates effects of graded concentrations of AP on nuclear ERK response to SP (5 nM). **D**) shows AP concentration-response curves. (n=7-8 experiments).

Figure 23 illustrates the assessment of the analgesic potential of acid-responsive DIPMA-(MK-3207) nanoparticles in a CFA model with mechanical allodynia assessed by Von Frey hairs following administration of **A**) 30 nM, 100 nM, 300 nM, and 1000 nM of MK-3207 (n = 4 per concentration), and **B**) acid-responsive DIPMA nanoparticles loaded with 30 nM, 50 nM, and 100 nM of MK-3207 (n=4 per each concentration).

Figure 24 illustrates the concentration of loaded aprepitant and MK-3207 (μM) in 1mg/mL DIPMA polymer composition against the initial loading ratio of aprepitant/MK-3207 (LC-MS assessment). Mean -/+ SD, n = 4

Figure 25 illustrates the assessment of the analgesic potential of dual-loaded DIPMA-(Ap+MK-3207) nanoparticles in a CFA model with mechanical allodynia assessed by Von Frey hairs following administration of 120 nM of aprepitant + 120 nM of MK-3207 (n=4); and acid-responsive DIPMA nanoparticles loaded with (82 nM of aprepitant + 12 nM of MK-3207), (81 nM of aprepitant + 25 nM of MK-3207), and (80 nM of aprepitant + 81 nM of MK-3207) (* $P<0.05$, ** $P<0.01$, #$P<0.001$; 2-way ANOVA, Dunnett's post-test).

Figure 26 illustrates the assessment of the analgesic potential of co-administering DIPMA-Ap nanoparticles and DIPMA-(MK3207) nanoparticles in a CFA model with mechanical allodynia assessed by Von Frey hairs following administration of 120 nM of aprepitant + 120 nM of MK-3207 (n=4); and DIPMA-Ap nanoparticles (30 nM in Ap) + DIPMA-(MK-3207) nanoparticles (30 nM in MK-3207), DIPMA-Ap nanoparticles (60 nM in Ap) + DIPMA-(MK-3207) nanoparticles (60 nM in MK-3207), and DIPMA-Ap nanoparticles (120 nM in Ap) + DIPMA-(MK-3207) nanoparticles (120 nM in MK-3207) (* $P<0.05$, #P<0.001; 2way ANOVA, Dunnett's post-test).

**Detailed description of the invention**

In this specification a number of terms are used which are well known to a skilled addressee. Nevertheless for the purposes of clarity a number of terms will be defined.

The term "halo" refers to fluoro (F), chloro (Cl), bromo (Br), or iodo (I).

The term "acyl" refers to a radical of formula -C(=O)-R wherein R is a chemical substituent. As non-limiting examples, R can be independently selected from H, alkyl, haloalkyl, aryl, heteroaryl, cycloalkyl, and heterocyclyl, wherein each R is optionally substituted as defined elsewhere herein. Non-limiting examples of acyl include -C(=O)Me and -C(=O)Ph.

The term "acyloxy" refers to a radical of formula -C(=O)-OR wherein R is a chemical substituent. R can be independently selected from H, alkyl, haloalkyl, aryl, heteroaryl, cycloalkyl, and heterocyclyl, wherein each R is optionally substituted as defined elsewhere herein. Non-limiting examples of acyloxy include -C(=O)OMe and -C(=O)OPh.

The term "amino" refers to a nitrogen centered radical of the formula $-NR_2$, wherein each R is independently a substituent. As non-limiting examples, each R can be independently selected from H, alkyl, haloalkyl, aryl, heteroaryl, cycloalkyl, and heterocyclyl, wherein each R is optionally substituted as defined elsewhere herein. Non-limiting examples of amino include $N(alkyl)_2$ (e.g., $NMe_2$, $NiPr_2$), $NH_2$, NH(alkyl), and $N(Ph)_2$.

The term "alkyl" refers to a hydrocarbon chain that may be a straight chain or branched chain, containing the indicated number of carbon atoms. For example, $C_{1-10}$ indicates that the group may have from 1 to 10 (inclusive) carbon atoms in it. Non-limiting examples include methyl, ethyl, *iso*-propyl, *tert*-butyl, *n*-hexyl.

The term "haloalkyl" refers to an alkyl, in which one or more hydrogen atoms is/are replaced with an independently selected halo.

The term "alkoxy" refers to an -O-alkyl radical (e.g., $-OCH_3$).

The term "haloalkoxy" refers to an -O-haloalkyl radical (e.g., $-OCH_3$).

The term "alkylene" refers to a branched or unbranched divalent alkyl (e.g., $-CH_2-$).

The term "arylene" and the like refer to divalent forms of the ring system, here divalent aryl.

The term "alkenyl" refers to a hydrocarbon chain that may be a straight chain or branched chain having one or more carbon-carbon double bonds. The alkenyl moiety contains the indicated number of carbon atoms. For example, $C_2$-$_6$ indicates that the group may have from 2 to 6 (inclusive) carbon atoms in it.

The term "alkynyl" refers to a hydrocarbon chain that may be a straight chain or branched chain having one or more carbon-carbon triple bonds. The alkynyl moiety contains the indicated number of carbon atoms. For example, $C_{2-6}$ indicates that the group may have from 2 to 6 (inclusive) carbon atoms in it.

The term "aryl" refers to a 6-carbon monocyclic, 10-carbon bicyclic, or 14-carbon tricyclic aromatic ring system

wherein 0, 1, 2, 3, or 4 atoms of each ring may be substituted by a substituent, and wherein the ring comprising a monocyclic radical is aromatic and wherein at least one of the fused rings comprising a bicyclic or tricyclic radical is aromatic e.g. tetrahydronaphthyl. Examples of aryl groups also include phenyl, naphthyl and the like.

The term "cycloalkyl" or "carbocyclyl" as used herein includes saturated cyclic hydrocarbon groups having 3 to 10 carbons, preferably 3 to 8 carbons, and more preferably 3 to 6 carbons, wherein the cycloalkyl group may be optionally substituted. Preferred cycloalkyl groups include, without limitation, cyclopropyl, cyclobutyl, cyclopentyl, cyclopentenyl, cyclohexyl, cyclohexenyl, cycloheptyl, and cyclooctyl.

The term "heteroaryl" refers to an aromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2, 3, or 4 atoms of each ring may be substituted by a substituent, and wherein the ring comprising a monocyclic radical is aromatic and wherein at least one of the fused rings comprising a bicyclic or tricyclic radical is aromatic (but does not have to be a ring which contains a heteroatom, e.g. tetrahydroisoquinolinyl. Exemplary heteroaryl systems are derived from, but not limited to, the following ring systems: pyrrole, furan, thiophene, imidazole, pyrazole, oxazole (=[1,3]oxazole), isoxazole (=[1,2]oxazole), thiazole (=[1,3]thiazole), isothiazole (=[1,2]thiazole), [1,2,3]triazole, [1,2,4]triazole, [1,2,4]oxadiazole, [1,3,4]oxadiazole, [1,2,4]thiadiazole, [1,3,4]thiadiazole, tetrazole, pyridine, pyridazine, pyrimidine, pyrazine, [1,2,3]triazine, [1,2,4]triazine, [1,3,5]triazine, indole, isoindole, benzofuran, benzothiophene [1,3]benzoxazole, [1,3]benzothiazole, benzoimidazole, indazole, quinoline, isoquinoline, cinnoline, quinazoline, quinoxaline, phthalazine, different naphthyridines, e.g. [1,8]naphthyridine, different thienopyridines, e.g. thieno[2,3-b]pyridine and purine. The term "heterocyclyl" refers to a nonaromatic 5-8 membered monocyclic, 8-12 membered bicyclic, or 11-14 membered tricyclic ring system having 1-3 heteroatoms if monocyclic, 1-6 heteroatoms if bicyclic, or 1-9 heteroatoms if tricyclic, said heteroatoms selected from O, N, or S (e.g., carbon atoms and 1-3, 1-6, or 1-9 heteroatoms of N, O, or S if monocyclic, bicyclic, or tricyclic, respectively), wherein 0, 1, 2 or 3 atoms of each ring may be substituted by a substituent. Examples of heterocyclyl groups include piperazinyl, pyrrolidinyl, dioxanyl, morpholinyl, tetrahydrofuranyl, and the like.

The term "substituted" refers to moieties having substituents replacing a hydrogen on one or more non-hydrogen atoms of the molecule. It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc. Substituents can include, for example, -(Ci-9 alkyl) optionally substituted with one or more of hydroxyl, $-NH_2$, -NH($C_{1-3}$ alkyl), and - N($C_{1-3}$ alkyl)$_2$; -($C_{1-9}$ haloalkyl); a halide; a hydroxyl; a carbonyl [such as -C(O)OR, and - C(O)R]; a thiocarbonyl [such as -C(S)OR, -C(O)SR, and -C(S)R]; -($C_{1-9}$ alkoxy) optionally substituted with one or more of halide, hydroxyl, $-NH_2$, -NH($C_{1-3}$ alkyl), and -N($C_{1-3}$ alkyl)$_2$; -OPO(OH)$_2$; a phosphonate [such as -PO(OH)$_2$ and -PO(OR')$_2$]; -OPO(OR')R"; -NRR'; - C(O)NRR'; -C(NR)NR'R"; -C(NR')R"; a cyano; a nitro; an azido; -SH; -S-R; -OSO$_2$(OR); a sulfonate [such as -SO$_2$(OH) and -SO$_2$(OR)]; -SO$_2$NR'R"; and -SO$_2$R; in which each occurrence of R, R' and R" are independently selected from H; -($C_{1-9}$ alkyl); $C_{6-10}$ aryl optionally substituted with from 1-3R'''; 5-10 membered heteroaryl having from 1-4 heteroatoms independently selected from N, O, and S and optionally substituted with from 1-3 R'''; $C_{3-7}$ carbocyclyl optionally substituted with from 1-3 R'''; and 3-8 membered heterocyclyl having from 1-4 heteroatoms independently selected from N, O, and S and optionally substituted with from 1-3 R'''; wherein each R''' is independently selected from -($C_1$-$_6$ alkyl), -($C_1$-$_6$ haloalkyl), a halide (e.g., F), a hydroxyl, -C(O)OR, -C(O)R, -($C_1$-$_6$ alkoxyl), -NRR', -C(O)NRR', and a cyano, in which each occurrence of R and R' is independently selected from H and -($C_1$-$_6$ alkyl). In some embodiments, the substituent is selected from -($C_1$-$_6$ alkyl), -($C_1$-$_6$ haloalkyl), a halide (e.g., F), a hydroxyl, -C(O)OR, - C(O)R, -($C_{1-6}$ alkoxyl), -NRR', -C(O)NRR', and a cyano, in which each occurrence of R and R' is independently selected from H and -($C_1$-$_6$ alkyl).

The terms "aprepitant", "vofopitant", "L-733060", "MK-3207", and "olcegepant" refer to chemical entities having structures, chemical names, and CAS numbers in the Table below.

| Name | Structure | Chemical Name | CAS # |
|---|---|---|---|
| aprepitant | 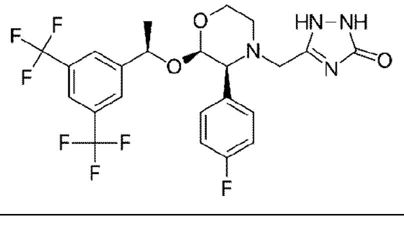 | 5-([(2R,3S)-2-((R)-1-[3,5-Bis (trifluoromethyl)phenyl]ethoxy)-3-(4-fluorophenyl)morpholino]methyl)-1H-1,2,4-triazol-3(2H)-one | 170729-80-3 |

(continued)

| Name | Structure | Chemical Name | CAS # |
|---|---|---|---|
| vofopitant | 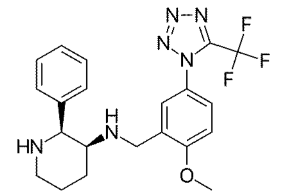 | (2S,3S)-N-[(2-Methoxy-5-[5-(trifluoromethyl)tetrazol-1-yl]phenyl) methyl]-2-phenylpiperidin-3-amine | 168266-90-8 |
| L-733060 | 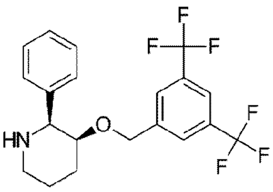 | (2S,3S)-3-{[3,5-bis(Trifluoromethyl) benzyl]oxy}-2-phenylpiperidine | 148700-85-0 |
| MK-3207 | | 2-[(8R)-8-(3,5-difluorophenyl)-10-oxo-6,9-diazaspiro[4.5]decan-9-yl]-N-[(2R)-2'-oxospiro[1,3-dihydroindene-2,3'-1H-pyrrolo[2,3-b]pyridine]-5-yl]acetamide | 957118-49-9 |
| olcegepant | | N-[(2R)-1-[[(2S)-6-amino-1-oxo-1-(4-pyridin-4-ylpiperazin-1-yl)hexan-2-yl] amino]-3-(3,5-dibromo-4-hydroxyphenyl)-1-oxopropan-2-yl] -4-(2-oxo-1,4-dihydroquinazolin-3-yl) piperidine-1-carboxamide | 204697-65-4 |

As used herein, the term "DIPMA nanoparticles" or "acid-responsive DIPMA nanoparticles" means nanoparticles formed from P(PEGMA-co-DMAEMA)-b-P(DIPMA-co-DEGMA) diblock copolymer. As illustrated in Fig. 1, DIPMA nanoparticles are comprised by a hydrophobic core of P(PEGMA-co-DMAEMA) and a hydrophilic shell of P(DIPMA-co-DEGMA).

As used herein, the term "DIPMA-Ap nanoparticles" (or "DIPMA-AP nanoparticles") means nanoparticles formed from P(PEGMA-co-DMAEMA)-b-P(DIPMA-co-DEGMA) diblock copolymers loaded with aprepitant (i.e., aprepitant is contained within the core of the nanoparticles). Accordingly, the term "DIPMA-(MK-3207) nanoparticles" means nanoparticles formed from P(PEGMA-co-DMAEMA)-b-P(DIPMA-co-DEGMA) diblock copolymers loaded with MK-3207; and the term "DIPMA-(Ap+MK-3207) nanoparticles" means nanoparticles formed from P(PEGMA-co-DMAEMA)-b-P(DIPMA-co-DEGMA) diblock copolymers dual-loaded with aprepitant and MK-3207 (i.e., both aprepitant and MK-3207 are contained within the core of the nanoparticles).

As used herein, the term "BMA-Ap nanoparticles" means nanoparticles formed from P(PEGMA-co-DMAEMA)-b-P(BMA-co-DEGMA) diblock copolymer loaded with aprepitant (i.e., aprepitant is contained within the core of the nanoparticles).

By studying the substance P (SP) neurokinin 1 receptor (NKiR) as a prototypical GPCR that robustly traffics to endosomes upon activation, it has now been shown that endosomal GPCRs convey sustained signals that underlie excitation and nociceptive transmission in spinal neurons. SP was found to initiate rapid transient signaling at the cell surface which was followed by arrestin-mediated internalisation into endosomes and recruitment of signaling complexes to promote sustained signaling. This ultimately results in distinct cellular outcomes including prolonged activity and nuclear translocation of the MAP kinase ERK1/2, concomitant with physiological outcomes such as sustained spinal neuron excitability and central pain transmission.

These findings suggest that targeting GPCRs in endosomes is required for optimal pharmacological intervention. The concept that endosomes are platforms for compartmentalized GPCR signaling that underlies pathophysiologically important processes has implications for receptor signal-specificity and therapeutic targeting. Endosomal trafficking allows GPCRs to generate signals in subcellular compartments that may explain how different receptors that activate the same G-proteins and βarrs can specifically regulate responses. Delivery of GPCR modulators to endosomes may enable targeting of signals that underlie disease-relevant processes with enhanced efficacy and selectivity.

It has now been surprisingly found that the polymeric nanoparticles according to the invention can be utilised to effectively deliver hydrophobic modulators of GPCRs intact into the endosomal lumen, where they are able to efficiently

interact with the endocytosed receptor.

*In vitro* assessment of the polymeric nanoparticles of the invention indicate that the administration of empty nanoparticles neither interfere nor trigger i[$Ca^{+2}$] influx, and only control nanoparticles increased nuclear ERK. On the contrary, polymeric nanoparticles loaded with the NKiR antagonist aprepitant (Ap) were able to diminish SP triggered nuclear ERK, previously described by Jensen *et al* to mediate endosomal $NK_1R$ signaling (Jensen, D.D. et al. Sci. Transl. Med. 2017, 31(9) 392). An assessment using Rotarod also found that intrathecal administration of nanoparticles did not modify locomotor patterns of mice. When the polymeric nanoparticles of the invention were tested on a capsaicin acute pain model (Sakurada, T. et al. Neuropharmacology 1992, 31(12), 1279-85) evoked primarily through the activation of the TRPV1 receptor (Story, G.M. et al. Cell. 2003, 112(6), 819-29), sustained and significant analgesia was observed with DIPMA-Ap nanoparticles throughout the 4 hours of capsaicin effect. Capsaicin is also known to increase the expression of the $NK_1R$ (Sakurada, T. et al. Neuropharmacology 1992, 31(12), 1279-85) and phospho-ERK protein (pERK) (Jensen, D.D. et al. Sci. Transl. Med. 2017, 31(9) 392) onNKiR-expressing dorsal horn neurons in lamina I/II, indicating that the analgesic effect of nanoparticles could be attributed to downregulation of the endosomal signaling of the NKiR. These findings suggest that delivery of hydrophobic GPCR modulators via polymeric nanoparticles may be a suitable and efficient option to selectively inhibit endosomal signaling of GPCRs.

In one aspect the present invention provides an aqueous liquid comprising polymeric nanoparticles of copolymer chains assembled to form a core / shell structure, the copolymer chains having:

(i) an acid-responsive hydrophobic polymer block that forms the core of the nanoparticles, wherein the acid-responsive hydrophobic polymer block comprises tertiary amine functional groups that are protonated in an acidic environment; and

(ii) a hydrophilic polymer block that forms the shell of the nanoparticles and is solvated by the aqueous liquid,

wherein the nanoparticles contain within their core a hydrophobic modulator of endosomal GPCR signaling or a pharmaceutically acceptable salt thereof.

In one embodiment, the polymeric nanoparticles disclosed herein are micelles.

Disclosed herein but not part of the invention is also a method of modulating endosomal GPCR signaling in a subject in need thereof comprising administering to the subject an aqueous liquid comprising polymeric nanoparticles of copolymer chains assembled to form a core / shell structure, the copolymer chains having:

(i) an acid-responsive hydrophobic polymer block that forms the core of the nanoparticles; and
(ii) a hydrophilic polymer block that forms the shell of the nanoparticles and is solvated by the aqueous liquid,

wherein the nanoparticles contain within their core a hydrophobic modulator of endosomal GPCR signaling, or a pharmaceutically acceptable salt thereof,
and wherein the acid-responsive hydrophobic polymer block undergoes a transition in an acidic environment of the endosomal lumen that causes the assembled copolymer chains to disassemble and release the hydrophobic modulator of endosomal GPCR signaling, or the pharmaceutically acceptable salt thereof, into the endosome.

The method further comprises administering to the subject an effective amount of a second modulator of endosomal GPCR signalling or a pharmaceutically acceptable salt thereof. The second modulator of endosomal GPCR signalling can be as described anywhere herein..

Reference to an "aqueous liquid" or "aqueous environment" will be understood to mean a liquid medium that comprises at least 50 wt. % of water. An aqueous liquid medium may therefore comprise one or more other miscible co-solvents. In some embodiments, the liquid medium will predominantly comprise water, for example, at least about 70 wt. %, or at least about 80 wt. %, or at least about 90 wt. %, or at least about 95 wt. % of water. In some embodiments of the invention it may be desirable that the liquid medium consists essentially of water and/or heavy water. The "aqueous liquid" or "aqueous environment" will include, for example, pharmaceutical compositions as herein defined as well as physiological environments including, but not limited to, extracellular and intracellular environments and the environment within the endosomal lumen.

For avoidance of any doubt, reference herein to the aqueous liquid or aqueous environment is not intended to exclude the presence of other components in that medium. The aqueous liquid comprises the polymeric nanoparticles (e.g., polymeric micellar nanoparticles), and may also comprise one or more additives that may, for example, regulate pH. A liquid medium that consists essentially of water may therefore comprise one or more soluble or insoluble non-liquid additives.

It has now been shown that the polymeric nanoparticles disclosed herein can rapidly internalize into cells via clathin-dependent and independent endocytosis. Organellar pH within endosomes is tightly regulated, ranging from

mildly acid in early endosomes to highly acidic in late endosomes/lysosomes. pH regulation can be modulated by a number of factors such as proton leak, CIC chloride channels or Na,K-ATPases. However, it is primarily determined by the activity of vacuolar ATPase, the function of which is to pump protons from the cytoplasm to the lumen of the endosome (Kane, P.M., Microbiol. Mol. Biol. Rev. 2006, 70, 177-91).

Unlike conventional nanoparticles, polymeric nanoparticles used in accordance with the invention are formed of an assembly of copolymer chains, the copolymer chains having an acid-responsive polymer block that is hydrophobic at physiologic or basic pH and forms the core of the nanoparticles, and a hydrophilic polymer block that is solvated by an aqueous liquid. Those skilled in the art will appreciate that (a) the hydrophilic polymer block that is solvated by the liquid medium represents the "shell" or "corona" of the nanoparticles, and (b) the acid-responsive hydrophobic polymer block that forms the core of the nanoparticles is poorly solvated by an aqueous liquid at physiologic or basic pH. Those skilled in the art will also appreciate that acid-responsive polymers are polymers that undergo a physical change or transition in an acidic environment.

As will be discussed in more detail below, the acid-responsive hydrophobic polymer block of the copolymer chains not only forms the core of the nanoparticles but also provides a means to promote disassembly of the nanoparticles. The acid-responsive hydrophobic polymer block provides this means for disassembly by undergoing a transition upon being subjected to an acidic environment.

It will be understood that reference to the acid-responsive hydrophobic polymer block "undergoing a transition" in an acidic environment of the endosomal lumen refers to the acid-responsive polymer block undergoing a hydrophilic transition in the acidic environment of the endosomal lumen, leading to solvation of the polymeric core, dissociation of the copolymer chains and release of the hydrophobic modulator of an endosomal GPCR, or the pharmaceutically acceptable salt thereof, into the endosome.

As an example, it is believed that acid-responsive functional groups contained within the hydrophobic core of the nanoparticles of the invention become protonated in the acidic environment of the endosomal lumen, resulting in a hydrophilic transition of the acid-responsive polymer block, leading to solvation of the core and dissociation of the copolymer chains, which in turn releases the hydrophobic modulator of an endosomal GPCR into the endosomal lumen.

It will be appreciated that the "acidic environment" described herein refers to an aqueous environment having a pH below physiological pH or pH 7.4. Accordingly, it will be understood that the term "acidic environment" denotes strongly acidic environments as well as weakly acidic environments. Suitable pH ranges will lie in the range of about pH 1 and pH 7.3. In one embodiment, the pH range will lie in the range of about pH 2 and about pH 7. In another embodiment, the pH will be in the range of about pH 3 and about pH 7, about pH 4 and about pH 7, or about pH 5 and about pH 7. Preferably, the pH will be in the range of about pH 5.5 and about pH 7. More preferable, the pH will be in the range of about pH 5.9 and about pH 6.5 (e.g., about pH 6.1).

In one embodiment, the polymeric nanoparticles disclosed herein are micelles. The term "micelle" is well known in the art to define an assembly or aggregate of amphipathic molecules dispersed within a liquid medium. A conventional micelle in an aqueous liquid medium is made up of assembled molecules having a section or region that exhibits hydrophilic character and is solvated by the surrounding aqueous liquid medium, sequestering a hydrophobic section or region of the molecules so as to form the centre or core of the micelle. This type of micelle is commonly referred to as a "normal phase micelle" or an "oil-in water micelle".

Micelles are typically spherical or spheroidal in shape, but can also include cylindrical and bilayer shapes. The shape and size of a given micelle is typically determined by the nature of the amphipathic molecule from which it is formed and liquid medium properties such as amphipathic molecule concentration, temperature, pH and ionic strength. The process of forming micelles is commonly referred to as micellisation. The assembled amphipathic molecules may also be referred to as "micellar" assembled amphipathic molecules.

In some embodiments, the nanoparticles used in accordance with the invention have a diameter from 1 nm-1000 nm. In one embodiment, the nanoparticles used in accordance with the invention have a diameter from 5 nm-200 nm. In one embodiment, the nanoparticles used in accordance with the invention have a diameter from 5 nm-150 nm. In one embodiment, the nanoparticles used in accordance with the invention have a diameter from 15-120 nm (e.g., from 15-100 nm, from 20-90 nm, from 25-80 nm, from 25-70 nm, from 25-60 nm, from 25-50 nm, or from 25-45 nm).

The nanoparticles used in accordance with the invention are nanoparticles of assembled copolymer chains. In other words, the nanoparticles are formed from or formed of copolymer chains. The copolymer chains comprise an acid-responsive polymer block that forms the core of the nanoparticles, and a polymer block that is solvated by the liquid medium. Those skilled in the art will therefore appreciate that the copolymer chains exhibit amphipathic character. The nanoparticles used in accordance with the invention may be further described with reference to Figure 1A. Thus, the copolymer chains may be described as an A-B diblock copolymer with one block represented by an acid-responsive hydrophobic polymer block, and the other block represented by a hydrophilic polymer that is capable of being solvated by the aqueous liquid. Assembly of the copolymer chains gives rise to nanoparticles where the acid-responsive hydrophobic polymer block forms the core of the nanoparticles, and the hydrophilic polymer block that is solvated by the liquid medium forms the shell or corona.

Although the copolymer chain illustrated in Figure 1A and described herein is an A-B diblock copolymer, the copolymer chains used in accordance with the invention are not limited to such a structure. In particular, provided that the copolymer chains are capable of forming a polymeric nanoparticle and comprise (a) an acid-responsive hydrophobic polymer block that forms the core of the nanoparticle, and (b) a hydrophilic polymer block that is solvated by the liquid medium at physiologic or basic pH, there is no particular limitation regarding the composition or structure of the copolymer.

As an example, the copolymer chains may have an A-B-A triblock copolymer structure where the A blocks, which may be the same or different, represent hydrophilic polymers that are capable of being solvated by the liquid medium, and the B block represents a hydrophobic acid-responsive polymer that forms the core of the nanoparticle.

The copolymer chains may be linear or branched.

The copolymer chains may comprise one or more acid-responsive hydrophobic polymer blocks, and one or more hydrophilic polymer blocks that are solvated by the liquid medium at physiologic or basic pH. Each polymer block may be independently linear or branched. Each polymer block may be independently a homo- or co-polymer.

The copolymer chains used in accordance with the invention will generally have an overall number average molecular weight ranging from about 600 to about 130,000. In some embodiments, the copolymer chains used in accordance with the invention have an overall number average molecular weight ranging from about 1,000 to about 100,000. In certain embodiments, the copolymer chains used in accordance with the invention have an overall number average molecular weight ranging from about 5,000 to about 75,000. In certain embodiments, the copolymer chains used in accordance with the invention have an overall number average molecular weight ranging from about 10,000 to about 50,000.

In connection with this, the number average molecular weight of the polymer block that is solvated by the liquid medium will generally range from about 100 to about 50,000 (e.g., from 500 to about 50,000, from 1,000 to about 40,000, from 2,000 to about 30,000, from 5,000 to about 30,000), and the number average molecular weight of the stimulus responsive polymer block that forms the core of the nanoparticles will generally range from about 500 to about 80,000 (e.g., from 1,000 to about 70,000, from 2,000 to about 60,000, from 3,000 to about 50, 000, from 5,000 to about 40,000, from 5,000 to about 30,000, from 10,000 to about 30,000).

Reference herein to number average molecular weight is intended to mean that determined by gel permeation chromatography (GPC).

Acid-responsive polymer blocks (e.g., acid-responsive hydrophobic polymer blocks) may be derived from ethylenically unsaturated monomers comprising functional groups such as amine groups including primary, secondary, and tertiary alkyl amines, nitrogen containing heterocyclyl (e.g., morpholinyl, pyrrolidinyl, piperazinyl, and piperidinyl), pyridine, and other nitrogen-containing heteroaryls (e.g., imidazole) which are protonated in the acidic environment of the endosomal lumen. Acid-responsive polymer blocks may be prepared from ethylenically unsaturated monomers such as N-vinyl formamide, N-vinyl acetamide, acrylamide and methacrylamide, N-methylacrylamide, N,N-dimethylacrylamide, dimethylaminoethyl or aminoethyl. Acid-responsive polymers may also be prepared as polypeptides from amino acids (e.g. polylysine) or derived from naturally occurring polymers such as proteins (e.g. lysozyme, albumin, casein), or nucleic acids such as DNA.

In one embodiment, acid-responsive polymer blocks may be derived from ethylenically unsaturated monomers selected from the following:

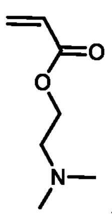
,
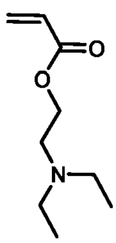
,
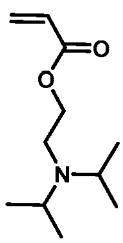
,
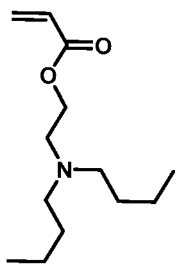
,

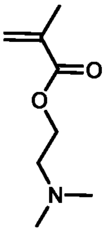
,
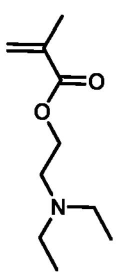
,
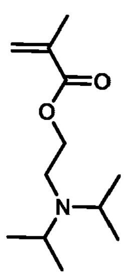
,
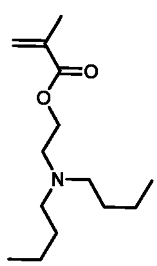
,

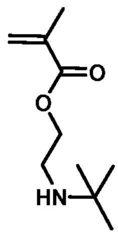, and 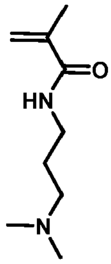.

In one embodiment, acid-responsive polymer blocks may be derived from ethylenically unsaturated monomers selected from the following:

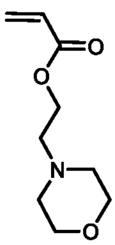, 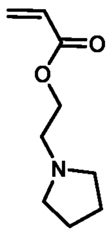, 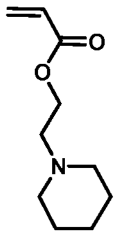, 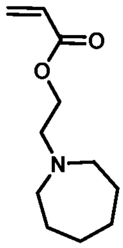,

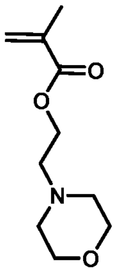, 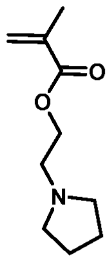, 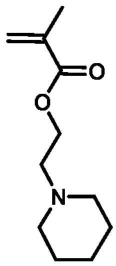, 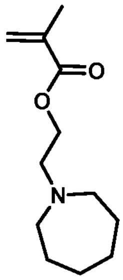,

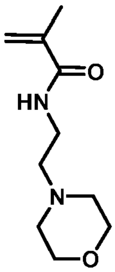, 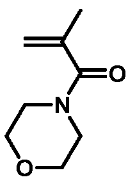, 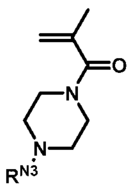
Examples of $R^{N1}$ and $R^{N2}$ include methyl, ethyl, and propyl, and 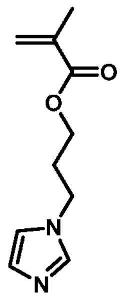.

In one embodiment, acid-responsive polymer blocks may be derived from ethylenically unsaturated monomers selected from the following:

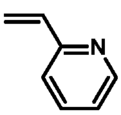, 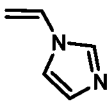, 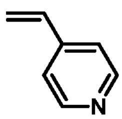, and 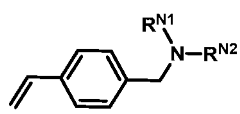
Examples of $R^{N1}$ and $R^{N2}$ include methyl, ethyl, and propyl.

In one embodiment, the acid-responsive polymer blocks may be derived from ethylenically unsaturated monomers comprising functional groups such as amine groups copolymerised with polyalkyleneoxide methacrylate monomers. Without wishing to be bound by theory, it is believed that copolymerisation of monomers comprising functional groups with polyalkyleneoxide methacrylate monomers may help to reduce any cellular toxicity associated with the protonated polymers once dissociation of the polymeric nanoparticles has occurred.

Provided that the copolymer chains assemble to form nanoparticles in the aqueous liquid, there is no particular limitation regarding the nature or composition of the polymer block that is solvated by the aqueous liquid at physiologic or basic pH. This polymer block will generally not be an acid-responsive polymer block.

The block must contain sufficient polymerised monomer residues that impart hydrophilic character to the block and collectively promote an ability to be solvated in the aqueous liquid. Those skilled in the art will be aware of ethylenically unsaturated monomers that can provide hydrophilic character to a polymer chain. Such ethylenically unsaturated monomers include, but are not limited to, acrylic acid, methacrylic acid, hydroxyethyl methacrylate, hydroxypropyl methacrylate, hydroxyethyl acrylate, or copolymers thereof.

Provided that the copolymer chains can form nanoparticles that function as herein described, each block of the copolymer chain may contain polymerised monomer residues derived from a range of ethylenically unsaturated monomers.

Common ethylenically unsaturated monomers used in forming polymer chains include those that can be polymerised by a radical polymerisation process. The monomers should be capable of being polymerised with other monomers. The factors which determine copolymerisability of various monomers are well documented in the art, for example, see: Greenlee, R.Z., in Polymer Handbook 3rd Edition (Brandup, J., and Immergut. E.H. Eds) Wiley: New York, 1989 p II/53.

Examples of such ethylenically unsaturated monomers include those of formula (I):

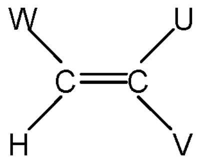

(I)

where U and W are independently selected from $-CO_2R^1$, $-COR^1$, $-CSR^1$, $-CSOR^1$, $-COSR^1$, $-CONH_2$, $-CONHR^1$, $-CONR^1_2$, hydrogen, halogen and optionally substituted Ci-$C_4$ alkyl or U and W form together a lactone, anhydride or imide ring that may itself be optionally substituted, where the optional substituents are independently selected from hydroxy, $-CO_2H$, $-CO_2R^1$, $-COR^1$, $-CSR^1$, $-CSOR^1$, $-COSR^1$, -CN, $-CONH_2$, $-CONHR^1$, - $CONR^1_2$, $-OR^1$, $-SR^1$, $-O_2CR^1$, $-SCOR^1$, and $-OCSR^1$;

V is selected from hydrogen, $R^1$, $-CO_2H$, $-CO_2R^1$, $-COR^1$, $-CSR^1$, $-CSOR^1$, $-COSR^1$, - $CONH_2$, $-CONHR^1$, $-CONR^1_2$, $-OR^1$, $-SR^1$, $-O_2CR^1$, $-SCOR^1$, and $-OCSR^1$;

U and V, taken together with the carbon to which each is attached, forms a heterocyclic ring that includes from 5-8 ring atoms, wherein from 1-3 ring atoms are heteroatoms each independently selected from N, N(H), O, and S, wherein the heterocyclic ring is optionally substituted with one or more substituents selected from $C_1$-$C_6$ alkyl, oxo, and $C_1$-$C_6$ alkoxy; and

each $R^1$ is independently selected from optionally substituted alkyl, optionally substituted alkenyl, optionally substituted alkynyl, optionally substituted aryl, optionally substituted heteroaryl, optionally substituted carbocyclyl, optionally substituted heterocyclyl, optionally substituted arylalkyl, optionally substituted heteroarylalkyl, optionally substituted alkylaryl, optionally substituted alkylheteroaryl, and an optionally substituted polymer chain.

Each $R^1$ may also be independently selected from optionally substituted $C_1$-$C_{22}$ alkyl (e.g., alkyl optionally substituted with one or more $C_{1-10}$ dialkylamino; alkyl optionally substituted with one or more heterocyclyl; alkyl optionally substituted with one or more carbocyclyl; alkyl optionally substituted with one or more aryl; or alkyl optionally substituted with one or more heteroaryl wherein the heterocyclyl, carbocyclyl, aryl, and heteroaryl are each further optionally substituted as defined elsewhere herein), optionally substituted $C_2$-$C_{22}$ alkenyl, optionally substituted $C_2$-$C_{22}$ alkynyl, optionally substituted $C_6$-$C_{18}$ aryl, optionally substituted $C_3$-$C_{18}$ heteroaryl, optionally substituted $C_3$-$C_{18}$ carbocyclyl, optionally substituted $C_2$-$C_{18}$ heterocyclyl, optionally substituted $C_7$-$C_{24}$ arylalkyl, optionally substituted $C_4$-$C_{18}$ heteroarylalkyl, optionally substituted $C_7$-$C_{24}$ alkylaryl, optionally substituted $C_4$-$C_{18}$ alkylheteroaryl, and an optionally substituted polymer chain.

$R^1$ may also be selected from optionally substituted Ci-Cis alkyl, optionally substituted $C_2$-$C_{18}$ alkenyl, optionally

substituted aryl, optionally substituted heteroaryl, optionally substituted carbocyclyl, optionally substituted heterocyclyl, optionally substituted aralkyl, optionally substituted heteroarylalkyl, optionally substituted alkaryl, optionally substituted alkylheteroaryl and a polymer chain.

In one embodiment, $R^1$ may be independently selected from optionally substituted $C_1$-$C_6$ alkyl.

In some embodiments, examples of optional substituents for $R^1$ include those selected from alkyleneoxidyl (epoxy), hydroxy, alkoxy, acyl, acyloxy, formyl, alkylcarbonyl, carboxy, sulfonic acid, alkoxy- or aryloxy-carbonyl, isocyanato, cyano, silyl, halo, amino (e.g., dialkylamino), aryl, heteroaryl, carbocyclyl, and heterocyclyl, including salts and derivatives thereof, wherein each optional substituent for $R^1$ is further optionally substituted with one or more substituents selected from Ci-Cis alkyl, Ci-Cis alkenyl, Ci-Cis alkynyl, hydroxy, Ci-Cis alkoxy, acyl, and acyloxy.

Examples polymer chains include those selected from polyalkylene oxide, polyarylene ether and polyalkylene ether.

Examples of monomers of formula (I) include maleic anhydride, N-alkylmaleimide, N-arylmaleimide, dialkyl fumarate and cyclopolymerisable monomers, acrylate and methacrylate esters, acrylic and methacrylic acid, styrene, acrylamide, methacrylamide, and methacrylonitrile, mixtures of these monomers, and mixtures of these monomers with other monomers.

Other examples of monomers of formula (I) include: methyl methacrylate, ethyl methacrylate, propyl methacrylate (all isomers), butyl methacrylate (all isomers), 2-ethylhexyl methacrylate, isobornyl methacrylate, benzyl methacrylate, phenyl methacrylate, methacrylonitrile, alpha-methylstyrene, methyl acrylate, ethyl acrylate, propyl acrylate (all isomers), butyl acrylate (all isomers), 2-ethylhexyl acrylate, isobomyl acrylate, benzyl acrylate, phenyl acrylate, acrylonitrile, styrene, functional methacrylates, acrylates and styrenes selected from glycidyl methacrylate, 2-hydroxyethyl methacrylate, hydroxypropyl methacrylate (all isomers), hydroxybutyl methacrylate (all isomers), N,N-dimethylaminoethyl methacrylate, N,N-diethylaminoethyl methacrylate, triethyleneglycol methacrylate, itaconic anhydride, glycidyl acrylate, 2-hydroxyethyl acrylate, hydroxypropyl acrylate (all isomers), hydroxybutyl acrylate (all isomers), N,N-dimethylaminoethyl acrylate, N,N-diethylaminoethyl acrylate, triethyleneglycol acrylate, methacrylamide, N-methylacrylamide, N,N-dimethylacrylamide, N-tert-butylmethacrylamide, N-n-butylmethacrylamide, N-methylolmethacrylamide, N-ethylolmethacrylamide, N-tert-butylacrylamide, N-N-butylacrylamide, N-methylolacrylamide, N-ethylolacrylamide, diethylamino styrene (all isomers), diethylamino alpha-methylstyrene (all isomers), p-vinylbenzene sulfonic acid, p-vinylbenzene sulfonic sodium salt, trimethoxysilylpropyl methacrylate, triethoxysilylpropyl methacrylate, tributoxysilylpropyl methacrylate, dimethoxymethylsilylpropyl methacrylate, diethoxymethylsilylpropyl methacrylate, dibutoxymethylsilylpropyl methacrylate, diisopropoxymethylsilylpropyl methacrylate, dimethoxysilylpropyl methacrylate, diethoxysilylpropyl methacrylate, dibutoxysilylpropyl methacrylate, diisopropoxysilylpropyl methacrylate, trimethoxysilylpropyl acrylate, triethoxysilylpropyl acrylate, tributoxysilylpropylacrylate, dimethoxymethylsilylpropyl acrylate, diethoxymethylsilylpropyl acrylate, dibutoxymethylsilylpropyl acrylate, diisopropoxymethylsilylpropyl acrylate, dimethoxysilylpropyl acrylate, diethoxysilylpropyl acrylate, dibutoxysilylpropyl acrylate, diisopropoxysilylpropyl acrylate, vinyl acetate, vinyl butyrate, vinyl benzoate, vinyl chloride, vinyl fluoride, vinyl bromide, maleic anhydride, N-phenylmaleimide, N-butylmaleimide, N-vinylpyrrolidone, N-vinylcarbazole, butadiene, ethylene and chloroprene. This list is not exhaustive.

In some embodiments, each block of the copolymer chain may contain polymerised monomer residues derived from ethylenically unsaturated monomers of Formula (**I-a**):

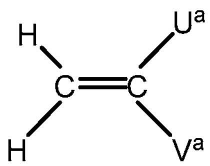

**(I-a)**

wherein $\mathbf{U^a}$ is selected from $-CO_2H$, $-CO_2\mathbf{R^{1U}}$, $-CO\mathbf{R^{1U}}$, $-CS\mathbf{R^{1U}}$, $-CSO\mathbf{R^{1U}}$, $-COS\mathbf{R^{1U}}$, $-CONH_2$, $-CN$, $-CONH\mathbf{R^{1U}}$, $-CON\mathbf{R^{1U}}_2$, hydrogen, halogen and optionally substituted $C_1$-$C_4$ alkyl, $-O\mathbf{R^{1U}}$, $-S\mathbf{R^{1U}}$, $-O_2C\mathbf{R^{1U}}$, $-SCO\mathbf{R^{1U}}$, and $-OCS\mathbf{R^{1U}}$;

$\mathbf{V^a}$ is selected from hydrogen, $\mathbf{R^{1V}}$, $-CO_2H$, $-CO_2\mathbf{R^{1V}}$, $-CO\mathbf{R^{1V}}$, $-CS\mathbf{R^{1V}}$, $-CSO\mathbf{R^{1V}}$, $-COS\mathbf{R^{1V}}$, $-CONH_2$, $-CONH\mathbf{R^{1V}}$, $-CON\mathbf{R^{1V}}_2$, $-O\mathbf{R^{1V}}$, $-S\mathbf{R^{1V}}$, $-O_2C\mathbf{R^{1V}}$, $-SCO\mathbf{R^{1V}}$, and $-OCS\mathbf{R^{1V}}$; or

$\mathbf{U^a}$ and $\mathbf{V^a}$, taken together with the carbon to which each is attached, forms a heterocyclic ring that includes from 5-8 ring atoms, wherein from 1-3 ring atoms are heteroatoms each independently selected from N, N(H), O, and S, wherein the heterocyclic ring is optionally substituted with one or more substituents selected from $C_1$-$C_6$ alkyl, oxo,

and $C_1$-$C_6$ alkoxy;
and

each of $\mathbf{R^{1U}}$ and $\mathbf{R^{1V}}$ is an independently selected $\mathbf{R^1}$, wherein $\mathbf{R^1}$ is as defined for Formula (**I**), above.

In certain embodiments of Formula (**I-a**), $\mathbf{U^a}$ is selected from $-CO_2H$, $-CO_2\mathbf{R^{1U}}$, - CO$\mathbf{R^{1U}}$, $-CONH_2$, -CONH$\mathbf{R^{1U}}$, and-CON$\mathbf{R^{1U}}_2$.
In certain embodiments of the foregoing, $\mathbf{U^a}$ is selected from $-CO_2\mathbf{R^{1U}}$.
In certain embodiments of the foregoing (when $\mathbf{U^a}$ is selected from $-CO_2H$, $-CO_2\mathbf{R^{1U}}$, -CO$\mathbf{R^{1U}}$, $-CONH_2$, -CONH$\mathbf{R^{1U}}$, and -CON$\mathbf{R^{1U}}_2$), each $\mathbf{R^{1U}}$ is selected from optionally substituted $C_1$-$C_{22}$ alkyl (e.g., $C_1$-$C_{22}$ alkyl substituted with amino, e.g., dialkylamino; or Ci-$C_{22}$ alkyl substituted with alkoxy wherein the alkoxy is optionally substituted, e.g., $\mathbf{R^{1U}}$ is

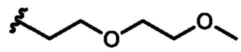
)

and an optionally substituted polymer chain (e.g., polyalkylene oxide such as polyethylene oxide).
In certain embodiments of Formula (**I-a**), $\mathbf{V^a}$ is selected from hydrogen and $\mathbf{R^{1V}}$.
In certain embodiments of the foregoing (when $\mathbf{V^a}$ is selected from hydrogen and $\mathbf{R^{1V}}$), $\mathbf{R^{1V}}$ is independently selected from optionally substituted $C_1$-$C_{22}$ alkyl (e.g., unsubstituted $C_1$-$C_6$ alkyl, e.g., methyl).
In some embodiments, an ethylenically unsaturated monomer of Formula (**I-a**) is of Formula (**I-a0**):

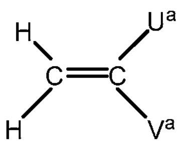

**(I-a0)**

wherein $\mathbf{U^a}$ is hydrogen, -CO$\mathbf{R^{1U}}$, $-CO_2\mathbf{R^{1U}}$, or -C(O)NH$\mathbf{R^{1U}}$;

$\mathbf{V^a}$ is H or $\mathbf{R^{1V}}$;
$\mathbf{R^{1U}}$ is selected from the group consisting of:

(i) $C_1$-$C_{22}$ alkyl substituted with one or more $N(C_1$-$C_{10}$ alkyl$)_2$ or $NH(C_1$-$C_{10}$ alkyl);
(ii) $C_1$-$C_{22}$ alkyl substituted with heterocyclyl including from 4-10 ring atoms, wherein from 1-3 ring atoms are heteroatoms, each independently selected from the group consisting of N, N(H), $N(C_{1-10}$ alkyl), and O, provided that the heterocyclyl comprises one or more ring nitrogen atoms, and wherein one or more of the heterocyclyl ring carbon atoms are optionally substituted;
(iii) $C_1$-$C_{22}$ alkyl substituted with heteroaryl including from 5-10 ring atoms, wherein from 1-4 ring atoms are heteroatoms, each independently selected from the group consisting of N, N(H), $N(C_{1-10}$ alkyl), O, and S, provided that the heteroaryl comprises one or more ring nitrogen atoms and wherein one or more of the heteroaryl ring carbon atoms are optionally substituted; and
(iv) heterocyclyl including from 4-10 ring atoms, wherein from 1-3 ring atoms are heteroatoms, each independently selected from the group consisting of N, N(H), $N(C_{1-10}$ alkyl), and O, provided that the heterocyclyl comprises one or more ring nitrogen atoms, and wherein one or more of the heterocyclyl ring carbon atoms are optionally substituted;

$\mathbf{R^{1V}}$ is selected from the group consisting of:

(i) optionally substituted $C_{1-6}$ alkyl;
(ii) heteroaryl including from 5-10 ring atoms, wherein from 1-4 ring atoms are heteroatoms, each independently selected from the group consisting of N, N(H), $N(C_{1-10}$ alkyl), O, and S, provided that the heteroaryl comprises one or more ring nitrogen atoms and wherein one or more of the heteroaryl ring carbon atoms are optionally substituted; and
(iii) optionally substituted $C_{6-10}$ aryl wherein the aryl is substituted with one or more aminoalkyl, mono-aminoalkyl,

or diaminoalkyl.

In some embodiments of Formula (**I-a0**), **U^a** is -CO**R^1U**, -$CO_2$**R^1U**, or -C(O)NH**R^1U** (e.g., -$CO_2$**R^1U**, or -C(O)NH**R^1U**).

In certain embodiments of Formula **(I-a0), V^a** is H or **R^1V;** and **R^1V** is optionally substituted $C_{1-6}$ alkyl (e.g., unsubstituted $C_{1-6}$ alkyl, e.g., methyl).

In certain embodiments of Formula (**I-a0**), **R^1U** is $C_1$-$C_{22}$ alkyl substituted with one or more N($C_1$-$C_{10}$ alkyl)$_2$ (e.g., N($C_3$-$C_{10}$ alkyl)$_2$).

In certain embodiments of Formula (**I-a0**), **R^1U** is $C_1$-$C_{22}$ alkyl substituted with one or more NH($C_1$-$C_{10}$ alkyl) (e.g., NH($C_3$-$C_{10}$ alkyl)).

In certain embodiments of Formula (**I-a0**), **R^1U** is $C_1$-$C_{22}$ alkyl substituted with heterocyclyl including from 5-7 ring atoms, wherein from 1-3 ring atoms are heteroatoms, each independently selected from the group consisting of N, N(H), N($C_{1-10}$ alkyl), and O, provided that the heterocyclyl comprises one or more ring nitrogen atoms, and wherein one or more of the heterocyclyl ring carbon atoms are optionally substituted. As non-limiting examples of the foregoing embodiments, **R^1U** can be $C_2$-$C_6$ alkyl substituted with pyrrolidinyl, morpholinyl, or piperiazinyl.

In certain embodiments of Formula (**I-a0**), **R^1U** is $C_1$-$C_{22}$ alkyl substituted with heteroaryl including from 5-10 (e.g, 5 or 6) ring atoms, wherein from 1-4 ring atoms are heteroatoms, each independently selected from the group consisting of N, N(H), N($C_{1-10}$ alkyl), O, and S, provided that the heteroaryl comprises one or more ring nitrogen atoms and wherein one or more of the heteroaryl ring carbon atoms are optionally substituted. As non-limiting examples of the foregoing embodiments, **R^1U** can be $C_2$-$C_6$ alkyl substituted with imidazolyl or pyridinyl.

In certain embodiments of Formula (**I-a0**), **U^a** is -$CO_2$**R^1U**; and **R^1U** is $C_1$-$C_{22}$ alkyl (e.g., $C_{2-4}$) substituted with N($C_1$-$C_{10}$ alkyl)$_2$ (e.g., N($C_3$-$C_{10}$ alkyl)$_2$).

In certain embodiments of Formula (**I-a0**), **U^a** is -$CO_2$**R^1U**; and **R^1U** is $C_1$-$C_{22}$ alkyl (e.g., $C_{2-4}$) substituted with NH($C_1$-$C_{10}$ alkyl) (e.g., NH($C_3$-$C_{10}$ alkyl)).

In certain embodiments of Formula (**I-a0**), **U^a** is -$CO_2$**R^1U**; and **R^1U** is $C_1$-$C_{22}$ alkyl substituted with heterocyclyl including from 5-7 ring atoms, wherein from 1-3 ring atoms are heteroatoms, each independently selected from the group consisting of N, N(H), N($C_{1-10}$ alkyl), and O, provided that the heterocyclyl comprises one or more ring nitrogen atoms, and wherein one or more of the heterocyclyl ring carbon atoms are optionally substituted.

In certain embodiments of Formula (**I-a0**), **U^a** is -$CO_2$**R^1U**; and **R^1U** is $C_1$-$C_{22}$ alkyl substituted with heteroaryl including from 5-10 ring atoms, wherein from 1-4 ring atoms are heteroatoms, each independently selected from the group consisting of N, N(H), N($C_{1-10}$ alkyl), O, and S, provided that the heteroaryl comprises one or more ring nitrogen atoms and wherein one or more of the heteroaryl ring carbon atoms are optionally substituted.

In certain embodiments of Formula (**I-a0**), **U^a** is -C(O)NH**R^1U**; and **R^1U** is $C_1$-$C_{22}$ alkyl substituted with N($C_1$-$C_{10}$ alkyl)$_2$ (e.g., N($C_3$-$C_{10}$ alkyl)$_2$).

In certain embodiments of Formula (**I-a0**), **U^a** is -C(O)NH**R^1U**; and **R^1U** is $C_1$-$C_{22}$ alkyl substituted with heterocyclyl including from 5-7 ring atoms, wherein from 1-3 ring atoms are heteroatoms, each independently selected from the group consisting of N, N(H), N($C_{1-10}$ alkyl), and O, provided that the heterocyclyl comprises one or more ring nitrogen atoms, and wherein one or more of the heterocyclyl ring carbon atoms are optionally substituted.

In certain embodiments of Formula (**I-a0**), **U^a** is -C(O)**R^1U**; and **R^1U** is heterocyclyl including from 5-7 ring atoms, wherein from 1-3 ring atoms are heteroatoms, each independently selected from the group consisting ofN, N(H), N($C_{1-10}$ alkyl), and O, provided that the heterocyclyl comprises one or more ring nitrogen atoms, and wherein one or more of the heterocyclyl ring carbon atoms are optionally substituted.

In some embodiments of Formula (**I-a0**), **U^a** is hydrogen; and **V^a** is **R^1V,** wherein **R^1V** is heteroaryl including from 5-10 ring atoms, wherein from 1-4 ring atoms are heteroatoms, each independently selected from the group consisting of N, N(H), N($C_{1-10}$ alkyl), O, and S, provided that the heteroaryl comprises one or more ring nitrogen atoms and wherein one or more of the heteroaryl ring carbon atoms are optionally substituted. As non-limiting examples of the foregoing embodiments, **R^1U** can be imidazolyl or pyridinyl.

In some embodiments of Formula (**I-a0**), **U^a** is hydrogen; and **V^a** is **R^1V,** wherein **R^1V** is optionally substituted $C_{6-10}$ aryl wherein the aryl is substituted with one or more aminoalkyl, mono-aminoalkyl, or diaminoalkyl.

Non-limiting examples of ethylenically unsaturated monomer of Formula (**I-a**) or (**I-a0**) include:

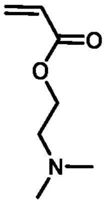, 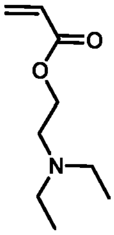, 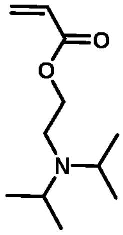, 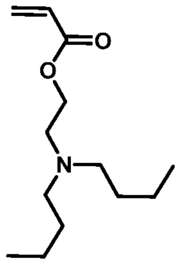,

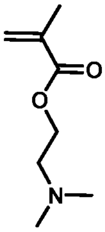, 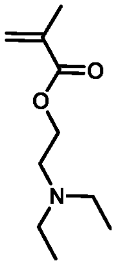, 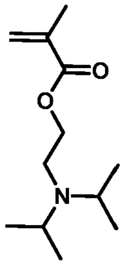, 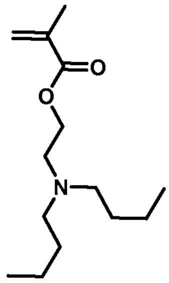,

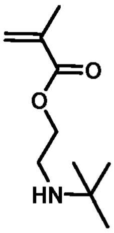, and 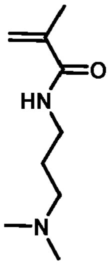.

Non-limiting examples of ethylenically unsaturated monomer of Formula (**I-a**) or (**I-a0**) also include:

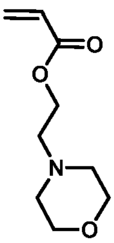, 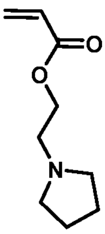, 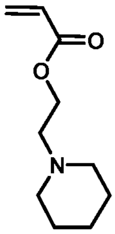, 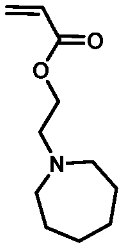,

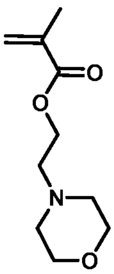, 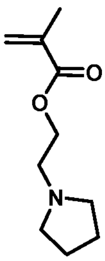, 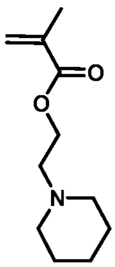, 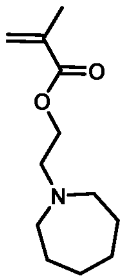,

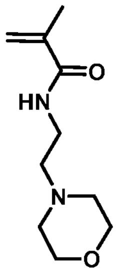, 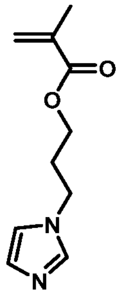, 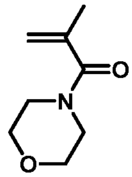, and 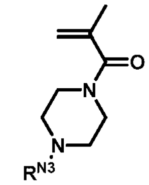 Examples of $R^{N1}$ and $R^{N2}$ include methyl, ethyl, and propyl .

Further non-limiting examples of ethylenically unsaturated monomer of Formula (**I-a**) or (**I-a0**) include:

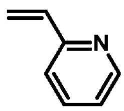, 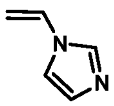, 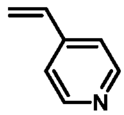, and 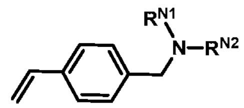 Examples of $R^{N1}$ and $R^{N2}$ include methyl, ethyl, and propyl .

In some embodiments, an ethylenically unsaturated monomer of Formula (**I-a**) is of Formula (**I-a1**):

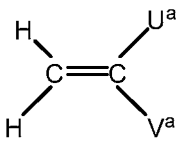

**(I-a1)**

wherein **U^a^** is $-CO_2$**$R^{1U}$**;

- **$V^a$** is H or **$R^{1V}$;**
- **$R^{1U}$** is $C_1$-$C_{22}$ alkyl substituted with one or more $N(C_3$-$C_{10}$ alkyl$)_2$; and
- **$R^{1V}$** is optionally substituted $C_1$-$C_6$ alkyl.

In certain embodiments of Formula (**I-a1**), **$R^{1U}$** is $C_2$-$C_6$ alkyl substituted with one or more $N(C_3$-$C_5$ alkyl$)_2$ (e.g., $CH_2CH_2N(iPr)_2$).

In certain embodiments of Formula (**I-a1**), **$R^{1V}$** is unsubstituted $C_1$-$C_3$ alkyl (e.g., methyl).

In certain embodiments of Formula (**I-a1**), **$R^{1U}$** is $C_2$-$C_6$ alkyl substituted with one or more $N(C_3$-$C_5$ alkyl$)_2$ (e.g., $CH_2CH_2N(iPr)_2$); and **$R^{1V}$** is unsubstituted $C_1$-$C_3$ alkyl (e.g., methyl).

As a non-limiting example of the foregoing embodiments, a monomer of Formula **(I-a1)** can have the following formula:

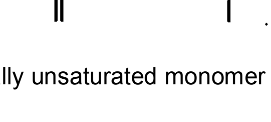.

In some embodiments, an ethylenically unsaturated monomer of Formula (**I-a**) is of Formula (**I-a2**):

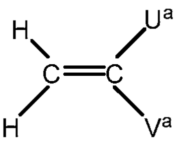

**(I-a2)**

wherein $\mathbf{U^a}$ is $-CO_2\mathbf{R^{1U}}$;

$\mathbf{V^a}$ is H or $\mathbf{R^{1V}}$;
$\mathbf{R^{1U}}$ is $C_1$-$C_{22}$ alkyl substituted with one or more Ci-Cis alkoxy, wherein the Ci-Cis alkoxy is optionally substituted with one or more substituents selected from Ci-Cis alkoxy; and
$\mathbf{R^{1V}}$ is optionally substituted $C_1$-$C_6$ alkyl.

In certain embodiments of Formula (**I-a2**), $\mathbf{R^{1U}}$ is $C_2$-$C_6$ alkyl optionally substituted one or more Ci-Cis alkoxy, wherein the Ci-Cis alkoxy is optionally substituted with one or more substituents selected from Ci-Cis alkoxy (e.g., $\mathbf{R^{1U}}$ can be n-butyl; or $\mathbf{R^{1U}}$ can be

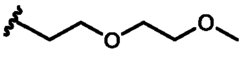).

In certain embodiments of Formula (**I-a2**), $\mathbf{R^{1V}}$ is unsubstituted $C_1$-$C_3$ alkyl (e.g., methyl).
In certain embodiments of Formula (**I-a2**), $\mathbf{R^{1U}}$ is $C_2$-$C_6$ alkyl optionally substituted one or more Ci-Cis alkoxy, wherein the Ci-Cis alkoxy is optionally substituted with one or more substituents selected from Ci-Cis alkoxy (e.g., $\mathbf{R^{1U}}$ can be n-butyl; or $\mathbf{R^{1U}}$ can be

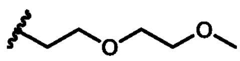);

and $\mathbf{R^{1V}}$ is unsubstituted $C_1$-$C_3$ alkyl (e.g., methyl).
As a non-limiting example of the foregoing embodiments, a monomer of Formula (I-**a2**) can have the following formula:

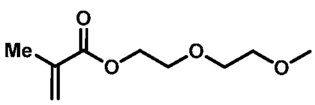 or 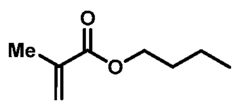.

In some embodiments, an ethylenically unsaturated monomer of Formula (**I-a**) is of Formula (**I-a3**):

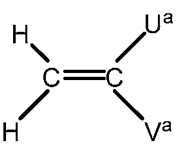

**(I-a3)**

wherein $\mathbf{U^a}$ is $-CO_2\mathbf{R^{1U}}$;

$\mathbf{V^a}$ is H or $\mathbf{R^{1V}}$;
$\mathbf{R^{1U}}$ is $C_1$-$C_{22}$ alkyl substituted with one or more $N(C_{1-}C_2\ alkyl)_2$; and
$\mathbf{R^{1V}}$ is optionally substituted $C_1$-$C_6$ alkyl.

In certain embodiments of Formula (**I-a3**), $\mathbf{R^{1U}}$ is $C_2$-$C_6$ alkyl substituted with one or more $N(C_{1-}C_2\ alkyl)_2$ (e.g.,

$CH_2CH_2N(Me)_2$).

In certain embodiments of Formula (**I-a3**), $\mathbf{R^{1V}}$ is unsubstituted $C_1$-$C_3$ alkyl (e.g., methyl).

In certain embodiments of Formula (**I-a3**), $\mathbf{R^{1U}}$ is $C_2$-$C_6$ alkyl substituted with one or more $N(C_1$-$C_2$ alkyl$)_2$ (e.g., $CH_2CH_2N(Me)_2$); and $\mathbf{R^{1V}}$ is unsubstituted $C_1$-$C_3$ alkyl (e.g., methyl).

As a non-limiting example of the foregoing embodiments, a monomer of Formula (I-**a1**) can have the following formula:

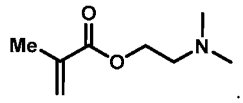

In some embodiments, an ethylenically unsaturated monomer of Formula (**I-a**) is of Formula (**I-a4**):

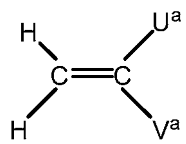

**(I-a4)**

wherein $\mathbf{U^a}$ is $-CO_2\mathbf{R^{1U}}$;

$\mathbf{V^a}$ is H or $\mathbf{R^{1V}}$;
$\mathbf{R^{1U}}$ is an optionally substituted polymer chain; and
$\mathbf{R^{1V}}$ is optionally substituted $C_1$-$C_6$ alkyl.

In certain embodiments of Formula (**I-a4**), $\mathbf{R^{1U}}$ is a polyalkylene oxide chain (e.g., $\mathbf{R^{1U}}$ can be polyethylene oxide).

In certain embodiments of Formula (**I-a4**), $\mathbf{R^{1V}}$ is unsubstituted $C_1$-$C_3$ alkyl (e.g., methyl).

In certain embodiments of Formula (**I-a4**), $\mathbf{R^{1U}}$ is is a polyalkylene oxide chain (e.g., $\mathbf{R^{1U}}$ can be polyethylene oxide); and $\mathbf{R^{1V}}$ is unsubstituted $C_1$-$C_3$ alkyl (e.g., methyl).

As a non-limiting example of the foregoing embodiments, a monomer of Formula (I-**a4)** can have the following formula:

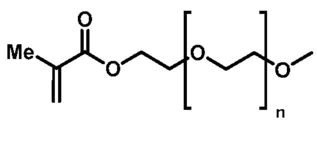,

wherein n is an integer from 3-100 (e.g., n = 5).

In some embodiments, an ethylenically unsaturated monomer of Formula (I-a) is of Formula (**I-a5**):

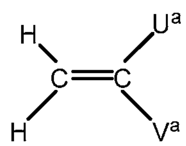

**(I-a5)**

wherein $\mathbf{U^a}$ and $\mathbf{V^a}$, taken together with the carbon to which each is attached, forms a heterocyclic ring that includes from 5-6 ring atoms, wherein from 1-3 ring atoms are heteroatoms each independently selected from N, N(H), and O, wherein the heterocyclic ring is optionally substituted with one or more substituents selected from $C_1$-$C_6$ alkyl, and oxo.

As a non-limiting example of foregoing embodiments, a monomer of Formula (**I-a5**) can have the following formula:

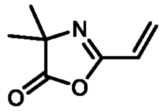

In some embodiments, the hydrophilic block of the co-polymer chain comprises polymerized monomer residues derived from monomers of Formula (**I-a3**).

In some embodiments, the hydrophilic block of the co-polymer chain comprises polymerized monomer residues derived from monomers of Formula (**I-a4**).

In certain embodiments, the hydrophilic block of the co-polymer chain comprises polymerized monomer residues derived from monomers of Formula (**I-a3**) and polymerized monomer residues derived from monomers of Formula (**I-a4**).

In some embodiments, the hydrophobic block of the co-polymer chain comprises polymerized monomer residues derived from monomers of Formula (**I-a0**).

In some embodiments, the hydrophobic block of the co-polymer chain comprises polymerized monomer residues derived from monomers of Formula (**I-a1**).

In some embodiments, the hydrophobic block of the co-polymer chain comprises polymerized monomer residues derived from monomers of Formula (**I-a2**).

In certain embodiments, the hydrophobic block of the co-polymer chain comprises polymerized monomer residues derived from monomers of Formula (**I-a1**) and polymerized monomer residues derived from monomers of Formula (**I-a2**).

In some embodiments, the co-polymer chain comprises polymerized monomer residues derived from monomers of Formula (**I-a1**); and polymerized monomer residues derived from monomers of Formula (**I-a3**).

In some embodiments, the co-polymer chain comprises:

1) a hydrophibilic block which comprises polymerized monomer residues derived from monomers of Formula (**I-a3**) and polymerized monomer residues derived from monomers of Formula (**I-a4**); and
2) a hydrophobic block which comprises polymerized monomer residues derived from monomers of Formula (**I-a1**) and polymerized monomer residues derived from monomers of Formula (**I-a2**).

The copolymer chains used in accordance with the invention may be prepared by any suitable method known to those skilled in the art. For example, the copolymer chains may be prepared by polymerising ethylenically unsaturated monomers (such as those herein described) by radical, coordination or ionic polymerisation techniques well known to those skilled in the art.

The polymerisation technique employed to prepare the copolymer chains may be living or non-living.

Living polymerisation is generally considered in the art to be a form of chain polymerisation in which irreversible chain termination is substantially absent. An important feature of living polymerisation is that polymer chains will continue to grow while monomer and the reaction conditions to support polymerisation are provided. Polymer chains prepared by living polymerisation can advantageously exhibit a well defined molecular architecture, a predetermined molecular weight and narrow molecular weight distribution or low polydispersity.

Examples of living polymerisation include ionic polymerisation and controlled radical polymerisation (CRP). Examples of CRP include, but are not limited to, iniferter polymerisation, stable free radical mediated polymerisation (SFRP), atom transfer radical polymerisation (ATRP), and reversible addition fragmentation chain transfer (RAFT) polymerisation.

It is envisaged that the copolymer chains of the nanoparticles described herein may be prepared using living polymerisation techniques. Equipment, conditions, and reagents for performing living polymerisation to prepare copolymers well known to those skilled in the art and will be discussed in more detail below.

Where the one or more ethylenically unsaturated monomers are to be polymerised by a living polymerisation technique, it will generally be necessary to include as the one or more reactants a living polymerisation agent. By "living polymerisation agent" is meant a compound that can participate in and control or mediate the living polymerisation of one or more ethylenically unsaturated monomers so as to form a living polymer chain (i.e. a polymer chain that has been formed according to a living polymerisation technique).

Living polymerisation agents that may be included as the one or more reactants in accordance with the invention include, but are not limited to, those which promote a living polymerisation technique selected from ionic polymerisation and CRP such as iniferter polymerisation agents, SFRP agents, ATRP agents and RAFT polymerisation agents.

RAFT agents suitable for use in accordance with the invention comprise a thiocarbonylthio group (which is a divalent moiety represented by: -C(S)S-). Examples of RAFT agents are described in Moad G.; Rizzardo, E; Thang S, H. Polymer 2008, 49, 1079-1131 and include xanthate, dithioester, dithiocarbonate, dithiocarbanate and trithiocarbonate compounds.

Contained within the core of the polymeric nanoparticles is a hydrophobic modulator of endosomal GPCR signaling. The term "modulator of endosomal GPCR signaling" as herein used refers to agonists and antagonists or inhibitors of GPCRs that have been endocytosed into endosomes. The modulator of endosomal GPCR signaling may be in any form including, but not limited to, an organic molecule, a polypeptide sequence, a hormone, a protein fragment or a derivative of any of these.

The term "endosomal GPCR signaling" as herein used refers to the signal transduced by an activated GPCR that has been endocytosed into an endosome, preferably an early endosome.

In one embodiment endosomal GPCR signaling will be signaling that is first transduced at the plasma membrane and is maintained when the receptor is endocytosed into early endosomes.

In another embodiment, the endosomal GPCR signaling will be signaling that requires receptor endocytosis and/or occurs exclusively on endosomal membranes, for example, β-arrestin mediated signaling. It is believed that βarrs interact with agonist-occupied G protein-coupled receptor kinase (GRK)-phosphorylated GPCRs at the cell surface and promote the transfer of ligand-bound receptor from the cell surface to early endosomes via dynamin- and clathrin-dependent endocytosis. It has recently been discovered that this pathway can mediate a second series of endosomal GPCR signaling that is distinct from G protein-dependent signaling at the plasma membrane. It is believed that the importance of this mechanism depends on the affinity with which GPCRs interact with βarrs, which varies depending on the extent of GPCR phosphorylation by GRKs. "Class A" GPCRs (e.g., $\beta_2$AR, $\alpha_{1b}$AR) have few phosphorylation sites, and transiently interact with βarr1 and βarr2, mostly at the plasma membrane, with a higher affinity for βarr2. "Class B"GPCRs (e.g., $AT_{1A}$R, NKiR, $PAR_2$) are phosphorylated at multiple sites and interact with both βarr1 and 2 with high affinity for prolonged periods at plasma and endosomal membranes. "Class C"GPCRs (e.g., bradykinin $B_2$ receptor) internalize with βarrs into endosomes followed by rapid dissociation of βarr upon agonist removal.

It is believed that the extent of Parr-induced MAPK signaling depends on the affinity of the receptor for βarrs, which depends on the receptor structure and on which of the seven mammalian GRKs phosphorylate the receptor. Thus, activation of $AT_{1A}$R and $V_2$R causes greater phosphorylation of βarr-bound ERK1/2 than activation of $\alpha_{1b}$AR and $\beta_2$AR, suggesting that the class B receptors signal more robustly through this pathway. Class B GPCRs include the secretin receptor, VPACi receptor, $VPAC_2$ receptor, PACi receptor, glucagon receptor, growth hormone releasing hormone (GHRH) receptor, glucagon-related peptide 1 (GLP-1) receptor, glucagon-related peptide 2 (GLP-2) receptor, gastric inhibitory polypeptide (GIP) receptor, corticotropin releasing factor 1 (CRF1) receptor, corticotropin releasing factor 2 (CRF2) receptor, parathyroid hormone 1 (PTH1) receptor, parathyroid hormone 2 (PTH2) receptor, calcitonin receptor-like receptor (CLR), calcitonin receptor, angiotensin II receptor type 1, vasopressin receptor 2, calcitonin gene related peptide (CGRP) receptor, neurokinin 1 receptor (NKiR), and protease activated-2 receptor ($PAR_2$).

In one embodiment, the modulator of endosomal GPCR signaling is a modulator of the endosomal NKi receptor.

In some embodiments, the modulator of endosomal GPCR signaling or pharmaceutically acceptable salt thereof is a modulator of the endosomal NKiR signaling. In certain of these embodiments, the hydrophobic modulator of endosomal GPCR signalling or pharmaceutically acceptable salt thereof is selected from the group consisting of aprepitant, fosaprepitant, tradipitant, maropitant, HTX-019, netupitant, serlopitant, orvepitant, NAS-911B, ZD-6021, KD-018, DNK-333, NT-432, NK-949, NT-814, EU-C-001, vestipitant, 1144814, SCH-900978, AZD-2738, BL-1833, casopitant, AV-810, KRP-103, 424887, cizolirtine, vofopitant, L-742694, capsazepine, GR-82334, MEN-11149, L-732138, NiK-004, TA-5538, CP-96345, lanepitant, LY-2590443, dapitant, burapitant, befetupitant, CJ-17493, AVE-5883, CGP-49823, CP-122721, CP-99994, SLV-317, TAK-637, L-733060, L-703606, dilopetine, MPC-4505, L-742311, FK-888, WIN-64821, NIP-530, SLV-336, ezlopitant, TKA-457, figopitant, ZD-4794, CP-100263, GR-203040, L-709210, MEN-10930, MEN-11467, LY-306740, FK-355, WIN-67689, WIN-51708, FK-224, BL-1832, CAM-6108, CP-98984, WS-9326A, L-741671, L-737488, L-740141, L-760735, L-161664, YM-49244, Sch-60059, SDZ-NKT-343, S-18523, RPR-111905, S-19752, L-161644, LY-297911, RPR-107880, L-736281, anthrotainin, RP-73467, WIN-64745, WIN-68577, WIN-62577 WIN-66306, RP-67580, CP-0364, L-743986, S-16474, CGP-47899, FR-113680, YM-44778, GR-138676, CGP-73400, CAM-2445, MDL-105172A, L-756867, isbufylline, R-673, SR-48968 and SR-14033, GW679769, CP-0578, and a pharmaceutically acceptable salt thereof.

As a non-limiting example, the hydrophobic modulator endosomal GPCR signalling or pharmaceutically acceptable salt thereof can be selected from the group consisting of: aprepitant, vofopitant, L-733060, and a pharmaceutically acceptable salt thereof. For example, the hydrophobic modulator endosomal GPCR signalling or pharmaceutically acceptable salt thereof is aprepitant or a pharmaceutically acceptable salt thereof.

In a further embodiment, the present invention provides the aqueous liquid according to the invention for use in the treatment of a disease or disorder mediated by endosomal NKiR signalling (e.g., when the modulator of endosomal GPCR signalling is a modulator (e.g., inhibitor) of endosomal NKiR signalling as described *supra*).

In a preferred embodiment, the disease or disorder mediated by endosomal NKiR signaling is selected from chemotherapy-induced nausea and vomiting (CINV), cyclic vomiting syndrome, postoperative nausea and vomiting, affective and addictive disorders including depression and anxiety, generalised anxiety disorder (GAD), gastrointestinal disorders including inflammatory bowel disease, irritable bowel syndrome, gastroparesis and functional dyspepsia, chronic inflammatory disorders including arthritis, respiratory disorders including COPD and asthma, urogenital disorders, sensory disorders and pain including somatic pain and visceral pain, pruritus, viral and bacterial infections and proliferative disorders (cancer), and combinations thereof.

In one embodiment, the disease or disorder mediated by endosomal NKiR signaling is somatic pain or visceral pain.

Within the context of the present invention, the term "pain" includes chronic inflammatory pain (e.g. pain associated with rheumatoid arthritis, osteoarthritis, rheumatoid spondylitis, gouty arthritis and juvenile arthritis); musculoskeletal pain, lower back and neck pain, sprains and strains, neuropathic pain, sympathetically maintained pain, myositis, pain associated with cancer and fibromyalgia, pain associated with migraine, pain associated with cluster and chronic daily headache, pain associated with influenza or other viral infections such as the common cold, rheumatic fever, pain associated with functional bowel disorders such as non-ulcer dyspepsia, non-cardiac chest pain and irritable bowel syndrome, pain associated with myocardial ischemia, post-operative pain, headache, toothache, dysmenorrhea, neuralgia, fibromyalgia syndrome, complex regional pain syndrome (CRPS types I and II), neuropathic pain syndromes (including diabetic neuropathy, chemoterapeutically induced neuropathic pain, sciatica, non-specific lower back pain, multiple sclerosis pain, HIV-related neuropathy, post-herpetic neuralgia, trigeminal neuralgia) and pain resulting from physical trauma, amputation, cancer, toxins or chronic inflammatory conditions. In a preferred embodiment the pain is somatic pain or visceral pain.

In another embodiment, the disease or disorder mediated by endosomal NKiR signaling is a chronic disease or disorder.

In some embodiments, the use in the method of treatment above further comprises administering to the subject an effective amount of a second modulator of endosomal GPCR signalling or a pharmaceutically acceptable salt thereof.

In certain embodiments, the second modulator of endosomal GPCR signalling or a pharmaceutically acceptable salt thereof is an inhibitor of endosomal CGRP and/or CLR signalling, such as MK-3207, olcegepant, or a pharmaceutically acceptale salt thereof.

In some embodiments, the second modulator of endosomal GPCR signalling or pharmaceutically acceptable salt thereof is contained within the cores of polymeric nanoparticles of copolymer chains,
wherein the copolymer chains assemble to form a core / shell structure, the copolymer chains having:

(i) an acid-responsive hydrophobic polymer block that forms the core of the nanoparticles, wherein the acid-responsive hydrophobic polymer block comprises tertiary amine functional groups that are protonated in an acidic environment; and
(ii) a hydrophilic polymer block that forms the shell of the nanoparticles and is solvated by the aqueous liquid.

In some embodiments, the hydrophobic modulator of endosomal GPCR signaling or pharmaceutically acceptable salt thereof is an inhibitor of endosomal CGRP and/or CLR signalling or pharmaceutically acceptabl salt.

In certain of these embodiments, the hydrophobic modulator of endosomal GPCR signaling or pharmaceutically acceptable salt thereof is selected from the group consisting of BI 44370, MK-3207, olcegepant, ubrogepant, rimegepant, SB-268262, telcagepant, and a pharmaceutically acceptable salt thereof.

As a non-limiting example, the hydrophobic modulator of endosomal GPCR signaling or pharmaceutically acceptable salt thereof can be selected from the group consisting of olcegepant, MK-3207, and a pharmaceutically acceptable salt thereof. For example, the hydrophobic modulator of endosomal GPCR signaling or pharmaceutically acceptable salt is MK-3207 or a pharmaceutically acceptable salt thereof.

In a further embodiment, the present disclosure provides the aqueous liquid according to the invention for use in the treatment of a disease or disorder mediated by endosomal CGRP receptor signalling and/or CLR signalling (e.g., when the endosomal GPCR signalling modulator is a modulator (e.g., inhibitor) of endosomal CGRP receptor signalling and/or CLR signalling as described supra).

In certain embodiments, the disease or disorder mediated by endosomal CGRP receptor signalling and/or CLR signaling is selected from the group consisting of: migraine and symptoms associated with migraine including pain, photophobia, phonophobia, nausea and vomiting, sensory disorders, pain including somatic pain and visceral pain, pain associated with cluster and chronic daily headache, respiratory disorders including COPD and asthma, gastrointestinal disorders including inflammatory bowel disease, irritable bowel syndrome, gastroparesis and functional dyspepsia, and chronic inflammatory disorders including osteoarthritis and rheumatoid arthritis.

In certain embodiments, the disease or disorder mediated by endosomal CGRP receptor signalling and/or CLR signaling is somatic pain or visceral pain.

In certain embodiments, the disease or disorder mediated by endosomal CGRP receptor signalling and/or CLR signaling is a chronic disease or disorder.

In certain embodiments, the use in the treatment above further comprises administering to the subject an effective amount of a second modulator of endosomal GPCR signalling or a pharmaceutically acceptable salt thereof.

In certain embodiments, the second modulator of endosomal GPCR signalling or a pharmaceutically acceptable salt thereof is an inhibitor of endosomal NKiR signalling, such as aprepitant, vofopitant, L-733060, or a pharmaceutically acceptale salt thereof.

In certain embodiments, the second modulator of endosomal GPCR signalling or pharmaceutically acceptable salt thereof is contained within the cores of polymeric nanoparticles of copolymer chains,
wherein the copolymer chains assemble to form a core / shell structure, the copolymer chains having:

(i) an acid-responsive hydrophobic polymer block that forms the core of the nanoparticles, wherein the acid-responsive hydrophobic polymer block comprises tertiary amine functional groups that are protonated in an acidic environment; and
(ii) a hydrophilic polymer block that forms the shell of the nanoparticles and is solvated by the aqueous liquid.

In some embodiments, the aqueous liquid herein further comprises a second hydrophobic modulator of endosomal GPCR signalling or a pharmaceutically acceptable salt thereof, wherein the second hydrophobic modulator of endosomal GPCR signalling or pharmaceutically acceptable salt thereof is contained within the core of the polymeric nanoparticles.

In certain embodiments, the second hydrophobic modulator of endosomal GPCR signaling or pharmaceutically acceptable salt thereof is an inhibitor of CGRP receptor signalling, e.g., CLR signaling.

In certain embodiments, the second hydrophobic modulator of endosomal GPCR signaling or pharmaceutically acceptable salt thereof is selected from the group consisting of BI 44370, MK-3207, olcegepant, ubrogepant, rimegepant, SB-268262, telcagepant, and a pharmaceutically acceptable salt thereof.

As a non-limiting example, the second hydrophobic modulator of endosomal GPCR signaling or pharmaceutically acceptable salt thereof can be selected from the group consisting of olcegepant, MK-3207, and a pharmaceutically acceptable salt thereof. For example, the second hydrophobic modulator of endosomal GPCR signaling or pharmaceutically acceptable salt thereof is MK-3207, or a pharmaceutically acceptable salt thereof.

In some embodiments, the molar ratio of the first hydrophobic modulator of endosomal GPCR signalling or pharmaceutically acceptable salt thereof and the second hydrophobic modulator of endosomal GPCR signalling or pharmaceutically acceptable salt thereof is from 1:10 to 10:1.

In some embodiments, the molar ratio of the first hydrophobic modulator of endosomal GPCR signalling or pharmaceutically acceptable salt thereof and the second hydrophobic modulator of endosomal GPCR signalling or pharmaceutically acceptable salt thereof is from 1:4 to 4:1 (e.g., from 1:2 to 2:1 (e.g., 1:1)).

In certain embodiments, the first hydrophobic modulator of endosomal GPCR signalling or pharmaceutically acceptable salt thereof is an inhibitor of endosomal $NK_1R$ signalling or pharmaceutically acceptable salt thereof; and the second hydrophobic modulator of endosomal GPCR signaling or pharmaceutically acceptable salt thereof is an inhibitor of endosomal CGRP receptor signalling, e.g., CLR signaling or pharmaceutically acceptable salt thereof.

As a non-limiting example, the first hydrophobic modulator of endosomal GPCR signalling or pharmaceutically acceptable salt thereof can be aprepitant, vofopitant, L-733060, or a pharmaceutically acceptable salt thereof; and the second hydrophobic modulator of endosomal GPCR signaling or pharmaceutically acceptable salt thereof can be olcegepant, MK-3207, or a pharmaceutically acceptable salt thereof.

Accordingly, in another aspect, provided herein is an aqueous liquid comprising polymeric nanoparticles of copolymer chains assembled to form a core / shell structure, the copolymer chains having:

(i) an acid-responsive hydrophobic polymer block that forms the core of the nanoparticles, wherein the acid-responsive hydrophobic polymer block comprises tertiary amine functional groups that are protonated in an acidic environment; and
(ii) a hydrophilic polymer block that forms the shell of the nanoparticles and is solvated by the aqueous liquid,

wherein the nanoparticles contain within their core:

a) a first hydrophobic modulator of endosomal GPCR signaling, or a pharmaceutically acceptable salt thereof; and
b) a second hydrophobic modulator of endosomal GPCR signaling, or a pharmaceutically acceptable salt thereof.

In certain of these embodiments, the aqueous liquid can have one or more features as described anywhere herein. For example,

The acid-responsive hydrophobic polymer block can comprise tertiary amine functional groups that are protonated in an acidic environment (e.g., at about pH = 6.1);

the acid-responsive hydrophobic polymer block can comprise a homopolymer or copolymer of 2-(diisopropylamino)ethyl methacrylate (DiPAEMA);

the acid-responsive hydrophobic polymer block can comprise a copolymer of 2-(diisopropylamino)ethyl methacrylate (DiPAEMA) and a polyalkyleneoxide methacrylate;

the acid-responsive hydrophobic polymer block can comprise a copolymer of 2-(diisopropylamino)ethyl methacrylate (DiPAEMA) and diethylene glycol methacrylate (DEGMA);

the hydrophilic polymer block can comprise a copolymer of poly(ethylene glycol)methacrylate (PEGMA) and 2-(dimethylamino)ethyl methacrylate (DMAEMA); and/or

the nanoparticle diameter can be from 15 to 120 nm (e.g., from 15-100 nm (e.g., from 25-50 nm)).

In certain embodiments, the first hydrophobic modulator of endosomal GPCR signaling or pharmaceutically acceptable salt thereof is an inhibitor of an endosomal GPCR.

In certain embodiments, the first hydrophobic modulator of endosomal GPCR signaling or pharmaceutically acceptable salt thereof is an inhibitor of endosomal $NK_1R$ signalling or pharmaceutically acceptable salt thereof.

In certain of these embodiments, the first hydrophobic modulator of endosomal GPCR signalling or pharmaceutically acceptable salt thereof is selected from the group consisting of: aprepitant, fosaprepitant, tradipitant, maropitant, HTX-019, netupitant, serlopitant, orvepitant, NAS-911B, ZD-6021, KD-018, DNK-333, NT-432, NK-949, NT-814, EU-C-001, vestipitant, 1144814, SCH-900978, AZD-2738, BL-1833, casopitant, AV-810, KRP-103, 424887, cizolirtine, vofopitant, L-742694, capsazepine, GR-82334, MEN-11149, L-732138, NiK-004, TA-5538, CP-96345, lanepitant, LY-2590443, dapitant, burapitant, befetupitant, CJ-17493, AVE-5883, CGP-49823, CP-122721, CP-99994, SLV-317, TAK-637, L-733060, L703,606, dilopetine, MPC-4505, L-742311, FK-888, WIN-64821, NIP-530, SLV-336, ezlopitant, TKA-457, figopitant, ZD-4794, CP-100263, GR-203040, L-709210, MEN-10930, MEN-11467, LY-306740, FK-355, WIN-67689, WIN-51708, FK-224, BL-1832, CAM-6108, CP-98984, WS-9326A, L-741671, L-737488, L-740141, L-760735, L-161664, YM-49244, Sch-60059, SDZ-NKT-343, S-18523, RPR-111905, S-19752, L-161644, LY-297911, RPR-107880, L-736281, anthrotainin, RP-73467, WIN-64745, WIN-68577, WIN-62577 WIN-66306, RP-67580, CP-0364, L-743986, S-16474, CGP-47899, FR-113680, YM-44778, GR-138676, CGP-73400, CAM-2445, MDL-105172A, L-756867, isbufylline, R-673, SR-48968 and SR-14033, GW679769, CP-0578, and a pharmaceutically acceptable salt thereof.

As a non-limiting example, the first hydrophobic modulator endosomal GPCR signalling or pharmaceutically acceptable salt thereof can be selected from the group consisting of: aprepitant, vofopitant, L-733060, and a pharmaceutically acceptable salt thereof.

In certain embodiments, the second hydrophobic modulator of endosomal GPCR signaling or pharmaceutically acceptable salt thereof is an inhibitor of CGRP and/or CLR signalling.

In certain embodiments, the second hydrophobic modulator of endosomal GPCR signaling or pharmaceutically acceptable salt thereof is selected from the group consisting of BI 44370, MK-3207, olcegepant, ubrogepant, rimegepant, SB-268262, telcagepant, and a pharmaceutically acceptable salt thereof.

As a non-limiting example, the second hydrophobic modulator of endosomal GPCR signaling or pharmaceutically acceptable salt thereof can be selected from the group consisting of olcegepant, MK-3207, and a pharmaceutically acceptable salt thereof.

In certain embodiments, the molar ratio of the first hydrophobic modulator of endosomal GPCR signalling or pharmaceutically acceptable salt thereof and the second hydrophobic modulator of endosomal GPCR signalling or pharmaceutically acceptable salt thereof is from 1:10 to 10:1.

In certain embodiments, the molar ratio of the first hydrophobic modulator of endosomal GPCR signalling or pharmaceutically acceptable salt thereof and the second hydrophobic modulator of endosomal GPCR signalling or pharmaceutically acceptable salt thereof is from 1:4 to 4:1 (e.g., from 1:2 to 2:1 (e.g., 1:1)).

In certain embodiments, the first hydrophobic modulator of endosomal GPCR signalling or pharmaceutically acceptable salt thereof is an inhibitor of endosomal NKiR signalling or pharmaceutically acceptable salt thereof; and the second hydrophobic modulator of endosomal GPCR signaling or pharmaceutically acceptable salt thereof is an inhibitor of endosomal CGRP receptor signalling, e.g., CLR signaling or pharmaceutically acceptable salt thereof.

As a non-limiting example, the first hydrophobic modulator of endosomal GPCR signalling or pharmaceutically acceptable salt thereof can be aprepitant, vofopitant, L-733060, or a pharmaceutically acceptable salt thereof; and the second hydrophobic modulator of endosomal GPCR signaling or pharmaceutically acceptable salt thereof can be olcegepant, MK-3207, or a pharmaceutically acceptable salt thereof.

Accordingly, in another aspect, provided herein but not part of the invention is a method of modulating endosomal GPCR signaling in a subject in need thereof comprising administering to the subject an effective amount of the aqueous liquid according to the present disclosure, wherein the core of the polymeric nanoparticles contains a first modulator of endosomal GPCR signalling and a second moluator of endosomal GPCR signalling as described *supra.*

In another aspect, provided herein but not part of the invention is a method of modulating endosomal GPCR signaling in a subject in need thereof comprising administering to the subject an aqueous liquid comprising polymeric nanoparticles of copolymer chains assembled to form a core / shell structure, the copolymer chains having:

(i) an acid-responsive hydrophobic polymer block that forms the core of the nanoparticles, wherein the acid-responsive hydrophobic polymer block comprises tertiary amine functional groups that are protonated in an acidic environment; and
(ii) a hydrophilic polymer block that forms the shell of the nanoparticles and is solvated by the aqueous liquid,

wherein the nanoparticles contain within their core

a) a first hydrophobic modulator of endosomal GPCR signaling, or a pharmaceutically acceptable salt thereof; and
b) a second hydrophobic modulator of endosomal GPCR signaling, or a pharmaceutically acceptable salt thereof;

and wherein the acid-responsive polymer block undergoes a transition in an acidic environment of the endosomal lumen that causes the assembled copolymer chains to disassemble and release the hydrophobic modulators of endosomal GPCR signaling, or the pharmaceutically acceptable salt thereof, into the endosome.

In another aspect, provided herein but not part of the invention is a method for the treatment of a disease or disorder mediated by endosomal GPCR signalling, wherein the disease or disorder is mediated by CGRP receptor signalling, e.g., CLR signaling, and/or NKiR signalling, comprising administering to a subject in need thereof an effective amount of the aqueous liquid according to the present disclosure, wherein the core of the polymeric nanoparticles contains a first modulator of endosomal GPCR signalling and a second moluator of endosomal GPCR signalling as described *supra.*

In some embodiments, the disease or disorder is a disease or disorder mediated by endosomal CGRP receptor signalling, e.g., CLR signaling, such as migraine and symptoms associated with migraine including pain, photophobia, phonophobia, nausea and vomiting, sensory disorders, pain including somatic pain and visceral pain, pain associated with cluster and chronic daily headache, respiratory disorders including COPD and asthma, gastrointestinal disorders including inflammatory bowel disease, irritable bowel syndrome, gastroparesis and functional dyspepsia, or chronic inflammatory disorders including osteoarthritis and rheumatoid arthritis.

In some embodiments, the disease or disorder is a disease or disorder mediated by endosomal NKiR signalling, such as chemotherapy-induced nausea and vomiting (CINV), cyclic vomiting syndrome, postoperative nausea and vomiting, affective and addictive disorders including depression and anxiety, generalised anxiety disorder (GAD), gastrointestinal disorders including inflammatory bowel disease, irritable bowel syndrome, gastroparesis and functional dyspepsia, chronic inflammatory disorders including arthritis, respiratory disorders including COPD and asthma, urogenital disorders, sensory disorders and pain including somatic pain and visceral pain, pruritus, viral and bacterial infections and proliferative disorders (cancer), and combinations thereof.

In certain embodiments, the disease or disorder is somatic pain or visceral pain.

In certain embodiments, the disease or disorder is a chronic disease or disorder.

In another aspect, provided herein but not part of the invention is a method of modulating endosomal GPCR signaling in a subject in need thereof comprising administering to the subject an effective amount of:

1) a first aqueous liquid or a pharmaceutical composition thereof, wherein the first aqueous liquid contains an NKiR inhibitor within the core of the polymeric nanoparticles as described anywhere herein; and
2) a second aqueous liquid or a pharmaceutical composition thereof, wherein the second aqueous liquid contains a CGRP receptor, e.g., CLR inhibitor within the core of the polymeric nanoparticles as described anywhere herein.

In another aspect, provided herein but not part of the invention is a method for the treatment of a disease or disorder mediated by endosomal GPCR signalling comprising administering to the subject an effective amount of:

1) a first aqueous liquid or a pharmaceutical composition thereof, wherein the first aqueous liquid contains an NKiR inhibitor within the core of the polymeric nanoparticles as described anywhere herein; and
2) a second aqueous liquid or a pharmaceutical composition thereof, wherein the second aqueous liquid contains a CGRP receptor, e.g., CLR inhibitor within the core of the polymeric nanoparticles as described anywhere herein.

In certain embodiments, the NKiR inhibitor; the CGRP receptor, e.g., CLR inhibitor; and/or the first and/or second aqueous liquid can have one or more features as described anywhere herein.

In certain embodiments, the disease or disorder mediated by endosomal GPCR signalling can be as described anywhere herein.

In certain of the foregoing embodiments described herein, the inhibitor is an antagonist.

Where the hydrophobic modulator of an endosomal GPCR comprises one or more functional groups that may be protonated or deprotonated (for example at physiological pH) the modulator may be prepared and/or isolated as a pharmaceutically acceptable salt. It will be appreciated that the modulator may be zwitterionic at a given pH. As used herein the expression "pharmaceutically acceptable salt" refers to the salt of a given compound, wherein the salt is suitable for administration as a pharmaceutical. Such salts may be formed, for example, by the reaction of an acid or a base with an amine or a carboxylic acid group respectively.

Pharmaceutically acceptable acid addition salts may be prepared from inorganic and organic acids. Examples of inorganic acids include hydrochloric acid, hydrobromic acid, sulfuric acid, nitric acid, phosphoric acid and the like. Examples of organic acids include acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

Pharmaceutically acceptable base addition salts may be prepared from inorganic and organic bases. Corresponding counter ions derived from inorganic bases include the sodium, potassium, lithium, ammonium, calcium and magnesium salts. Organic bases include primary, secondary and tertiary amines, substituted amines including naturally-occurring substituted amines, and cyclic amines, including isopropylamine, trimethyl amine, diethylamine, triethylamine, tripropylamine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, and N-ethylpiperidine.

The present invention also provides a pharmaceutical composition comprising a therapeutically effective amount of the aqueous liquid according to the invention, together with at least one pharmaceutically acceptable carrier or diluent.

While the aqueous liquid according to the invention, comprising polymeric nanoparticles containing a hydrophobic modulator of endosomal GPCR signaling, or a pharmaceutically acceptable salt thereof, may be the sole active ingredient administered to the subject, the administration of other active ingredient(s) with the hydrophobic modulator of endosomal GPCR signaling is within the scope of the invention. In one or more embodiments it is envisaged that a combination of two or more modulators of endosomal GPCR signaling will be administered to the subject. It is envisaged that the modulator(s) could also be administered with one or more additional therapeutic agents in combination. The combination may allow for separate, sequential or simultaneous administration of the hydrophobic modulator(s) of endosomal GPCR signaling as hereinbefore described with the other active ingredient(s). The combination may be provided in the form of a pharmaceutical composition.

The term "combination", as used herein refers to a composition or kit of parts where the combination partners as defined above can be dosed dependently or independently or by use of different fixed combinations with distinguished amounts of the combination partners, i.e., simultaneously or at different time points. The combination partners can then, e.g., be administered simultaneously or chronologically staggered, that is at different time points and with equal or different time intervals for any part of the kit of parts. The ratio of the total amounts of the combination partners to be administered in the combination can be varied, e.g. in order to cope with the needs of a patient sub-population to be treated or the needs of the single patient which different needs can be due to age, sex, body weight, etc. of the patients.

As will be readily appreciated by those skilled in the art, the route of administration and the nature of the pharmaceutically acceptable carrier will depend on the nature of the condition and the mammal to be treated. It is believed that the choice of a particular carrier or delivery system, and route of administration could be readily determined by a person skilled in the art. In the preparation of any formulation containing the aqueous liquid according to the invention care should be taken to ensure that the activity of the hydrophobic modulator of endosomal GPCR signaling is not destroyed in the process and that the modulator is able to reach its site of action without being destroyed. Similarly the route of administration chosen should be such that the compound reaches its site of action.

Those skilled in the art may readily determine appropriate formulations for the aqueous liquids of the present invention using conventional approaches. Identification of preferred pH ranges and suitable pharmaceutically acceptable excipients, for example antioxidants, is routine in the art. Buffer systems are routinely used to provide pH values of a desired range and include carboxylic acid buffers for example acetate, citrate, lactate and succinate. A variety of antioxidants are available for such formulations including phenolic compounds such as BHT or vitamin E, reducing agents such as methionine or sulphite, and metal chelators such as EDTA.

It is envisaged that the aqueous liquids according to the invention will be prepared in parenteral dosage forms, including those suitable for intravenous, intrathecal, and intracerebral or epidural delivery. The pharmaceutical forms suitable for injectable use include sterile injectable solutions or dispersions, and sterile powders for the extemporaneous preparation of sterile injectable solutions. They should be stable under the conditions of manufacture and storage and may be preserved against reduction or oxidation and the contaminating action of microorganisms such as bacteria or fungi.

The solvent or dispersion medium for the injectable solution or dispersion may contain any of the conventional solvent or carrier systems for the active compound, and may contain, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as lecithin, by the maintenance of the required

particle size in the case of dispersion and by the use of surfactants. The prevention of the action of microorganisms can be brought about where necessary by the inclusion of various antibacterial and antifungal agents, for example, parabens, chlorobutanol, phenol, sorbic acid, thimerosal and the like. In many cases, it will be preferable to include agents to adjust osmolarity, for example, sugars or sodium chloride. Preferably, the formulation for injection will be isotonic with blood. Prolonged absorption of the injectable compositions can be brought about by the use in the compositions of agents delaying absorption, for example, aluminium monostearate and gelatin. Pharmaceutical forms suitable for injectable use may be delivered by any appropriate route including intravenous, intramuscular, intracerebral, intrathecal, epidural injection or infusion.

Sterile injectable solutions are prepared by incorporating the aqueous liquids of the invention in the required amount in the appropriate solvent with various of the other ingredients such as those enumerated above, as required, followed by filtered sterilization. Generally, dispersions are prepared by incorporating the various sterilised active ingredient into a sterile vehicle which contains the basic dispersion medium and the required other ingredients from those enumerated above.

Pharmaceutically acceptable vehicles and/or diluents include any and all solvents, dispersion media, antibacterial and antifungal agents, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated. Supplementary active ingredients can also be incorporated into the compositions.

It is especially advantageous to formulate the compositions in unit dosage form for ease of administration and uniformity of dosage. Unit dosage form as used herein refers to physically discrete units suited as unitary dosages for the mammalian subjects to be treated; each unit containing a predetermined quantity of active material calculated to produce the desired therapeutic effect in association with the required pharmaceutically acceptable vehicle. The specification for the novel unit dosage forms of the invention are dictated by and directly dependent on (a) the unique characteristics of the active material and the particular therapeutic effect to be achieved, and (b) the limitations inherent in the art of compounding active materials for the treatment of disease in living subjects having a diseased condition in which bodily health is impaired as herein disclosed in detail.

As mentioned above the principal active ingredient may be compounded for convenient and effective administration in therapeutically effective amounts with a suitable pharmaceutically acceptable vehicle in unit dosage form. A unit dosage form can, for example, contain the principal active compound in amounts ranging from 0.25 μg to about 2000 mg. Expressed in proportions, the active compound may be present in from about 0.25 μg to about 2000 mg/mL of carrier. In the case of compositions containing supplementary active ingredients, the dosages are determined by reference to the usual dose and manner of administration of the said ingredients.

As used herein, the term "effective amount" refers to an amount of compound which, when administered according to a desired dosing regimen, provides the desired therapeutic activity. Dosing may occur once, or at intervals of minutes or hours, or continuously over any one of these periods. Suitable dosages may lie within the range of about 0.1 ng per kg of body weight to 1 g per kg of body weight per dosage. A typical dosage is in the range of 1 μg to 1 g per kg of body weight per dosage, such as is in the range of 1 mg to 1 g per kg of body weight per dosage. In one embodiment, the dosage may be in the range of 1 mg to 500 mg per kg of body weight per dosage. In another embodiment, the dosage may be in the range of 1 mg to 250 mg per kg of body weight per dosage. In yet another embodiment, the dosage may be in the range of 1 mg to 100 mg per kg of body weight per dosage, such as up to 50 mg per body weight per dosage.

The terms "treatment" and "treating" as used herein cover any treatment of a condition or disease in an animal, preferably a mammal, more preferably a human. The terms "prevention" and "preventing" as used herein cover the prevention or prophylaxis of a condition or disease in an animal, preferably a mammal, more preferably a human.

Throughout this specification and claims which follow, unless the context requires otherwise, the word "comprise", and variations such as "comprises" or "comprising", will be understood to imply the inclusion of a stated integer or group of integers or steps but not the exclusion of any other integer or group of integers.

The reference in this specification to any prior publication (or information derived from it), or to any matter which is known, is not, and should not be taken as an acknowledgment or admission or any form of suggestion that that prior publication (or information derived from it) or known matter forms part of the common general knowledge in the field of endeavour to which this specification relates.

The invention will now be described with reference to the following examples:

**Example 1: Synthesis and characterisation of diblock copolymers**

Diblock copolymers were synthesised according to Scheme 5/Scheme 5B and the methods outlined below. These diblock copolymers both comprised the same hydrophilic copolymer P(PEGMA-co-DMAEMA) but different hydrophobic copolymers. P(DIPMA-co-DEGMA) was used to give acid-responsive properties to the polymer and P(BMa) to form a control polymer with non-acid responsive properties. Prior to each synthesis, monomers were deinhibited using

basic aluminum oxide.

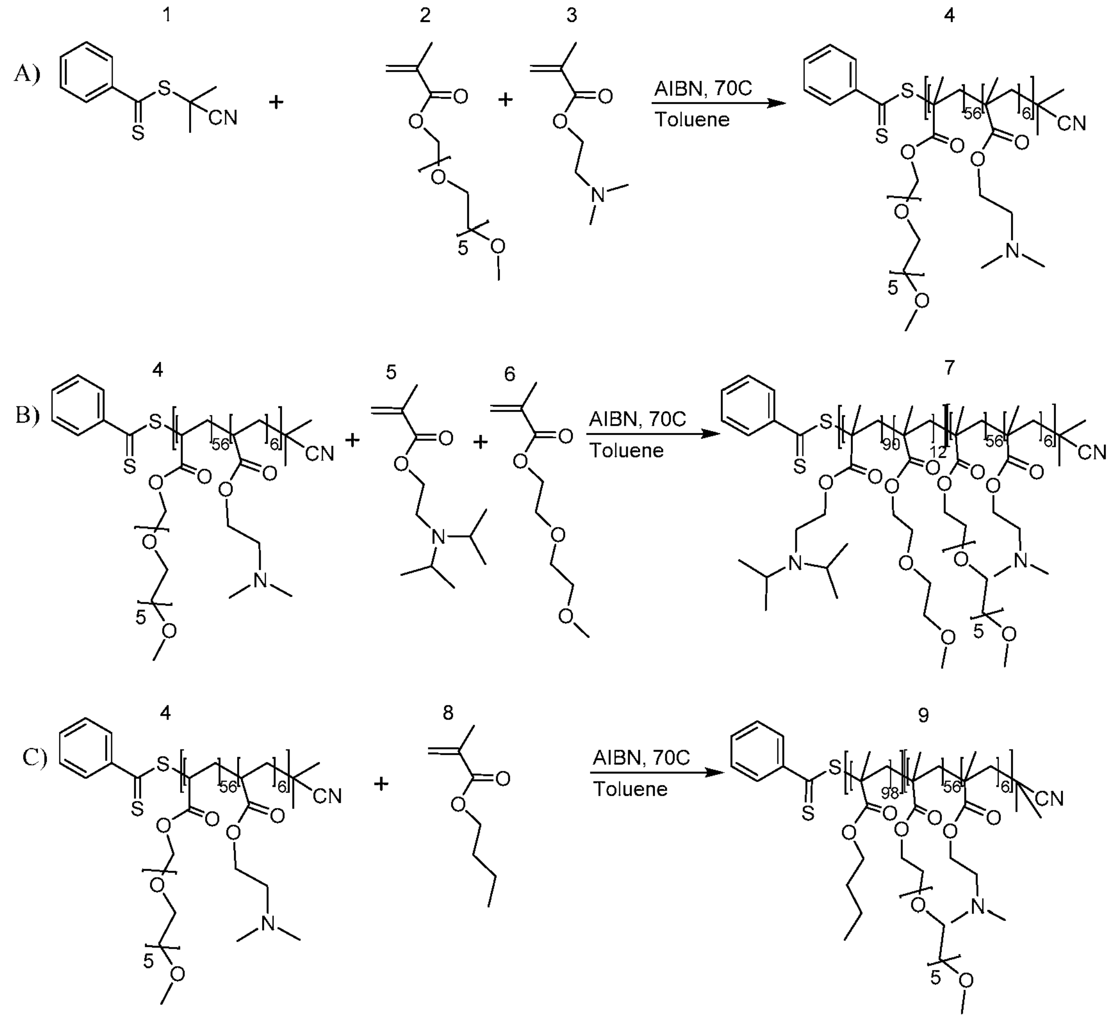


**Scheme 5:** Sequential polymerization of P(PEGMA-co-DMAEMA)-b-P(DIPMA-co-DEGMA) and P(PEGMA-co-DMAEMA)-b-P(BMA) diblock copolymers. A) Synthesis of the hydrophilic block using (1) CPDB; (2) PEGMA and (3) DMAEMA to form (4) the hydrophilic block p(PEGMA-co-DMAEMA). B) Synthesis of P(PEGMA-co-DMAEMA)-b-P(DIPMA-co-DEGMA) by chain extension reaction, where addition of (5) DIPMA and (6) DEGMA to (4) the hydrophilic block forms (7) the diblock P(PEGMA-co-DMAEMA)-b-P(DIPMA-co-DEGMA) and C) the addition of (8) BMA to (4) the hydrophilic block forms (9) P(PEGMA-co-DMAEMA)-b-P(BMA). The initiator utilised is AIBN.

P(PEGMA-co-DMAEMA)-b-P(DIPMA-co-DEGMA-co-Cy5) diblock copolymer was synthesized according to Scheme 5B and the methods outlined below.

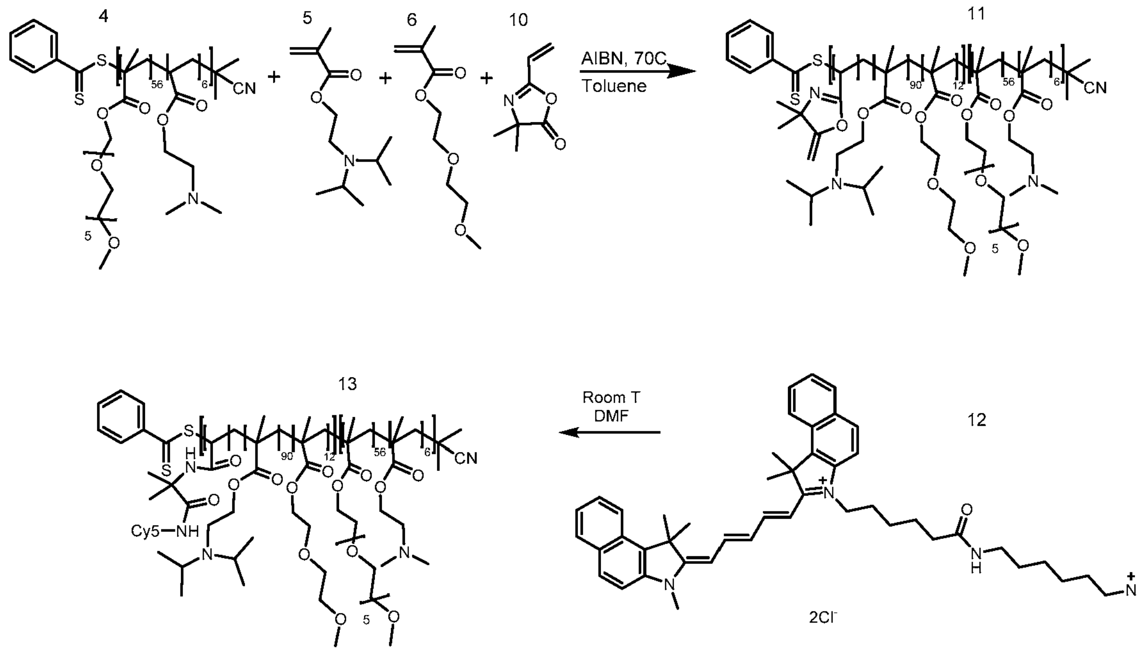


**Scheme 5B:** synthesis of the P(PEGMA-co-DMAEMA)-b-P(DIPMA-co-DEGMA-co-Cy5) diblock copolymer. Polymerization using (4) the hydrophilic block p(PEGMA-co-DMAEMA), (5) DIPMA, (6) DEGMA, and (10)VDM forms (11). Subsequent coupling of (11) with Cy5 (12) forms (13).

**Macromolecular chain transfer agent: P(PEGMA-co-DMAEMA) hydrophilic block copolymer.** The macromolecular chain transfer agent (Macro-CTA) was synthesised by reversible addition fragmentation chain transfer (RAFT) polymerisation using 2-cyanoprop-2-yl dithiobenzoate (CPBD) as a RAFT agent and azobisisobutyronitrile (AIBN) as the initiator in a ratio of 2:0.2. Poly(ethylene glycol) methyl ether methacrylate average Mn ~ 500 (PEGMA), 2-(dimethylamino)ethyl methacrylate (DMAEMA, 98%), 2-(diethylamino)ethyl methacrylate (DEAEMA, 99%), 2-(diisopropyllamino)ethyl methacrylate (DIPAEMA, 97%), butyl methacrylate (BMA, 99%), and 4,4-dimethyl-2-vinyl-2-oxazolin-5-one (VDM) were obtained from Sigma Aldrich.

**P(PEGMA-co-DMAEMA) hydrophilic block copolymer**

The macromolecular chain transfer agent (Macro-CTA), P(PEGMA-co-DMAEMA), was synthesized by the reversible addition fragmentation chain (RAFT) polymerization method using 2-cyanoprop-2-yl dithiobenzoate (CPBD) as a RAFT agent and azobisisobutyronitrile (AIBN) as the initiator in a ratio of 2:0.2. PEGMA and DMAEMA (at a ratio of 120:12) were dissolved in 30 mL of toluene with CPBD and AIBN. The solution was deoxygenated by sparging with nitrogen for 30 min. After polymerizing for 21 h at 70 °C and 400 RPM, the reaction was cooled to 0 °C in an ice bath and exposed to air. An aliquot of the solution was sampled to determine conversion by $^{1}$H NMR. The solution was then dialyzed (MWCO 3500) against acetone for 96 h to remove any remaining monomer. Solvent was evaporated and the product was dried for 24 in a vacuum oven at 37°C and 1000 mbar.

**P(PEGMA-co-DMAEMA)-b-P(DIPMA-co-DEGMA) diblock copolymer.** The chain extension reaction was performed in the presence of AIBN using the hydrophilic block P(PEGMA-co-DMAEMA) and the monomers DIPMA and DEGMA at a ratio of 1:0.15:100:11. The mixture was dissolved in toluene and once deoxygenated, was left to react at 70 °C, 400 RPM for 17.5 h. The final product was purified as previously described.

**P(PEGMA-co-DMAEMA)-b-P(BMA) diblock copolymer.** BMA was polymerised from the hydrophilic P(PEGMA-co-DMAEMA) block by a chain extension reaction in the presence of AIBN in toluene. The ratio of [BMA]:[Macro-CTA]:[AIBN] was 120:1:0.2. The solution was deoxygenated and the contents of the vial were left to react for 15 h at 70 °C and 400 RPM. The final product was purified as previously described.

**P(PEGMA-co-DMAEMA)-b-P(DIPMA-co-DEGMA-co-Cy5) diblock copolymer.**

The chain extension of P(PEGMA-co-DMAEMA) was done in toluene by adding DIPMA, DEGMA and 4,4-dimethyl-2-vinyl-2-oxazolin-5-one (VDM) in the presence of AIBN at a ratio of 1:100:11:0.15. The mixture was deoxygenated and left to react at 70 °C, 400 RPM for 17.5 h. Cy5 coupling was performed in a second step by mixing the reaction with Cyanine5 amine. The mixture was left to react at room temperature, 400 RPM for 72h under dark conditions and the final product was purified as described.

**Gel permeation chromatography (GPC).** GPC analysis to determine the molecular weight of the polymer was performed on a Shimadzu (Kyoto, Japan) liquid chromatography system equipped with a (RID-10A) differential refractive index detector ($\lambda$ = 633 nM) and SPD-20A ultraviolet detector connected to a 5.0$\mu$m bead-size guard column (50 $\times$ 7.8 mm) followed by three Shodex KF-850L columns (300 $\times$ 8 mm, bead size: 10 $\mu$m, pore size maximum: 5000 Å) in series operating at 40 °C. The eluent was *N*,*N*-dimethylacetamide (DMAC, HPLC grade with 0.03% w/v LiBr) and running at 1 mL/min. A molecular weight calibration curve was produced using polystyrene standards with narrow molecular weights distribution ranging from 500 to 2 $\times$ $10^6$ Da.

**Proton-nuclear magnetic resonance ($^1$H-NMR).** $^1$H-NMR analysis to determine the conversion of the polymers was performed using a Bruker Avance III 400 Ultrashield Plus spectrometer (MA, USA) at 400 mHz running Topspin, version 1.3, using deuterated chloroform (chloroform-d) as solvent. The conversion for P(PEGMA-co-DMAEMA) was determined by the integral of the monomer peak at 5.7 ppm on the raw $^1$H-NMR. The composition of PEGMA and DMAEMA was determined using the peak of P(PEGMA-co-DMAEMA) at 4.25 ppm and the peak of DMAEMA at 2.5 ppm (Figure 2A). The composition of DIPMA and DEGMA monomers on P(PEGMA-co-DMAEMA)-b-(DIPMA-co-DEGMA) was determined by the integral of the DIPMA peak at 2.99 ppm and the ratio of the integrals of the peaks at 3.375 and 3.395 ppm of PEGMA and DEGMA monomers (Figure 2B). The conversion of P(PEGMA-co-DMAEMA)-b-(DIPMA-co-DEGMA) was determined using the initial ratio of DIPMA and DEGMA (100:11) and the ratio obtained by $^1$H-NMR (90:12). Similarly, P(PEGMA-co-DMAEMA)-b-(BMA) conversion was determined using the initial ratio of the BMA and Macro-CTA (120:1) and the ratio obtained by $^1$H-NMR (98:1). The units of BMA were determined by the integral of the peak at 3.94 ppm (Figure 2C).

Conversions (Conv%) and repeating monomer units (n) were calculated by $^1$H-NMR using peak integrals (I) where the subscript number indicates the location of the peak in ppm ($I_x$). The Conv% and n for P(PEGMA-co-DMAEMA) were calculated using the $^1$H-NMR spectra (Figure 2) with $Conv\% = 100 \times (1-I_{5.7})$, $PEGMA\ n = \frac{I_{4.25}-I_{2.5}}{2}$ and $DEGMA\ n = \frac{I_{2.5}}{2}$. Conv% and n for P(PEGMA-co-DMAEMA)-b-(DIPMA-co-DEGMA) were calculated using the $^1$H-NMR spectra (Figure 2) with $Conv\% = 100 \times \frac{(PEGMA\ n + DEGMA\ n)}{(PEGMA\ n_{theoretical} + DEGMA\ n_{theoretical})}$, $DIPMA\ n = \frac{I_3 + I_{2.6}}{4} \times (PEGMA\ n + DEGMA\ n)$ and $DEGMA\ n = \frac{I_{3.396}}{I_{3.378}} \times PEGMA\ n$. Conv% and n for P(PEGMA-co-DMAEMA)-b-(BMA) were calculated using the $^1$H-NMR spectra (Figure 2) with $Conv\% = 100 \times \frac{BMA\ n}{BMA\ n_{theoretical}}$ and $BMA\ n = \frac{I_{3.93}}{2} \times (PEGMA\ n + DEGMA\ n)$.

Table 1 summarises the resulting characteristics of the copolymers and di-block copolymers synthesised. The molecular weights determined by GPC were found to be lower than the ones obtained by $^1$H-NMR due to GPC being calibrated to polystyrene standards. The GPC chromatogram shows unimodal peaks, where the diblock copolymers shifted toward higher retention times as the molecular weight increases (Figure 3). The chemical structures of the polymers were verified by $^1$H-NMR (Figure 2) and the copolymerisation of the macro-CTA resulted in a 54% conversion, while the diblock copolymers P(PEGMA-co-DMAEMA)-b-(DIPMA-co-DEGMA) and P(PEGMA-co-DMAEMA)-b-(BMA) reached higher conversion (92% and 82%). The detailed composition of every copolymer is described in Table 1.

**Table 1** Characterisation of the hydrophilic block copolymer P(PEGMA-co-DMAEMA) and the diblock copolymers P(PEGMA-co-DMAEMA)-b-P(DIPMA-co-DEGMA) and P(PEGMA-co-DMAEMA)-b-P(BMA-co-DEGMA).

| Polymer | Conversion (%) | GPC | | $^1$H-NMR | | | | | |
|---|---|---|---|---|---|---|---|---|---|
| | | $M_n$ (g/mL) | PDI | $M_n$ (g/mL) | Composition | | | | |
| | | | | | PEGMA | DMAEMA | DIPMA | DEGMA | BMA |
| P(PEGMA-co-DMAEMA) | 54 | 12377 | 1.20 | 17,600 | 56 | 6 | - | - | - |
| P(PEGMA-co-DMAEMA)-b-(DIPMA-co-DEGMA) | 92 | 17581 | 1.36 | 38,930 | 56 | 6 | 90 | 12 | - |
| P(PEGMA-co-DMAEMA)-b-(BMA) | 82 | 18225 | 1.37 | 31,530 | 56 | 6 | - | | 98 |

Conversion and composition were calculated by $^1$H NMR monomer ratio.
Mn was measured by GPC using a polystyrene calibration curve, resulting in differing average number molecular weight values by $^1$H -NMR.

**Example 2: Self-assembly and characterisation of polymeric nanoparticles according to the invention and control nanoparticles.**

**Self-assembly of nanoparticles.** P(PEGMA-co-DMAEMA)-b-P(DIPMA-co-DEGMA) was used as the diblock copolymer to self-assemble acid-responsive polymeric nanoparticles according to the invention and P(PEGMA-co-DMAEMA)-b-P(BMA) to self-assemble control nanoparticles that do not possess acid responsive properties.

For the self-assembly of polymeric nanoparticles loaded with aprepitant (Ap), a mixture of 53.5 μg of Ap and 5 mg of diblock copolymer was dissolved in 0.5 mL of tetrahydrofuran (THF), while empty nanoparticles were self-assembled without adding Ap. The mixture was then added into 4.5 mL of phosphate-buffered saline (PBS) under vigorous stirring at a flow rate of 1.2 mL/h, using a Harvard apparatus syringe pump (MA, USA) at room temperature. Assemblies of acid-responsive polymeric nanoparticles loaded with Ap (DIPMA-Ap) and non-acid responsive nanoparticles loaded (BMA-Ap) were dialyzed against PBS under nitrogen flow for 24 h, using dialysis bags (MWCO 3500, Membrane Filtration Products, USA). Assemblies of acid-responsive nanoparticles without Ap (DIPMA-empty and BMA-empty) were dialyzed using Slide-A-Lyzer mini dialysis devices MWCO 3.5K (Thermo Fisher Scientific, MA, USA), for 24 h. The assembly of nanoparticles for live cell imaging was done as described for nanoparticles without AP. The diblock copolymer used was P(PEGMA-co-DMAEMA)-b-P(DIPMA-co-DEGMA-co-Cy5) that couples Cy5 on the hydrophobic portion, resulting in nanoparticles with Cy5 localized in the core (DIPMA-Cy5).

P(PEGMA-co-DMAEMA)-b-P(BMA) polymeric nanoparticles loaded with MK-3207 (DIMPA-(MK-3207) nanoparticles) were prepared in a similar fashion as above.

**Dynamic light scattering.** Size distribution of nanoparticles was determined by dynamic light scattering (DLS) using a Zetasizer Nano ZS ZEN3600 particle size analyser (Malvern, UK). DIPMA-Ap, DIPMA-empty and BMA-$A_P$ at a concentration of 1 mg/mL were filtered using 0.45 μm nylon filters and added to polystyrene cuvettes. Measurements were performed at 25 °C and 173° backscatter angle.

**Ultra-performance liquid chromatography coupled to mass spectrometry (UPLC-MS).** Ap loaded in the core of polymeric nanoparticles was determined by UPLC-MS using a Waters Micromass Quattro Premier triple quadrupole mass spectrometer coupled to a Waters Acquity UPLC (MA, USA). Freeze-dried DIPMA-$A_P$ and BMA-$A_P$ at a concentration of 1 mg/mL were dissolved in a 5:2 mixture of DMSO:0.1% formic acid in water. The samples were prepared for analysis by mixing an aliquot of each preparation with internal standard solution (diazepam, 5 μg/mL) in a 5:2 proportion and made up to 500 μL with the dilution solvent (1:1 mixture of 50% acetonitrile and 0.1% formic acid). Chromatographic separation was conducted on a Supelco Ascentis Express RP Amide column (50 mm by 2.1 mm, 2.7

μm particle size) equipped with a Phenomenex Security Guard pre-column fitted with a Synergi Polar cartridge and both maintained at a column temperature of 40°C. AP loading was quantified against AP standards (0.016 to 20 μM). The mobile phase consisted of 0.05% formic acid in water and acetonitrile and compounds were eluted under gradient conditions. Mass spectrometry was conducted in positive electrospray ionization conditions and elution of compounds monitored with multiple-reaction monitoring.

**pH-dependent disassembly.** Nile Red (Nr) is a solvatochromic dye that fluoresces only in non-polar solvents, allowing determination of the pH of disassembly for the nanoparticles.

Specifically, the pH of disassembly is identified by observing the loss of fluorescence of Nr due to release of NR from the core of nanoparticles. Nanoparticles were self-assembled as previously described and Ap was replaced by 0.5 mg of Nile red per mg of polymer and dialyzed as described. Acid-responsive polymeric nanoparticles loaded with Nile red (DIPMA-Nr) and non-acid responsive polymeric nanoparticles (BMA-Nr) were prepared at a concentration of 200 μg/mL. PBS solutions with a pH range from 7.6 to 5.0 were prepared and pH disassembly properties were assessed measuring Nile red (Sigma-Aldrich, MO, USA) fluorescence (excitation/emission 552/636nm) using a Flex-Station 3 (Molecular Devices, CA, USA).

**Critical micelle concentration (CMC).** CMC was determined by the pyrene $I_1/I_3$ ratio. A pyrene stock solution (50 μM) was prepared in THF and 5 μL of pyrene stock were added to 995 μL of graded concentrations of nanoparticles (400 to 0.5 μg/mL), obtained by diluting nanoparticle stock solutions in PBS. The mixture was stirred for 3 h at room temperature and the fluorescence spectrum of pyrene was recorded from 360 to 410 nm using an excitation wavelength of 336 nm in a Shimadzu Espectrofluorophotometer RF5301PC (Kyoto, Japan). The emission intensities measured at 373 nm ($I_1$) and 384 nm ($I_3$) were used to calculate the pyrene $I_1/I_3$ ratio.

Table 2 summarizes the characterisation of nanoparticles self-assembled using P(PEGMA-co-DMAEMA)-b-P(DIPMA-co-DEGMA) for acid-responsive nanoparticles and P(PEGMA-co-DMAEMA)-b-P(BMA) for control nanoparticles DIPMA and BMA nanoparticles were self-assembled in the presence of Aprepitant (AP, MK-869), a hydrophobic NKiR antagonist, forming P(PEGMA-co-DMAEMA)-b-P(DIPMA-co-DEGMA)-Ap (abbreviated as DIPMA-AP) and P(PEGMA-co-DMAEMA)-b-P(BMA)-Ap nanoparticles (abbrieviated as BMA-AP), respectively. DIPMA-AP nanoparticles loaded 11.4 ± 2.3 μM AP, which corresponds to 57 ± 11.4% of the AP initially added in the self-assembly process (Table 2). BMA-AP nanoparticles loaded 12.3 ± 2.7 μM of AP (60.9 ± 13.4%). Self-assembled nanoparticles are dynamic structures that remain assembled when the concentration of polymer is high enough to favor their assembled state (critical micelle concentration, CMC). The CMC of DIPMA-empty (DIPMA-empty) and BMA-empty nanoparticles was similar (1.9 ± 1.1 and 1.5 ± 1.1 μg/mL, respectively). The CMC of DIPMA-AP (2.4 ± 0.5 μg/mL) was slightly higher than BMA-AP (1.5 ± 0.8 μg/mL) nanoparticles. DIPMA-AP nanoparticles appeared to be uniformly spherical, assessed by transmission electron microscopy, where dynamic light scattering indicated 40.4 ± 5.1 nm diameter and a surface ζ-potential of -0.2 ± 1.6 mV (Table 2, Figure 1C). DIPMA-empty nanoparticles were 37 ± 4.2 nm diameter, with a surface ζ-potential of -0.5 ± 2.0 mV. BMA-AP and BMA-empty nanoparticles were of smaller diameter (28 ± 2.5 and 30 ± 4.4 nm diameter, respectively) but similar ζ-potential (-1.1 ± 2.8 and -0.3 ± 0.3 mV, respectively).

**Table 2** Characterisation of acid-responsive and control nanoparticles self assembled using the diblock copolymers P(PEGMA-co-DMAEMA)-b-P(DIPMA-co-DEGMA) and P(PEGMA-co-DMAEMA)-b-P(BMA-co-DEGMA).

| Nanoparticle | Diameter (nm) | ζ-potential (mV) | Aprepitant loading (%) | CMC (μg/mL) | pH of disassembly |
|---|---|---|---|---|---|
| DIPMA-Ap | 40.4 ± 5.1 | -0.2 ± 1.6 | 57.0 ± 11.4 | 2.4 ± 0.5 | 6.08 ± 0.064 |
| DIPMA-empty | 37.0 ± 4.2 | -0.5 ± 2.0 | N/A | 1.9 ± 1.1 | 6.08 ± 0.064 |
| BMA-Ap | 28.0 ± 2.5 | -1.1 ± 2.8 | 60.9 ± 13.4 | 1.5 ± 0.8 | N/A |
| BMA-empty | 30.0 ± 4.4 | -0.3 ± 0.3 | N/A | 1.5 ± 1.1 | N/A |
| N/A: not applicable. Mean ± SD | | | | | |

### Example 3: DIPMA nanoparticles are internalized into SP $NK_1R$ positive endosomes.

HEK-293 cells transfected with rNKiR tagged with GFP (rNKiR-GFP) were incubated with DIPMA-empty nanoparticles conjugated with Cy5 (DIPMA-Cy5) at 40 min. Cells were then challenged with 10 nM SP to promote activation and internalization of the rNKiR-GFP and imaged at 30 and 60 min post SP addition. At 30 min, rNKiR-GFP and DIPMA-Cy5 nanoparticles were co-localized in endosomes (Figure 4A). This co-localization increased 60 min after SP addition (Figure 4B) indicating that DIPMA nanoparticles are not only able to be internalized by HEK-293 cells, but also to distribute into positive $rNK_1R$-GFP endosomes.

**Example 4: Characterization of nuclear ERK signaling.**

SP concentration response curve showed a rapid nuclear ERK activation that increases over time. Interestingly, SP 10 nM showed a rapid increase followed by a gradual decrease on the signal (Figure 5A). The area under the curve (AUC) showed an $EC_{50}$ of 14.9 nM of SP when calculated for the duration of the experiment (65 min) (Figure 5C) and 5 nM of SP ($EC_{30}$) was selected to stimulate HEK-hNKiR cells on following experiments. The Ap concentration response curve showed that after 5 nM SP stimulation, only the highest concentrations of Ap used (10 μM, 1 μM and 100 nM) were able to decrease nuclear ERK triggered by SP (Figure 5B). The AUC showed an $IC_{50}$ of 123.9 nM for Ap (Figure 5C).

**Example 5: Regulation of $NK_1R$ signaling by acid-responsive polymeric nanoparticles.**

Measurements of intracellular calcium were performed in HEK-293 and HEK-hNKiR cells with DIPMA-empty and BMA-empty nanoparticles to investigate the effect of nanoparticles and free polymer on the activation of channels and receptors that mediate increase of intracellular calcium. Nanoparticles were added in three different concentrations, above (10 μg/mL) and below (3.16 and 1 μg/mL) the CMC. In both cell lines, the effect observed was similar; DIPMA-empty and BMA-empty nanoparticles were not able to increase intracellular calcium influx during the 28 minutes of experiment. These findings demonstrate that nanoparticles do not increase intracellular calcium *in vitro* (Figure 6).

The effect of nanoparticles on ERK activity was assessed by pre-incubating HEK-h NKiR cells with DIPMA-empty and BMA-empty nanoparticles (10, 20 and 30 μg/mL) or vehicle for 30 min prior the addition of vehicle (Figure 7). DIPMA-empty and BMA-empty did not trigger nuclear ERK activation in any of the concentrations studied (Figure 7A).

Viability of cells was examined using alamar Blue, that assess the capability of viable cells to reduce the active compound Rezasurin to resofurin, which can be detected by fluorescence. Cells did not show any significant reduction on their ability to reduce Rezasurin after 24 (Figure 7C) and 48 h (Figure 7D) of incubation with nanoparticles (1, 3, 10, 30 and 100 μg/mL).

**Example 6: Effect of polymeric nanoparticles loaded with aprepitant on nuclear ERK activity.**

After SP stimulation and $NK_1R$ internalization, the $NK_1R$ recruits a series of proteins to form a signalosome from which it is able to signal, increasing nuclear ERK and cAMP (Jensen, D.D., et al., Neurokinin 1 receptor signaling in endosomes mediates sustained nociception and is a viable therapeutic target for prolonged pain relief, Sci. Transl. Med. 2017 31(9), 392). To test the ability of DIPMA-$A_P$ nanoparticles to decrease nuclear ERK signaling, HEK-hNKiR cells were pre-incubated for 30 min with DIPMA-$A_P$ (10 μg/mL -100 nM) nanoparticles and BMA-$A_P$ (10 μg/mL -100 nM) nanoparticles, followed by SP addition. It was found that 10 μg of DIPMA-$A_P$ nanoparticles load with 100 nM of aprepitant were able to decrease nuclear ERK triggered by SP. A similar result was observed for 10 μg/mL of BMA-$A_P$ nanoparticles loaded with 100 nM of aprepitant, while 100 nM of free aprepitant showed a lower decrease (Figure 8).

**Example 7: Effect of nanoparticles on locomotor patterns.**

The Rotarod test was used to assess the effect of nanoparticles on animal motor performance. Treatments did not produce significant differences in latency to fall compared to vehicle group at any of the time points examined, indicating that i.t administration of nanoparticles do not modify locomotor patterns and do not affect neuromuscular coordination in mice (Figure 9).

**Example 8: Distribution of DIPMA-Cy5 nanoparticles.**

Administration of DIPMA-Cy5 nanoparticles allowed tracking of the movement of nanoparticles *in vivo.* After a single administration, nanoparticles were localised within the injection site (L3-L4) throughout the time points evaluated (Figure 10). Decrease of the Cy5 fluorescence was observed between 4-6 h, weakening its signal by 8 h. At 24 h, no fluorescence was observed, which could indicate degradation of the nanoparticles. Nevertheless, after the disassembly of DIPMA-Cy5 nanoparticles and further degradation of the diblock copolymer forming units, there is a possibility that degraded Cy5 conjugated diblock copolymers could quench Cy5 fluorescence, leading to difficulties in accurately interpreting the loss of Cy5 fluorescence.

**Example 9: Intrathecal administration of polymeric nanoparticles relieve the nociceptive response of an acute pain model.**

To evaluate the efficacy of acid-responsive polymeric nanoparticles to selectively inhibit the endosomal signaling of the NKiR involved in the generation of acute pain, a single dose of DIPMA-$A_P$ nanoparticles, controls (DIPMA-empty,

DIPMA-empty mixed with free aprepitant, BMA-$A_P$ and free aprepitant) and vehicle were administered intrathecally 30 minutes prior to subcutaneous injection of capsaicin in the right hindpaw (ipsilateral) (Figure 11). Vehicle and DIPMA-empty showed a robust and rapid decrease in the von Frey response, consistent with capsaicin evoked mechanical allodynia, which returned to basal level at 24 h. A discrete analgesia (30%) was observed on the first hour in free aprepitant and DIPMA-empty nanoparticles mixed with free aprepitant groups, followed by a gradual decay on the analgesia, losing its effects completely at 3h. Interestingly, BMA-$A_P$ nanoparticles showed similar values of analgesia to free aprepitant on the first 30 minutes, extending the effect for 1.5 h. However, the same behaviour on the decay of the effect was observed. In contrast, DIPMA-$A_P$ nanoparticles showed higher analgesia than control groups in the first 60 min, increasing afterwards to reach a sustained effect for 4 h.

**Example 10: Intrathecal administration of polymeric nanoparticles relieves the nociceptive response in a chronic pain model.**

The chronic pain model was induced by ligation of the sciatic nerve, allowing sensitisation to pain (allodynia, hyperalgesia) to develop for 10 days. Sensitisation was confirmed measuring baseline (pre-surgery) and post-surgery paw withdrawal using a Randall-Selitto electronic algesimeter (Eva Santos-Nogueira, et al. J Neurotrauma. 2012, 29(5): 898-904).

Rats were then injected intrathecally (L3-L4 location in spine, total 10μL volume), with polymeric nanoparticles (DIPMA, BMA: empty or loaded with aprepitant), vehicle control (artificial cerebrospinal fluid) or increasing concentrations of aprepitant (100, 300 nM and 1μM, n=4 per each concentration). Three different concentrations of aprepitant were tested to determine which drug concentration is required to show analgesic effects in chronic pain model. 10, 30 or 50 μg of non-acid responsive BMA nanoparticles were loaded with 100, 300 or 500nM of aprepitant (n=6 per each concentration) and 10, 30 or 50 μg of acid-responsive DIPMA nanoparticles were loaded with 100, 300 or 500nM of aprepitant (n=6 per each concentration).

Randall-Selitto was performed at 15, 30 min 1 h, followed by measurements every 30min for 5 h then every hour until 7 hours, the results of which are shown in Figures 12A, 12B and 13. As is evident, aprepitant-loaded nanoparticles provide greater analgesia and longer lasting effects. While 300nM of free aprepitant resulted in a 20g improvement in pressure (-25% pain recovery), this effect lasted only 2 hrs. Whereas 300nM aprepitant loaded into DIPMA polymeric nanoparticles provided 60g improvement in pressure (<80% recovery), lasting for 5hrs.

**Example 11: Assessment of nanoparticle effect on ATP production**

Historically, the toxicity of nanoparticles has been assessed using a myriad of different assays for cell health. CellTiter Glo, propidium iodide and AlamarBlue are used to assess the effect of the nanoparticles on mitochondrial health, nuclear membrane integrity and the cell redox potential, respectively. These are commonly used assays that measure different indicators of cell health which need to be considered prior to selecting an assay.

The CellTiter Glo assay was used to measure the effect of polymeric nanoparticles on ATP production as an indicator of mitochondrial activity. The assay uses an ATP-dependent Ultra-Glo recombinant luciferase enzymatic conversion of Beetle Luciferin to produce oxyluciferin and light. The bioluminescence from this enzymatic interaction can be measured and correlated with ATP and cell viability. The advantage of using this analysis is that it is sensitive, is unaffected by serum or phenol red (common pH indicator in growth media), only involves one reagent addition, and only requires a minimum of 10 min of incubation at RT for a steady luminescent signal. This can limit the error as it reduces the number of steps and manual handling required. However, the CellTiter Glo reagent also contains a detergent and ATPase inhibitors to lyse the cells and release the ATP for detection and cannot be used monitor cell viability over time. Cells were treated with DEAEMA and DIPMA nanoparticles at increasing concentrations and DEGMA incorporated at 0-50%. Cells were incubated with the nanoparticles up to 24 h and viability was measured using the CellTiter Glo. Up to 8 h of DEAEMA and DIPMA nanoparticle incubations, toxicity was only observed at concentrations of 50 μg/mL (Figure 13). At 24 h, the 10 μg/mL and 50 μg/mL concentration of the nanoparticles reduced the cell viability. The introduction of 50% DEGMA increased the cell viability of the cells treated with 50 μg/mL of DEAEMA nanoparticles at all time points measured. In the DIPMA treated cells, the 0-20% DEGMA nanoparticles reduced the cell viability when compared to the 0% nanoparticles. Interestingly, only the 50% DEGMA-DIPMA nanoparticle improved the cell viability profile.

**Example 12: Assessment of nanoparticle effect on membrane integrity and permeability**

To determine if the polymeric nanoparticles disrupted membrane integrity, propidium iodide (PI) assays were performed. This cell impermeant dye counterstains the nucleus and chromosomes by intercalating nucleic bases and is used to detect cell death. At concentrations below the CMC, DEAEMA and DIPMA did not affect membrane integrity. Consistent with the ATP detection assay, 50μg/ mL of the DEAEMA nanoparticles incubated for 2-24 hours induced

-40% cell death in the PI detection assay (Figure 14). The toxicity of the DEAEMA nanoparticle was significantly reduced with the introduction of 50% DEGMA when incubated with cells for 2, 8 and 24 h. The DIPMA nanoparticles were less toxic and showed -15% death of the cell population at the highest concentration of particles after 24 h of incubation. This is low comparative to the ATP detection which showed -40% loss in cell viability with the DIPMA nanoparticle at the equivalent concentration and time. The differences in DEAEMA and DIPMA toxicity may be due to the pKa which can affect the fate of the particle translocation through the endosomal network. DEAEMA has a higher pKa than DIPMA with the reported pKa of 7.1 and 6.1, respectively. Therefore, DEAEMA is likely to disassemble earlier in the endosomal maturation process at a higher pH, whereas the DIPMA nanoparticle is likely to remain in the intact further in the endosomal network. The delayed dissociation may result in slower onset of polycationic-dependent cell death and may also determine the mechanism of cell death.

**Example 13: Assessment of nanoparticle effect on intracellular redox potential**

The membrane integrity and ATP production assays indicated that the DEAEMA and DIPMA nanoparticles did not lead to complete cytotoxicity using the concentration and time parameters tested. Therefore, to observe the full cytotoxic profile of the particles, the effect of higher concentrations (0.4-250 $\mu$g/mL) and prolonged exposure (12-72 h) of the nanoparticles on the intracellular redox potential was investigated using AlamarBlue reagent. AlamarBlue (resazurin) is a cell permeable redox indicator that is reduced to the fluorescent product, resorufin, in the cytoplasm of cells with active metabolisms. The fluorescence measured correlates to the number of viable cells in a population. Consistent with the membrane integrity and ATP production assays, DEAEMA nanoparticles induced cell death at 50 $\mu$g/mL over 72 h (Figure 15). 100% cytotoxicity was also observed at 250 $\mu$g/mL of the DEAEMA nanoparticle. The incorporation of 50% DEGMA completely recovered cell viability of 50 $\mu$g/mL up to and including 72 h and recovered 14.4 $\pm$ 5.5% cell viability at 250 $\mu$g/mL (12 h). The DIPMA nanoparticle induced cell death after 48 h of treatment with 50 $\mu$g/mL and 250 $\mu$g/mL and after 72 h of incubation with 10-250 $\mu$g/mL. The incorporation of 50% DEGMA improved the biocompatibility of the DIPMA particle in the 48 h treated cells. However, the shielding effect of the 50% DEGMA only partially improved cell viability when cells were incubated with concentrations of 50 $\mu$g/mL (22.5 $\pm$ 7.5%) and 250 $\mu$g/mL (19.6 $\pm$ 2.0%). At 72 h, when incubated with 10 $\mu$g/mL of the nanoparticles, 5% and 10% DEGMA prevented cytotoxicity, and 20% and 50% partially increased cell viability. After a 72 h incubation with higher concentrations of nanoparticles, DEGMA incorporation had a minimal effect on cell viability.

**Example 14: Clathrin- and dynamin-dependent cellular uptake and pH-dependent disassembly** of nanoparticles **in $NK_1R$-positive early endosomes**

The uptake and intracellular trafficking of DIPMA nanoparticles loaded with Cyanine 5 (DIPMA-Cy5) was examined by confocal microscopy. To examine trafficking to endosomes, DIPMA-Cy5 nanoparticles were incubated with HEK-293 cells expressing Rab5-GFP, which identifies early endosomes, or Rab7-GFP, which marks late endosomes. Uptake of DIPMA-Cy5 Nanoparticles was detected within 150 s, and by 300 s DIPMA-Cy5 nanoparticles were detected in RabS-GFP-positive early endosomes (Fig. 16A). After 30 and 60 min, DIPMA-Cy5 nanoparticles were extensively localized to Rab5-GFP early endosomes and Rab7-GFP late endosomes (Fig. 16B, Fig. 17A). To determine whether nanoparticles traffic to endosomes containing the NKiR, DIPMA-Cy5 nanoparticles were incubated with HEK-293 cells transfected with rNKiR-GFP. After 30 min, cells were challenged with 10 nM SP, to promote internalization of the $rNK_1R$-GFP. At 30 and 60 min after SP, rNKiR-GFP and DIPMA-Cy5 nanoparticles were co-localized in endosomes (Fig. 16C, Fig. 17B). Thus, DIPMA-Cy5 nanoparticles internalize and traffic to early endosomes containing rNKiR-GFP.

The uptake and disassembly of DIPMA nanoparticles loaded with Coumarin153 (DIPMA-CO), which emits in an aqueous environment but not in the hydrophobic core, were examined by high content imaging (confocal microscopy). When DIPMA-CO nanoparticles were incubated with HEK-293 cells, there was a marked increase in cellular fluorescence within 5 min that continued for 30 min (Fig. 16D, E). The clathrin inhibitor PitStop2 and the dynamin inhibitor Dyngo4a inhibited cellular fluorescence. Bafilomycin A1, which inhibits the vacuolar $H^+$ATPase that acidifies endosomes, and $NH_4Cl$, which alkalinizes endosomes, also suppressed fluorescence (Fig. 16F). These results are consistent with clathrin- and dynamin-dependent endocytosis of nanoparticles, and pH-dependent disassembly in acidified early endosomes.

**Example 15: Effects of** nanoparticle **encapsulated AP on nociception**

Whereas AP suppresses acute chemotherapy-induced nausea and vomiting, NKiR antagonists have been ineffective at reversing chronic disorders, including depression and pain. Since the NKiR redistributes to endosomes after continuous stimulation with SP, the failure of antagonists may be due to their inability to access and engage the NKiR in acidic endosomes. To examine the hypothesis that incorporation into nanoparticles amplifies the anti-nociceptive actions of AP due to delivery to NKiR-positive endosomes in spinal neurons, AP formulations were administered by

intrathecal injection to mice or rats, including free AP, or AP incorporated into pH-responsive (DIPMA-AP) and non-responsive (BMA-AP) nanoparticles (Fig. 18A). Empty nanoparticles or vehicle were used as controls. Acute and chronic inflammatory and neuropathic nociception were examined.

**Capsaicin-evoked mechanical allodynia.**

AP, nanoparticles or vehicle (5 μl) was administered by intrathecal injection to mice 30 min before intraplantar injection to the hind paw of capsaicin, which activates TRPV1 to cause acute allodynia and neurogenic inflammation. Withdrawal responses to stimulation of the plantar surface of the injected hindpaw with calibrated von Frey filaments (VFF) were measured before and after capsaicin. In mice receiving vehicle or DIPMA-empty nanoparticles, capsaicin decreased the VFF threshold from 0.5 - 4 h, consistent with mechanical allodynia, which returned to baseline after 24 h (Fig. 18B). Free AP (5 μl, 100 nM) and DIPMA-empty nanoparticles mixed with free AP (100 nM) caused a modest anti-allodynia after 1 h (16 ± 4% and 15 ± 3% inhibition, respectively), which was not detected after 1.5 h. BMA-AP nanoparticles (100 nM AP) had a similar effect after 0.5-1 h, although the effect was more sustained than free AP, extending for 2 h. DIPMA-AP nanoparticles that delivered the same dose of AP (5 μl, 100 nM) caused marked anti-allodynia at 0.5-1 h (1 h, 34 ± 3% inhibition) that was sustained for 4 h (35 ± 2% inhibition), when other treatments were ineffective. Analysis of the integrated response (0-4 h area under curve, AUC) confirmed that DIPMA-AP nanoparticles provided the most effective inhibition of capsaicin-evoked mechanical allodynia (Fig. 18C).

**Complete Freund's adjuvant-evoked mechanical hyperalgesia.**

Intraplantar injection of complete Freund's adjuvant (CFA) causes sustained mechanical hyperalgesia, which allowed examination of the capacity of nanoparticle-encapsulated AP administered in a therapeutic manner to reverse inflammatory pain (Fig. 18A). By 48 h after intraplantar CFA, there was a marked decrease in VFF threshold, consistent with mechanical hyperalgesia (Fig. 18D). Intrathecal injection of vehicle 48 h after CFA did not affect mechanical hyperalgesia, which persisted for 24 h. AP (5 μl, 100 or 300 nM) dose-dependently reversed hyperalgesia for 2-3 h (1.5 h, % inhibition: 100 nM, 30 ± 6; 300 nM, 47 ± 3%). BMA-AP nanoparticles (100 nM AP) were as effective as free AP (300 nM). DIPMA-AP nanoparticles (100 nM AP) produced a larger inhibition of allodynia than the same dose of free AP (1.5 h, % inhibition: 54 ± 4%), and the inhibition was maintained for 6 h, when other AP treatments were ineffective. Although systemic morphine (3 mg/kg, i.p.) fully reversed the mechanical hyperalgesia after 0.5 h, the effect quickly waned to baseline after 3 h. Analysis of integrated response (0-8 h AUC, half width response) indicated that DIPMA-AP nanoparticles produced the most sustained reversal of hyperalgesia (Fig. 18E, F).

**Nerve injury-evoked mechanical hyperalgesia.**

The sural nerve spared (SNS) model produces a stable and robust mechanical hyperalgesia in rats that can last for more than 50 days, which permitted examination of the efficacy of nanoparticle-encapsulated AP to relieve chronic neuropathic pain in another species. At 10 days after surgery, SNS reduced in the pressure that induced withdrawal of the hind paw (Randal-Siletto test) when compared to sham-operated rats, indicative of mechanical hyperalgesia (Fig. 18A, G). In rats receiving intrathecal injection of vehicle, mechanical hyperalgesia was maintained for 7 h (Fig. 18G). Although AP (10 μl, 100 nM) did not modify withdrawal threshold, AP (300 nM) inhibited withdrawal thresholds after 0.5 h to a maximum of 40 ± 2% inhibition after 1 h, and return to baseline after 2.5 h. AP (1 μM) almost fully reversed hyperalgesia after 1 h (75 ± 4% inhibition), although hyperalgesia returned to baseline after 3 h (Fig. 19). BMA-AP (10 μl, 100, 300 nM AP) inhibited hyperalgesia to a similar degree as free AP (300 nM). DIPMA-AP (100, 300 nM AP) strongly reversed hyperalgesia, with almost complete inhibition after 1.5 h (300 nM, 80 ± 4% inhibition) and maintenance for 4.5 h, when none of the other treatments were effective. DIPMA-AP (500 nM) provided complete relief from hyperalgesia for 4.5 h (Fig. 19). Although morphine fully reversed hyperalgesia for 2 h, the effect was short lived compared to DIPMA-AP and was absent after 2.5 h. Analysis of integrated response (0-7 h AUC, half width response) indicated that DIPMA-AP nanoparticles produced the most sustained reversal of hyperalgesia (Fig. 18H, I).

Thus, encapsulation into pH-responsive DIPMA nanoparticles enhances the anti-nociceptive actions of AP in models of acute (capsaicin), inflammatory (CFA) and neuropathic (SNS) pain in two species, increasing the magnitude and duration of the response. Since AP encapsulated into non-pH-responsive nanoparticles was no more effective than free AP, the enhanced efficacy of DIPMA-AP depends on pH-responsive delivery and not nanoparticle encapsulation *per se.*

**Example 16: Effects of nanoparticle encapsulated AP on sensitization and activation of primary sensory and spinal neurons**

SP, CGRP and glutamate, released from the central projections of primary sensory neurons (C-fiber nociceptors) in the dorsal horn of the spinal cord, activate receptors on second order spinal neurons to mediate nociceptive transmission. The sensitization of primary sensory neurons and second order spinal neurons is a hallmark of chronic pain. To examine sensitization, we measured the threshold current required to activate C-fiber reflexes, and assessed wind-up (frequency-dependent increase in the excitability of spinal cord neurons induced by electrical stimulation of C-fibers). The threshold current required for activation of the C-fiber-mediated reflex responses in the ipsilateral biceps femoris muscle was reduced in SNS rats compared to sham controls (SNS, 3.2 ± 2.8 mA; sham, 10.3 ± 1.2 mA, $P<0.05$). Application of electrical stimulus (0.1 Hz) lead to a constant and stable C-reflex activity over time as well as evoked wind-up activity (Fig. 20A-F). Administration of AP (10 μl, 1 μM intrathecal) to SNS rats decreased the C-reflex, but only at 30 min, but did not affect wind-up. DIPMA-AP nanoparticles (300 nM AP) decreased the C-reflex within 45 min and the wind-up activity within 15 min, and inhibited responses for the duration of observations (120 min).

The effectiveness of intrathecal injection of DIPMA-AP to suppress nociception may be due to antagonism of sustained SP-induced excitation of spinal neurons, which requires NKiR signaling from endosomes. To examine this possibility, we made cell-attached patch-clamp recordings of neurons in laminae I and II in slices of rat spinal cord. In vehicle-treated slices, SP (1 μM, 2 min) caused a rapid onset in action potential firing that was sustained for 16 min after washout (Fig. 20G-I). Preincubation with AP (100 nM) or BMA-AP (100 nM AP) did not affect the onset, rate or duration of SP-induced firing. DIPMA-AP (100 nM) did not affect the initial onset of SP-evoked firing, but inhibited the rate of discharge after washout and the duration of excitation. These results support the hypothesis that AP, when delivered in pH-responsive nanoparticles, antagonizes the NKiR in acidified endosomes and inhibits the signals that drive sustained excitation of spinal neurons and persistent pan transmission.

**Example 17: Antagonism of Endosomal $NK_1R$**

The use of genetically-encoded FRET biosensors enables evaluation of signaling pathways in living cells with high spatiotemporal fidelity. By using FRET biosensors to assess compartmentalized signaling, and genetic and pharmacological approaches to inhibit clathrin- and dynamin-mediated NKiR endocytosis, we have previously shown that the NKiR in endosomes activates extracellular signal-regulated kinase (ERK) in the nucleus and protein kinase C and cAMP in the cytosol. Nuclear ERK regulates transcription and mediates SP-induced excitation of spinal neurons. We therefore examined the capacity of nanoparticle encapsulated AP to antagonize SP-induced activation of nuclear ERK.

To examine activation of nuclear ERK, we expressed in HEK-hNKiR cells NucEKAR, an ERK biosensor that is targeted to the nucleus. SP (100 pM - 1 μM) stimulated a rapid (2 min), sustained (>35 min) and concentration-dependent activation of nuclear ERK ($EC_{50}$ 5.9 nM) (Fig. 22A, B). We examined the capacity of free AP to antagonize the nuclear ERK response to 5 nM SP (~$EC_{50}$). AP inhibited SP-evoked activation of nuclear ERK, but only the highest AP concentrations (0.1, 1, 10 μM; $IC_{50}$ 45 nM) (Fig. 22C, D). To determine the whether NKiR endocytosis and endosomal signaling is necessary for SP-induced activation of nuclear ERK, we transfected HEK-hNKiR with wild-type (WT) dynamin or dominant negative dynamin K44E, which inhibits NKiR endocytosis. In cells expressing WT dynamin, SP stimulated a rapid and sustained activation of nuclear ERK ($EC_{50}$ 11.1 nM) (Fig. 21A, C). Dynamin K44E attenuated responses to all concentrations of SP, inhibited the response to 10 nM SP at all times, reduced the potency of SP by ~ 2-fol ($EC_{50}$ 19.8 nM), and reduced the efficacy by -30% (Fig. 21B, C). Thus, NKiR endocytosis is necessary for persistent SP-induced activation of nuclear ERK.

To determine whether unloaded nanoparticles affect nuclear ERK activity, we incubated HEK293 cells for 30 min with DIPMA-empty and BMA-empty nanoparticles (10, 20, 30 μg/mL). DIPMA-empty and BMA-empty nanoparticles did not activate nuclear ERK at any concentration studied (Fig. 21D-E). Viability of cells was examined using alamar Blue, which assess the capability of viable cells to reduce the active compound rezasurin to resofurin. Exposure of HEK293 cells to DIPMA-empty and BMA-empty nanoparticles (1-100 μg/mL) for 24 h or 48 h did not affect cell viability (Fig. 21F). Thus, nanoparticles do not activate ERK or have cytotoxic actions in HEK293 cells.

To compare the capacity of free AP and nanoparticle-encapsulated AP to antagonize the $NK_1R$ in endosomes, we measured SP-induced activation of nuclear ERK in HEK-hNKiR cells. Cells were preincubated with vehicle, free AP (100 nM), BMA-AP (100 nM AP), DIPMA-AP (100 nM AP) for 30 min, and were then challenged with SP (5 nM). In vehicle-treated cells, SP rapidly activated nuclear ERK, which remained active for ~ 30 min (Fig. 21G, I). Free AP partially inhibited the response, whereas BMA-AP and DIPMA-AP abolished the response. To compare the capacity of free AP and nanoparticle-encapsulated AP to induce a sustained antagonism of the $NK_1R$ in endosomes, cells were incubated with vehicle, AP, BMA-AP or DIPMA-AP for 30 min, washed, recovered in medium without antagonist for 30 min, and then challenged with SP. Free AP was now inactive, whereas BMA-AP and DIPMA-AP abolished SP-induced activation of nuclear ERK (Fig. 21H, I). Thus, encapsulation into nanoparticles markedly enhances the duration of action of AP,

even after washout from cells.

**Example 18: Intrathecal Administration of DIPMA-(MK-3207) Nanoparticles**

The analgesic potential of acid-responsive DIPMA-MK-3207 nanoparticles was assessed in a CFA model. Mechanical allodynia was assessed by Von Frey hairs.

30 nM, 100 nM, 300 nM, and 1μM of MK-3207 (n = 4 per concentration) were dosed intrathecally (mouse). Paw withdrawal threshold (PWT/g) was measured over time. The results were summarized graphically in Fig. 23(A).

Acid-responsive DIPMA nanoparticle compositions with 30 nM, 50 nM, and 100 nM of MK-3207 (DIMPA-(MK-3207) nanoparticles) were dosed intrathecally (mouse) (compared to the administration of 100 nM MK-3207). Paw withdrawal threshold (PWT/g) were measured over time. The results are summarized graphically in Fig. 23(B).

**Example 19: Preparation of Dual-Loaded DIMPA Nanoparticles -- DIPMA-(Ap+MK-3207) Nanoparticles**

Acid-responsive DIPMA nanoparticles dual-loaded with aprepitant (Ap) and MK-3207 were prepared using methods similar to those described elsewhere herein (e.g., **Example 2**) with P(PEGMA-co-DMAEMA)-b-P(DIPMA-co-DEGMA) diblock polymers. Aprepitant and MK-3207 in loading ratios of 1:0.5, 1:1, 1:2, and 1:4 ratios led to dual-loaded nanoparticle compositions shown in the table below. Concentrations of loaded aprepitant and MK-3207 in the nanoparticle compositions were assessed with LC-MS and summarized in the table below (see second and third column) (also see Fig. 24).

| | Concentration in 1 mg/ml polymer solution (Mean, n=3-5) | | i.t. Dose (administering 5μL) | |
|---|---|---|---|---|
| Ap: MK-3207 loading ratio | Aprep (μM) | MK-3207 (μM) | Aprep (nM) | MK-3207 (nM) |
| 1:0.5 | 5.58 | 0.85 | 82 | 12 |
| 1:1 | 5.22 | 1.41 | 76 | 13 |
| 1:2 | 5.5 | 2.2 | 81 | 25 |
| 1:4 | 5.91 | 5.58 | 80 | 81 |
| Note: Mean -/+ SD, n=4 | | | | |

**Example 20: Intrathecal Administration of Dual-Loaded DIPMA-(Ap+MK-3207) Nanoparticles**

The analgesic potential of acid-responsive DIPMA nanoparticles dual-loaded with aprepitant and MK-3207 was assessed in a CFA model. Mechanical allodynia was assessed by Von Frey hairs.

Acid-responsive DIPMA nanoparticle compositions loaded with 82 nM of aprepitant + 12 nM of MK-3207, 81 nM of aprepitant + 25 nM of MK-3207, and 80 nM of aprepitant + 81 nM of MK-3207 were dosed intrathecally (mouse). The paw withdrawl threshold (PWT/g) was measured over time. This was compared with intrathecal dosing of 120 nM of aprepitant + 120 nM of MK-3207 (mouse). The results are summarized graphically in Fig. 25.

Administration of dual-loaded DIPMA-(Ap+MK-3207) nanoparticles resulted in enhanced efficacy and duration of action compared to administration of the free Ap and MK-3207.

**Example 21: Intrathecal Co-Administration of DIPMA-(MK-3207) Nanonparticles and DIPMA-Ap Nanoparticles**

Administration of a cholestanol conjugate of $CGRP_{8-37}$ (a membrane-impermeant CLR antagonist) (i.e., $CGRP_{8-37}$-Chol) but not unconjugated $CGRP_{8-37}$ reversed mechanical hyperalgesia (by -20%). Co-administration of spantide (an NKi inhibitor) cholestanol conjugate (i.e., Span-Chol) and $CGRP_{8-37}$-Chol caused a marked (-75%) reversal of CFA-induced mechanical hyperalgesia, whereas the combination of unconjugated Span and $CGRP_{8-37}$ had no effect (Proc. Natl. Acad. Sci. USA. 114(46):12309-12314. See e.g., Figures 7E and 7F therein).

The analgesic potential of co-administering DIPMA-(MK-3207) nanoparticles with DIPMA-$A_P$ nanoparticles was assessed in a CFA model. Mechanical allodynia was assessed by Von Frey hairs.

DIPMA-$A_P$ nanoparticles (30 nM in Ap) + DIPMA-(MK-3207) nanoparticles (30 nM in MK-3207), DIPMA-$A_P$ nanoparticles (60 nM in Ap) + DIPMA-(MK-3207) nanoparticles (60 nM in MK-3207), and DIPMA-$A_P$ nanoparticles (120 nM in Ap) + DIPMA-(MK-3207) nanoparticles (120 nM in MK-3207) were dosed intrathecally (mouse). The paw withdrawl threshold (PWT/g) was measured over time. This was compared with intrathecal dosing of 120 nM of aprepitant + 120

nM of MK-3207 (mouse). The results are summarized graphically in Fig. 26.

Co-dosing DIPMA-(MK-3207) nanonparticles and DIPMA-$A_P$ nanoparticles is efficacious in CFA pain model: improved analgesic efficacy and sustained effect are obsevrved when nanoparticles are co-dosed each at 120 nM compared to the administration of free AP and MK-3207 at the same concentration.

## Materials and Methods

**Transmission electron microscopy (TEM).** The morphology of nanoparticles was determined by TEM imaging. TEM imaging was performed using a Tecnai F20 transmission electron microscope at an accelerating voltage of 200 kV at ambient temperature. An aliquot (5 μL) of 0.1 wt% latex solution (diluted with MiliQ water) was deposited on a Formvar coated copper grid (GSCu100F-50, Proscitech) and was allowed to dry overnight in air and at room temperature.

**Cell line.** The human NKiR (h NKiR) ORF with a CD8 signal sequence and N-terminal FLAG-tag was cloned into pcDNA5 FRT/TO between KpnI and NotI restriction sites using Gibson Assembly (NEB). A stable cell line expressing hNKiR (HEK-hNKiR) was produced by co-transfecting Flpn HEK-293 cells with 0.5 μg of the hNKiR vector and 4 μg of pOG44, using polyethylenimine (PEI, Polysciences, USA) at a 1:6 DNA:PEI ratio. ~$0.7\times10^6$ cells were seeded into a T-25 tissue culture flask (Perkin Elmer, MA, USA) in Dulbecco's modified Eagle medium supplemented with penicillin (50U/mL) and streptomycin (50U/mL) (DMEM/pen/strep) and incubated for 24 h (37°C, 5% $CO_2$). The media was changed to fresh DMEM/pen/strep prior the transfection and the flask was then incubated for 24 h (37°C, 5% $CO_2$) before the media was changed to DMEM supplemented with 10% (v/v) fetal bovine serum (FBS) and hygromycin B (200 μg/ml, Invitrogen) (DMEM/FBS/Hygro).

**Cell culture.** HEK-hNKiR cells were cultured in DMEM supplemented with FBS and hygromycin B (5% CO2, 37°C). Cells were plated in poly-L-lysine coated black 96 well CulturPlate (Perkin Elmer, MA, USA) for foster resonance energy transfer (FRET) assays and in 96-well clear plastic plates (Corning, NY, USA) for Intracellular calcium measurements.

**Nanoparticle trafficking in cell lines.** HEK-293 cells were plated on poly-D-Lysine coated ibidi chambers (Germany) in DMEM supplemented with 10% (v/v) FBS (DMEM/FBS). After 24 h, cells were transfected with 300 ng of rat (r) $NK_1R$-GFP/chamber using PEI at a 1:6 ratio and cultured for further 48 h. To identify endosomal compartments, HEK-293 cells were infected with Rab5a-GFP (resident in early endosomes) or Rab7a-GFP (late endosomes) (CellLight, Thermo Fisher Scientific, USA) 16 h before imaging. To examine localization of nanoparticles, cells were incubated in Leibovitz's L-15 medium with DIPMA-Cy5 nanoparticles (20 μg/mL, 30 min, 37°C) or vehicle, followed by addition of SP (10 nM).

Cells were imaged at 30 and 60 min post-SP addition using a Leica SP8 confocal microscope equipped with HCX PL APO 40x (NA 1.30) and HCX PL APO 63x (NA 1.40) oil objectives. Images were analyzed using Fiji and deconvolved with Huygens Professional version 18.04 (Scientific Volume Imaging, The Netherlands), using the CMLE algorithm with signal to noise ratio 10 and 100 iterations.

**Determination of compartmentalized signaling using FRET biosensors.** HEK-hNKiR cells (~$2\times10^6$) were seeded into 90 mm Petri dish (Corning™, USA) in DMEM/FBS/Hygro and incubated for 24 h (37°C, 5% $CO_2$). Prior to the transfection, the medium was changed to fresh DMEM/FBS/Hygro and nuclear ERK (nucEKAR) sensor was transfected (2.5 μg nucEKAR DNA/dish) using PEI at a 1:6 ratio. After 24 h, cells were plated in poly-L-lysine coated black 96 well CulturPlate (Perkin Elmer, USA) and incubated for further 24 h (37°C, 5% $CO_2$). On the day of the assay, cells were serum-starved for 6-8 h, and were then equilibrated in HBSS, supplemented with 12 mM 4-(2-hydroxyethyl)piperazine-1-ethanesulfonic acid (HEPES) at 37°C in $CO_2$-free incubator. FRET was assessed using a PHERAstar FS (BMG LABTECH, Germany) with an optic module FI 430 530 480 and measurements were made every 1 min. Baseline was measured for 5 min followed by stimulation with SP, vehicle (HBSS) or the positive control, phorbol 12,13-dibutyrate (1 μM, PDBu), and further measurements for 30 min. For the SP concentration response curve, half logarithm dilutions of SP were added (1 μM to 100 pM) and the half-maximal effective concentration ($EC_{50}$) was determined using the area under the curve (AUC) after SP addition (30 min reading). For the AP concentration response curve, logarithmic dilutions of AP (10 μM to 1 pM) were added 30 min prior baseline measurements, followed by the addition of 5 nM of SP. The half maximal inhibitory concentration ($IC_{50}$) was determined for AP as described. To assess the effect of nanoparticles on nuclear ERK signaling, DIPMA-empty, DIPMA-AP or BMA-AP (30, 20, 10 μg/mL) were added 30 min prior baseline measurements, followed by the addition of SP 5 nM or vehicle. Data were expressed as vehicle corrected values, normalized by the maximum response to the positive control.

**Intracellular calcium ($iCa^{+2}$) measurements.** Cells were incubated in HEPES-buffered saline (HBS; 150mM NaCl, 2.6mM KCl, 1.2mM MgCl2, 2.2mM CaCl2, 10mM D-glucose, 0.5% BSA, 10mM HEPES, 4mM Probenecid; pH 7.4) supplemented with Fura2-Am ester dye 1 μM (Thermo Fisher Scientific, MA, USA) and Pluronic F-127 (0.02%) for 45 minutes at 37 °C in dark in $CO_2$-free incubator. Cells were washed 2 times with HBS and rested for 10 min at 37 °C. To observe changes in $iCa^{2+}$, Fura-2 fluorescence was measured using a FlexStation 3 (Molecular Devices, CA, USA)

every 1 min for 30 min. Baseline was measured during 5 min, followed by the addition of SP (5 nM), vehicle (HBS), the positive control Ionomycin (1 μM) or nanoparticles (10 μg, DIPMA-empty, DIPMA-$A_P$ and BMA-Ap) and further measurements for 25 min.

**Uptake and disassembly of** nanoparticles **in HEK-293 cells.** Coumarin 153 is a solvatochromic dye that fluoresces only in polar solvents, allowing detection of the release of Coumarin loaded in the core of nanoparticles observed as the appearance of Coumarin fluorescence in intact cells. nanoparticles were self-assembled using 0.5 mg of Coumarin 153 per mg of DIPMA polymer (DIPMA-CO). HEK-293 cells were pre-incubated for 30 min with vehicle (Hank's Balanced Salt Solution, HBSS), dynamin inhibitor (Dyngo4a, Dy4, 30 μM), clathrin inhibitor (Pitstop, PS2, 30 μM), vacuolar $H^+$ATPase inhibitor (Bafilomycin A1, BFA, 1 μM) or $NH_4Cl$ (20 mM) to alkalinize endosomes. Images were obtained with a Leica SP8 confocal microscope using HCX PL APO 63x (NA 2.0) oil objective. Images were taken every 10 sec for 30 min, where the first 5 readings correspond to baseline images prior the addition of DIPMA-CO nanoparticles (20 μg/mL). All images were analyzed using Fiji.

**Animals.** Adult male C57BL/6 mice (6-10 weeks) were purchased from the Monash Animal Research Platform and male Sprague-Dawley rats (225-250 g) were obtained from the facilities of the Faculty of Medicine of the University of Chile. All animals were housed in groups of 4, maintained in a temperature (22 ± 4°C) and humidity-controlled environment with a 12 h light/dark cycle. Food and water were available *ad libitum.* All behavioural testing on animals was performed in a blinded manner by an experimenter blinded to the treatment groups. Experiments were performed during the light cycle and animals in each cage were randomly allocated to different treatment groups. Animals were euthanized by anesthetic overdose and thoracotomy. The studies were conducted in accordance with the Guide for the Care and Use of Laboratory Animals of the National Institutes of Health (NIH) and adhered to the ethical guidelines of the IASP. Housing conditions and experimental procedures were approved by the animal ethics committee of Monash Institute of Pharmaceutical Sciences, Monash University and the Bioethics Committee of the University of Santiago of Chile.

**Drug administration.** *Mice.* The following drugs were administered by intrathecal (i.t.) injection (5 μL) into the intervertebral space (L4, L5) of conscious mice: AP (100, 300 nM), nanoparticles delivering an equivalent dose of AP (DIPMA-AP, BMA-AP, 10 μg/mL-100 nM, 30 μg/mL-300 nM), controls (10 μg/mL of DIPMA-empty and a mixture of 10 μg/mL of DIPMA-empty and AP 100 nM), or vehicle (artificial cerebrospinal fluid, aCSF). Treatments were administered 30 min prior to rotarod experiments and the induction of acute pain or 48 h after the establishment of inflammatory pain. For biodistribution studies, DIPMA-Cy5 nanoparticles (10 μg/mL) were administered i.t immediately after obtaining control images. *Rats.* Drugs were administered by i.t. injection (10 μL) into the intervertebral space (L4, L5) of conscious rats: AP (100, 300 nM, 1μM), nanoparticles loaded with AP (DIPMA-AP, BMA-AP, 10 μg/mL-100 nM, 30 μg/mL-300 nM, 50 μg/mL -500 nM), DIPMA-empty nanoparticles (10, 30, 50 μg/mL), or vehicle (aCSF). Treatments were administered 10 days after sural nerve transection. For electrophysiological studies, drugs were administered by i.t. injection under anesthesia (isoflurane 1.2-1.5%): AP (1 μM) or nanoparticles (DIPMA-AP, BMA-AP, 30 μg/mL-300 nM).

**Rotarod test.** Prior to experiments, mice were acclimatized and trained on the Rotarod apparatus for three consecutive runs on two successive days to assess motor performance. On the day of experiment, three baseline readings were recorded and a cut-off threshold of 120 second was pre-set. DIPMA-Ap, BMA-Ap, DIPMA-empty or vehicle (aCSF) was injected intrathecally (5 μL). Subsequently, the latency of mice to fall (seconds) were recorded at 30 min, 60 min, 90 min, 120 min, 180 min and 240 min post-injection.

**Biodistribution of NPs.** Mice were sedated (2% isoflurane) and placed in an *in vivo* imaging system (IVIS spectrum, Perkin Elmer, USA). Images were obtained prior to i.t administration of DIPMA-Cy5 NPs (10 μg/mL). Posterior images were collected from 2 groups of mice, the first group was imaged at 2 and 6 h and the second group at 4 and 8 h post DIPMA-Cy5 administration.

**Induction and assessment of acute and inflammatory pain in mice**

*Acute pain.* Capsaicin (CAP, 5 μg) or vehicle (0.9% NaCl) was administered by subcutaneous intraplantar (i.pl.) injection (10 μL) into the left hindpaw of sedated mice (2% isoflurane) 30 min after i.t. injection of drugs.

*Inflammatory pain.* Complete Freund's Adjuvant (CFA, 0.5 mg/mL) or vehicle (0.9% NaCl) was administered by i.pl. injection (10 μL) into the left hindpaw of sedated mice (2% isoflurane). Drugs were administered by i.t. injection 48 h after CFA.

*Mechanical allodynia and hyperalgesia.* Mechanical nociception was assessed by measuring withdrawal thresholds to stimulation of the plantar surfaces of the ipsilateral and contralateral hindpaws with calibrated von Frey filaments (VFF). Prior to experiments, mice were acclimatized to the experimental apparatus and environment for 2 h on 2 successive days. VFF withdrawal thresholds were measured in triplicate to establish a baseline for each mouse. For the CAP model, VFF withdrawal thresholds were measured at 30 min intervals for the first 2 h after i.t. drug administration, then at 60 min intervals for the next 2 h, and finally after 24 h. For the CFA model, VFF withdrawal thresholds were measured every 30 min for the first 3 h after i.t. drug administration, then at 60 min intervals for the next 5 h, and finally

after 24 h. Results were normalized to the baseline withdrawal thresholds of each mouse. Results are expressed as a percentage of baseline, as area under curve (AUC), and as the half width response (the duration of the effect of each treatment calculated as the time to attain 50% of the maximal analgesic response).

**Induction and assessment of neuropathic pain in rats**

*Neuropathic pain.* Neuropathic pain was induced in rats using a variation of the sural nerve spared (SNS) injury model in rats, which induces rapid onset and sustained mechanical and thermal hyperalgesia. Under anesthesia (2% isoflurane), the 3 terminal distal branches of the sciatic nerve (tibial, common peroneal, sural nerves) were identified and the sural nerve was transected. For controls (sham), rats underwent a similar surgery but without transection of the sural nerve. After surgery, ketoprofen (3 mg/kg) and enrofloxacin (5 mg/kg) were administered subcutaneously (s.c.) for 2 days.

*Mechanical hyperalgesia.* Mechanical hyperalgesia was assessed in rats by measuring hindpaw withdrawal pressure thresholds using an algesimeter (Ugo Basile, Italy) with a cut-off value of 570 g to prevent injury. Mechanical hyperalgesia was evaluated before (basal) and 5, 9 and 10 days after the surgery. After the evaluation at day 10, drugs were administered by i.t. injection, and withdrawal thresholds were recorded every 30 min for 7 h. Results are expressed as the paw withdrawal pressure threshold ($g.cm^2$), AUC and half-width response.

**Electrophysiological assessment of the C-fiber-evoked nociceptive reflex and wind-up activity in rats.** Nociceptive synaptic transmission was evaluated by measurement of electromyographic (EMG) activity associated with the hind limb-flexion nociceptive reflex evoked by electrical activation of C fibers of the sural nerve (C-reflex). Rats were maintained under anesthesia (1.2-1.5% isoflurane in oxygen using a diaphragm rodent facemask) and placed on a regulated thermal pad (37 ± 0.5°C). EMG activity was measured using a pair of platinum stimulation electrodes inserted subcutaneously into the lateral part of the third and fourth toes, and recording electrodes inserted through the skin into the ipsilateral biceps femoris muscle. The C-reflex corresponds to the integration of the reflex response into a 150-450 ms time window post-stimulus. Windup is a potentiation of the C-reflex response when the stimulating frequency is increased to 1 Hz. The windup corresponds to the slope of the regression line fitted to the integrated response of the first seven consecutive windows recorded at 1 Hz stimulation. After recording to obtain a stable C-reflex response (~30 min), the threshold for C-reflex was estimated and the rats remained stimulated at 2X the threshold intensity for the duration of the experiment. The C-reflex was evaluated by the mean of 15 consecutive stimuli at 0.1 Hz while the next 7 stimuli at 1 Hz were used to evaluate windup. Recordings were made 10 days after surgery before (basal) and 30, 60, 90 and 120 min after i.t. drug administration. The integrated C-reflex responses were expressed as a percentage of basal response.

**Cell-attached patch clamp recordings of rat spinal neurons.** Parasagittal slices (300-400 μm) were prepared from rat lumbar spinal cord as described. Slices were transferred to a recording chamber and superfused with aCSF (2 $ml.min^{-1}$, 36°C). Dodt-contrast optics were used to identify large (capacitance ≥ 20 pF), putative NKiR-positive neurons in lamina I based on their position, size and fusiform shape with dendrites that were restricted to lamina I. Spontaneous currents were recorded from NKiR-positive lamina I neurons in cell-attached configuration using patch electrodes. Slices were preincubated in DIPMA-AP (10 μg/ml-100 nM AP), BMA-AP (10 μg/ml-100 nM AP) or AP (100 nM) for 120 min, washed and incubated in antagonist-free aCSF for a further 30-60 min before recording. Slices were challenged with SP (1 μM, 2 min) and firing rate for each cell was normalized to the response between the 2-4 min time points, which was not significantly different between groups. The firing time was determined as the duration of the response to last action potential.

**Cell Viability** assays. HEK-h $NK_1R$ cells were incubated with DIPMA-empty and BMA-empty nanoparticles for 24 and 48 h at a concentration ranging from 1 to 100 μg/mL. Media was replaced by phenol red-free DMEM, followed by the addition of 10% (v/v) alamarBlue reagent (Thermofisher Scientific, USA). Cells were incubated with alamarBlue for 2 h (37°C, 5% $CO_2$) and fluorescence of the reduced active compound, resofurin was measured (510/610nm exc/em) using a ClarioStar (BMG LABTECH).

**Cell Titer Glo reagent** was prepared according to the instructions supplied by Promega. After treatment, the plate was incubated at room temperature for 30 min. 50 μL of cell media was first removed from each well and discarded. The remaining contents of each well were treated with 50 μL of the CellTiter Glo reagent. Cells were incubated at room temperature for a minimum of 10 min and the bioluminescence was measured at $\lambda$ = 555 ± 40 nm, using the CLARIOstar (BMG labtech). Cell viability was then calculated by subtracting the TritonX-100 control and expressing the values as a percentage of the vehicle control. Data are presented as mean ± S.E.M of 5 individual experiments.

The **propidium iodide** cell toxicity assay was performed using a final well concentration of 500 nM. Cells were incubated with the dye for 30 min at room temperature. The fluorescence was detected using the CLARIOstar (ex: $\lambda$ = 535 ± 10 nm and em: $\lambda$ = 617 ± 10 nm). Cell toxicity values were calculated by a vehicle baseline subtraction and expressed as a percentage of the TritonX-100 control for cell death. Data are presented as mean ± S.E.M of 5 individual experiments.

In the **AlamarBlue** cell viability assay, cell media was removed and replaced with 100 μL of fresh media. Cells were treated with a range of particle concentrations (0.4- 250 μg/mL) for 12, 24, 48 and 72 h at 37 °C, 5% $CO_2$. Cells were washed twice with chilled HEPES buffered saline solution (HBSS) (Sigma Aldrich) and then 10% (v/v) AlamarBlue reagent (Life Technologies) in media was added. Cells were incubated for 4 h further and the fluorescence was measured using the CLARIOstar (ex: λ = 570 ± 10 nm, em: λ = 605 ± 15 nm). Cell viability was then calculated by subtracting the Triton X-100 control and expressing the values as a percentage of the vehicle control. Data are then presented as the mean ± S.E.M of 3 individual experiments.

**Statistical analyses.** Data are presented as mean ± SEM, unless noted otherwise. Differences were assessed using Student's t test for two comparisons. For multiple comparisons, differences were assessed using one- or two-way ANOVA followed by Dunnett's multiple comparison test (mechanical hyperalgesia and edema) and Tukey's multiple comparison test (FRET and $iCa^{2+}$). A P<0.05 was considered significant.

## Claims

1. An aqueous liquid comprising polymeric nanoparticles of copolymer chains assembled to form a core / shell structure, the copolymer chains having:

   (i) an acid-responsive hydrophobic polymer block that forms the core of the nanoparticles, wherein the acid-responsive hydrophobic polymer block comprises tertiary amine functional groups that are protonated in an acidic environment; and
   (ii) a hydrophilic polymer block that forms the shell of the nanoparticles and is solvated by the aqueous liquid,

   wherein the nanoparticles contain within their core a hydrophobic modulator of endosomal GPCR signaling, or a pharmaceutically acceptable salt thereof.

2. The aqueous liquid according to claim 1, wherein the tertiary amine functional groups are protonated at about pH = 6.1.

3. The aqueous liquid according to claim 1 or 2, wherein the acid-responsive hydrophobic polymer block comprises a homopolymer or copolymer of 2-(diisopropylamino)ethyl methacrylate (DiPAEMA).

4. The aqueous liquid according to any one of claims 1 to 3, wherein the acid-responsive hydrophobic polymer block comprises a copolymer of 2-(diisopropylamino)ethyl methacrylate (DiPAEMA) and a polyalkyleneoxide methacrylate.

5. The aqueous liquid according to any one of claims 1 to 4, wherein the hydrophilic polymer block comprises a copolymer of poly(ethylene glycol)methacrylate (PEGMA) and 2-(dimethylamino)ethyl methacrylate (DMAEMA).

6. The aqueous liquid according to any one of claims 1 to 5, wherein the hydrophobic modulator of endosomal GPCR signaling or pharmaceutically acceptable salt thereof is an inhibitor of an endosomal GPCR.

7. The aqueous liquid according to any one of claims 1 to 6, wherein the hydrophobic modulator of endosomal GPCR signaling or pharmaceutically acceptable salt thereof is an inhibitor of endosomal $NK_1R$ signaling, or pharmaceutically acceptable salt thereof.

8. The aqueous liquid according to any one of claims 1 to 7, wherein the hydrophobic modulator of endosomal GPCR signaling or pharmaceutically acceptable salt thereof is selected from the group consisting of aprepitant, fosaprepitant, tradipitant, maropitant, HTX-019, netupitant, serlopitant, orvepitant, NAS-911B, ZD-6021, KD-018, DNK-333, NT-432, NK-949, NT-814, EU-C-001, vestipitant, 1144814, SCH-900978, AZD-2738, BL-1833, casopitant, AV-810, KRP-103, 424887, cizolirtine, vofopitant, L-742694, capsazepine, GR-82334, MEN-11149, L-732138, NiK-004, TA-5538, CP-96345, lanepitant, LY-2590443, dapitant, burapitant, befetupitant, CJ-17493, AVE-5883, CGP-49823, CP-122721, CP-99994, SLV-317, TAK-637, L-733060, L-703606, dilopetine, MPC-4505, L-742311, FK-888, WIN-64821, NIP-530, SLV-336, ezlopitant, TKA-457, figopitant, ZD-4794, CP-100263, GR-203040, L-709210, MEN-10930, MEN-11467, LY-306740, FK-355, WIN-67689, WIN-51708, FK-224, BL-1832, CAM-6108, CP-98984, WS-9326A, L-741671, L-737488, L-740141, L-760735, L-161664, YM-49244, Sch-60059, SDZ-NKT-343, S-18523, RPR-111905, S-19752, L-161644, LY-297911, RPR-107880, L-736281, anthrotainin, RP-73467, WIN-64745, WIN-68577, WIN-62577 WIN-66306, RP-67580, CP-0364, L-743986, S-16474, CGP-47899, FR-113680, YM-44778, GR-138676, CGP-73400, CAM-2445, MDL-105172A, L-756867, isbufylline, R-673, SR-48968 and SR-14033, GW679769, CP-0578, and a pharmaceutically acceptable salt thereof.

**9.** The aqueous liquid according to any one of claims 1 to 6, wherein the hydrophobic modulator of endosomal GPCR signaling or pharmaceutically acceptable salt thereof is an inhibitor of endosomal CGRP and/or CLR signalling or pharmaceutically acceptable salt thereof.

**10.** The aqueous liquid according to any one of claims 1 to 6 and 9, wherein the hydrophobic modulator of endosomal GPCR signaling or pharmaceutically acceptable salt thereof is selected from the group consisting of BI 44370, MK-3207, olcegepant, ubrogepant, rimegepant, SB-268262, telcagepant, and a pharmaceutically acceptable salt thereof.

**11.** The aqueous liquid according to any one of claims 1 to 10, further comprising a second hydrophobic modulator of endosomal GPCR signaling or a pharmaceutically acceptable salt thereof, wherein the second hydrophobic modulator of endosomal GPCR signalling or pharmaceutically acceptable salt thereof is contained within the core of the polymeric nanoparticles.

**12.** A pharmaceutical composition comprising the aqueous liquid according to any one of claims 1 to 11 and, optionally, a pharmaceutically acceptable excipient.

**13.** The aqueous liquid according to any one of claims 7 to 8 for use in a method for the treatment of a disease or disorder mediated by endosomal NKiR signalling, the method comprising administering to a subject in need thereof an effective amount of said aqueous liquid, wherein the disease or disorder mediated by endosomal $NK_1R$ signaling is selected from chemotherapy-induced nausea and vomiting (CINV), cyclic vomiting syndrome, postoperative nausea and vomiting, affective and addictive disorders including depression and anxiety, generalised anxiety disorder (GAD), gastrointestinal disorders including inflammatory bowel disease, irritable bowel syndrome, gastroparesis and functional dyspepsia, chronic inflammatory disorders including arthritis, respiratory disorders including COPD and asthma, urogenital disorders, sensory disorders and pain including somatic pain and visceral pain, pruritus, viral and bacterial infections and proliferative disorders (cancer), and combinations thereof.

**14.** The aqueous liquid for use of claim 13, further comprising administering to the subject an effective amount of a second modulator of endosomal GPCR signaling or a pharmaceutically acceptable salt thereof.

**15.** The aqueous liquid according to any one of claims 9 to 10 for use in a method for the treatment of a disease or disorder mediated by endosomal CGRP and/or CLR signalling, the method comprising administering to a subject in need thereof an effective amount of said aqueous liquid, wherein the disease or disorder mediated by endosomal CGRP receptor and/or CLR signaling is selected from the group consisting of migraine and symptoms associated with migraine including pain, photophobia, phonophobia, nausea and vomiting, sensory disorders, pain including somatic pain and visceral pain, pain associated with cluster and chronic daily headache, respiratory disorders including COPD and asthma, gastrointestinal disorders including inflammatory bowel disease, irritable bowel syndrome, gastroparesis and functional dyspepsia, and chronic inflammatory disorders including osteoarthritis and rheumatoid arthritis.

**Patentansprüche**

**1.** Wässrige Flüssigkeit, umfassend polymere Nanopartikel aus Copolymerketten, die zu einer Kern/Schale-Struktur zusammengesetzt sind, wobei die Copolymerketten Folgendes aufweisen:

(i) einen auf Säure reagierenden hydrophoben Polymerblock, der den Kern der Nanopartikel ausbildet, wobei der auf Säure reagierende hydrophobe Polymerblock tertiäre Aminfunktionsgruppen umfasst, die in einer sauren Umgebung protoniert werden; und
(ii) einen hydrophilen Polymerblock, der die Schale der Nanopartikel ausbildet und von der wässrigen Flüssigkeit solvatisiert wird,

wobei die Nanopartikel in ihrem Kern einen hydrophoben Modulator der endosomalen GPCR-Signalübertragung oder ein pharmazeutisch annehmbares Salz davon enthalten.

**2.** Wässrige Flüssigkeit nach Anspruch 1, wobei die tertiären Aminfunktionsgruppen bei etwa pH = 6,1 protoniert sind.

**3.** Wässrige Flüssigkeit nach Anspruch 1 oder 2, wobei der auf Säure reagierende hydrophobe Polymerblock ein Homopolymer oder Copolymer von 2-(Diisopropylamino)ethylmethacrylat (DiPAEMA) umfasst.

4. Wässrige Flüssigkeit nach einem der Ansprüche 1 bis 3, wobei der auf Säure reagierende hydrophobe Polymerblock ein Copolymer aus 2-(Diisopropylamino)ethylmethacrylat (DiPAEMA) und einem Polyalkylenoxidmethacrylat umfasst.

5. Wässrige Flüssigkeit nach einem der Ansprüche 1 bis 4, wobei der hydrophile Polymerblock ein Copolymer aus Poly(ethylenglykol)methacrylat (PEGMA) und 2-(Dimethylamino)ethylmethacrylat (DMAEMA) umfasst.

6. Wässrige Flüssigkeit nach einem der Ansprüche 1 bis 5, wobei der hydrophobe Modulator der endosomalen GPCR-Signalübertragung oder ein pharmazeutisch annehmbares Salz davon ein Inhibitor eines endosomalen GPCR ist.

7. Wässrige Flüssigkeit nach einem der Ansprüche 1 bis 6, wobei der hydrophobe Modulator der endosomalen GPCR-Signalübertragung oder ein pharmazeutisch annehmbares Salz davon ein Inhibitor der endosomalen $NK_1R$-Signalübertragung oder ein pharmazeutisch annehmbares Salz davon ist.

8. Wässrige Flüssigkeit nach einem der Ansprüche 1 bis 7, wobei der hydrophobe Modulator der endosomalen GPCR-Signalübertragung oder ein pharmazeutisch annehmbares Salz davon ausgewählt sind aus der Gruppe, bestehend aus Aprepitant, Fosaprepitant, Tradipitant, Maropitant, HTX-019, Netupitant, Serlopitant, Orvepitant, NAS-911B, ZD-6021, KD-018, DNK-333, NT-432, NK-949, NT-814, EU-C-001, Vestipitant, 1144814, SCH-900978, AZD-2738, BL-1833, Kasopitant, AV-810, KRP-103, 424887, Cizolirtin, Vofopitant, L-742694, Capsazepin, GR-82334, MEN-11149, L-732138, NiK-004, TA-5538, CP-96345, Lanepitant, LY-2590443, Dapitant, Burapitant, Befetupitant, CJ-17493, AVE-5883, CGP-49823, CP-122721, CP-99994, SLV-317, TAK-637, L-733060, L-703606, Dilopetin, MPC-4505, L-742311, FK-888, WIN-64821, NIP-530, SLV-336, Ezlopitant, TKA-457, Figopitant, ZD-4794, CP-100263, GR-203040, L-709210, MEN-10930, MEN-11467, LY-306740, FK-355, WIN-67689, WIN-51708, FK-224, BL-1832, CAM-6108, CP-98984, WS-9326A, L-741671, L-737488, L-740141, L-760735, L-161664, YM-49244, Sch-60059, SDZ-NKT-343, S-18523, RPR-111905, S-19752, L-161644, LY-297911, RPR-107880, L-736281, Anthrotainin, RP-73467, WIN-64745, WIN-68577, WIN-62577 WIN-66306, RP-67580, CP-0364, L-743986, S-16474, CGP-47899, FR-113680, YM-44778, GR-138676, CGP-73400, CAM-2445, MDL-105172A, L-756867, Isbufyllin, R-673, SR-48968 und SR-14033, GW679769, CP-0578 und einem pharmazeutisch annehmbaren Salz davon.

9. Wässrige Flüssigkeit nach einem der Ansprüche 1 bis 6, wobei der hydrophobe Modulator der endosomalen GPCR-Signalübertragung oder ein pharmazeutisch annehmbares Salz davon ein Inhibitor der endosomalen CGRP- und/oder CLR-Signalübertragung oder ein pharmazeutisch annehmbares Salz davon ist.

10. Wässrige Flüssigkeit nach einem der Ansprüche 1 bis 6 und 9, wobei der hydrophobe Modulator der endosomalen GPCR-Signalübertragung oder ein pharmazeutisch annehmbares Salz davon ausgewählt ist aus der Gruppe, bestehend aus BI 44370, MK-3207, Olcegepant, Ubrogepant, Rimegepant, SB-268262, Telcagepant und einem pharmazeutisch annehmbaren Salz davon.

11. Wässrige Flüssigkeit nach einem der Ansprüche 1 bis 10, ferner umfassend einen zweiten hydrophoben Modulator endosomaler GPCR-Signalübertragung oder ein pharmazeutisch annehmbares Salz davon, wobei der zweite hydrophobe Modulator endosomaler GPCR-Signalübertragung oder das pharmazeutisch annehmbare Salz davon im Kern der polymeren Nanopartikel enthalten ist.

12. Pharmazeutische Zusammensetzung, umfassend die wässrige Flüssigkeit nach einem der Ansprüche 1 bis 11 und optional einen pharmazeutisch annehmbaren Hilfsstoff.

13. Wässrige Flüssigkeit nach einem der Ansprüche 7 bis 8 zur Verwendung in einem Verfahren zur Behandlung einer Krankheit oder Störung, die durch endosomale $NK_1R$-Signalübertragung vermittelt wird, wobei das Verfahren das Verabreichen einer wirksamen Menge der wässrigen Flüssigkeit an ein Subjekt, das dies benötigt, umfasst, wobei die Krankheit oder Störung, die durch endosomale $NK_1R$-Signalübertragung vermittelt wird, ausgewählt ist aus Chemotherapie-induzierter Übelkeit und Erbrechen (CINV), zyklischem Erbrechensyndrom, postoperativer Übelkeit und Erbrechen, affektiven und Suchtstörungen einschließlich Depressionen und Angstzuständen, generalisierter Angststörung (GAD), Magen-Darm-Erkrankungen einschließlich entzündlicher Darmerkrankungen, Reizdarmsyndrom, Gastroparese und funktioneller Dyspepsie, chronisch entzündlicher Erkrankungen einschließlich Arthritis, Atemwegserkrankungen einschließlich COPD und Asthma, Urogenitalstörungen, sensorischer Störungen und Schmerzen einschließlich somatischer Schmerzen und viszeraler Schmerzen, Pruritus, viralen und bakteriellen Infektionen und proliferativen Störungen (Krebs) sowie Kombinationen daraus.

**14.** Wässrige Flüssigkeit zur Verwendung nach Anspruch 13, ferner umfassend das Verabreichen einer wirksamen Menge eines zweiten Modulators der endosomalen GPCR-Signalübertragung oder eines pharmazeutisch annehmbaren Salzes davon an das Subjekt.

**15.** Wässrige Flüssigkeit nach einem der Ansprüche 9 bis 10 zur Verwendung in einem Verfahren zur Behandlung einer Krankheit oder Störung, die durch endosomale CGRP- und/oder CLR-Signalübertragung vermittelt wird, wobei das Verfahren das Verabreichen einer wirksamen Menge der wässrigen Flüssigkeit an ein Subjekt, das dies benötigt, umfasst, wobei die Krankheit oder Störung, die durch endosomale CGRP-Rezeptor- und/oder CLR-Signalübertragung vermittelt wird, ausgewählt ist aus der Gruppe bestehend aus: Migräne und mit Migräne verbundenen Symptomen, einschließlich Schmerzen, Photophobie, Phonophobie, Übelkeit und Erbrechen, Sinnesstörungen, Schmerzen einschließlich somatischer Schmerzen und viszeraler Schmerzen, Schmerzen im Zusammenhang mit Cluster- und chronischen täglichen Kopfschmerzen, Atemwegserkrankungen einschließlich COPD und Asthma, Magen-Darm-Erkrankungen einschließlich entzündlicher Darmerkrankungen, Reizdarmsyndrom, Gastroparese und funktioneller Dyspepsie sowie chronisch entzündlicher Erkrankungen einschließlich Osteoarthritis und rheumatoider Arthritis.

## Revendications

**1.** Liquide aqueux comprenant des nanoparticules polymères de chaînes de copolymères assemblées pour former une structure noyau / enveloppe, les chaînes de copolymères comportant :

(i) un bloc de polymère hydrophobe sensible aux acides qui forme le noyau des nanoparticules, dans lequel le bloc de polymère hydrophobe sensible aux acides comprend des groupes fonctionnels amine tertiaire qui sont protonés dans un environnement acide ; et
(ii) un bloc de polymère hydrophile qui forme l'enveloppe des nanoparticules et est solvaté par le liquide aqueux,

dans lequel les nanoparticules contiennent dans leur noyau un modulateur hydrophobe de signalisation GPCR endosomique, ou un sel pharmaceutiquement acceptable de celui-ci.

**2.** Liquide aqueux selon la revendication 1, dans lequel les groupes fonctionnels amine tertiaire sont protonés à environ pH = 6,1.

**3.** Liquide aqueux selon la revendication 1 ou 2, dans lequel le bloc de polymère hydrophobe sensible aux acides comprend un homopolymère ou copolymère de méthacrylate de 2-(diisopropylamino)éthyle (DiPAEMA).

**4.** Liquide aqueux selon l'une quelconque des revendications 1 à 3, dans lequel le bloc de polymère hydrophobe sensible aux acides comprend un copolymère de méthacrylate de 2-(diisopropylamino)éthyle (DiPAEMA) et un méthacrylate de poly(oxyde d'alkylène).

**5.** Liquide aqueux selon l'une quelconque des revendications 1 à 4, dans lequel le bloc de polymère hydrophile comprend un copolymère de méthacrylate de poly(éthylène glycol) (PEGMA) et de méthacrylate de 2-(diméthylamino)éthyle (DMAEMA).

**6.** Liquide aqueux selon l'une quelconque des revendications 1 à 5, dans lequel le modulateur hydrophobe de signalisation GPCR endosomique ou un sel pharmaceutiquement acceptable de celui-ci est un inhibiteur d'un GPCR endosomique.

**7.** Liquide aqueux selon l'une quelconque des revendications 1 à 6, dans lequel le modulateur hydrophobe de signalisation GPCR endosomique ou un sel pharmaceutiquement acceptable de celui-ci est un inhibiteur de la signalisation NKiR endosomique, ou un sel pharmaceutiquement acceptable de celui-ci.

**8.** Liquide aqueux selon l'une quelconque des revendications 1 à 7, dans lequel le modulateur hydrophobe de signalisation GPCR endosomique ou un sel pharmaceutiquement acceptable de celui-ci est choisi dans le groupe constitué de : aprépitant, fosaprépitant, tradipitant, maropitant, HTX-019, nétupitant, serlopitant, orvépitant, NAS-911B, ZD-6021, KD-018, DNK-333, NT-432, NK-949, NT-814, EU-C-001, vestipitant, 1144814, SCH-900978, AZD-2738, BL-1833, casopitant, AV-810, KRP-103, 424887, cizolirtine, vofopitant, L-742694, capsazépine, GR-82334, MEN-11149, L-732138, NiK-004, TA-5538, CP-96345, lanépitant, LY-2590443, dapitant, burapitant, béfétupitant, CJ-17493, AVE-5883, CGP-49823, CP-122721, CP-99994, SLV-317, TAK-637, L- 733060, L-703606, dilopétine, MPC-4505, L-742311, FK-888, WIN-64821, NIP-530, SLV-336, ezlopitant, TKA-457, figopitant, ZD-4794, CP-100263, GR-203040, L-709210, MEN-10930, MEN-11467, LY-306740, FK-355, WIN-67689, WIN-51708, FK-224, BL-1832, CAM-6108, CP- 98984, WS-9326A, L-741671, L-737488, L-740141, L-760735, L-161664, YM-49244, Sch-60059, SDZ-NKT-343, S-18523, RPR-111905, S-19752, L-161644, LY-297911, RPR-107880, L- 736281, anthrotainin, RP-73467, WIN-64745, WIN-68577, WIN-62577 WIN-66306, RP-67580, CP-0364, L-743986, S-16474, CGP-47899, FR-113680, YM-44778, GR-138676, CGP-73400, CAM-2445, MDL-105172A, L-756867, isbufylline, R-673, SR-48968 et SR-14033, GW679769, CP-0578, et un sel pharmaceutiquement acceptable de ceux-ci.

**9.** Liquide aqueux selon l'une quelconque des revendications 1 à 6, dans lequel le modulateur hydrophobe de signalisation GPCR endosomique ou un sel pharmaceutiquement acceptable de celui-ci est un inhibiteur de la signalisation CGRP et/ou CLR endosomique ou un sel pharmaceutiquement acceptable de celui-ci.

**10.** Liquide aqueux selon l'une quelconque des revendications 1 à 6 et 9, dans lequel le modulateur hydrophobe de signalisation GPCR endosomique ou un sel pharmaceutiquement acceptable de celui-ci est choisi dans le groupe constitué de BI 44370, MK-3207, olcégépant, ubrogépant, rimégépant, SB-268262, telcagépant, et un sel pharmaceutiquement acceptable de ceux-ci.

**11.** Liquide aqueux selon l'une quelconque des revendications 1 à 10, comprenant en outre un deuxième modulateur hydrophobe de signalisation GPCR endosomique ou un sel pharmaceutiquement acceptable de celui-ci, dans lequel le deuxième modulateur hydrophobe de signalisation GPCR endosomique ou un sel pharmaceutiquement acceptable de celui-ci est contenu dans le noyau des nanoparticules polymères.

**12.** Composition pharmaceutique comprenant le liquide aqueux selon l'une quelconque des revendications 1 à 11 et, facultativement, un excipient pharmaceutiquement acceptable.

**13.** Liquide aqueux selon l'une quelconque des revendications 7 à 8 pour utilisation dans un procédé de traitement d'une maladie ou d'un trouble médié par la signalisation NKiR endosomique, le procédé comprenant l'administration à un sujet en ayant besoin d'une quantité efficace dudit liquide aqueux, la maladie ou le trouble médié par la signalisation NKiR endosomique étant choisi parmi les nausées et vomissements chimio-induits (NVCI), le syndrome des vomissements cycliques, les nausées et vomissements postopératoires, des troubles affectifs et addictifs comprenant la dépression et l'anxiété, un trouble anxieux généralisé (TAG), des troubles gastro-intestinaux comprenant des maladies inflammatoires de l'intestin, le syndrome du côlon irritable, la gastroparésie et la dyspepsie fonctionnelle, des troubles inflammatoires chroniques comprenant l'arthrite, des troubles respiratoires comprenant la BPCO et l'asthme, des troubles urogénitaux, des troubles sensoriels et des douleurs comprenant une douleur somatique et une douleur viscérale, le prurit, des infections virales et bactériennes et des troubles prolifératifs (cancer), et des combinaisons de ceux-ci.

**14.** Liquide aqueux pour utilisation selon la revendication 13, comprenant en outre l'administration au sujet d'une quantité efficace d'un deuxième modulateur de signalisation GPCR endosomique ou un sel pharmaceutiquement acceptable de celui-ci.

**15.** Liquide aqueux selon l'une quelconque des revendications 9 à 10 pour utilisation dans un procédé pour le traitement d'une maladie ou d'un trouble médié par la signalisation CGRP et/ou CLR endosomique, le procédé comprenant l'administration au sujet en ayant besoin d'une quantité efficace dudit liquide aqueux, la maladie ou le trouble médié par la signalisation du récepteur de CGRP et/ou CLR endosomique étant choisi dans le groupe constitué de : la migraine et les symptômes associés à la migraine comprenant la douleur, la photophobie, la phonophobie, les nausées et les vomissements, des troubles sensoriels, une douleur comprenant une douleur somatique et une douleur viscérale, une douleur associée à l'algie vasculaire de la face et aux céphalées quotidiennes chroniques, des troubles respiratoires comprenant la BPCO et l'asthme, des troubles gastro-intestinaux comprenant une maladie inflammatoire de l'intestin, le syndrome du côlon irritable, la gastroparésie et la dyspepsie fonctionnelle, et des troubles inflammatoires chroniques comprenant l'arthrose et la polyarthrite rhumatoïde.

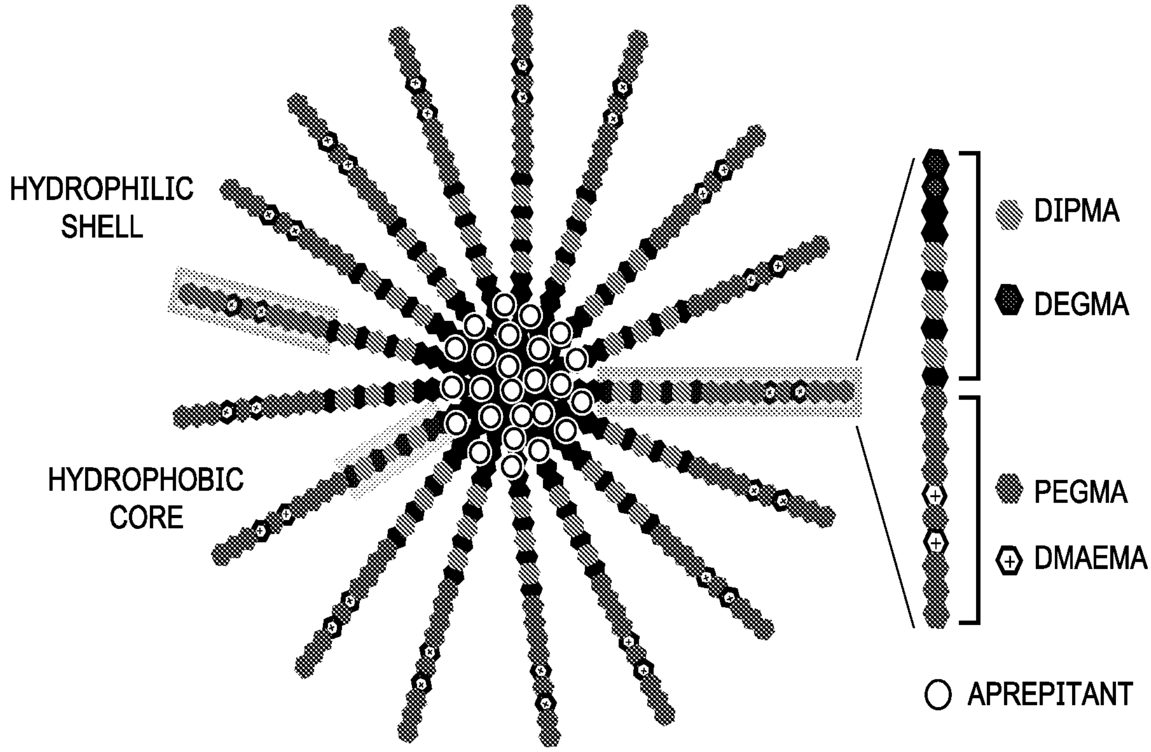
HYDROPHILIC
SHELL
HYDROPHOBIC
CORE
DIPMA
DEGMA
PEGMA
DMAEMA
APREPITANT

FIG. 1A

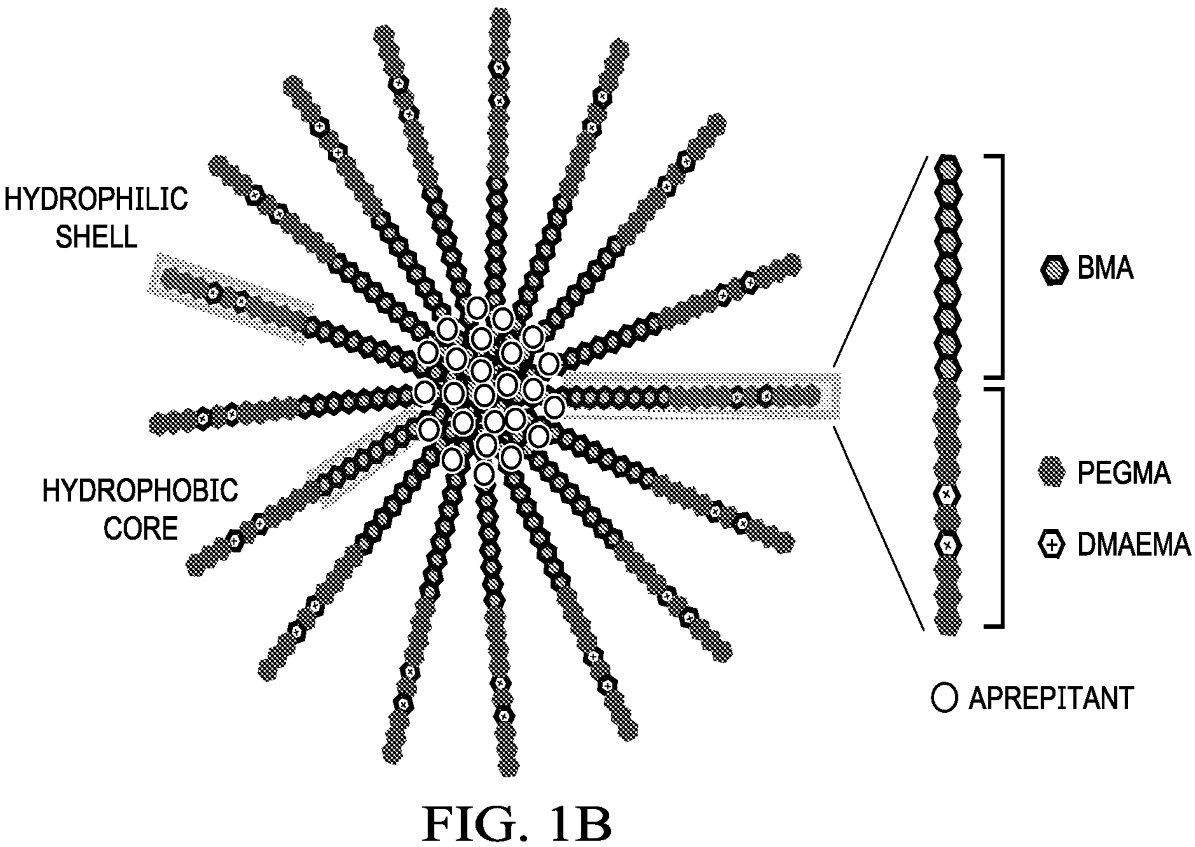
HYDROPHILIC
SHELL
HYDROPHOBIC
CORE
BMA
PEGMA
DMAEMA
APREPITANT

FIG. 1B

FIG. 1C

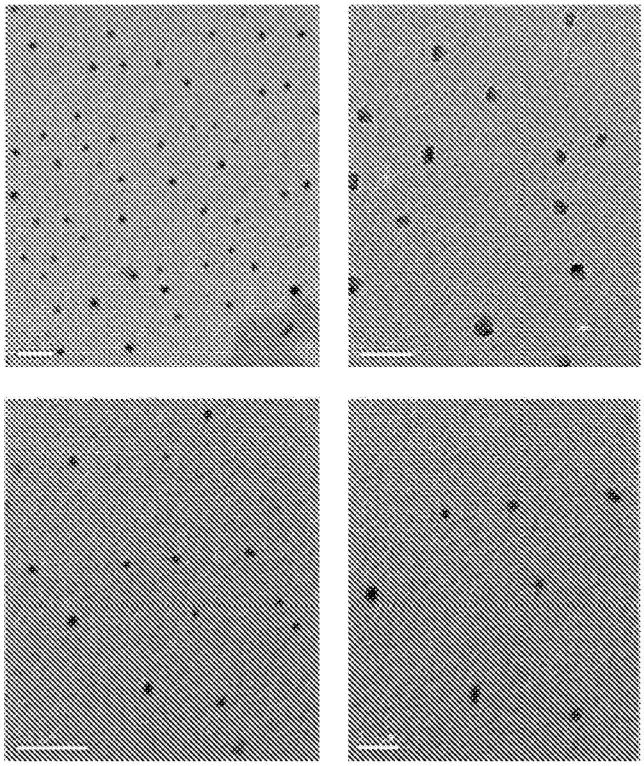

FIG. 1D

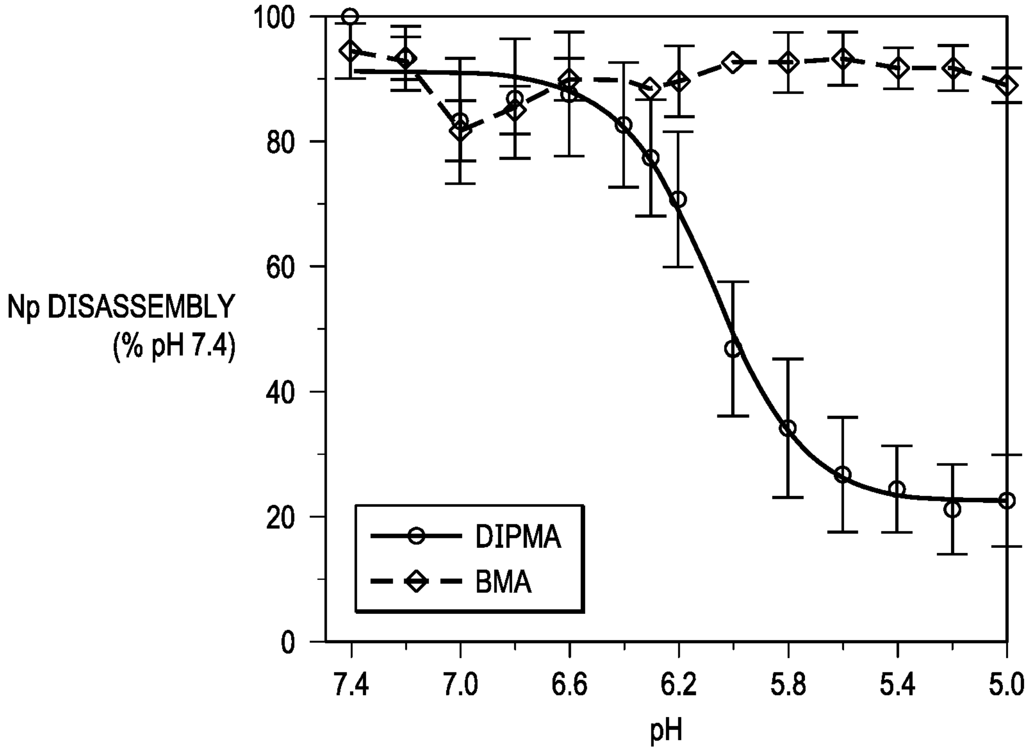
100
80
60
40
20
0
Np DISASSEMBLY
(% pH 7.4)
7.4
7.0
6.6
6.2
5.8
5.4
5.0
pH
DIPMA
BMA

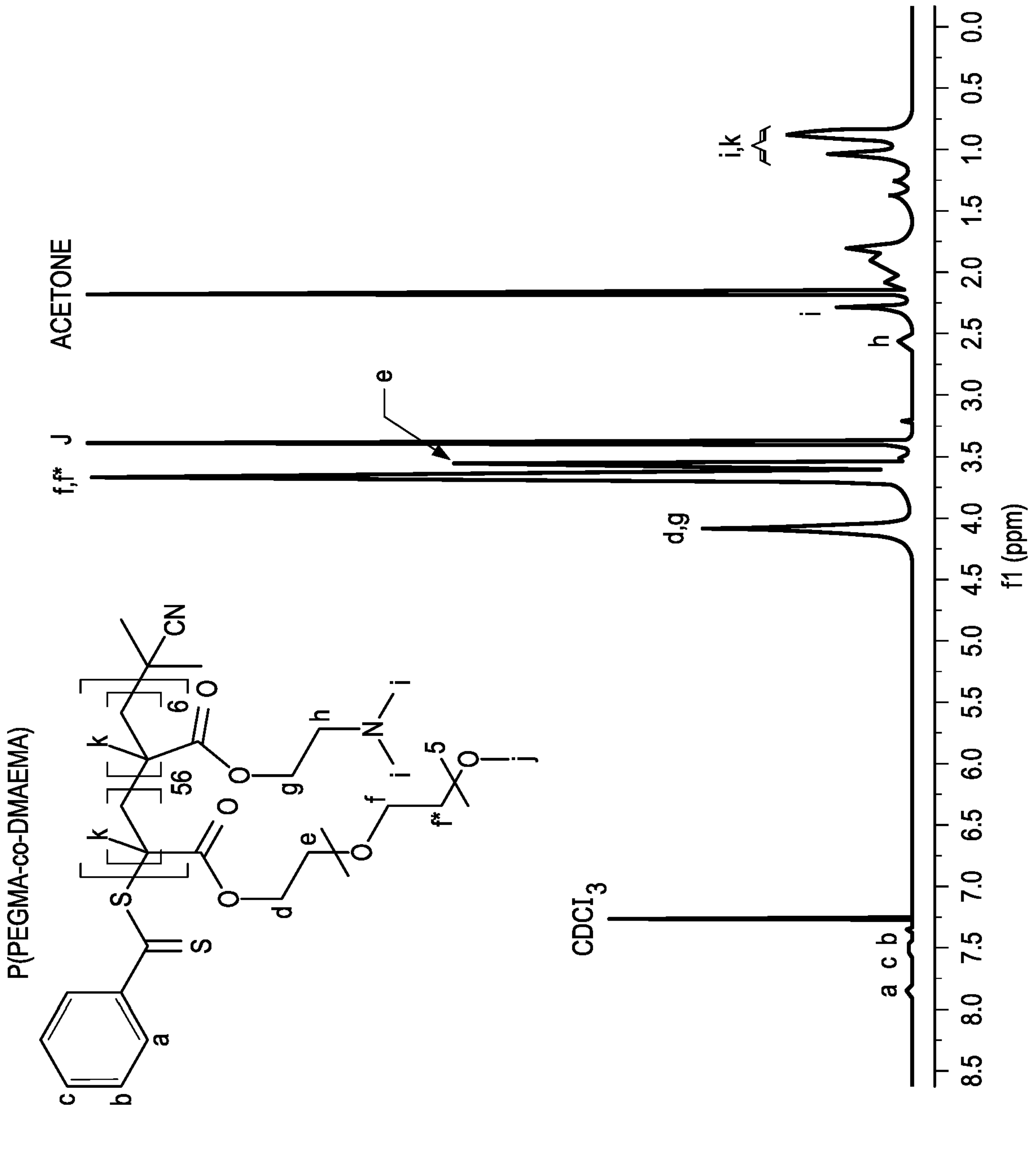
P(PEGMA-co-DMAEMA)
CDCl3
d,g
f,f*
J
e
ACETONE
h
i
i,k
a c b
8.5 8.0 7.5 7.0 6.5 6.0 5.5 5.0 4.5 4.0 3.5 3.0 2.5 2.0 1.5 1.0 0.5 0.0
f1 (ppm)

FIG. 2A

P(PEGMA-co-DMAEMA)-b-P-(DIPMA-co-DEGMA)

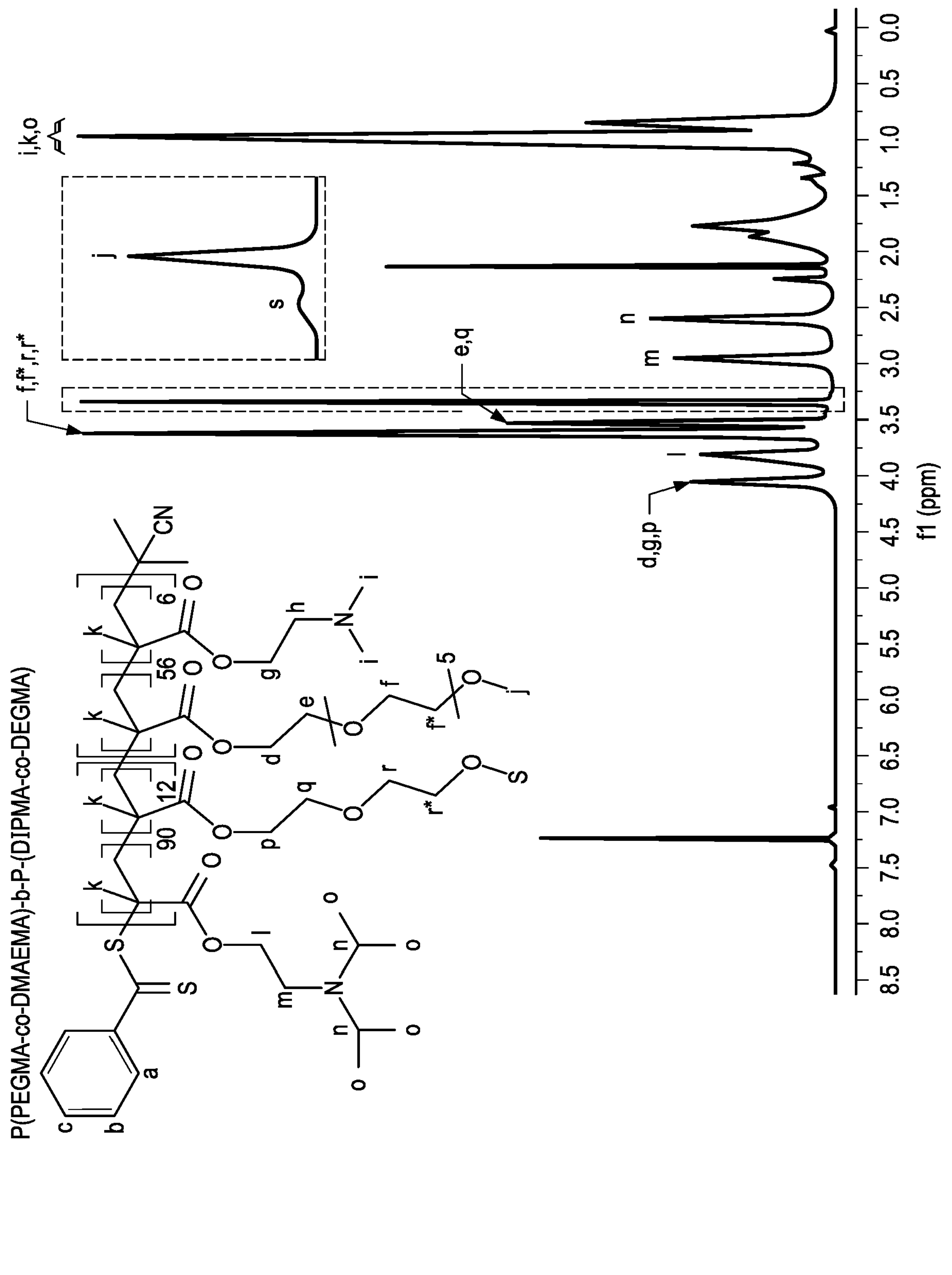


FIG. 2B

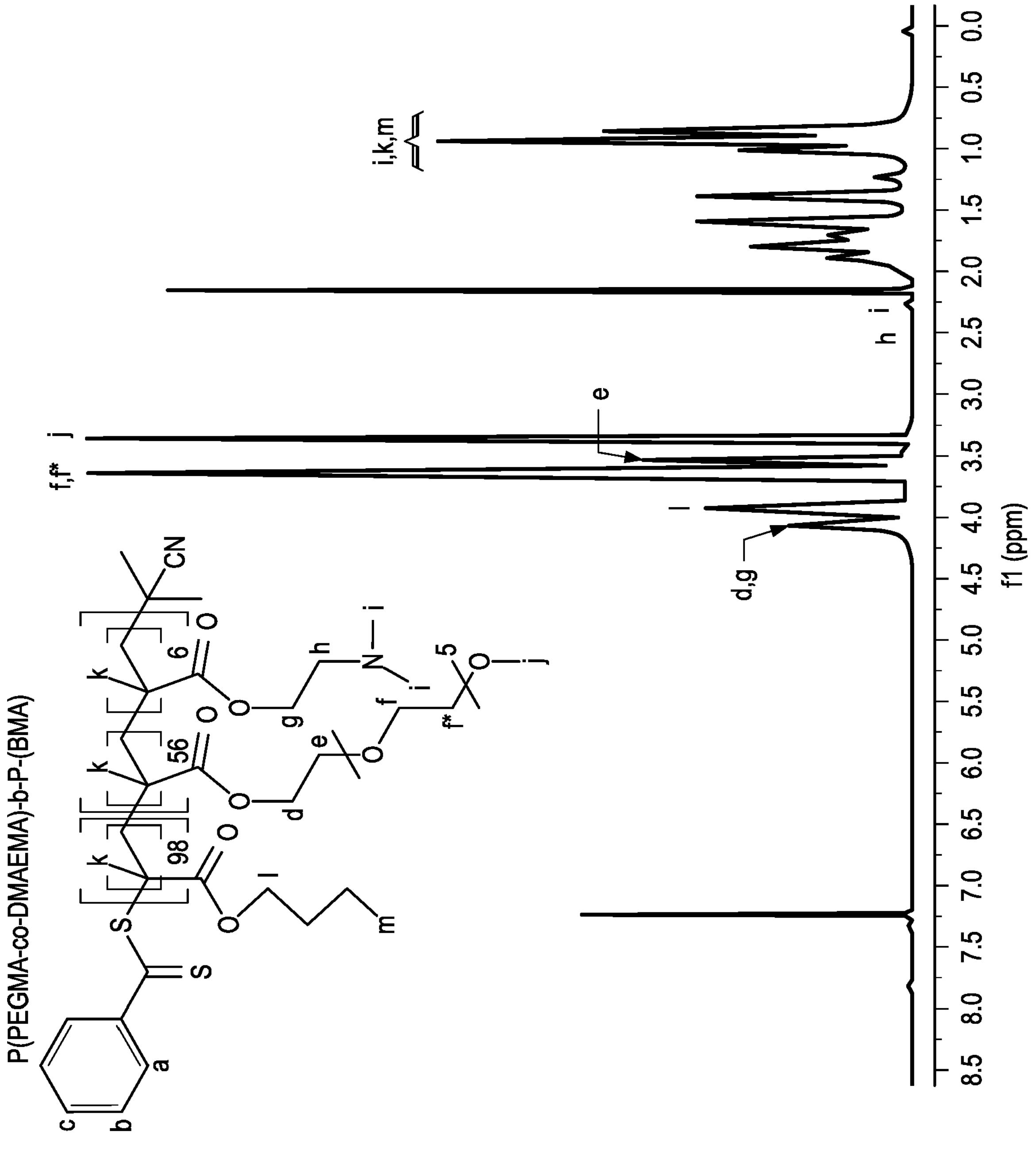
P(PEGMA-co-DMAEMA)-b-P-(BMA)
f1 (ppm)
8.5 8.0 7.5 7.0 6.5 6.0 5.5 5.0 4.5 4.0 3.5 3.0 2.5 2.0 1.5 1.0 0.5 0.0
i,k,m
f,f*
j
e
h i
l
d,g

FIG. 2C

FIG. 3

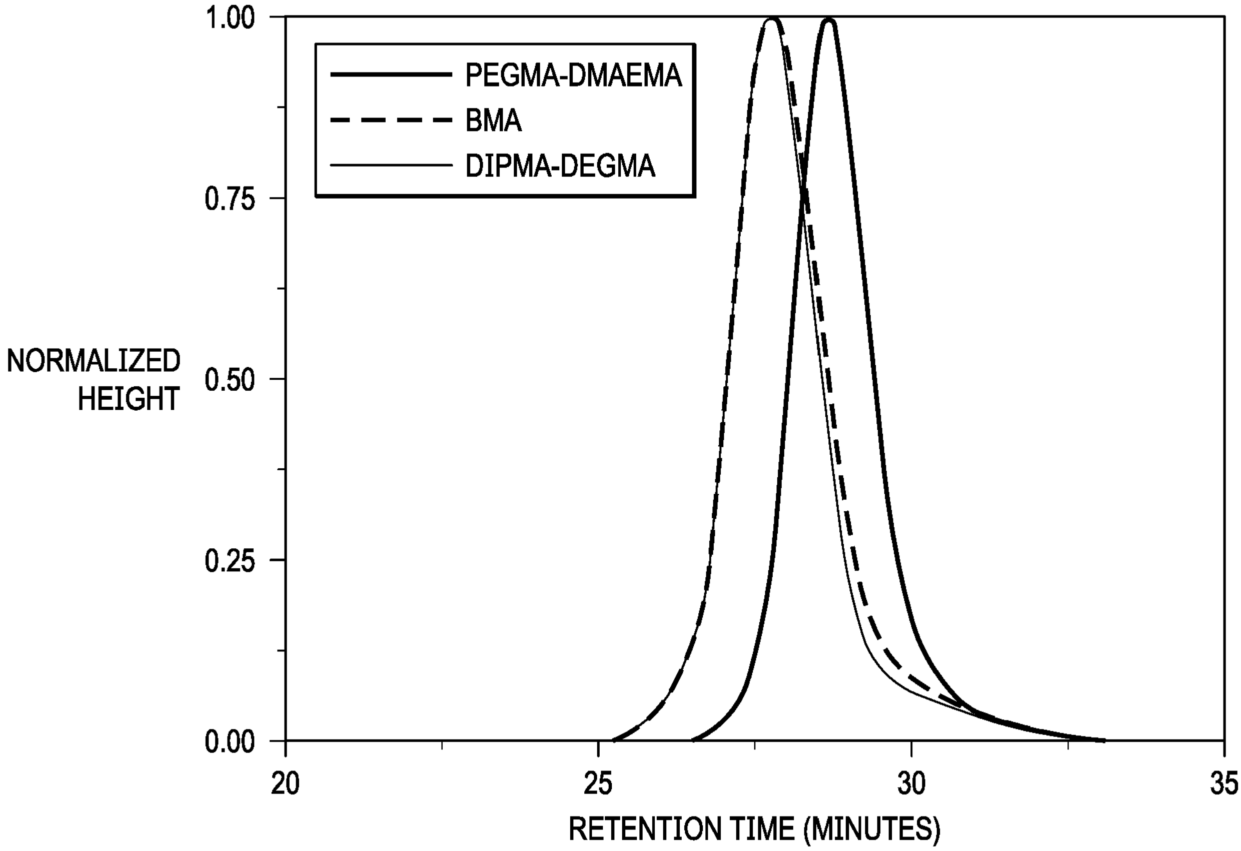

1.00
0.75
0.50
0.25
0.00
NORMALIZED HEIGHT
PEGMA-DMAEMA
BMA
DIPMA-DEGMA
20
25
30
35
RETENTION TIME (MINUTES)

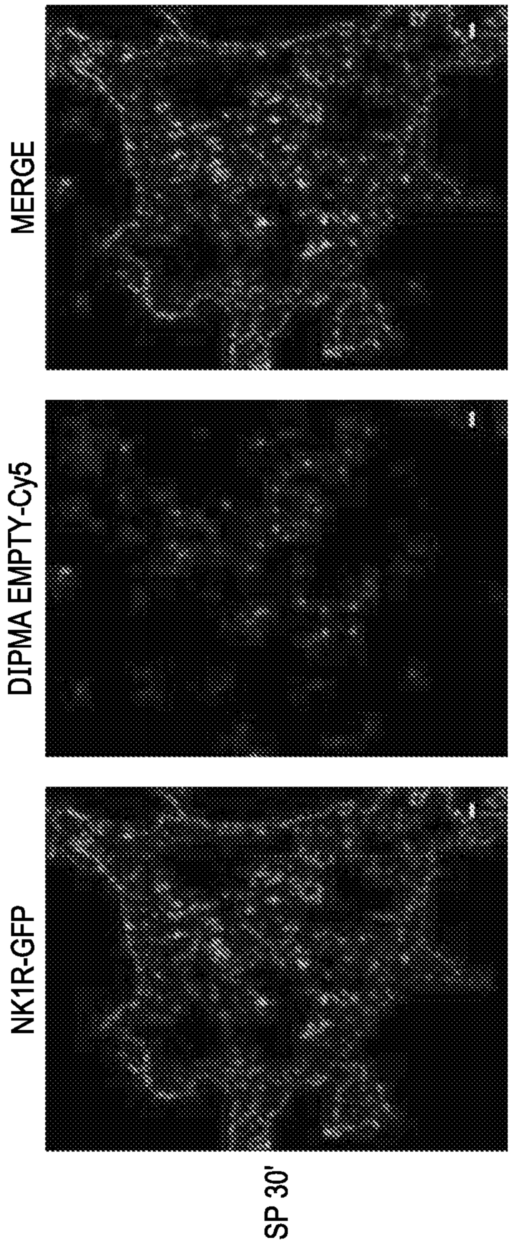
NK1R-GFP
DIPMA EMPTY-Cy5
MERGE
SP 30'

FIG. 4A

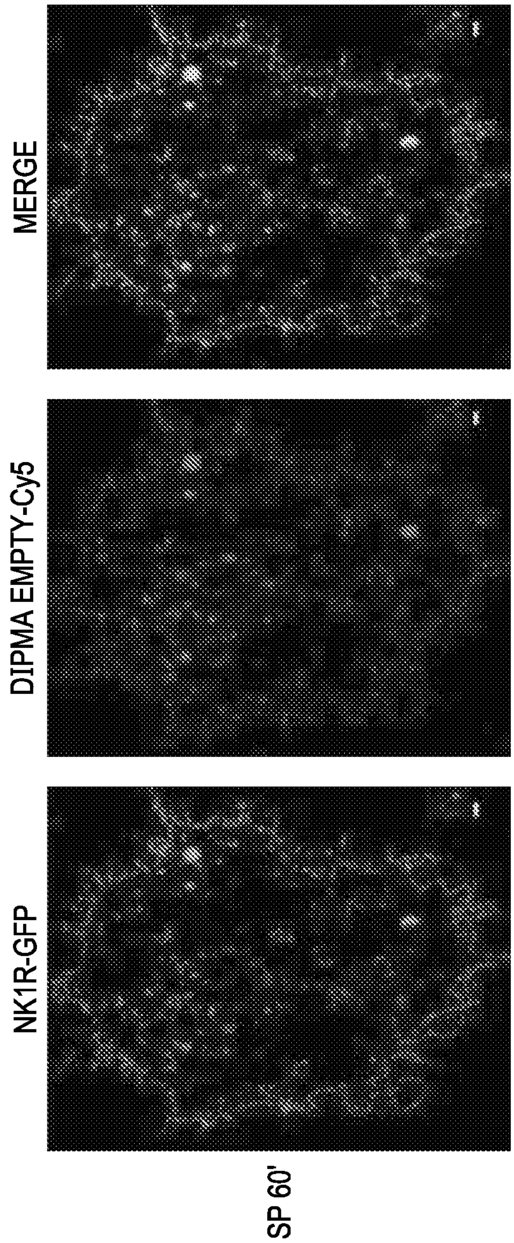
NK1R-GFP
DIPMA EMPTY-Cy5
MERGE
SP 60'

FIG. 4B

FIG. 5A

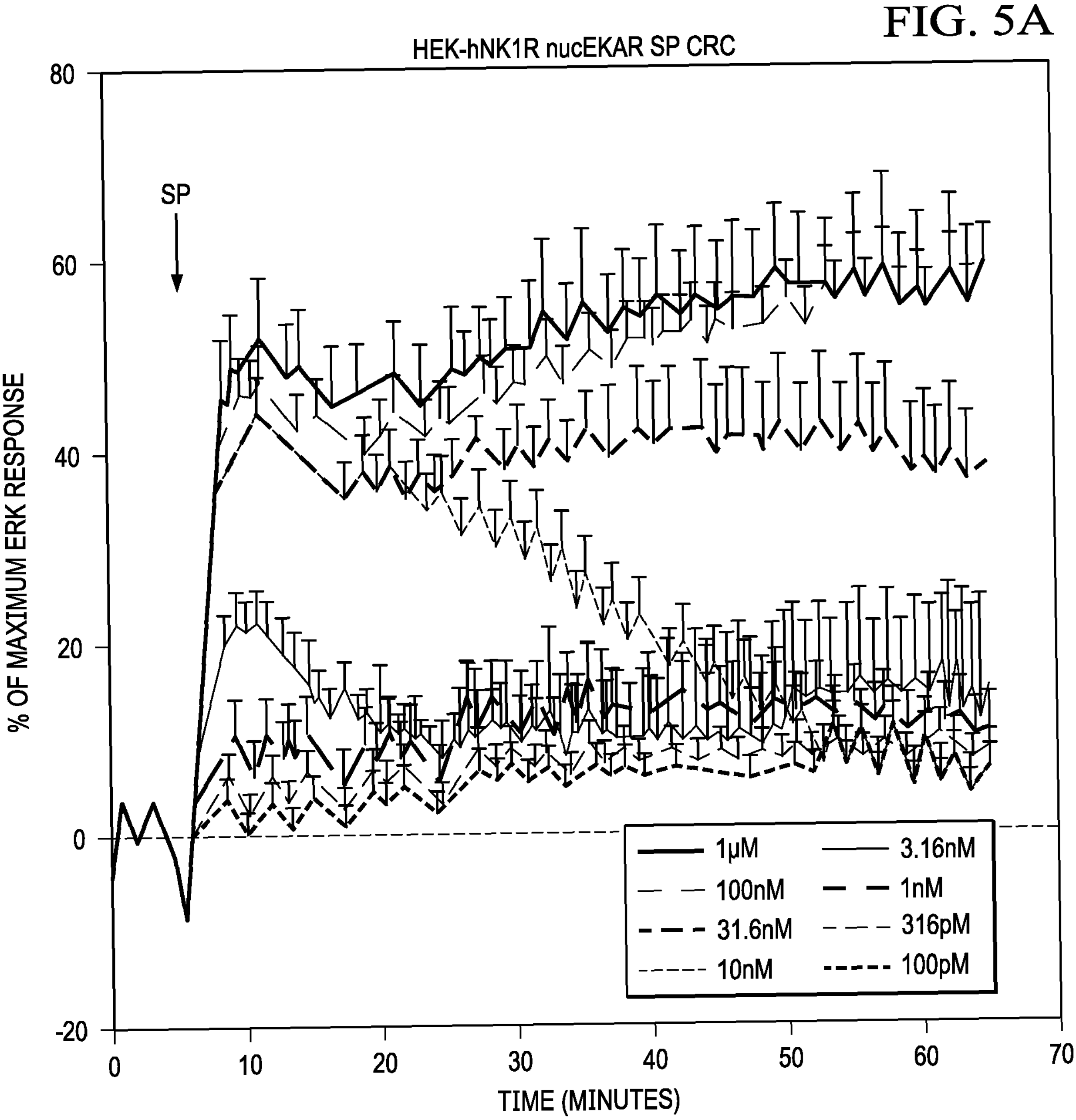
HEK-hNK1R nucEKAR SP CRC
SP
% OF MAXIMUM ERK RESPONSE
80
60
40
20
0
-20
0
10
20
30
40
50
60
70
TIME (MINUTES)
1μM
3.16nM
100nM
1nM
31.6nM
316pM
10nM
100pM

FIG. 5B

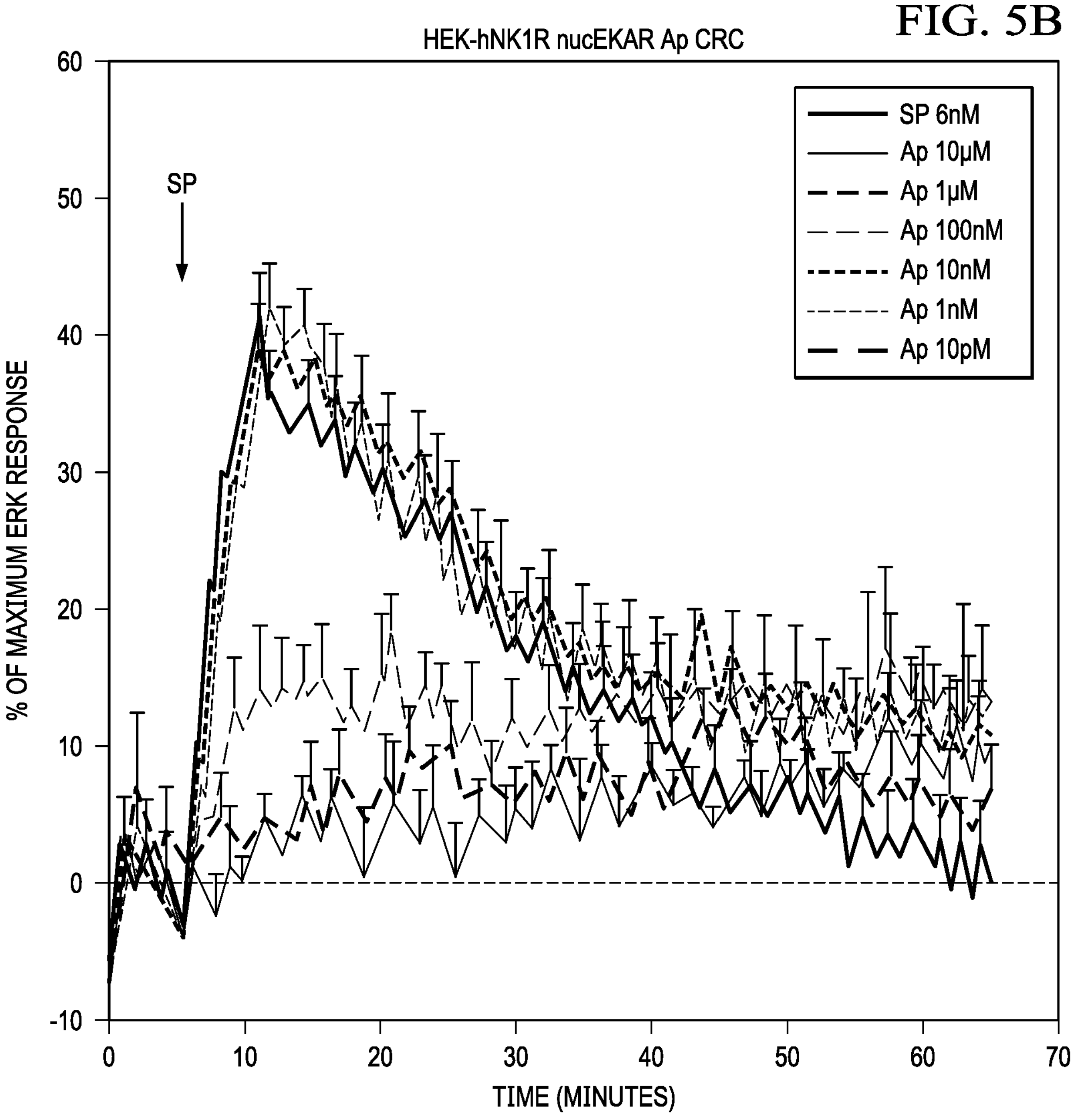
HEK-hNK1R nucEKAR Ap CRC
SP
SP 6nM
Ap 10μM
Ap 1μM
Ap 100nM
Ap 10nM
Ap 1nM
Ap 10pM
% OF MAXIMUM ERK RESPONSE
TIME (MINUTES)
60
50
40
30
20
10
0
-10
0
10
20
30
40
50
60
70

FIG. 5C

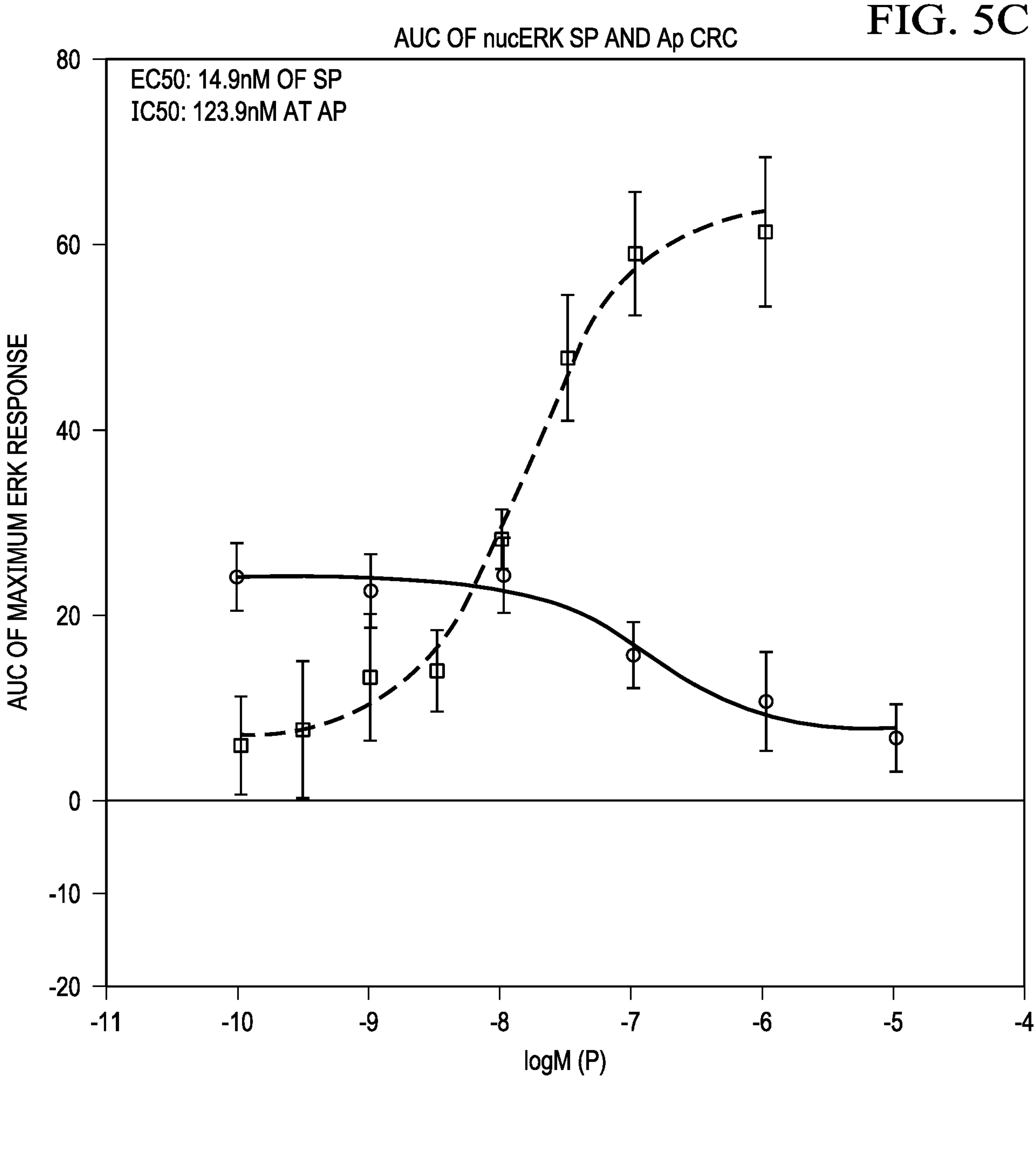
AUC OF nucERK SP AND Ap CRC
EC50: 14.9nM OF SP
IC50: 123.9nM AT AP
AUC OF MAXIMUM ERK RESPONSE
80
60
40
20
0
-10
-20
-11
-10
-9
-8
-7
-6
-5
-4
logM (P)

FIG. 6A

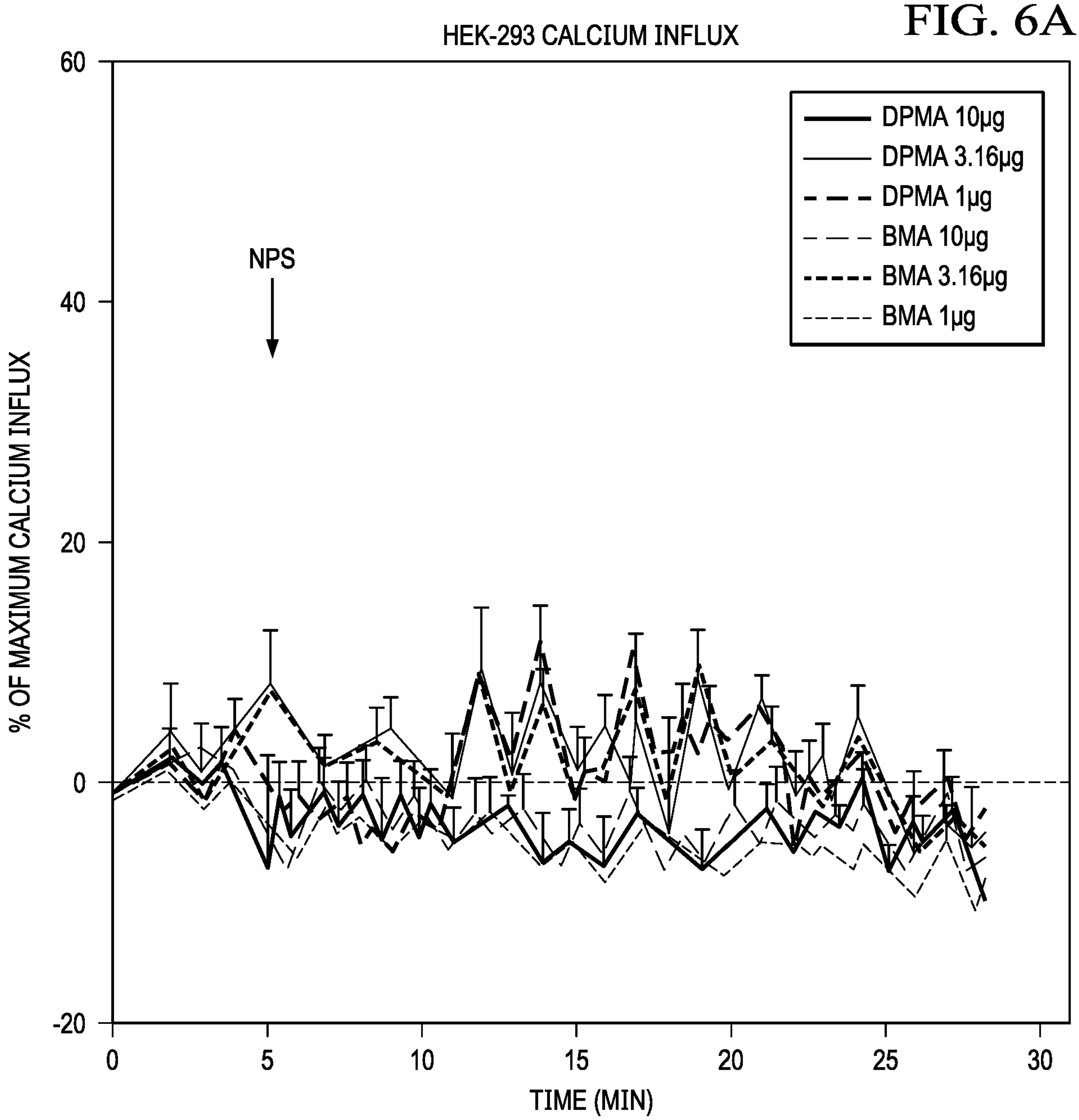
HEK-293 CALCIUM INFLUX
% OF MAXIMUM CALCIUM INFLUX
TIME (MIN)
NPS
DPMA 10μg
DPMA 3.16μg
DPMA 1μg
BMA 10μg
BMA 3.16μg
BMA 1μg
60
40
20
0
-20
0
5
10
15
20
25
30

FIG. 6B

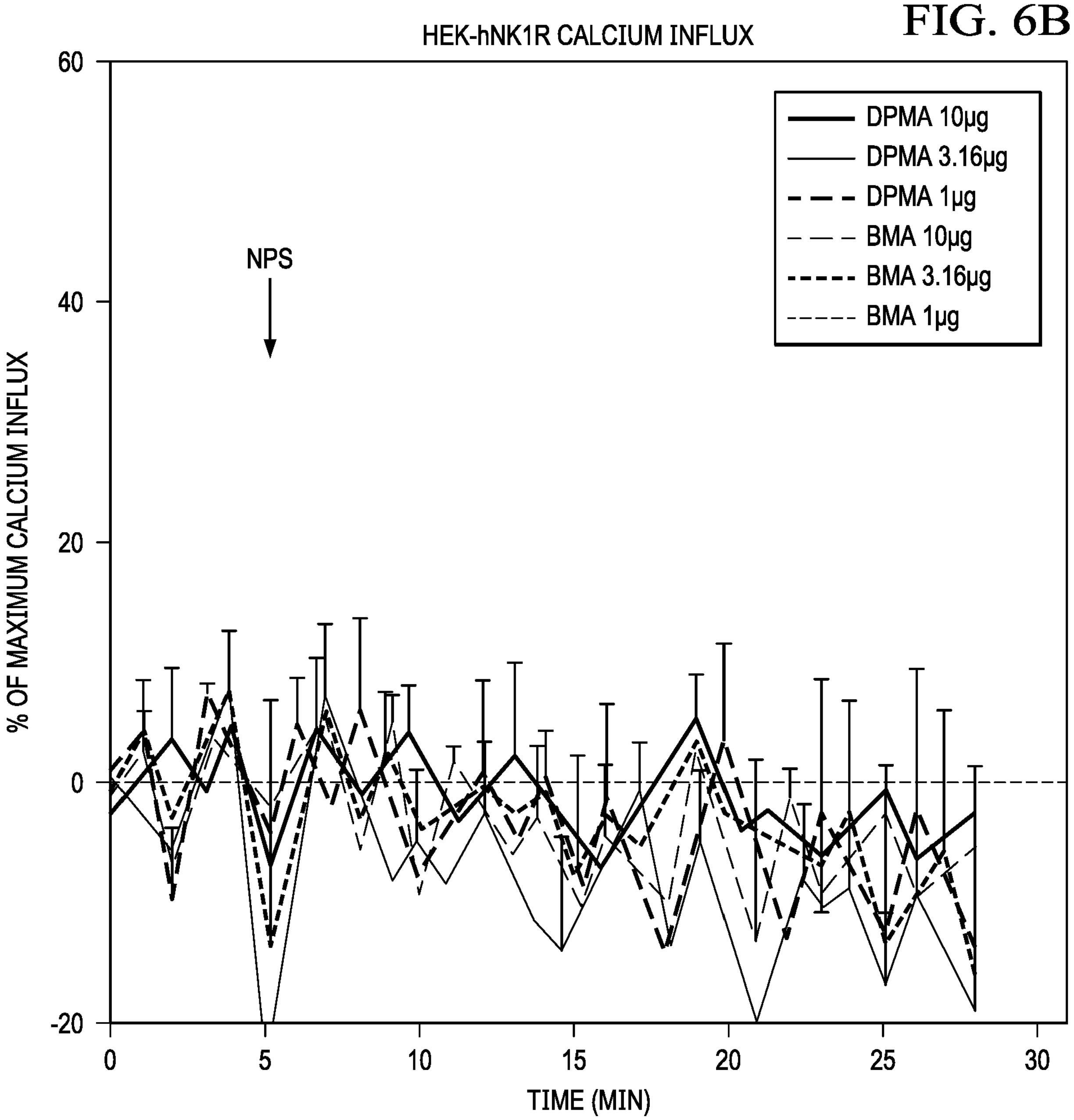
HEK-hNK1R CALCIUM INFLUX
60
40
20
0
-20
% OF MAXIMUM CALCIUM INFLUX
0
5
10
15
20
25
30
TIME (MIN)
NPS
DPMA 10µg
DPMA 3.16µg
DPMA 1µg
BMA 10µg
BMA 3.16µg
BMA 1µg

FIG. 6C

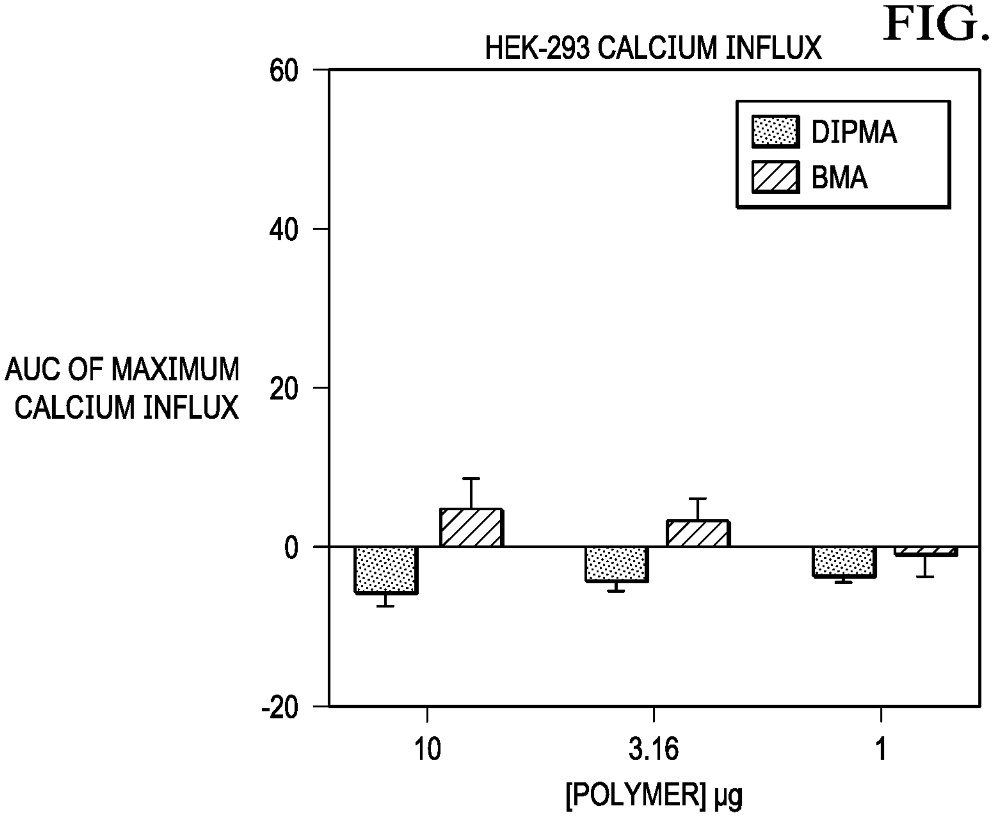
HEK-293 CALCIUM INFLUX
60
40
20
0
-20
AUC OF MAXIMUM CALCIUM INFLUX
DIPMA
BMA
10
3.16
1
[POLYMER] µg

FIG. 6D

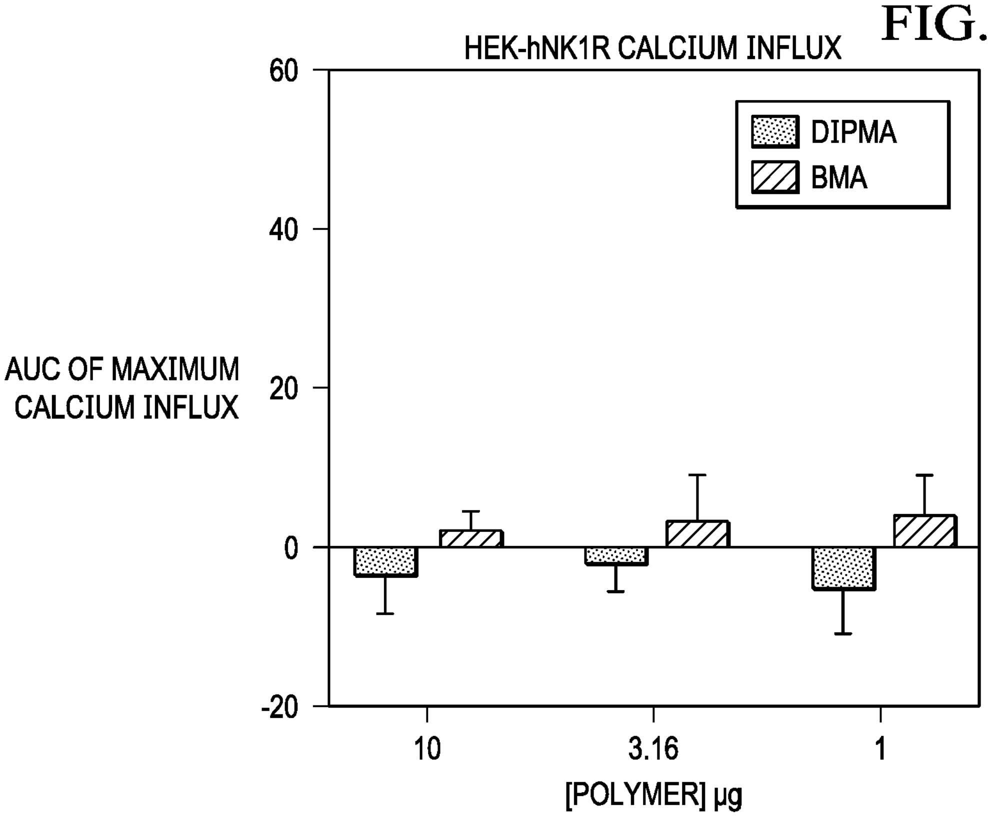
HEK-hNK1R CALCIUM INFLUX
60
40
20
0
-20
AUC OF MAXIMUM CALCIUM INFLUX
DIPMA
BMA
10
3.16
1
[POLYMER] µg

FIG. 7A

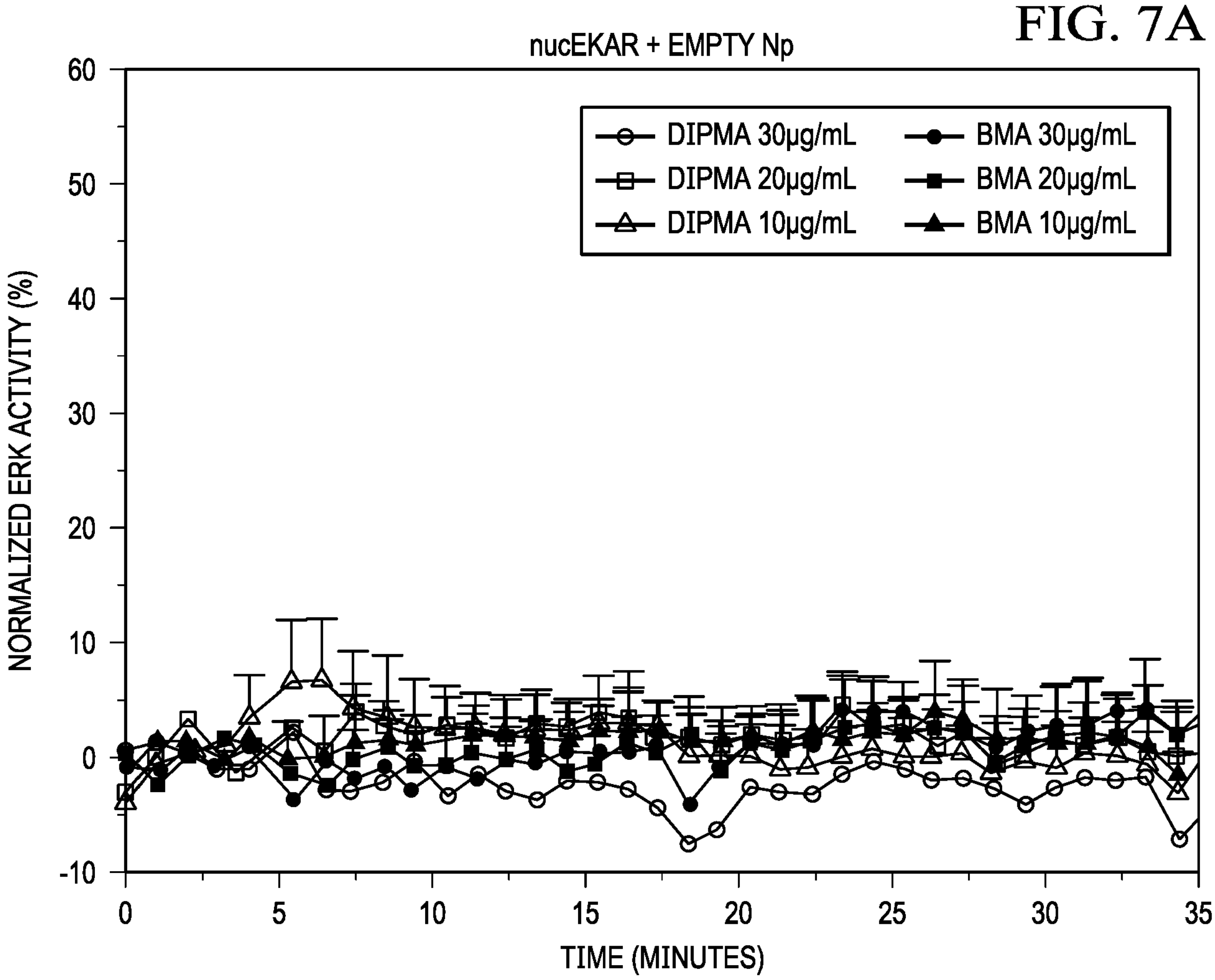
nucEKAR + EMPTY Np
DIPMA 30µg/mL
DIPMA 20µg/mL
DIPMA 10µg/mL
BMA 30µg/mL
BMA 20µg/mL
BMA 10µg/mL
NORMALIZED ERK ACTIVITY (%)
60
50
40
30
20
10
0
-10
0
5
10
15
20
25
30
35
TIME (MINUTES)

FIG. 7B

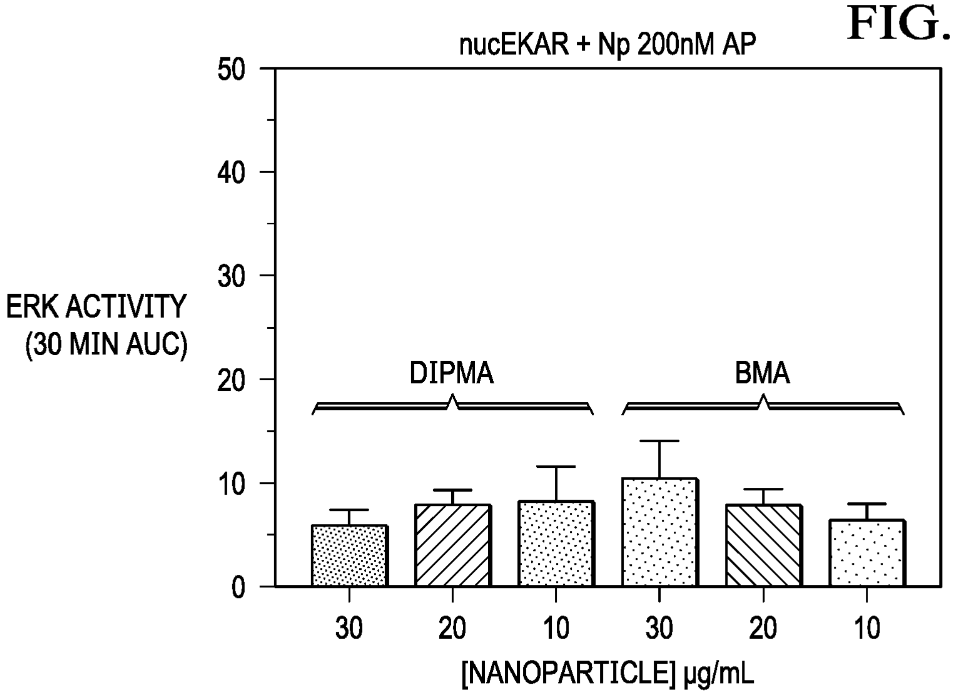
nucEKAR + Np 200nM AP
ERK ACTIVITY
(30 MIN AUC)
50
40
30
20
10
0
DIPMA
BMA
30
20
10
30
20
10
[NANOPARTICLE] µg/mL

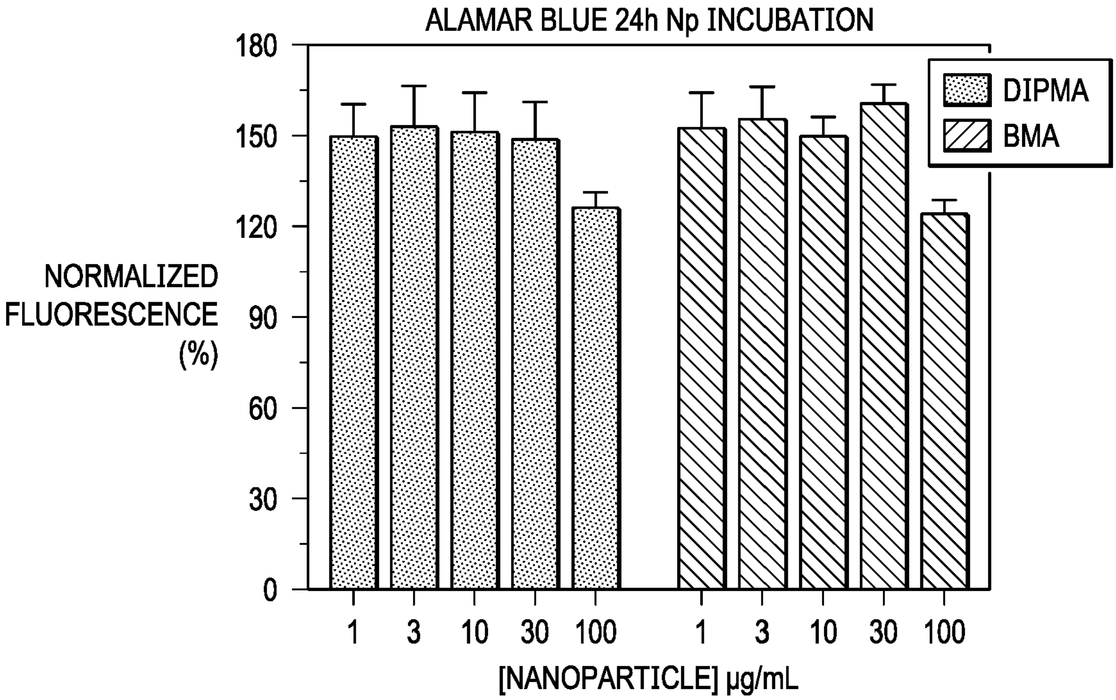
ALAMAR BLUE 24h Np INCUBATION
NORMALIZED FLUORESCENCE (%)
180
150
120
90
60
30
0
DIPMA
BMA
1 3 10 30 100 1 3 10 30 100
[NANOPARTICLE] µg/mL

FIG. 7C

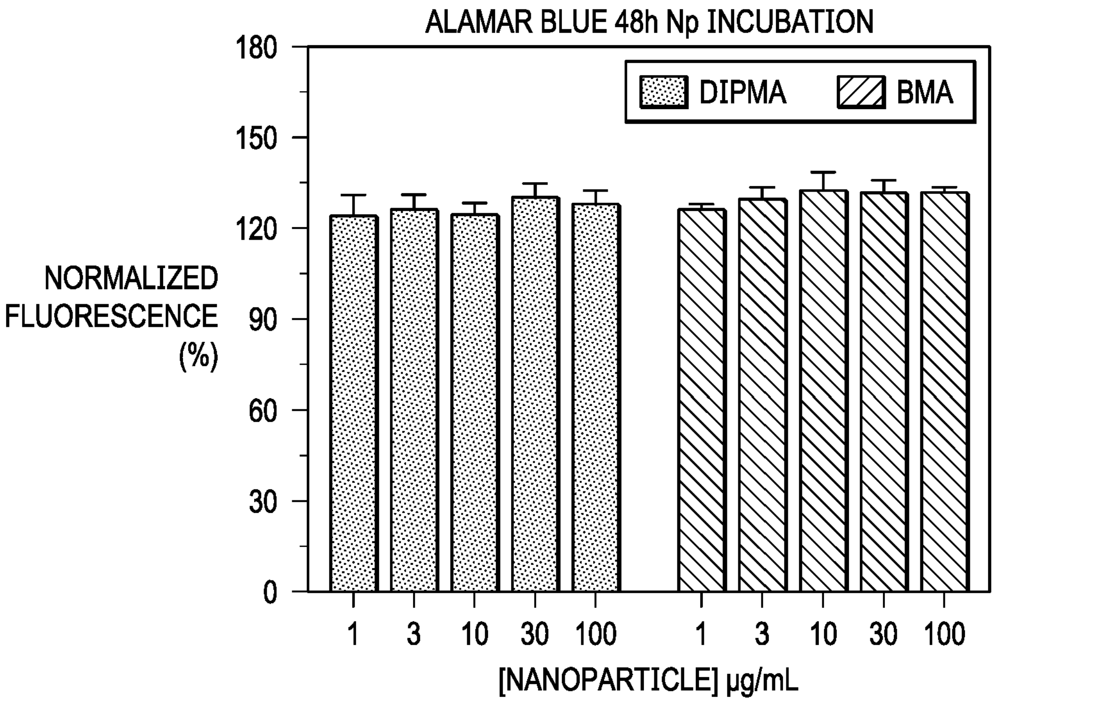
ALAMAR BLUE 48h Np INCUBATION
NORMALIZED FLUORESCENCE (%)
180
150
120
90
60
30
0
DIPMA
BMA
1 3 10 30 100 1 3 10 30 100
[NANOPARTICLE] µg/mL

FIG. 7D

FIG. 8A

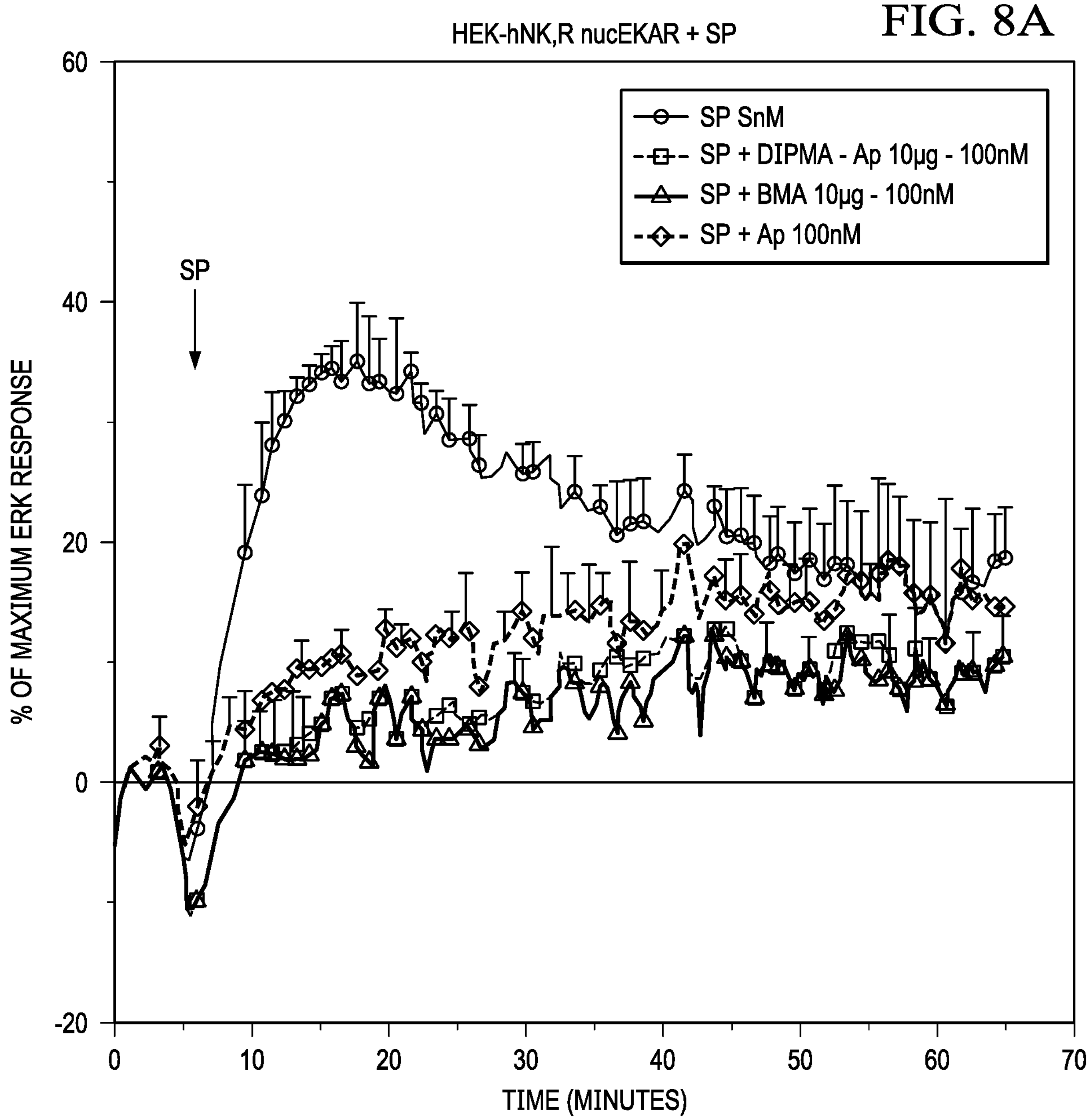
HEK-hNK,R nucEKAR + SP
SP SnM
SP + DIPMA - Ap 10µg - 100nM
SP + BMA 10µg - 100nM
SP + Ap 100nM
SP
% OF MAXIMUM ERK RESPONSE
TIME (MINUTES)
60
40
20
0
-20
0
10
20
30
40
50
60
70

FIG. 8B

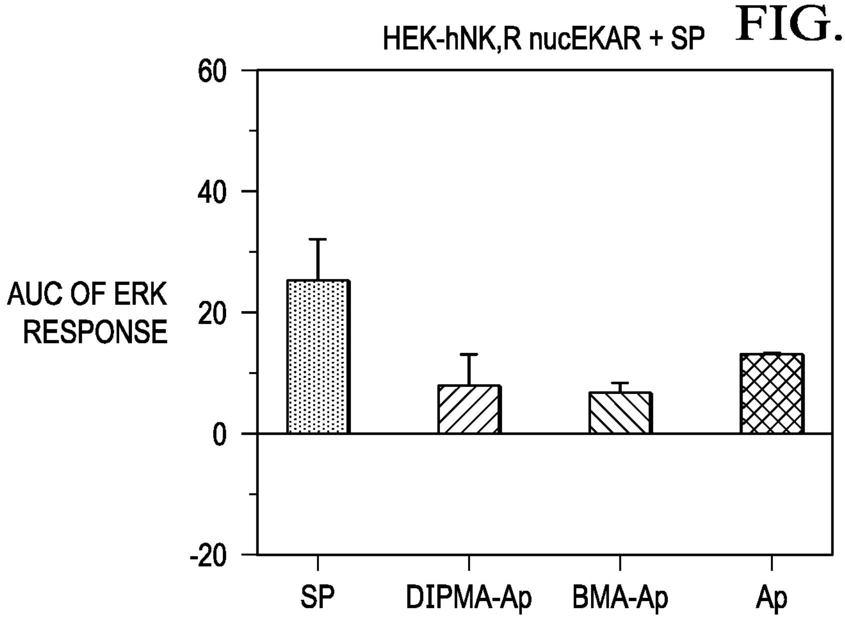
HEK-hNK,R nucEKAR + SP
AUC OF ERK RESPONSE
60
40
20
0
-20
SP
DIPMA-Ap
BMA-Ap
Ap

FIG. 9

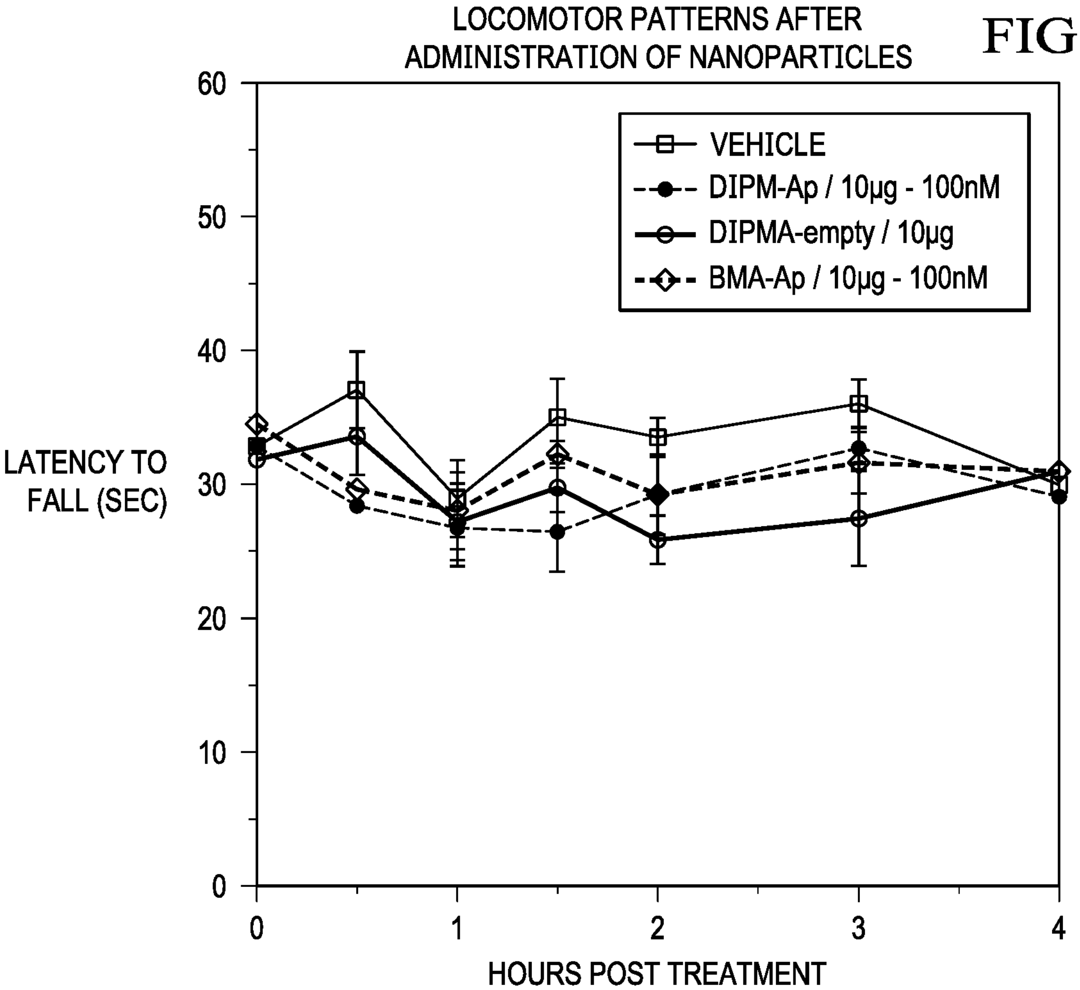
LOCOMOTOR PATTERNS AFTER
ADMINISTRATION OF NANOPARTICLES
VEHICLE
DIPM-Ap / 10µg - 100nM
DIPMA-empty / 10µg
BMA-Ap / 10µg - 100nM
LATENCY TO FALL (SEC)
60
50
40
30
20
10
0
0
1
2
3
4
HOURS POST TREATMENT

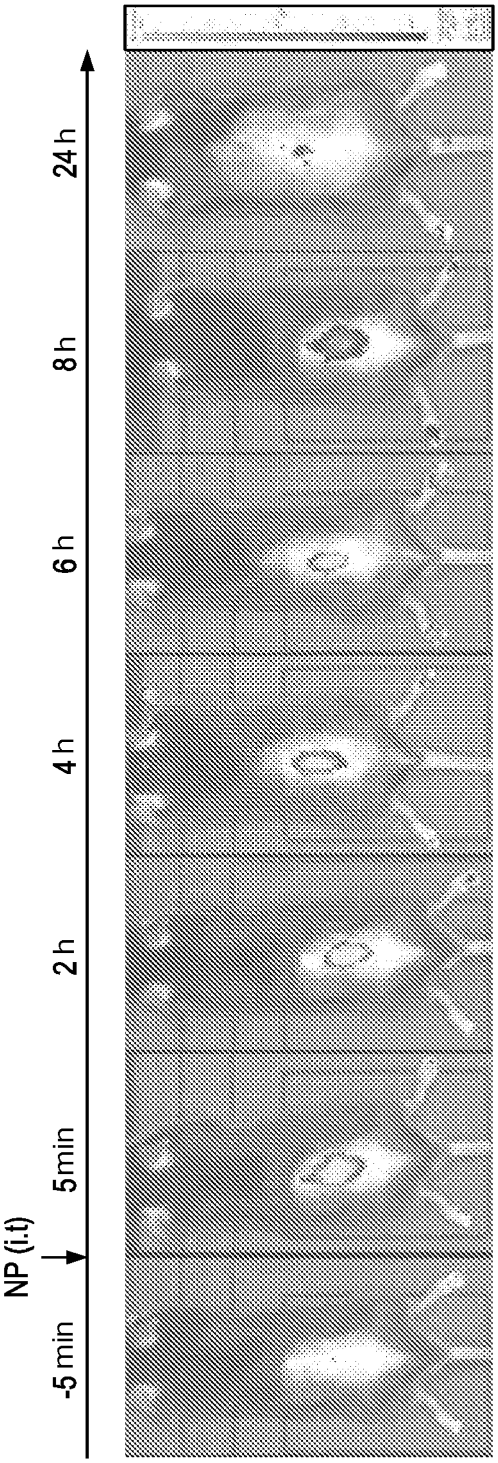


FIG. 10A

FIG. 10B

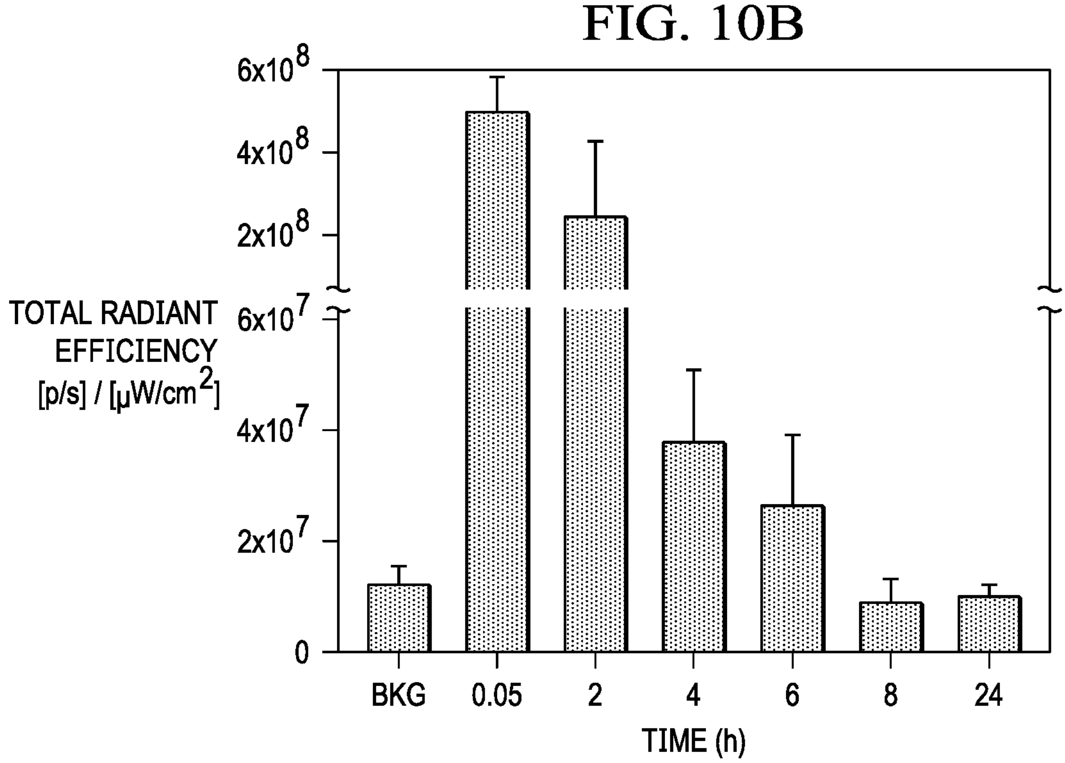


FIG. 10C

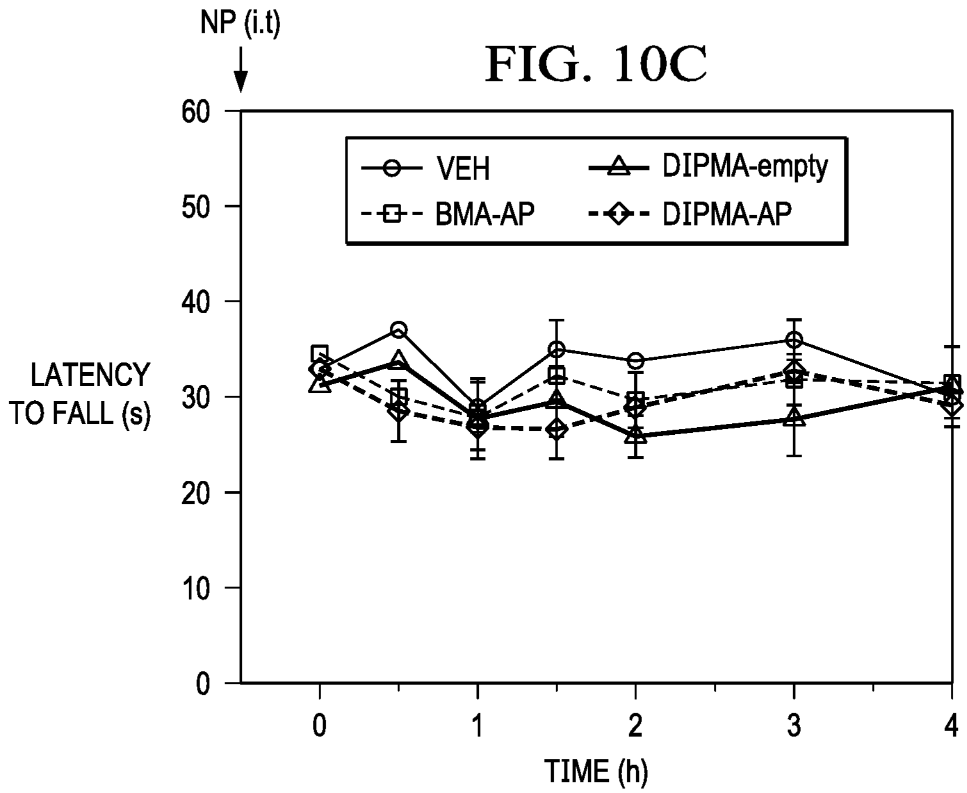

FIG. 11A

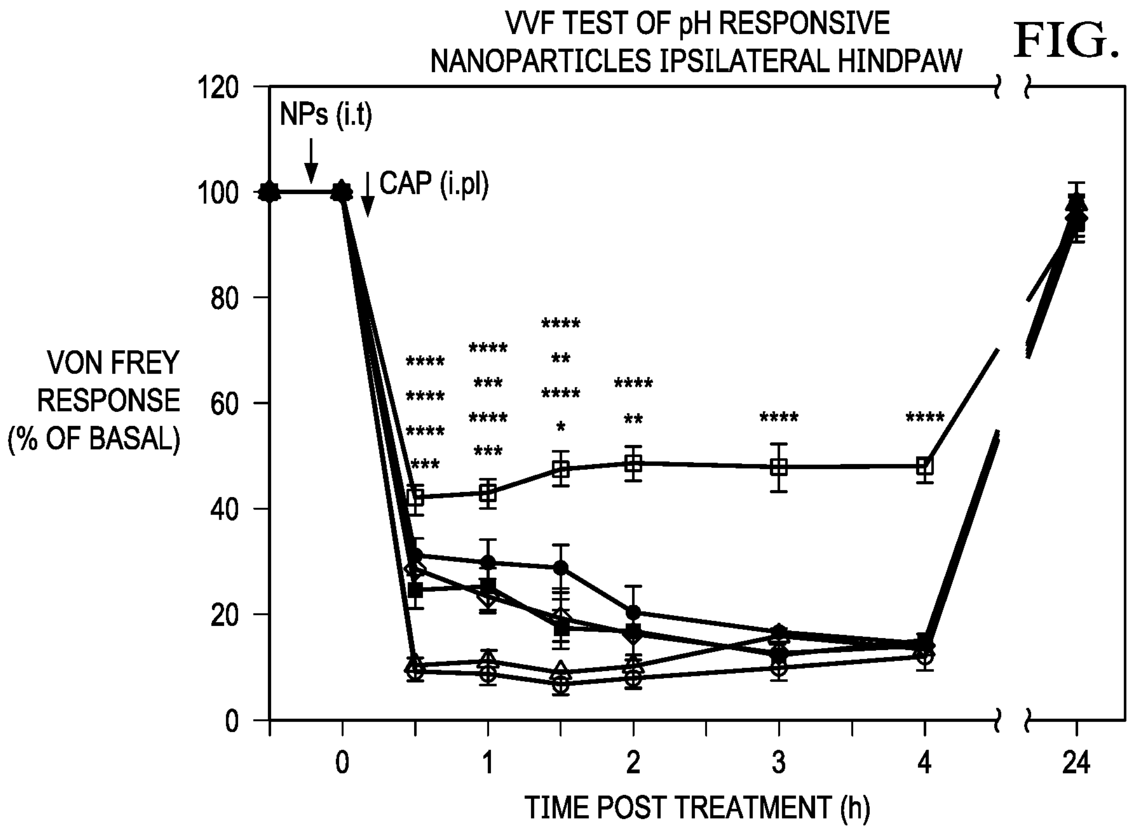


FIG. 11B

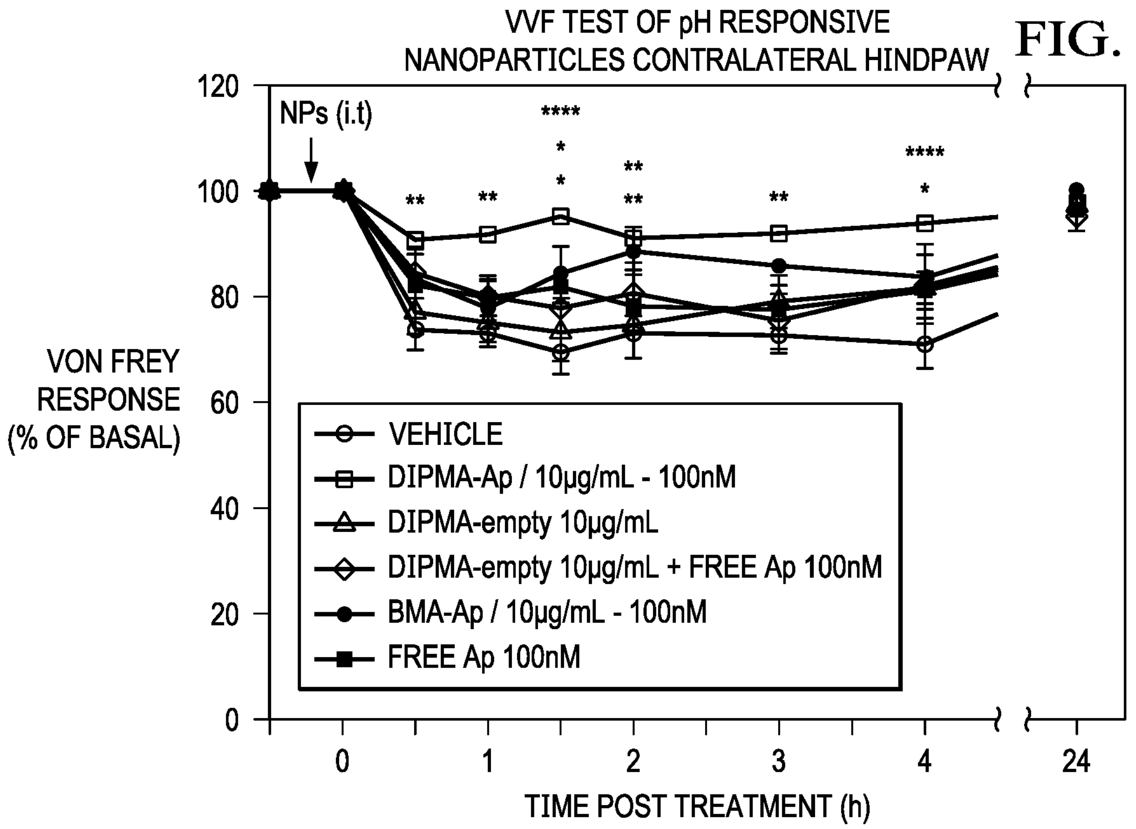

FIG. 12

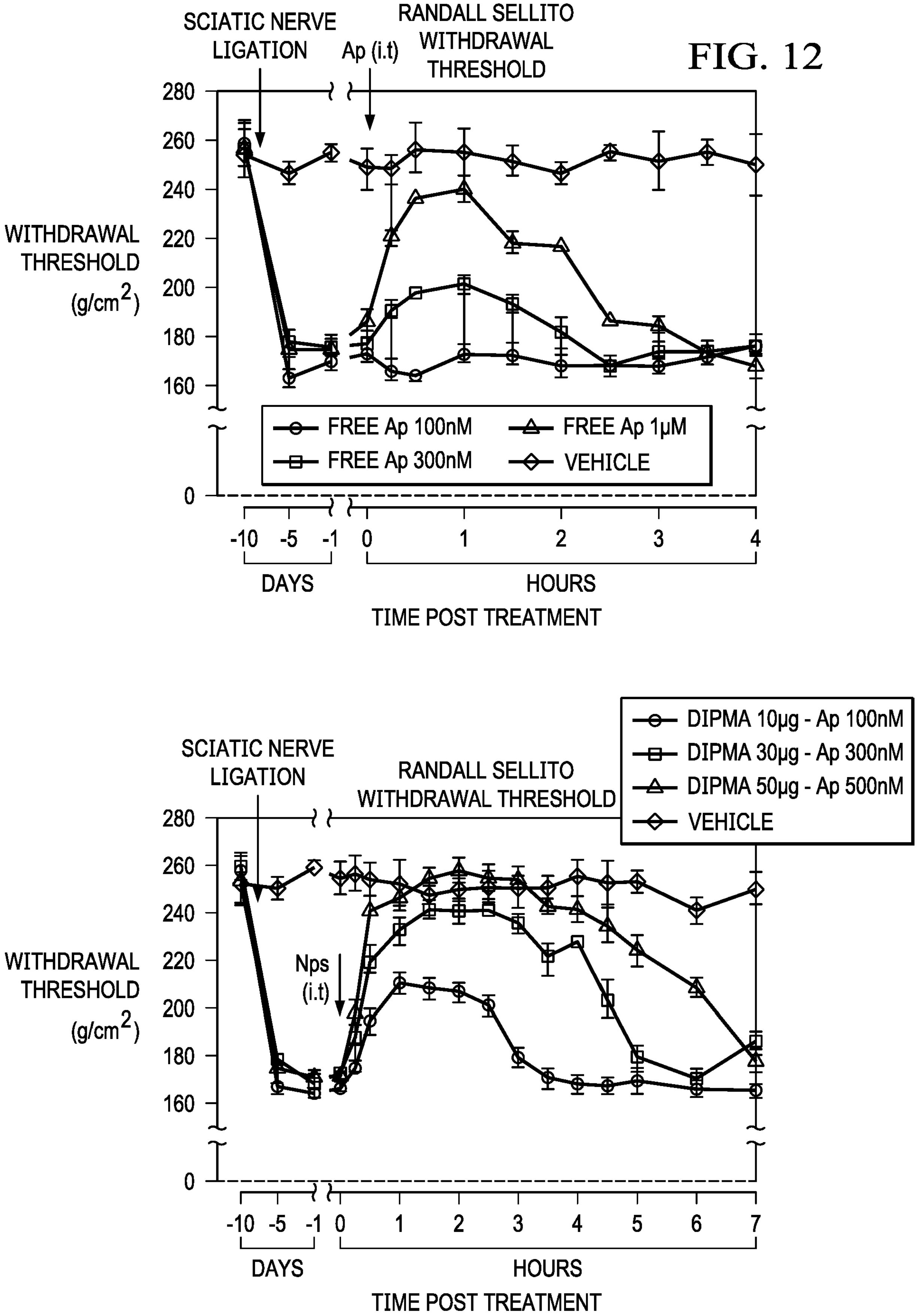
SCIATIC NERVE LIGATION
Ap (i.t)
RANDALL SELLITO WITHDRAWAL THRESHOLD
WITHDRAWAL THRESHOLD (g/cm2)
280
260
240
220
200
180
160
0
FREE Ap 100nM
FREE Ap 1μM
FREE Ap 300nM
VEHICLE
-10 -5 -1 0 1 2 3 4
DAYS
HOURS
TIME POST TREATMENT
DIPMA 10μg - Ap 100nM
DIPMA 30μg - Ap 300nM
DIPMA 50μg - Ap 500nM
VEHICLE
SCIATIC NERVE LIGATION
RANDALL SELLITO WITHDRAWAL THRESHOLD
Nps (i.t)
WITHDRAWAL THRESHOLD (g/cm2)
280
260
240
220
200
180
160
0
-10 -5 -1 0 1 2 3 4 5 6 7
DAYS
HOURS
TIME POST TREATMENT

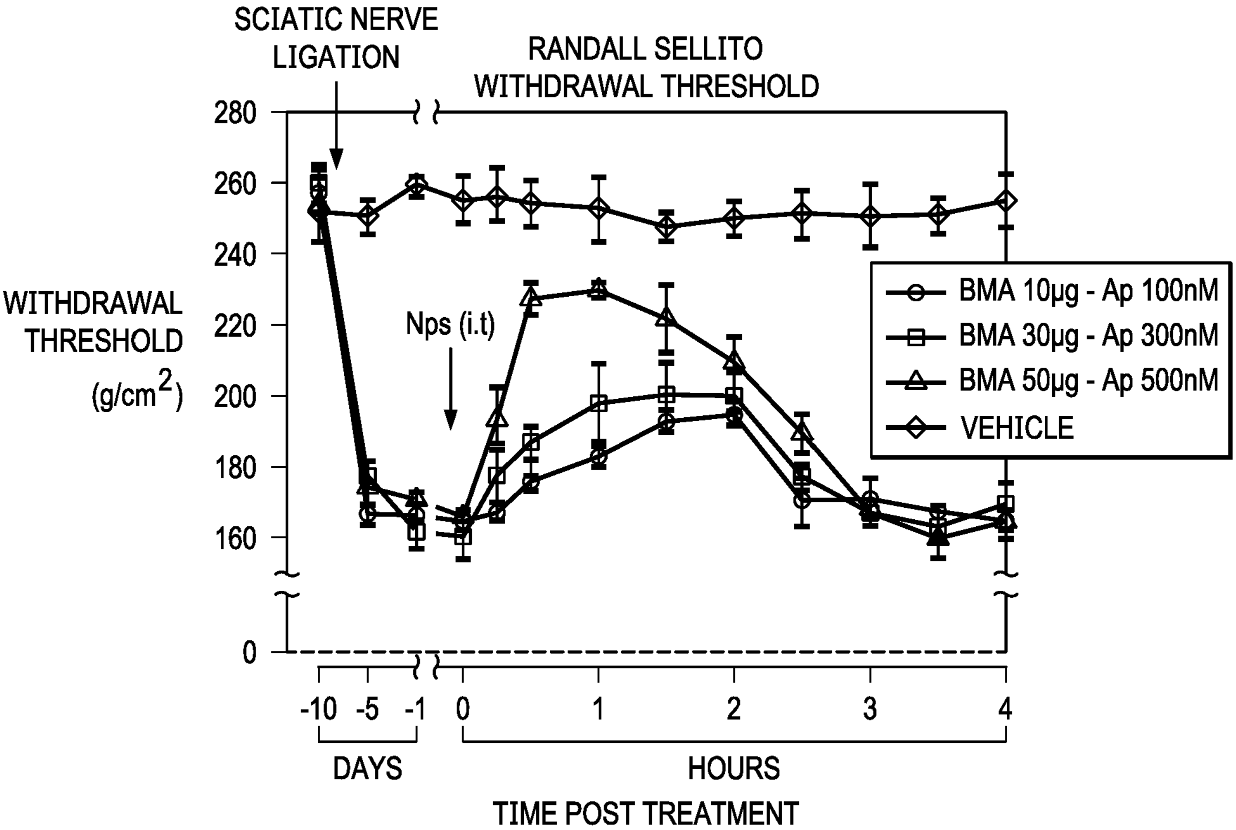


FIG. 12
(Continued)

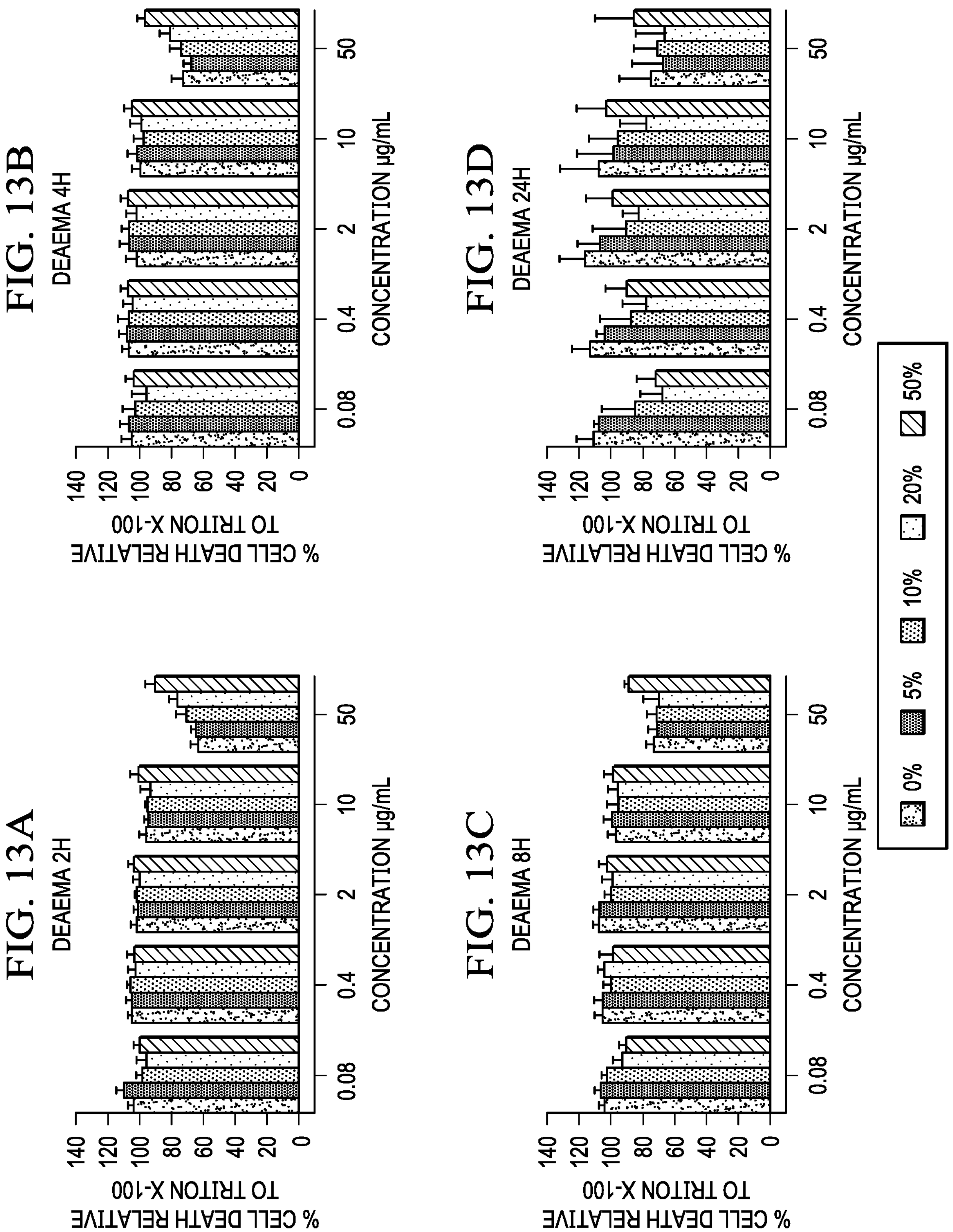
FIG. 13A
DEAEMA 2H
FIG. 13B
DEAEMA 4H
FIG. 13C
DEAEMA 8H
FIG. 13D
DEAEMA 24H
% CELL DEATH RELATIVE TO TRITON X-100
140
120
100
80
60
40
20
0
CONCENTRATION µg/mL
0.08
0.4
2
10
50
0%
5%
10%
20%
50%

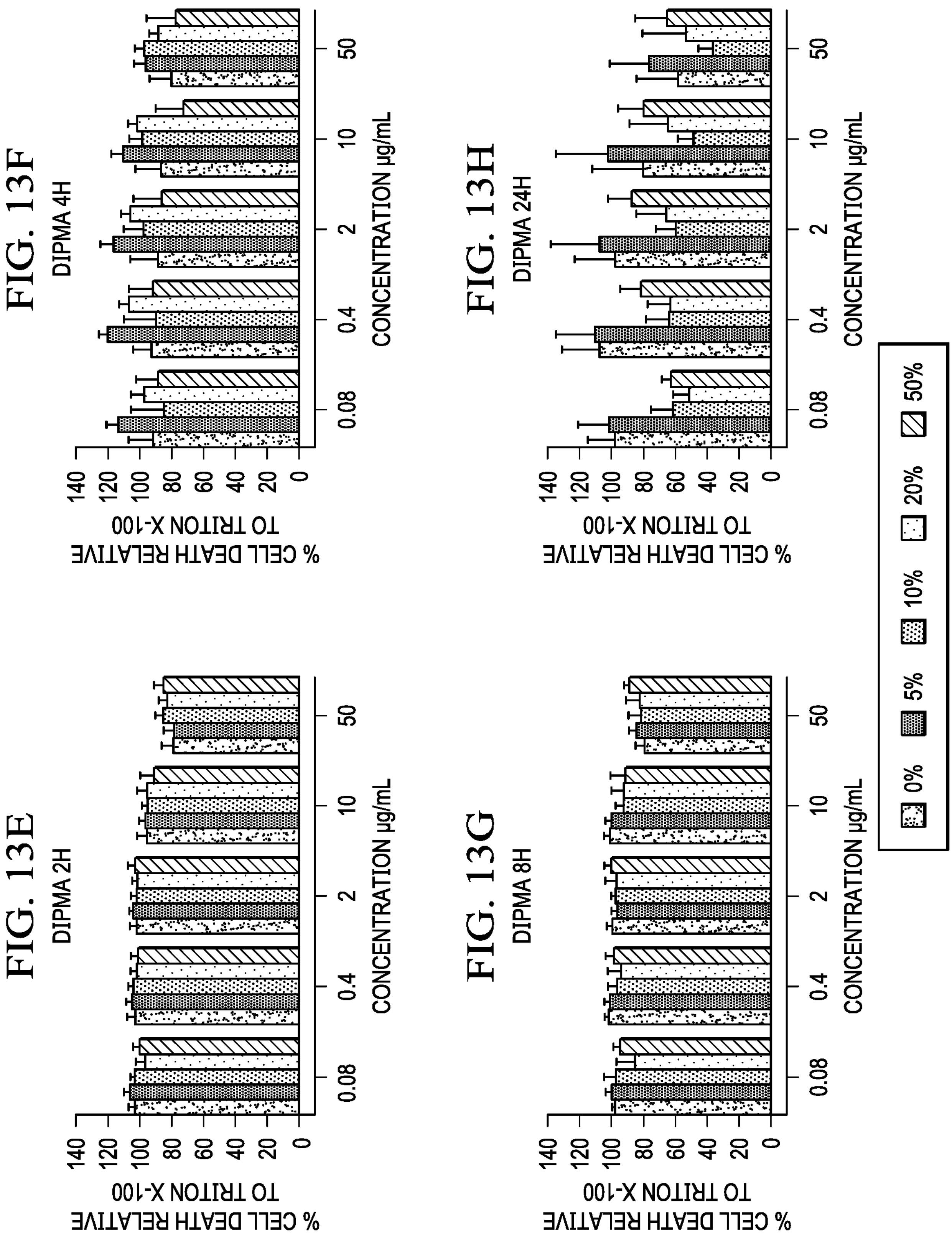
FIG. 13E
DIPMA 2H
FIG. 13F
DIPMA 4H
FIG. 13G
DIPMA 8H
FIG. 13H
DIPMA 24H
% CELL DEATH RELATIVE TO TRITON X-100
0
20
40
60
80
100
120
140
CONCENTRATION μg/mL
0.08
0.4
2
10
50
0%
5%
10%
20%
50%

FIG. 14A

DEAEMA 2H

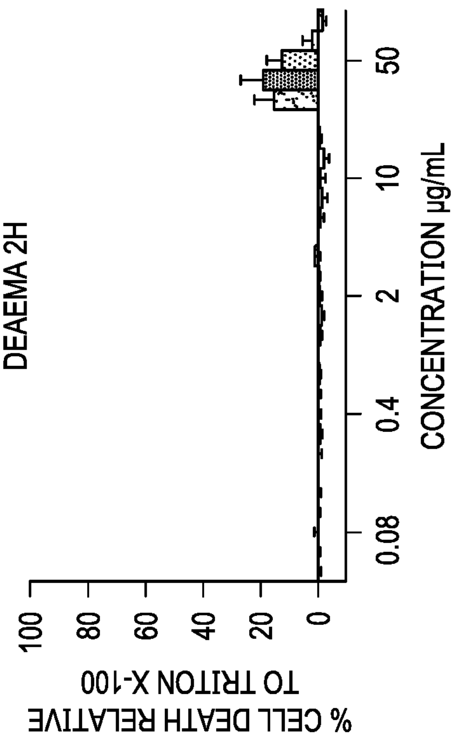


FIG. 14B

DEAEMA 4H

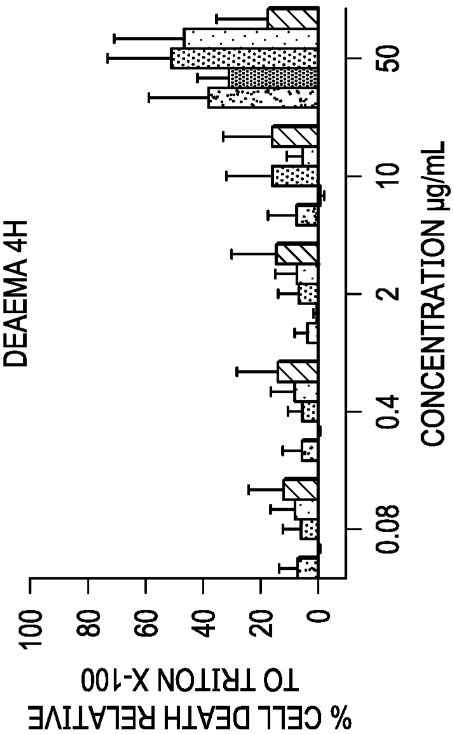


FIG. 14C

DEAEMA 8H

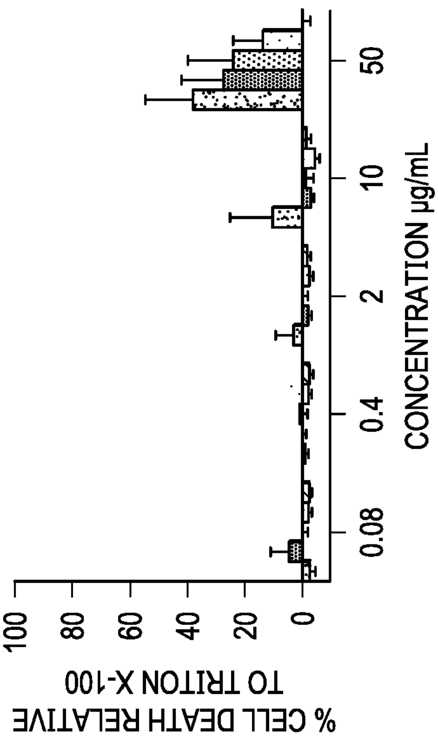


FIG. 14D

DEAEMA 24H

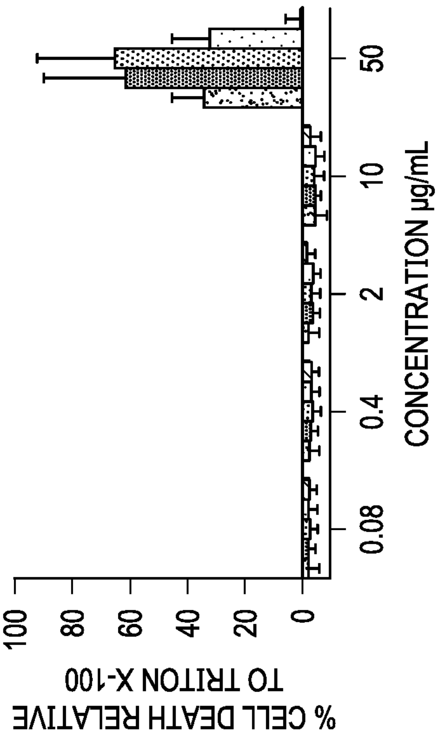


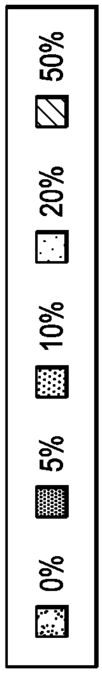

FIG. 14E

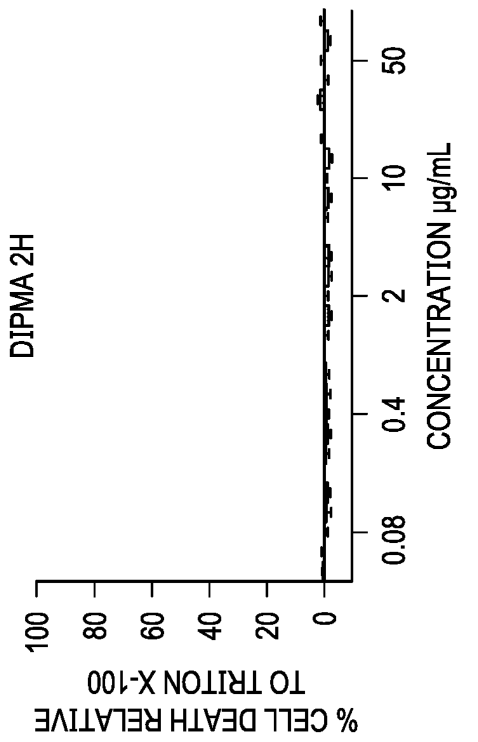


FIG. 14F

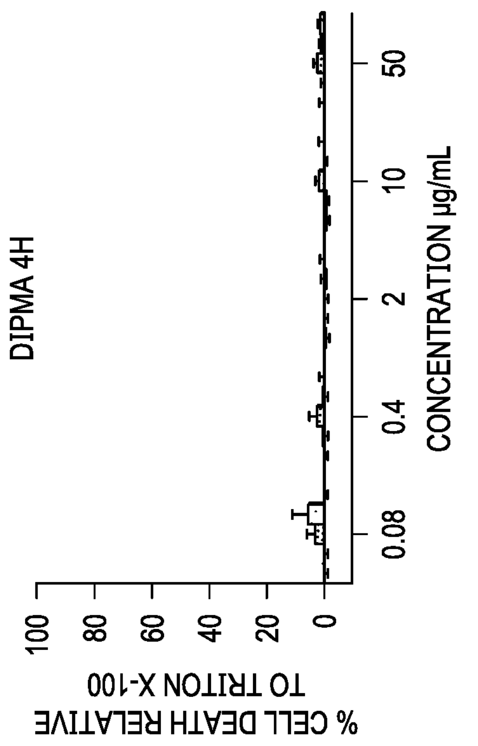


FIG. 14G

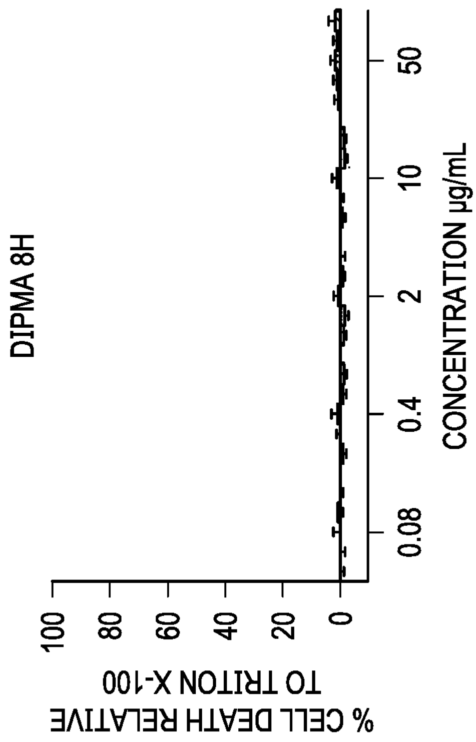


FIG. 14H

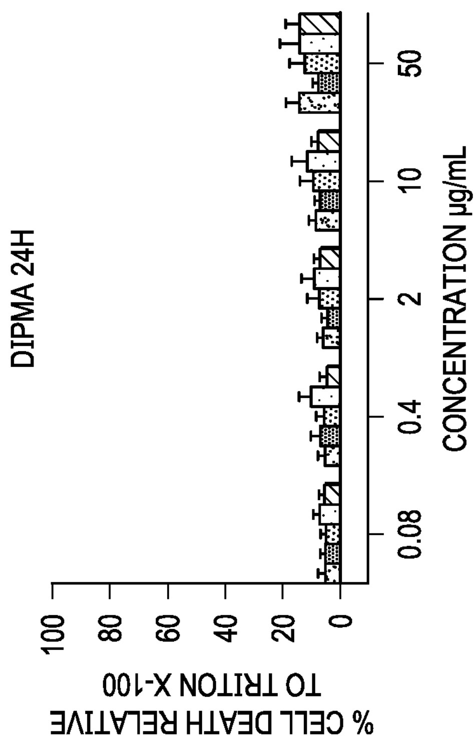


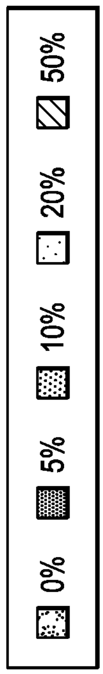

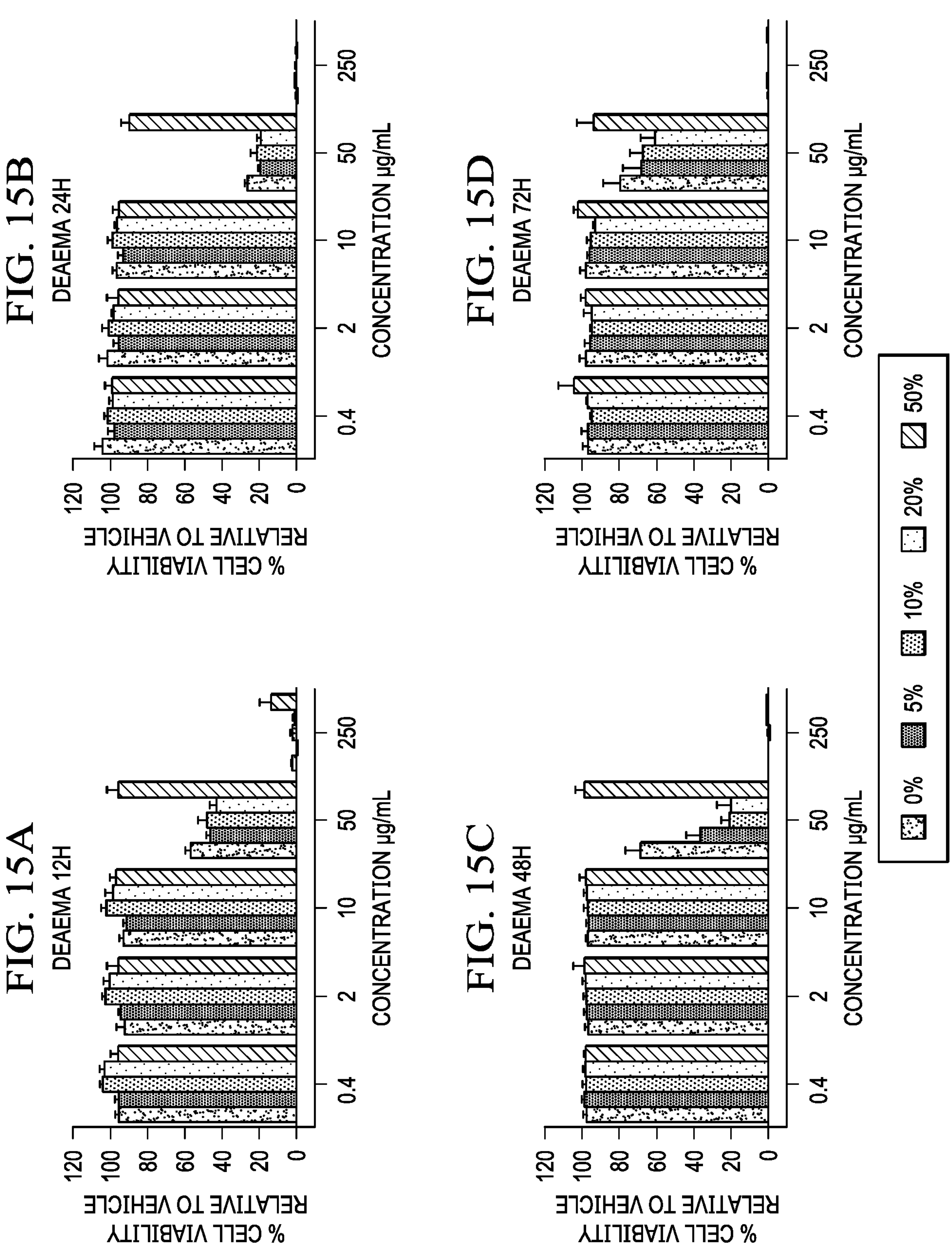


FIG. 15A

FIG. 15B

FIG. 15C

FIG. 15D

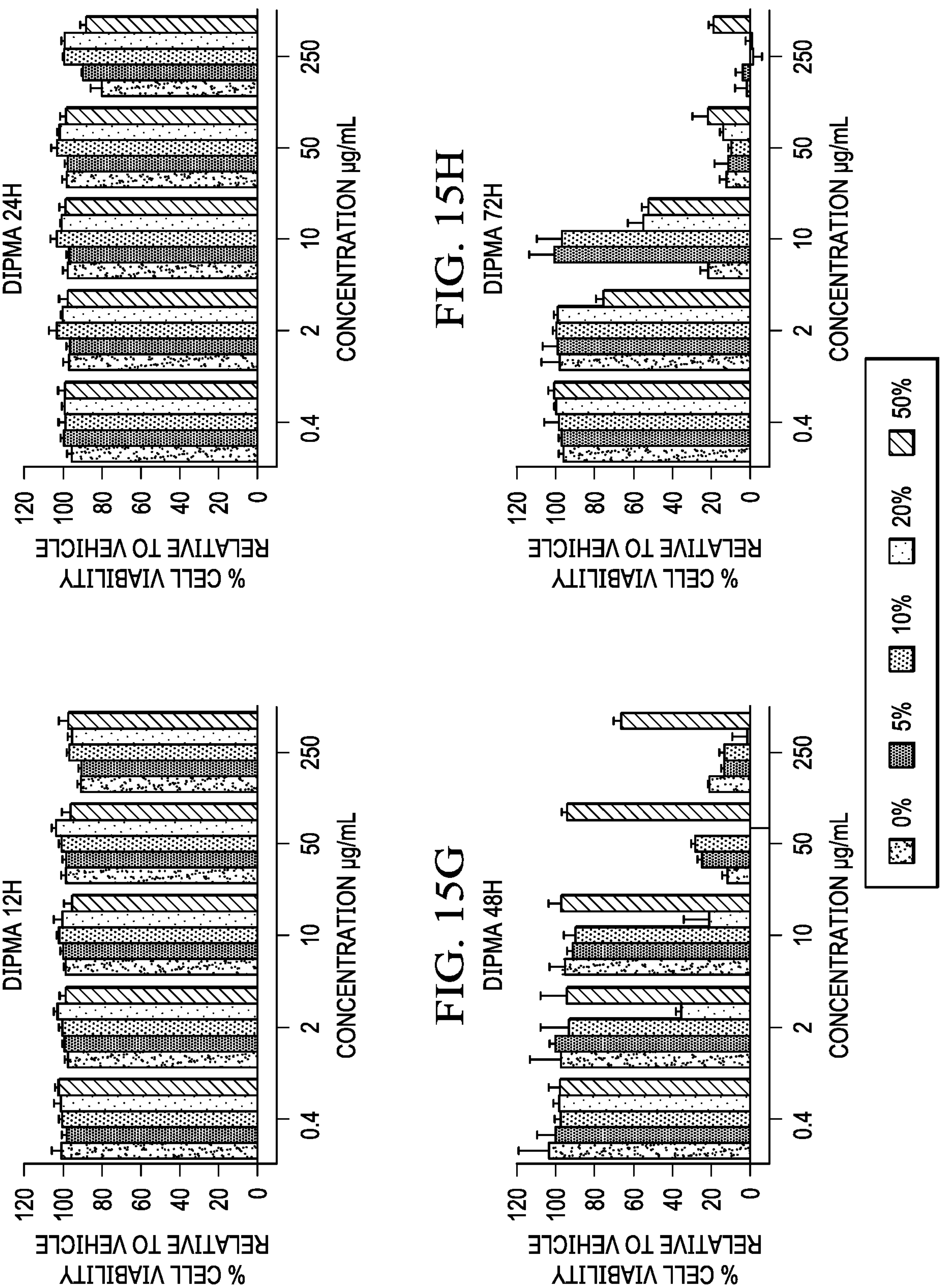


FIG. 15E

FIG. 15F

FIG. 15G

FIG. 15H

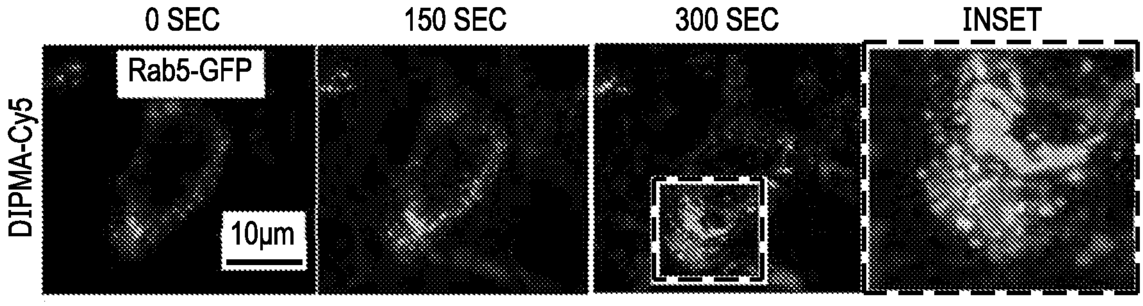


FIG. 16A

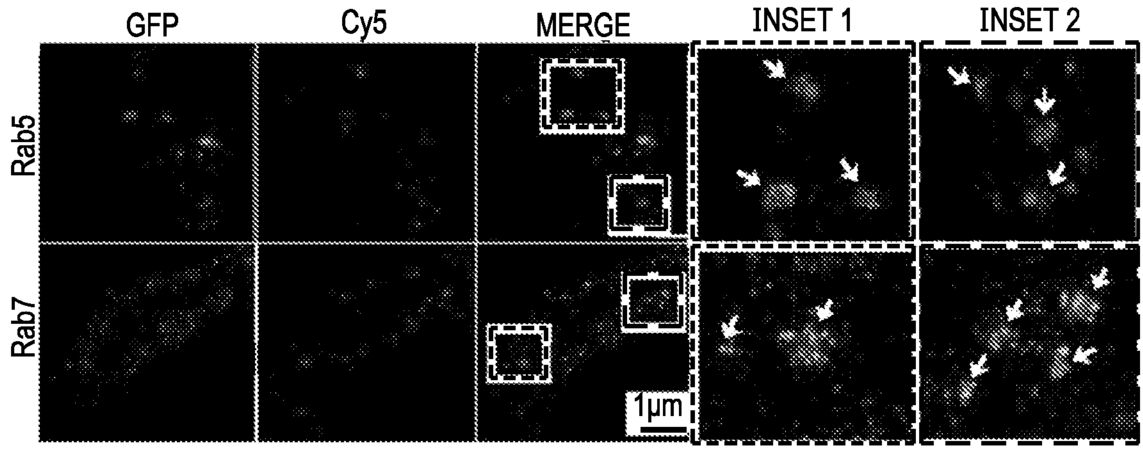


FIG. 16B

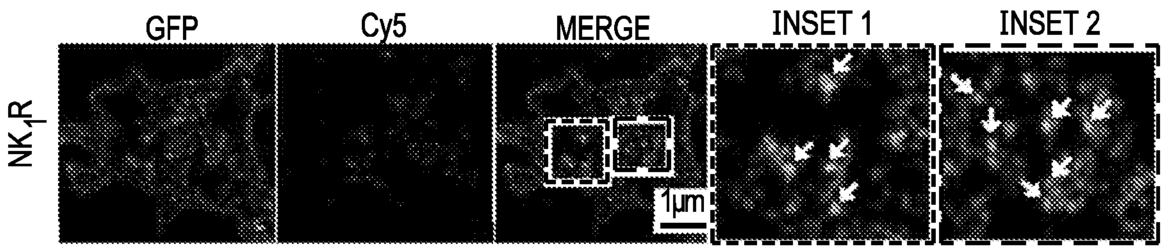


FIG. 16C

FIG. 16D

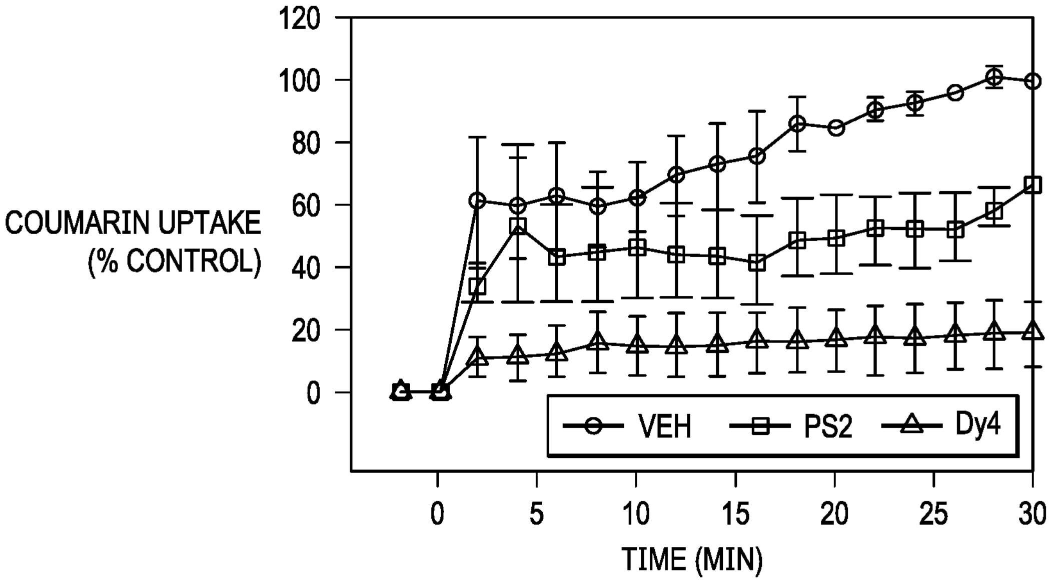


FIG. 16E

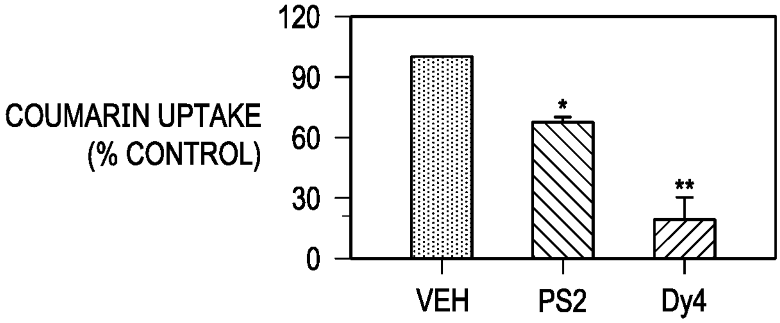


FIG. 16F

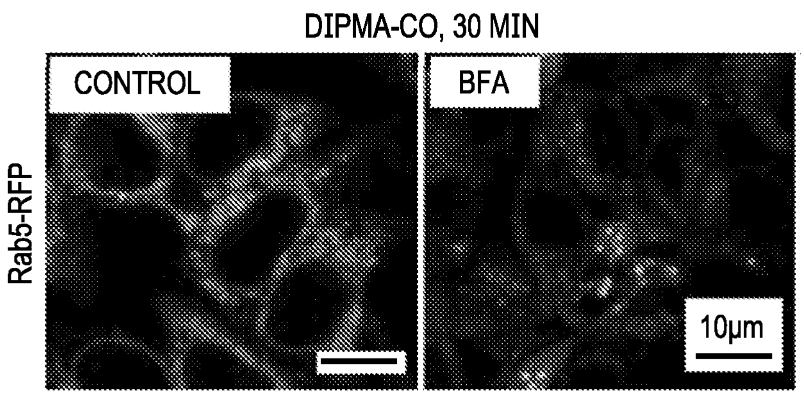

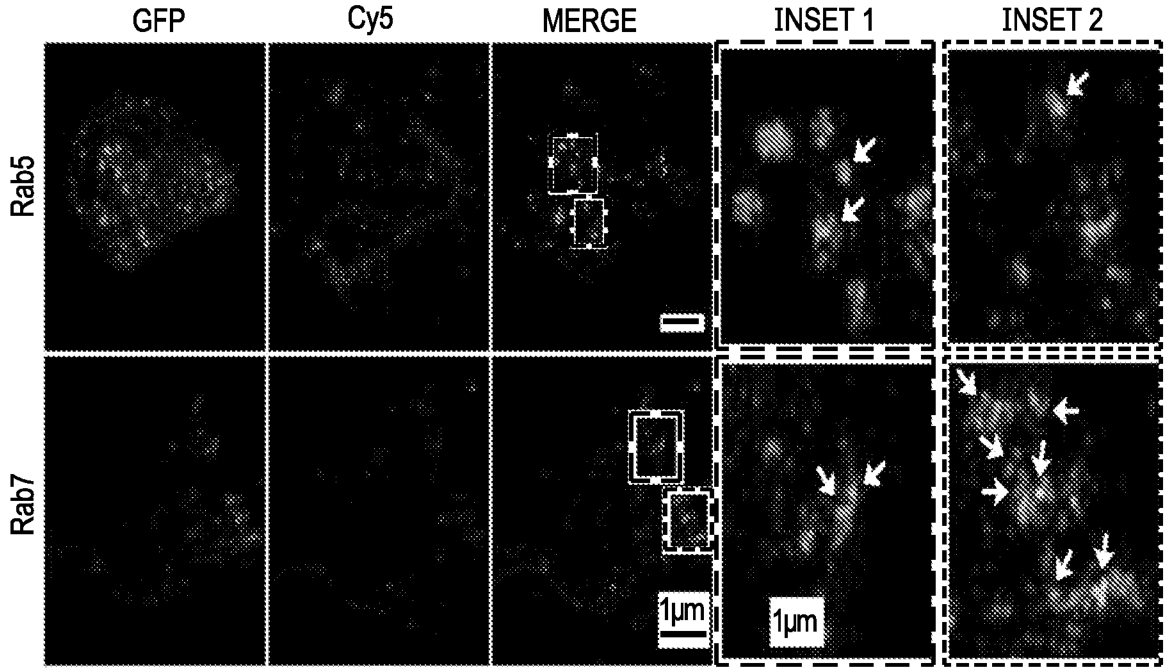


FIG. 17A

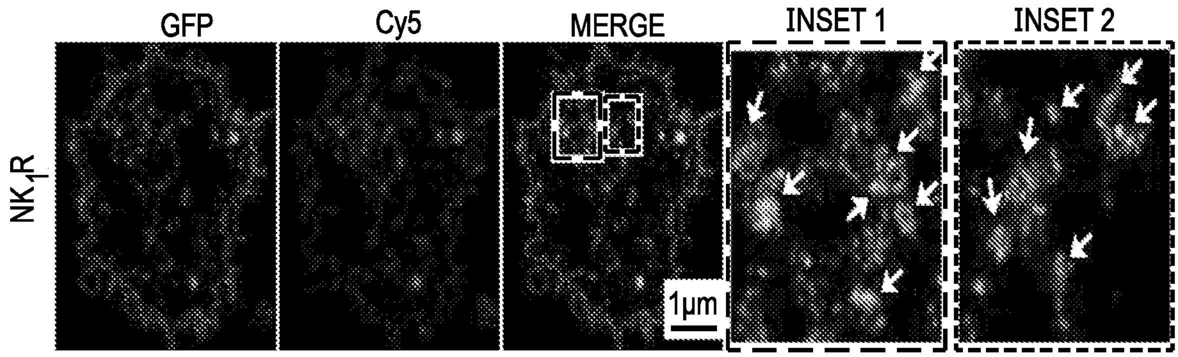


FIG. 17B

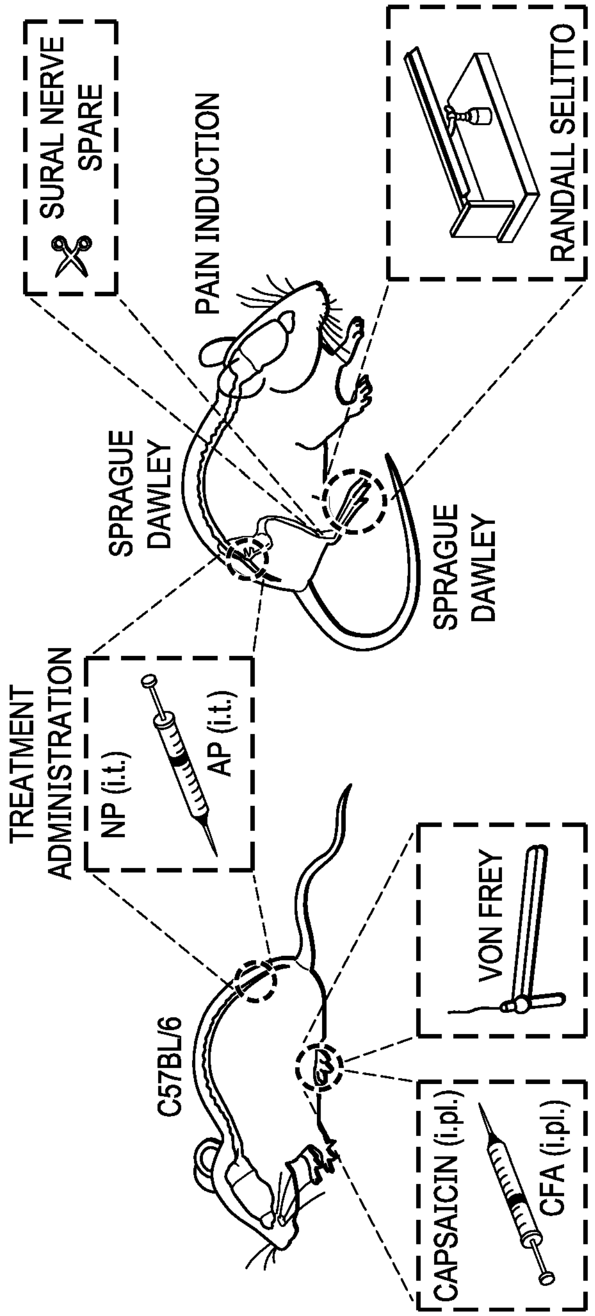
C57BL/6
CAPSAICIN (i.pl.)
CFA (i.pl.)
PAIN INDUCTION
VON FREY
BEHAVIOUR ASSESSMENT
TREATMENT ADMINISTRATION
NP (i.t.)
AP (i.t.)
SPRAGUE DAWLEY
SURAL NERVE SPARE
PAIN INDUCTION
SPRAGUE DAWLEY
RANDALL SELITTO
BEHAVIOUR ASSESSMENT

FIG. 18A

FIG. 18B

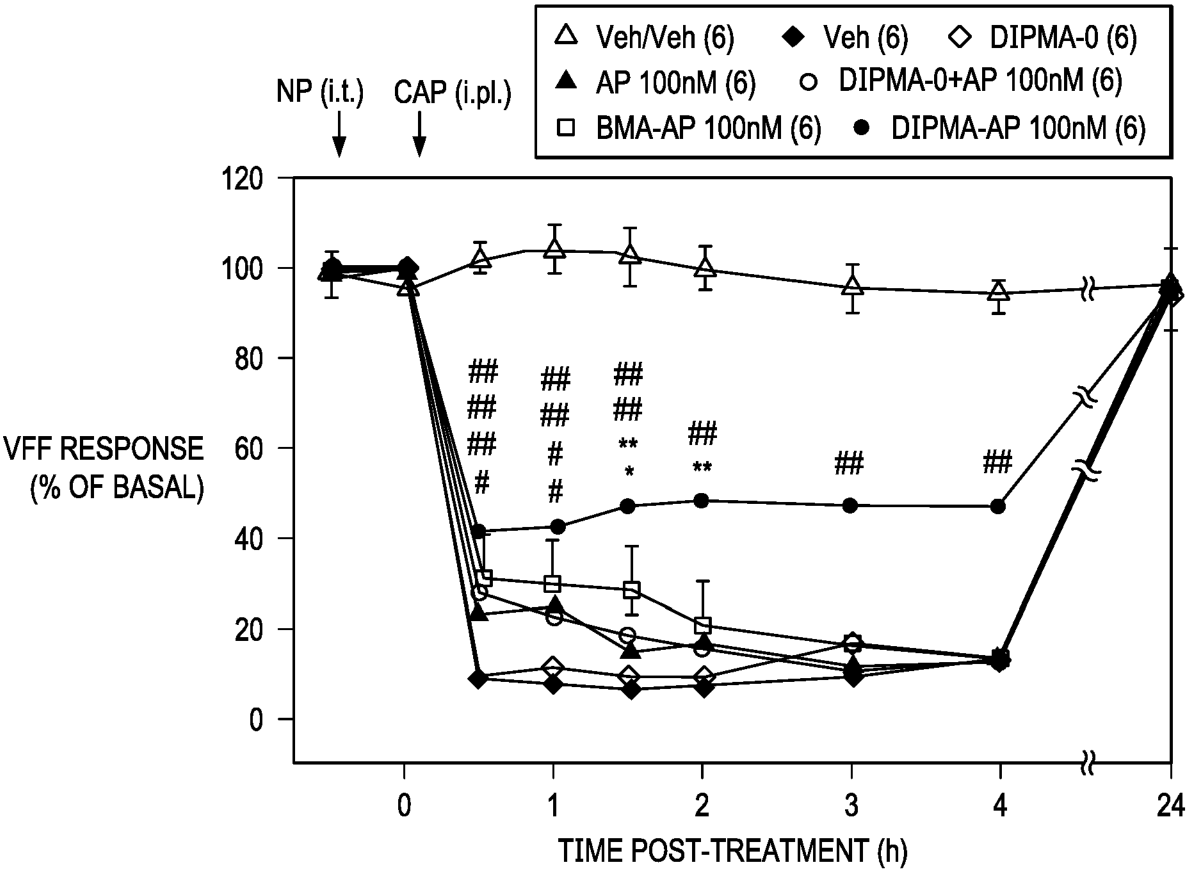


FIG. 18C

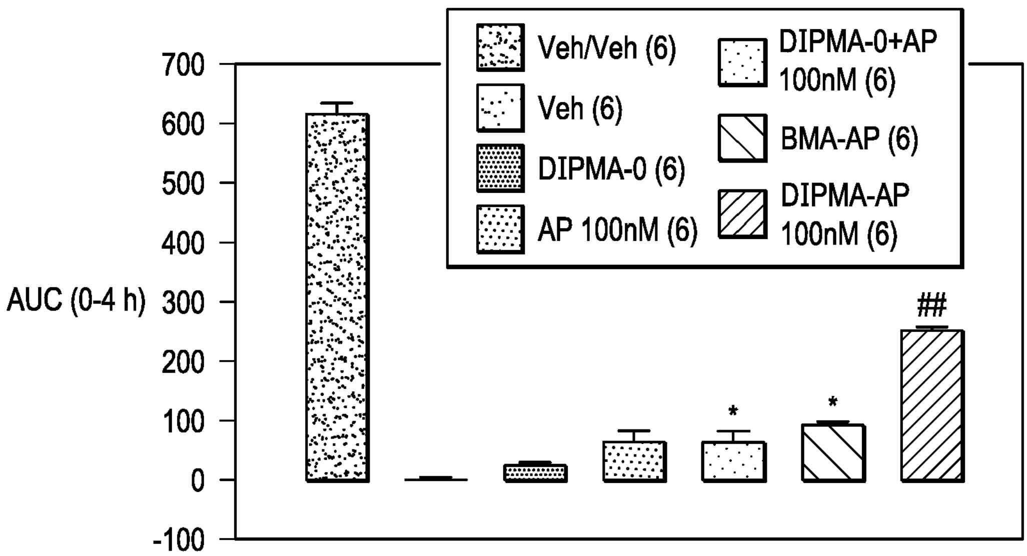

FIG. 18D

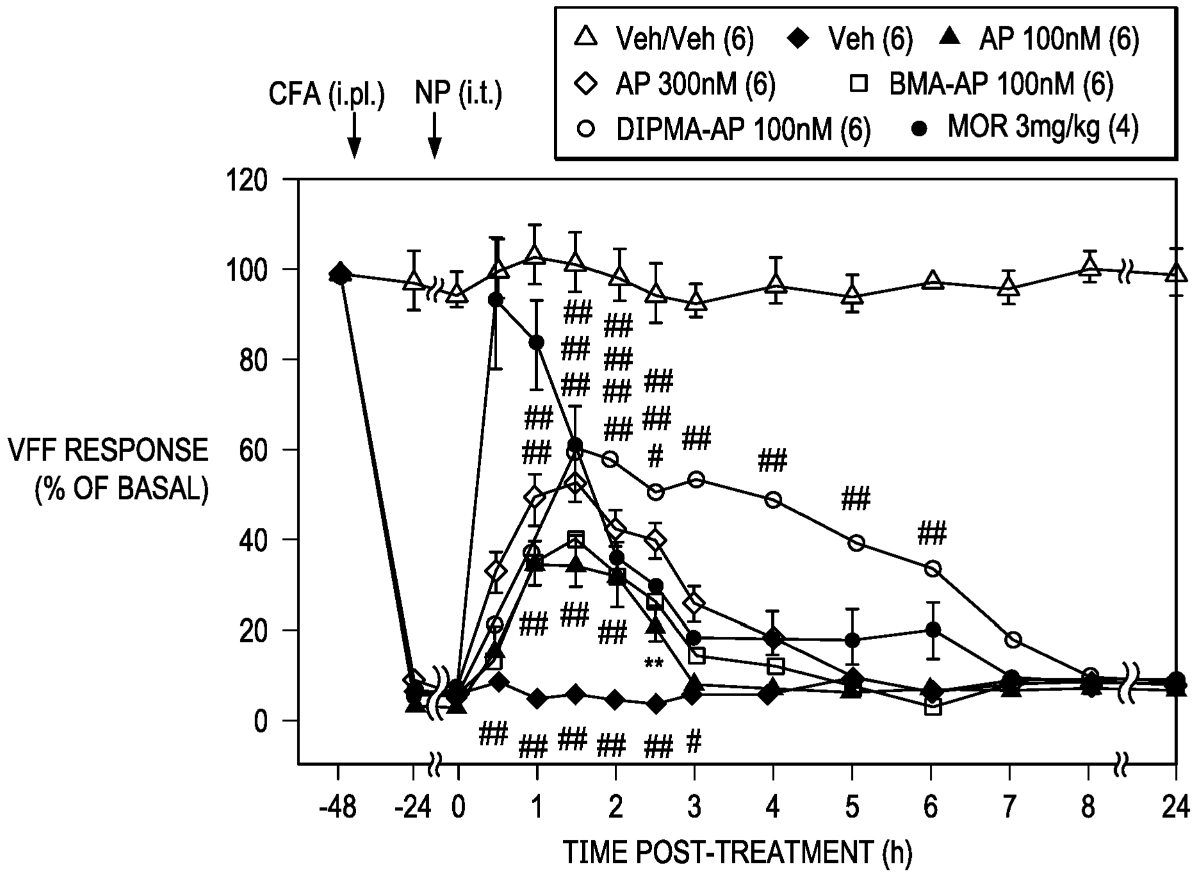


FIG. 18E

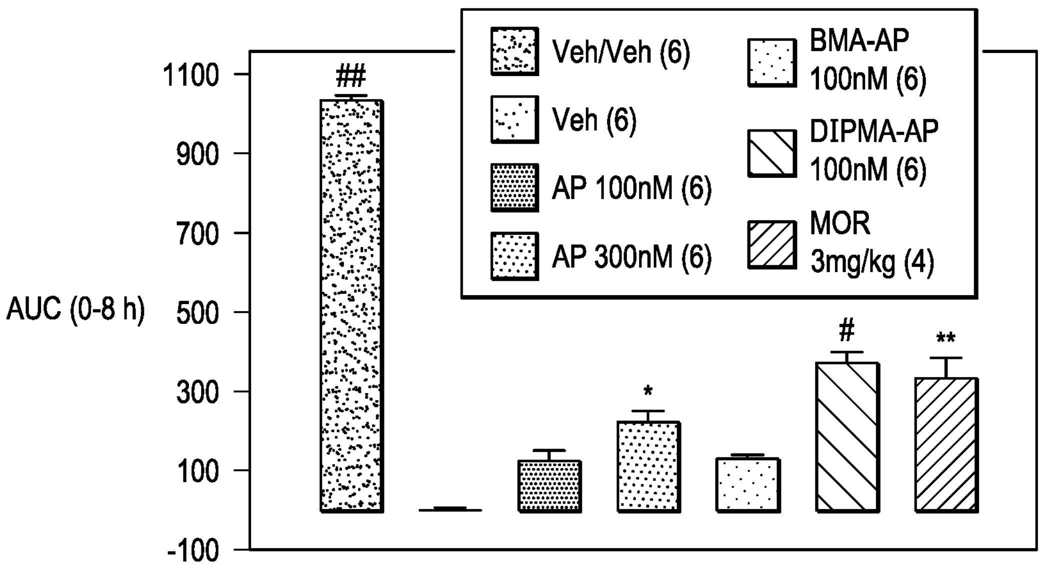

FIG. 18F

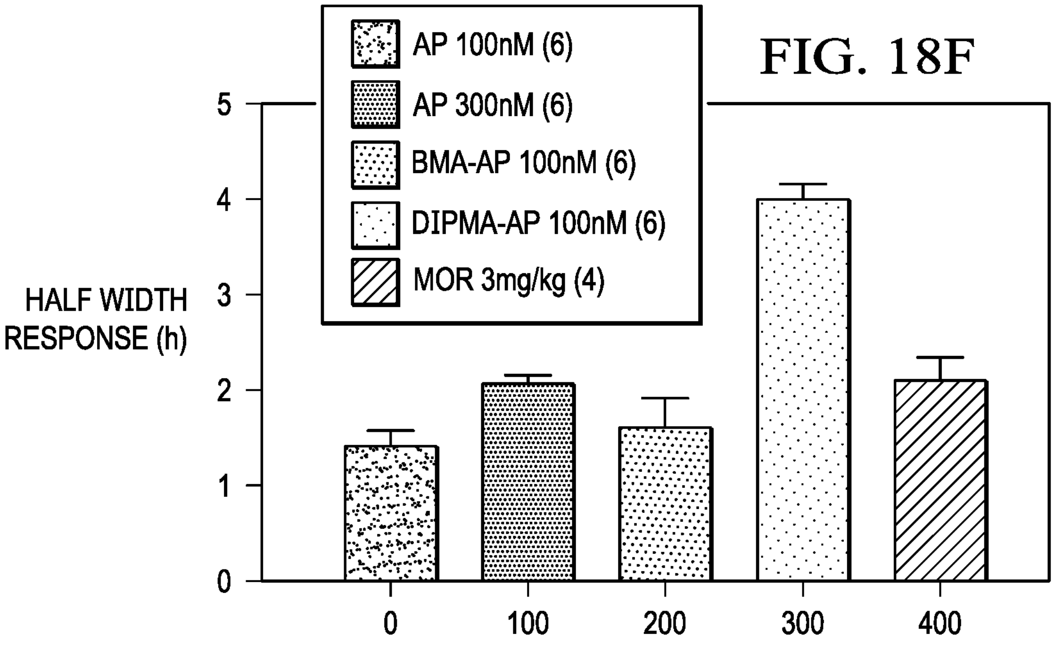


FIG. 18G

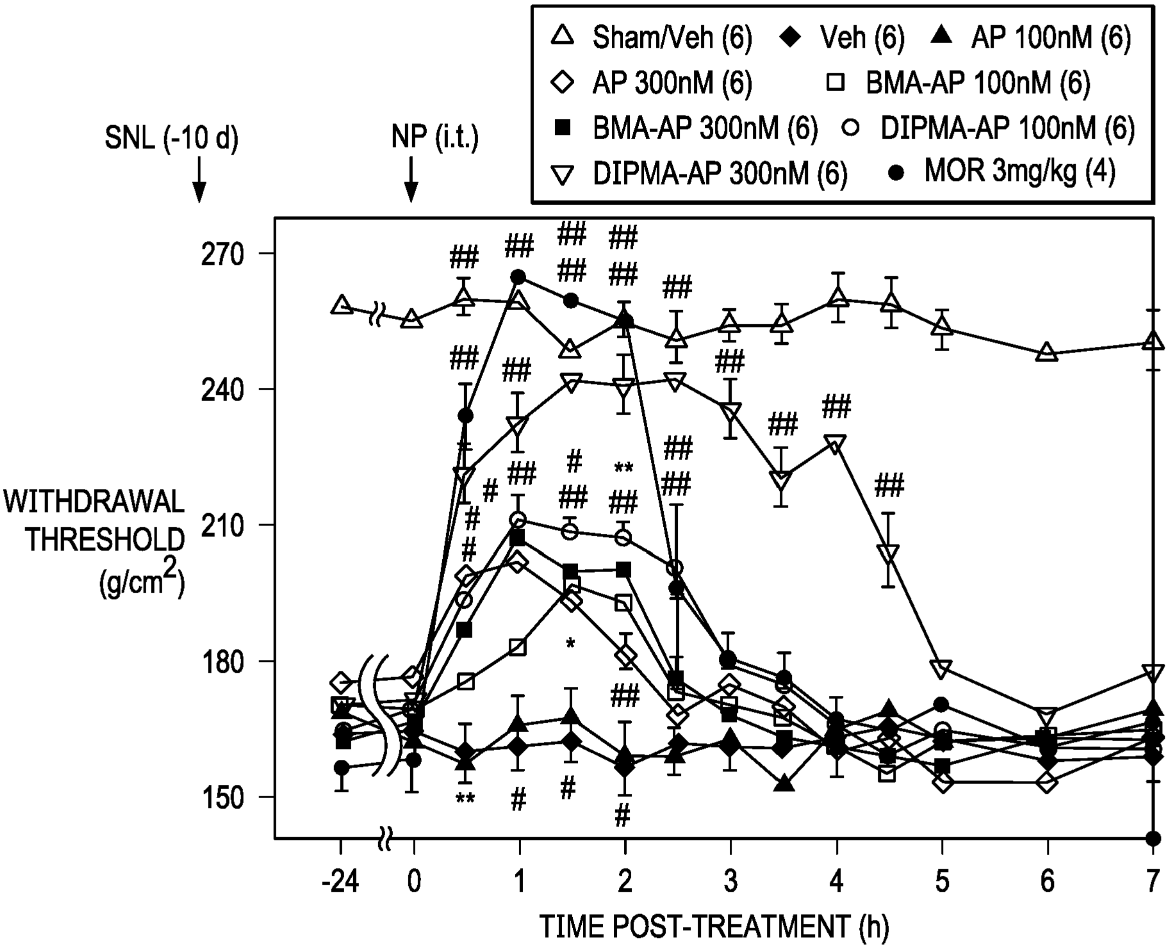

FIG. 18H

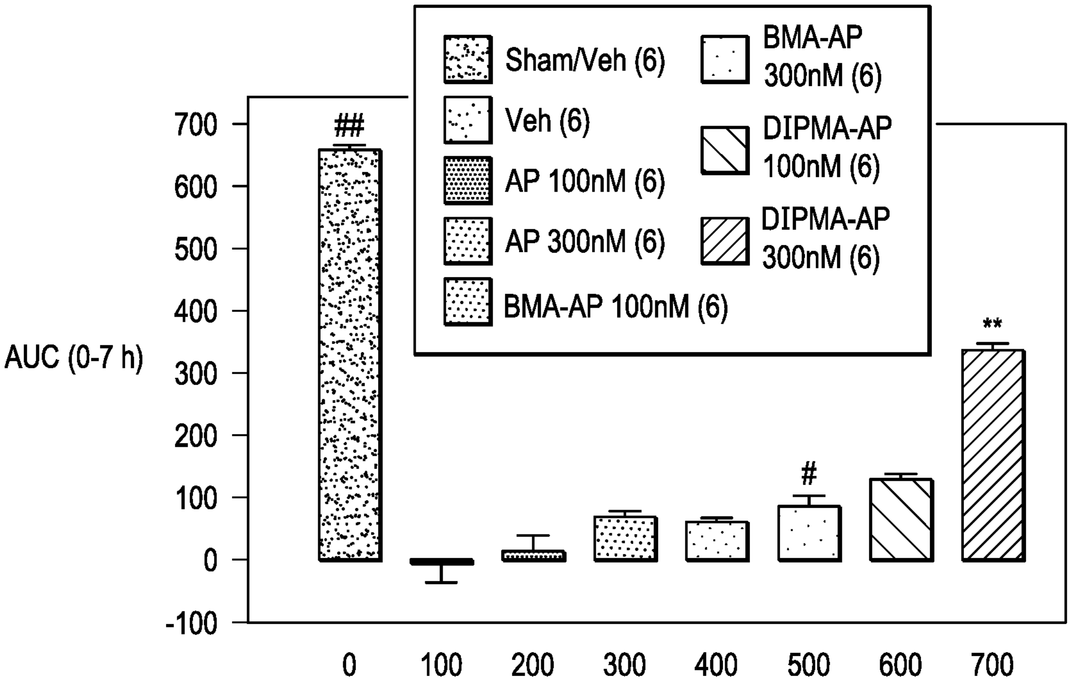


FIG. 18I

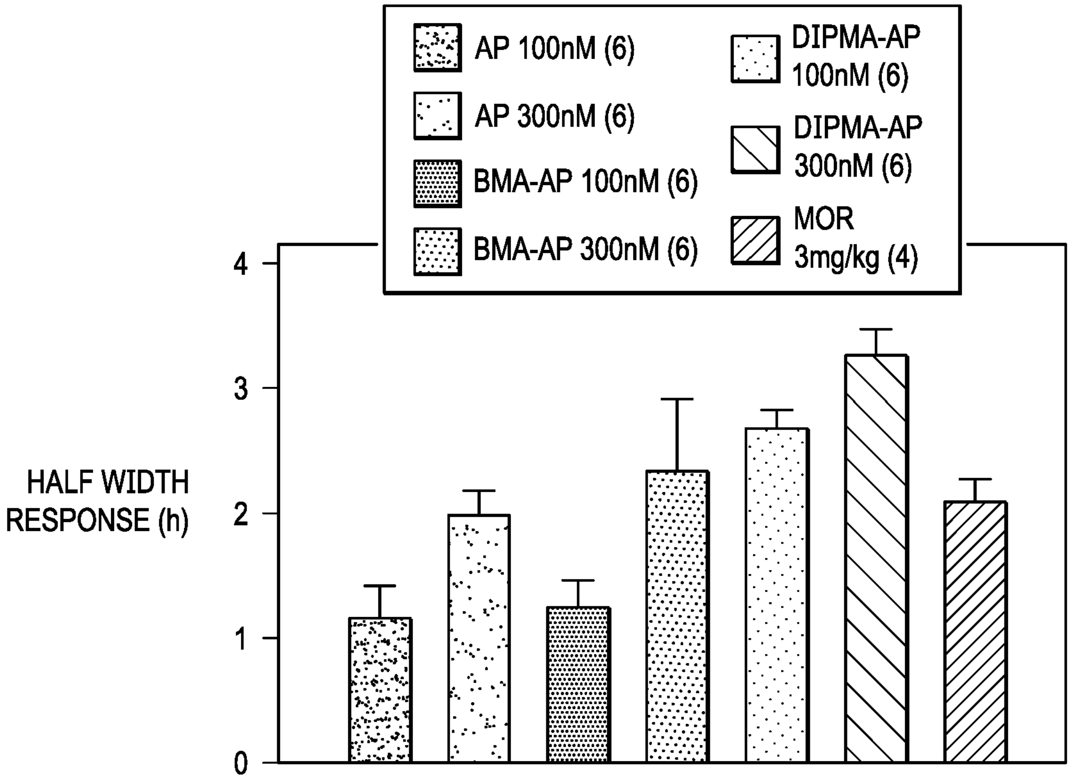

FIG. 19A

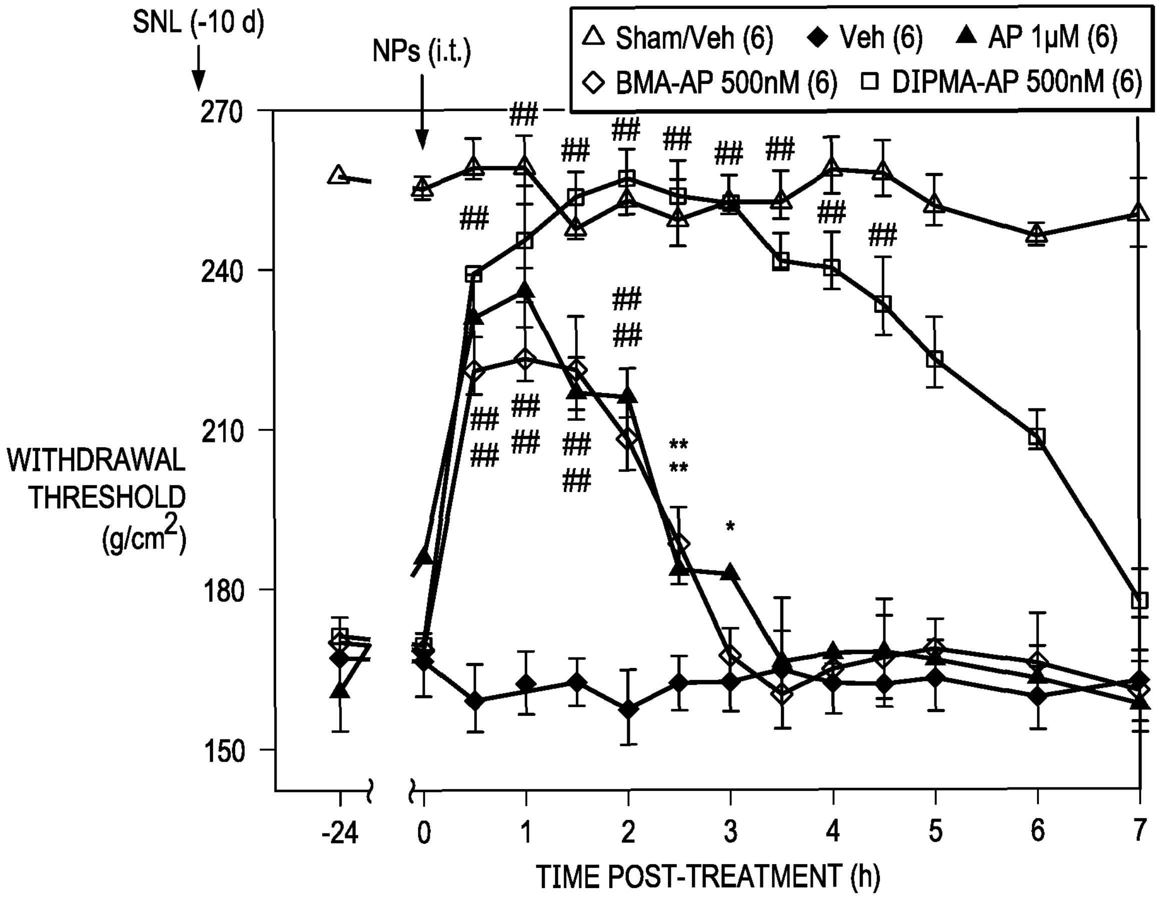


FIG. 19B

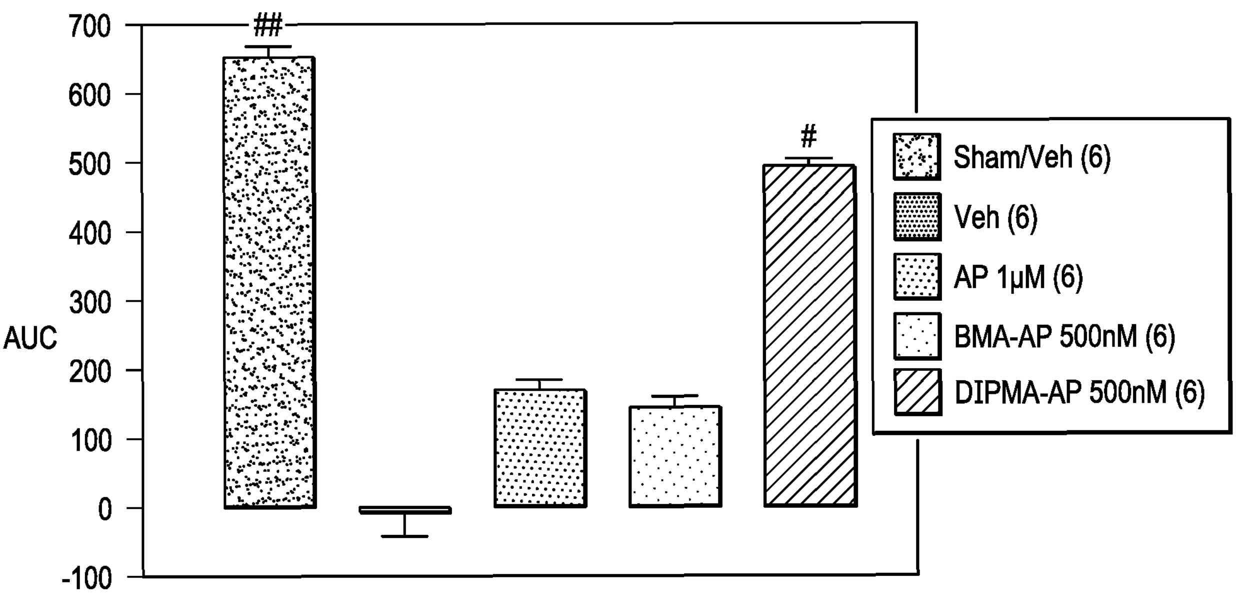

FIG. 20A

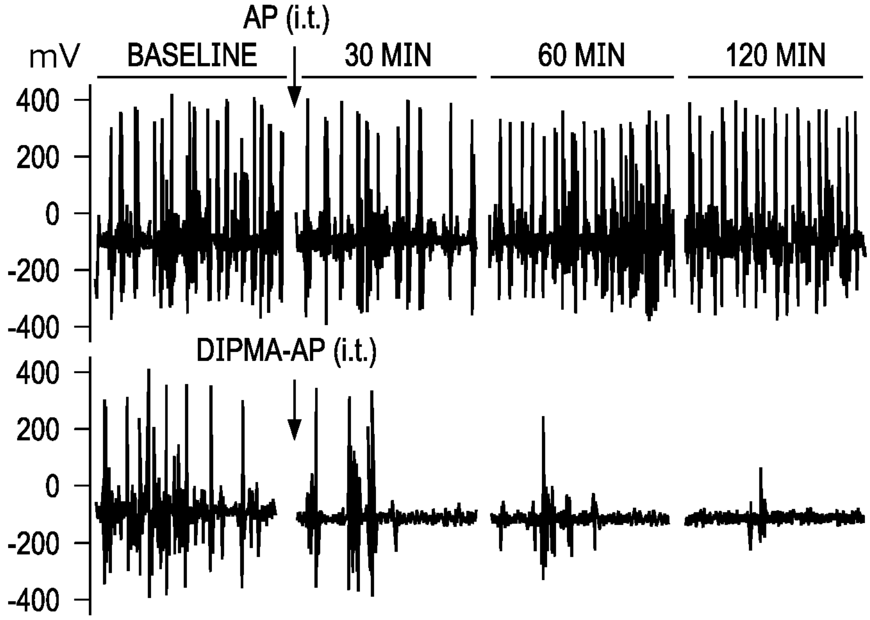


FIG. 20B

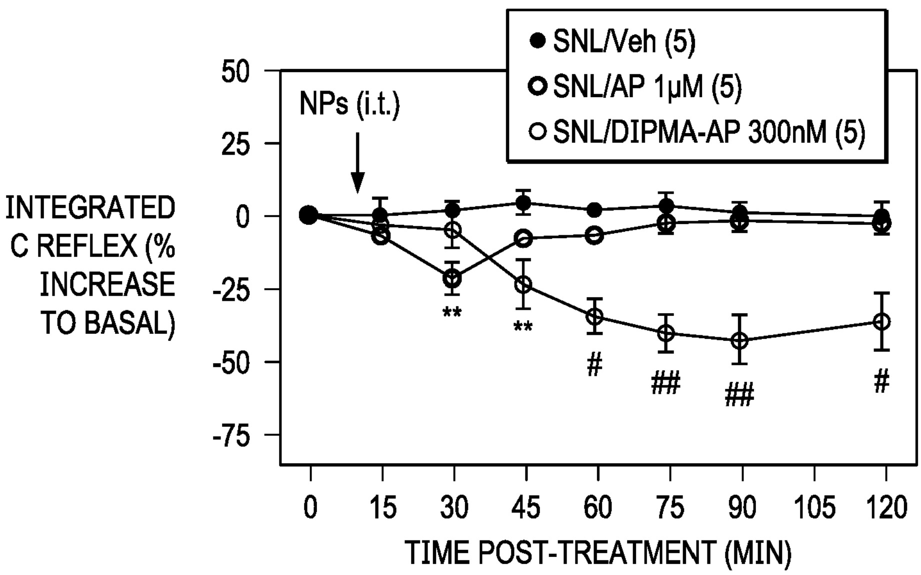

FIG. 20C

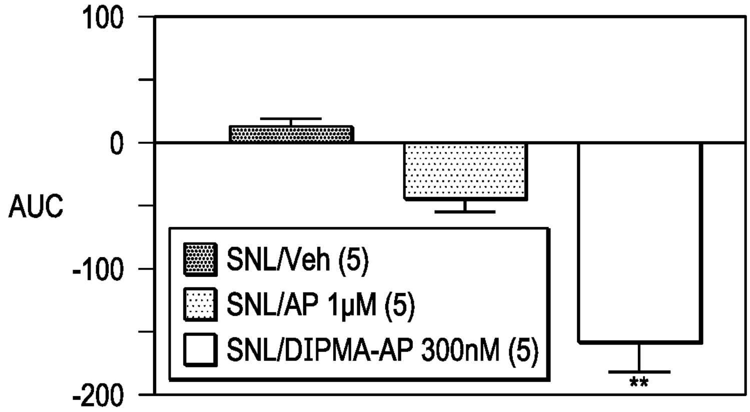


FIG. 20D

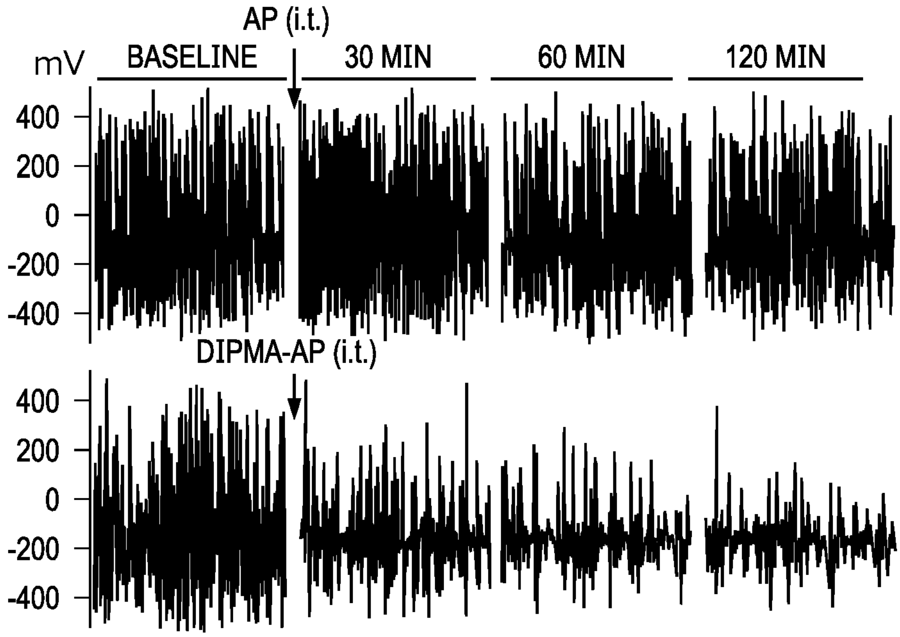

FIG. 20E

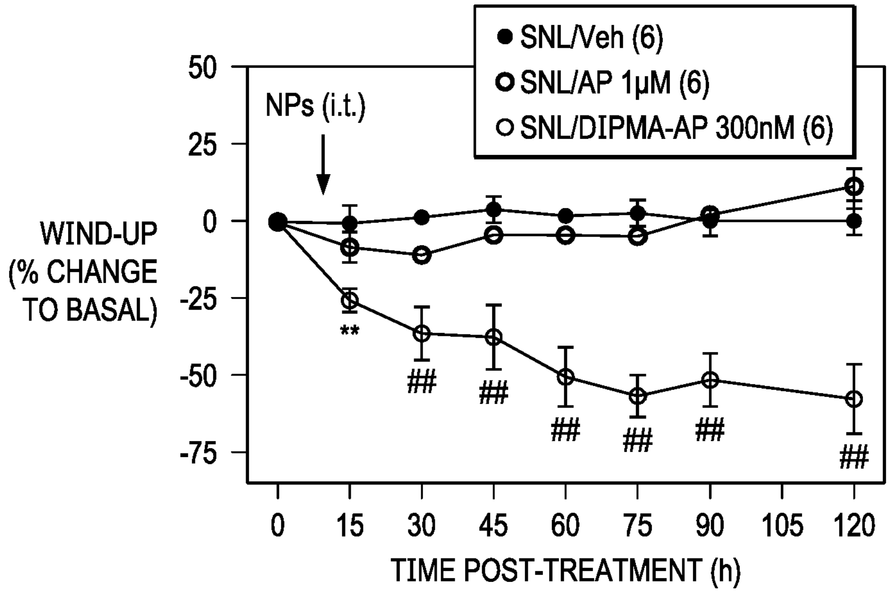


FIG. 20F

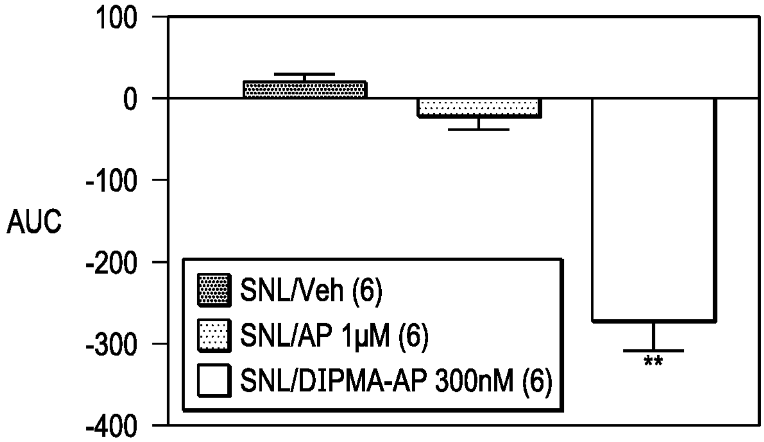

FIG. 20G

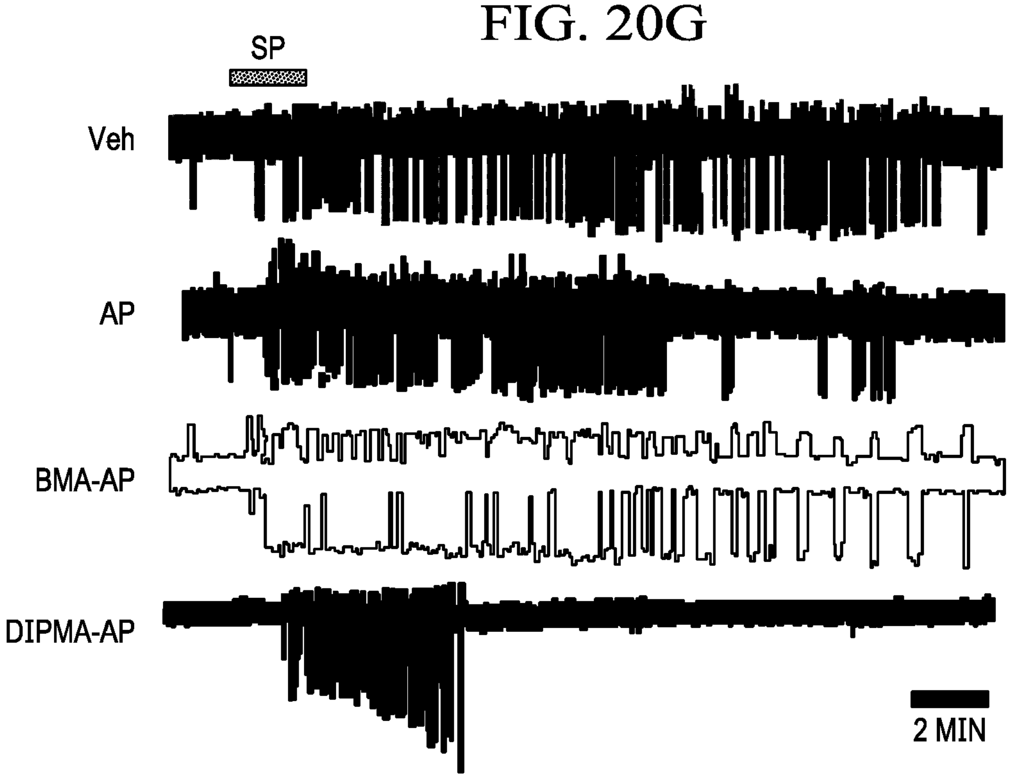

FIG. 20H

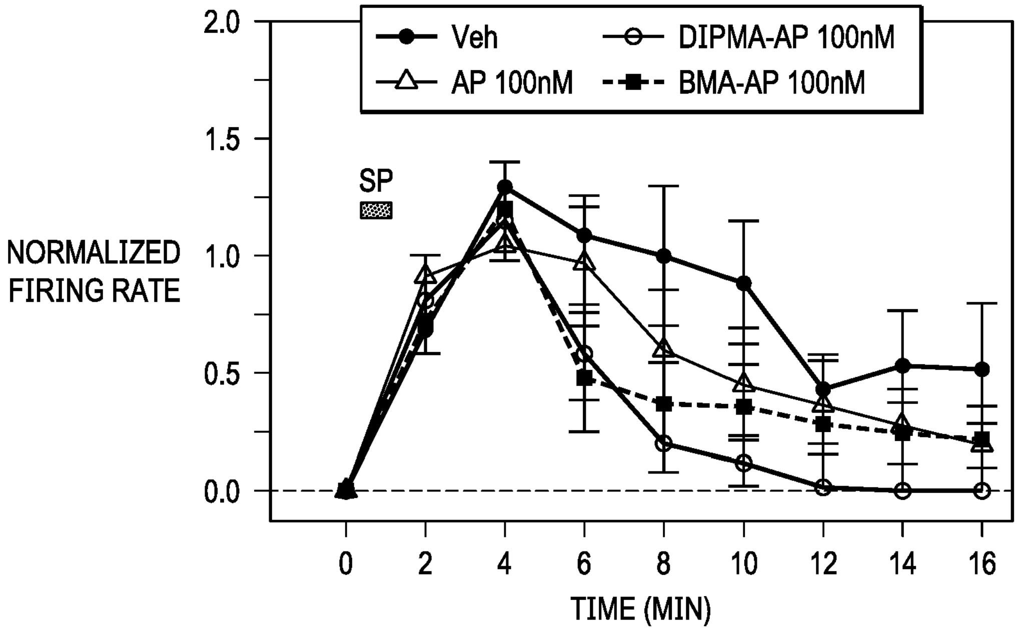


FIG. 20I

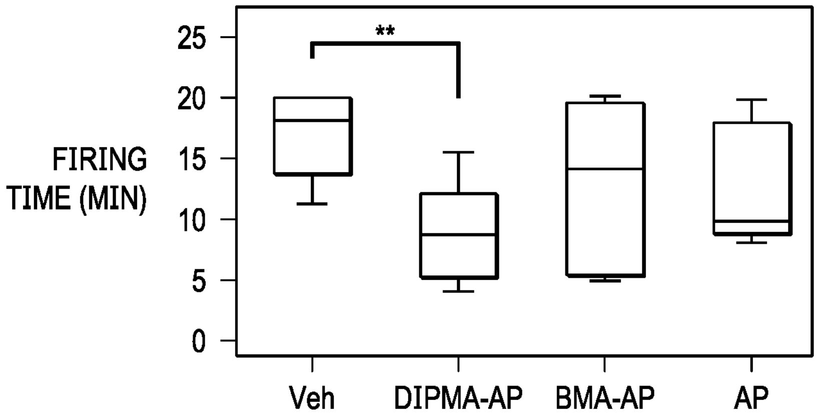

FIG. 21A

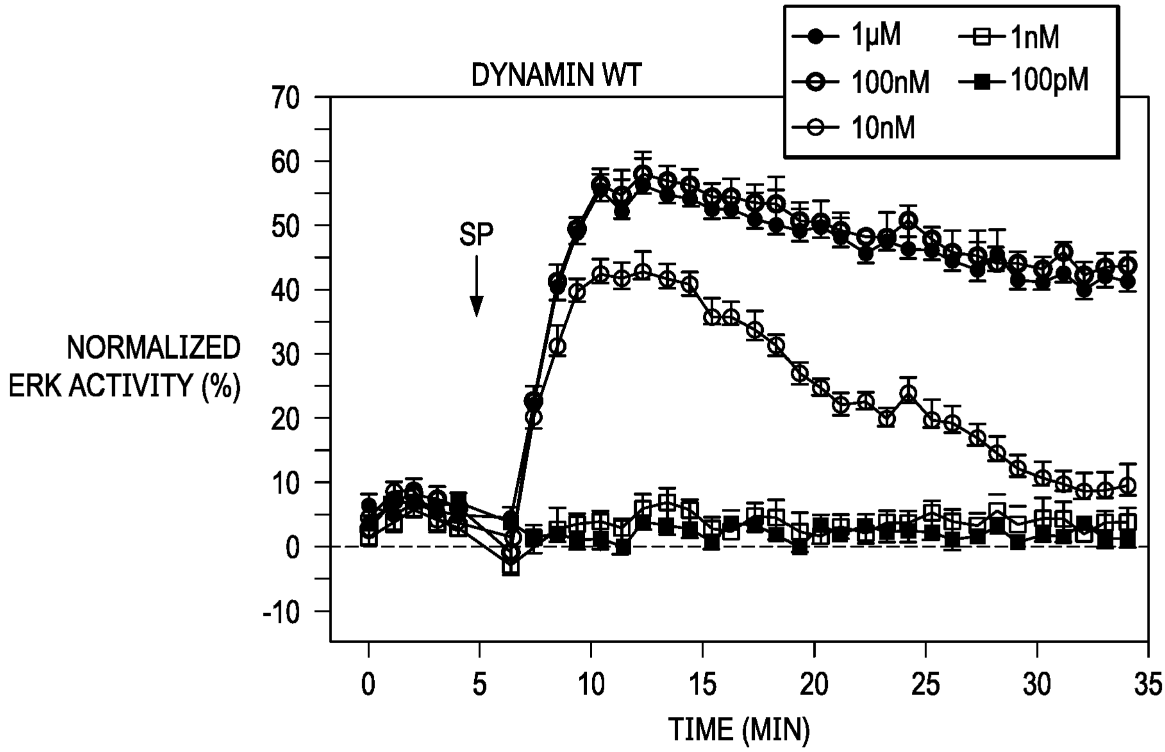


FIG. 21B

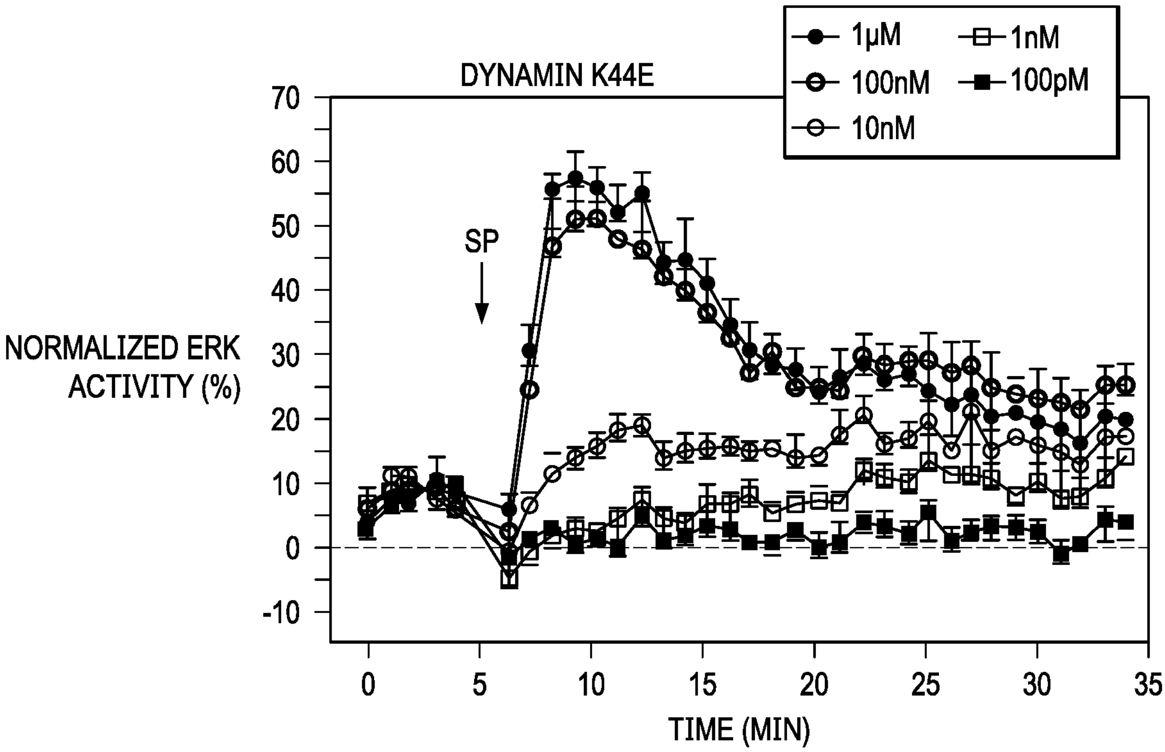

FIG. 21C

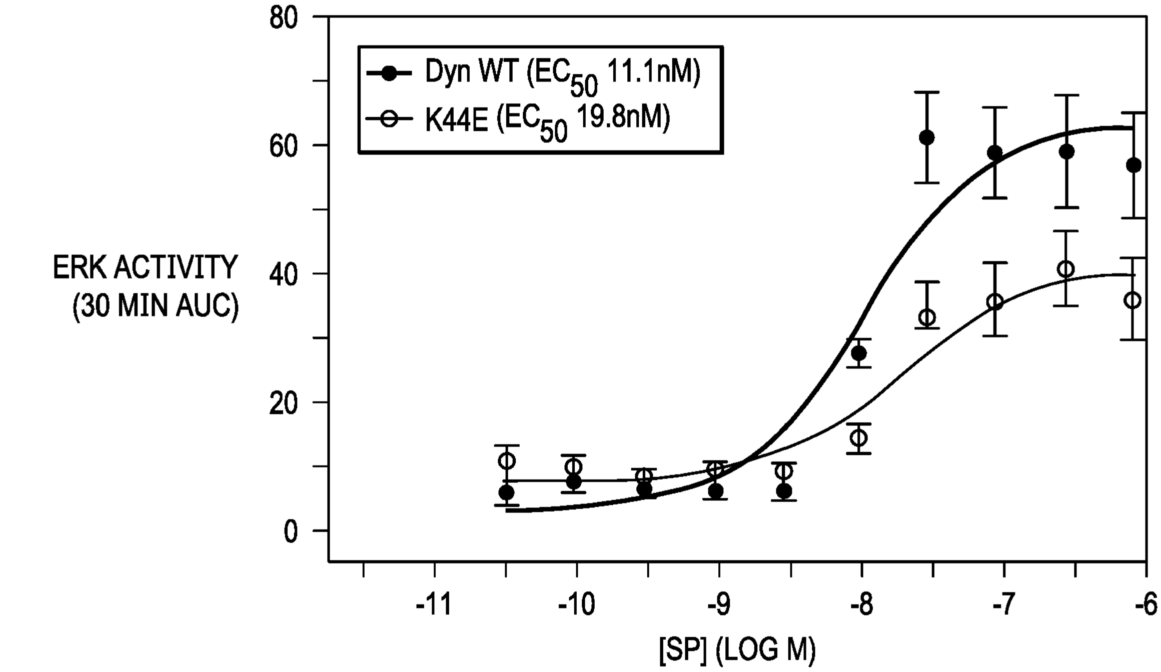


FIG. 21D

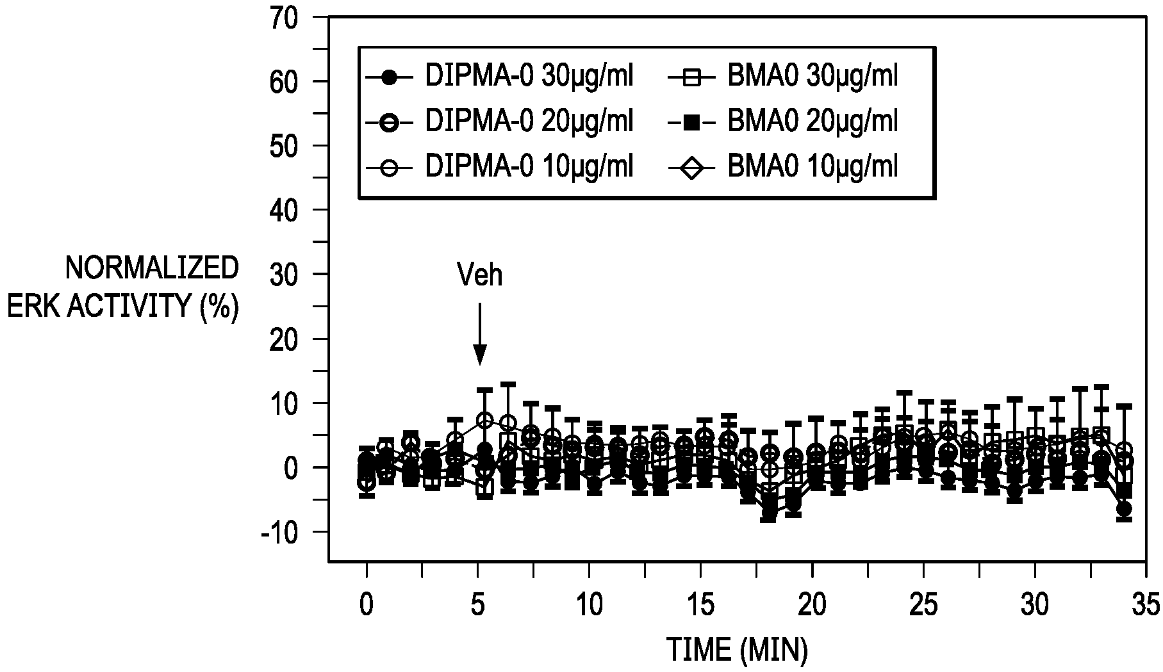

FIG. 21E

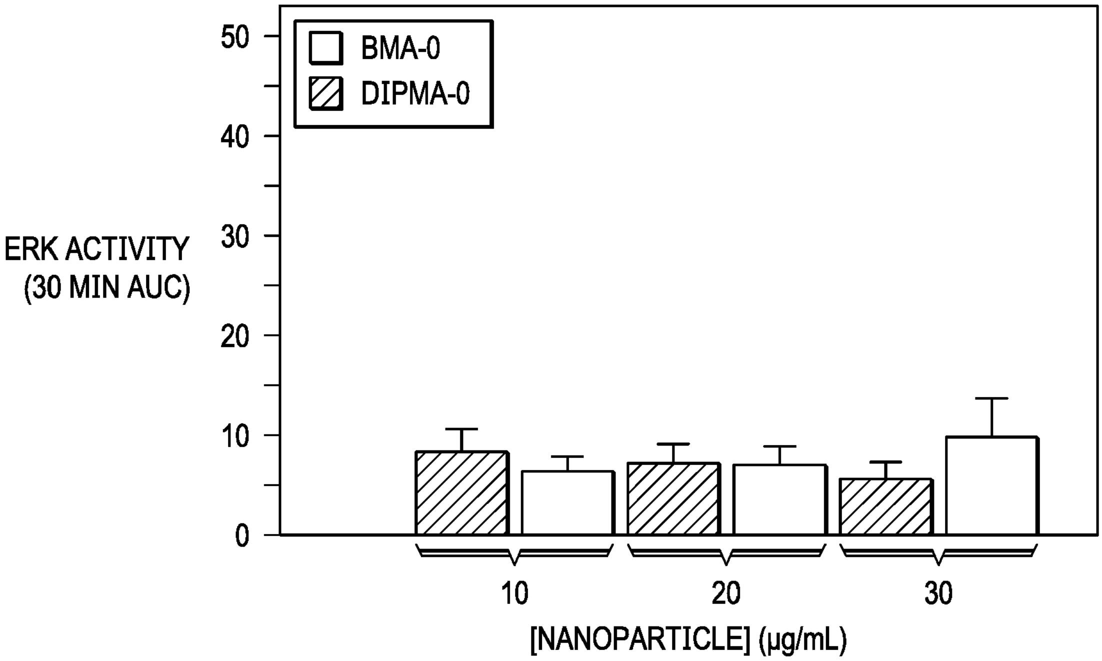


FIG. 21F

FIG. 21G

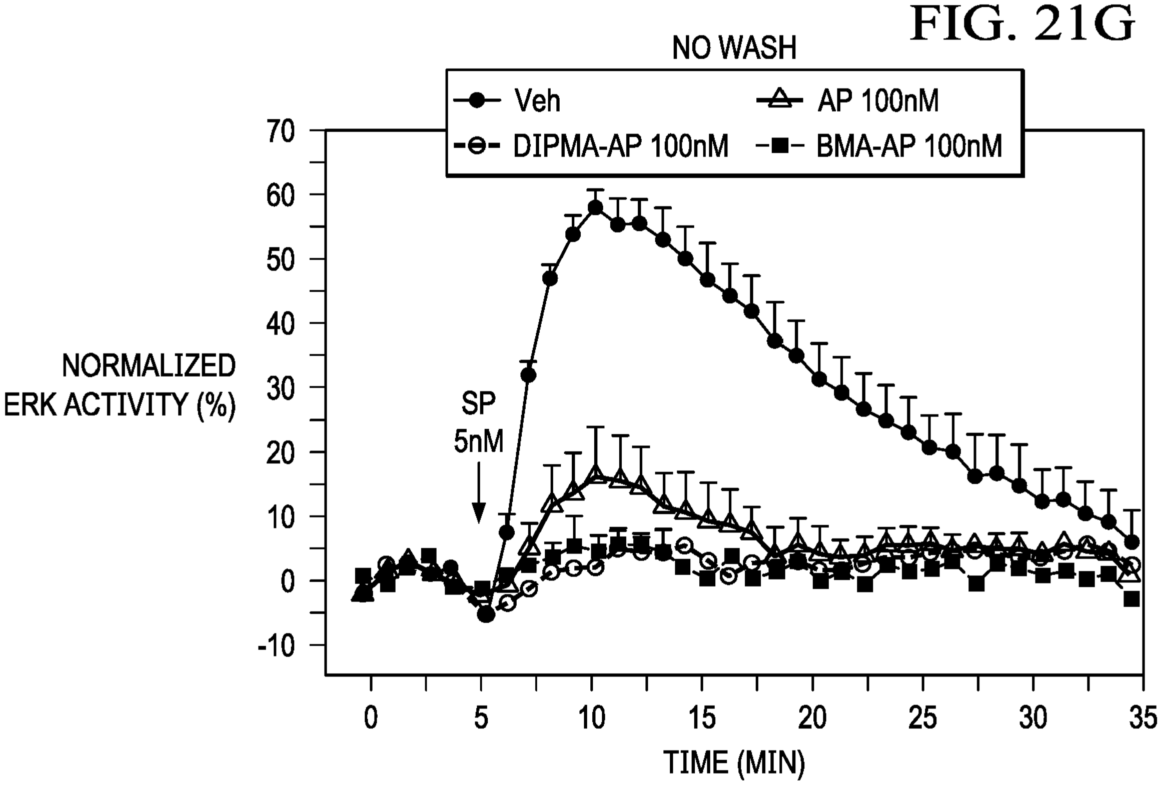

FIG. 21H

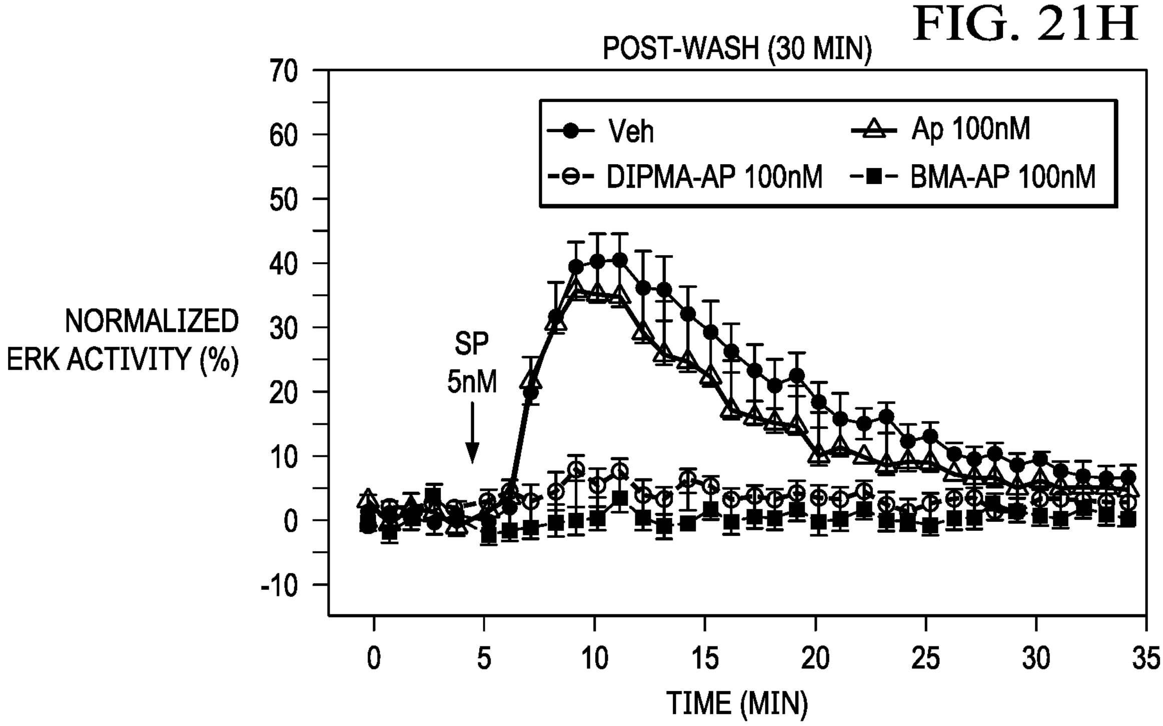


FIG. 21I

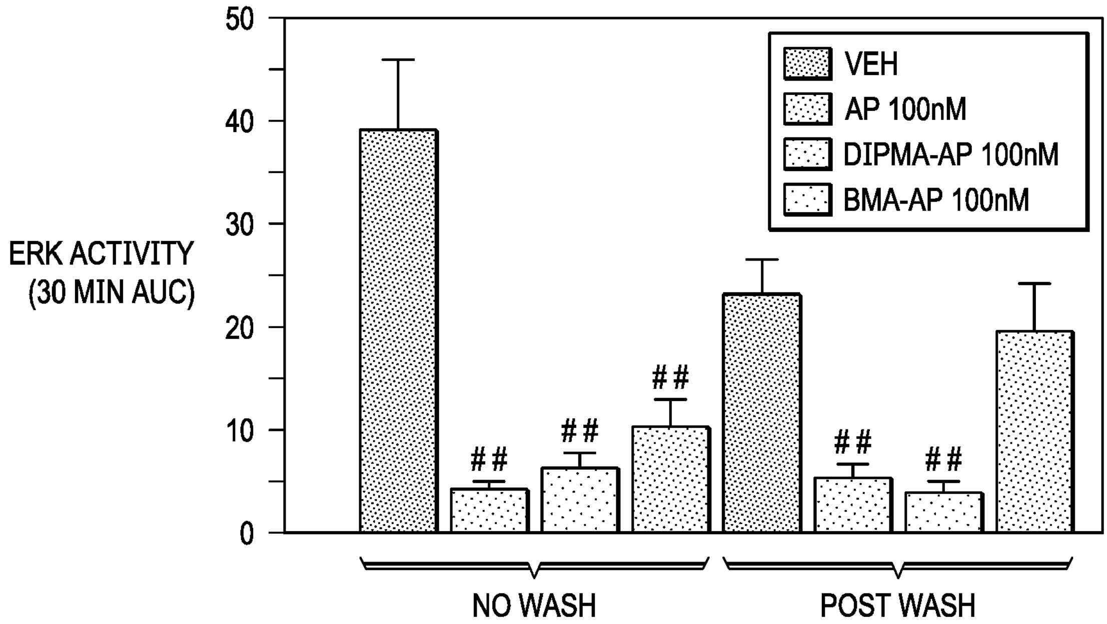

FIG. 22A

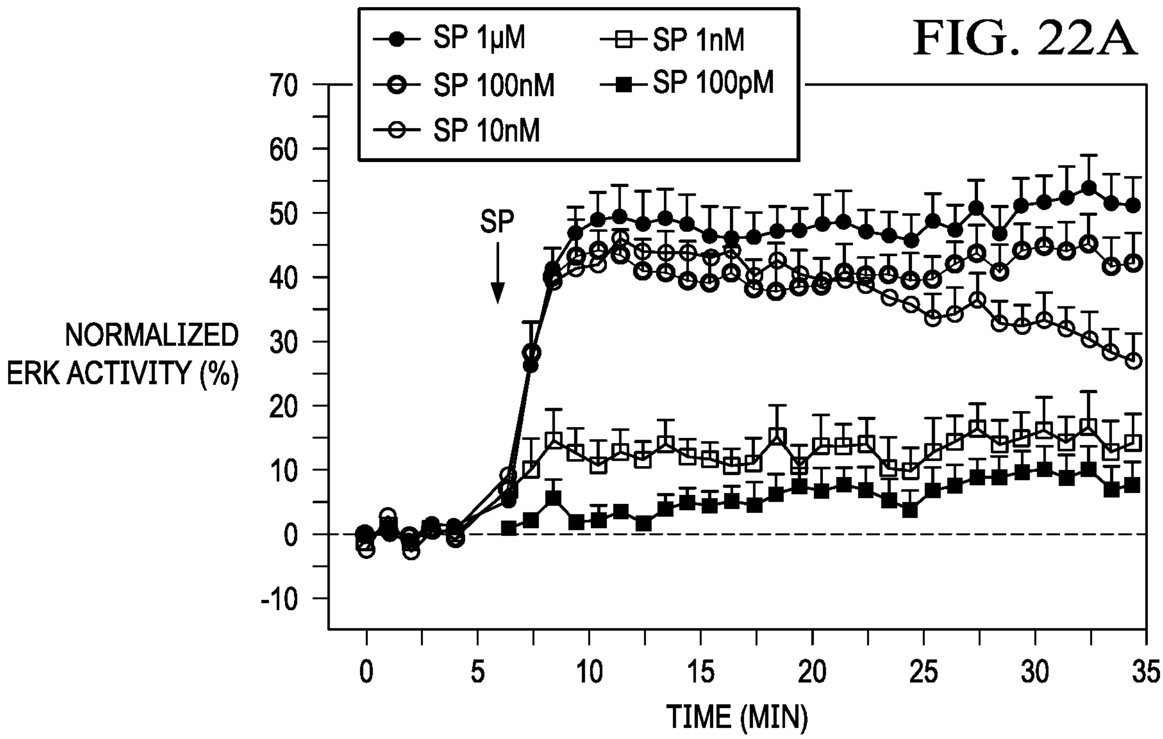


FIG. 22B

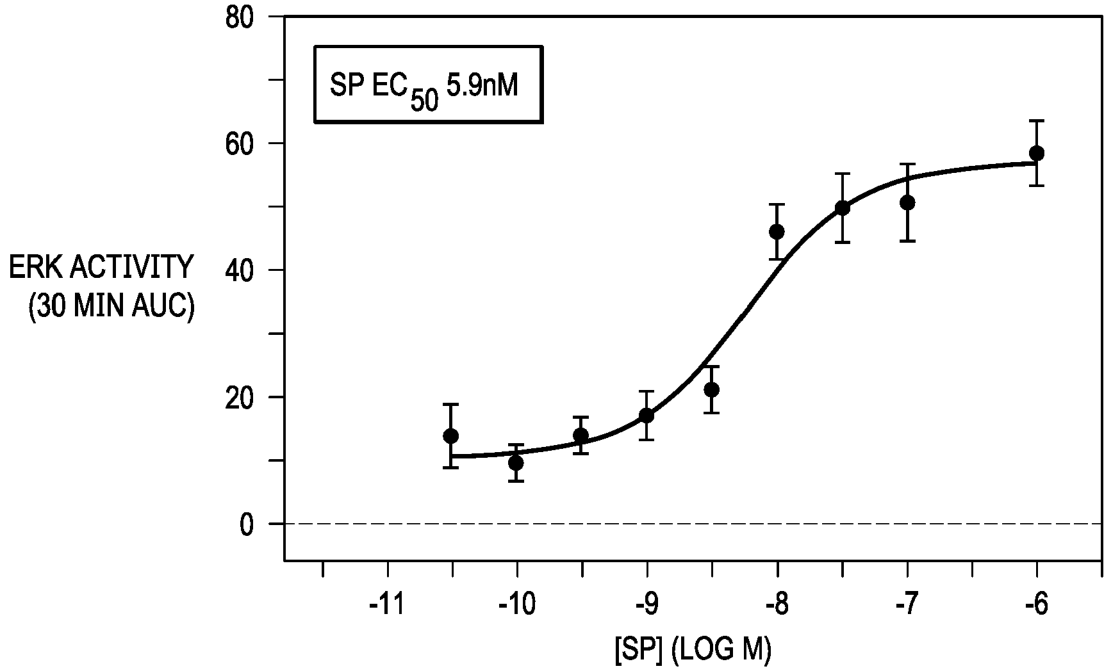

FIG. 22C

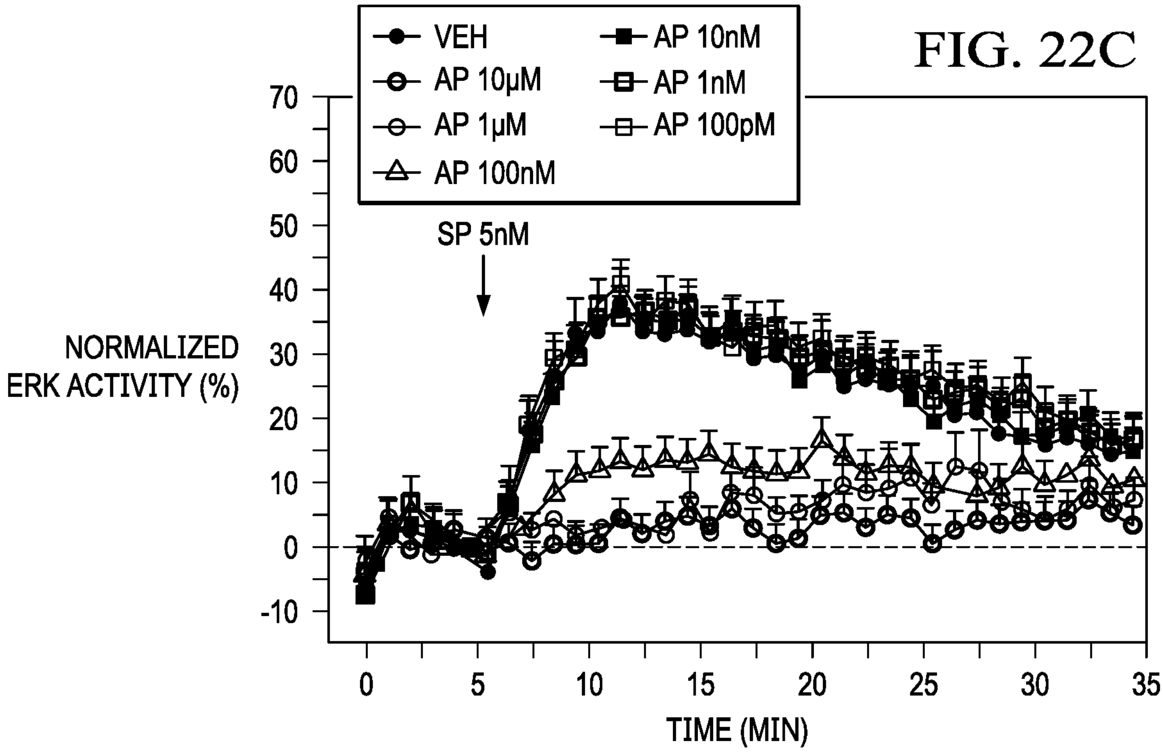


FIG. 22D

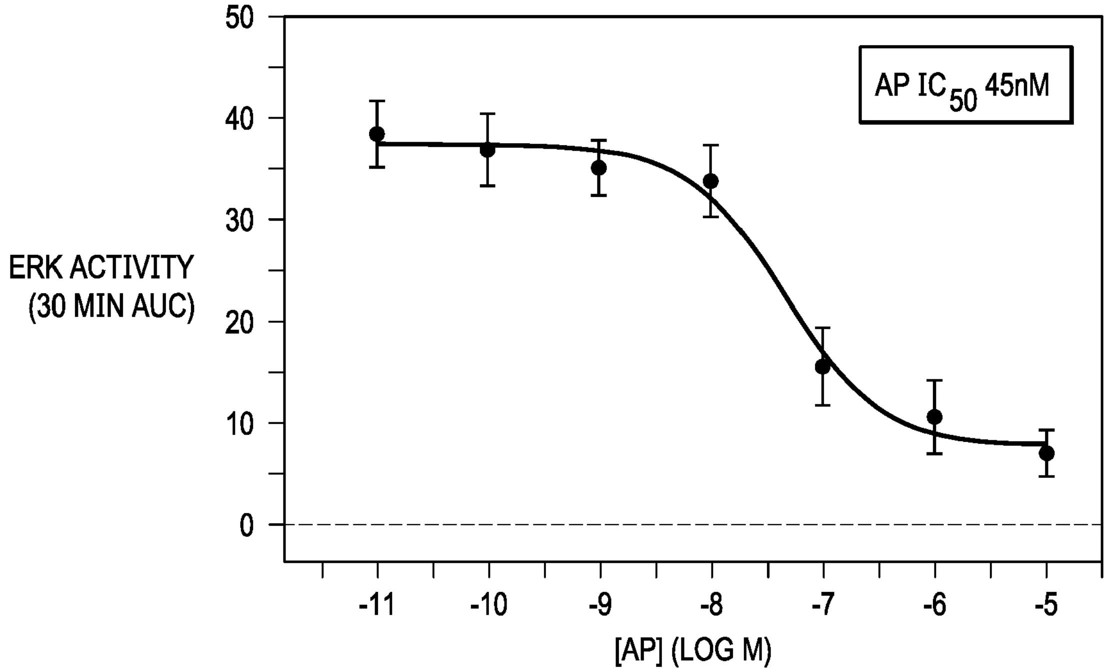

FIG. 23A

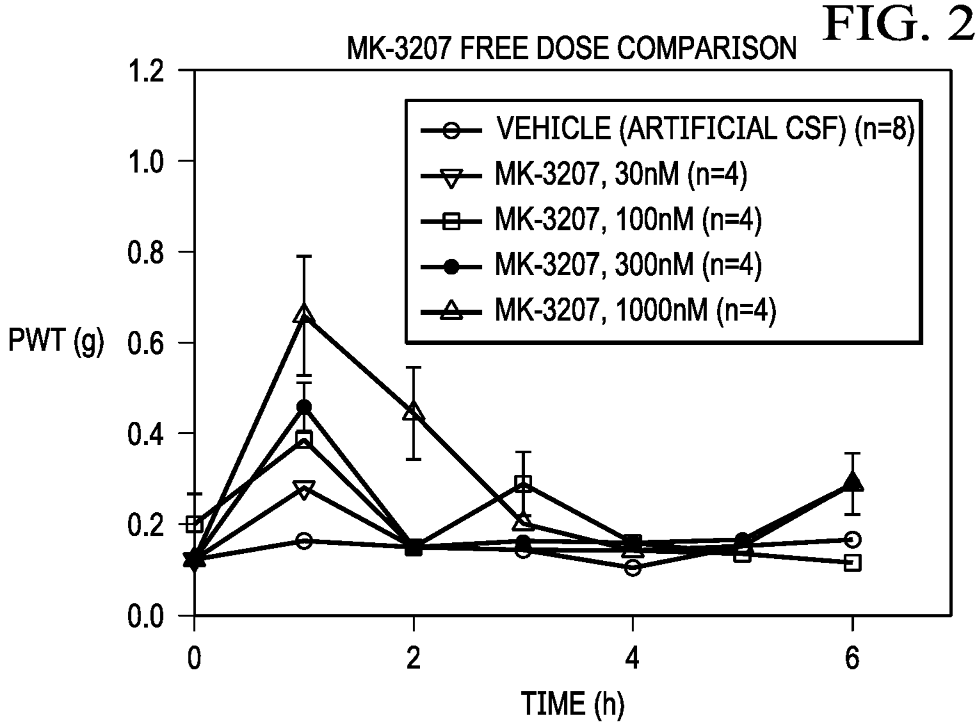


FIG. 23B

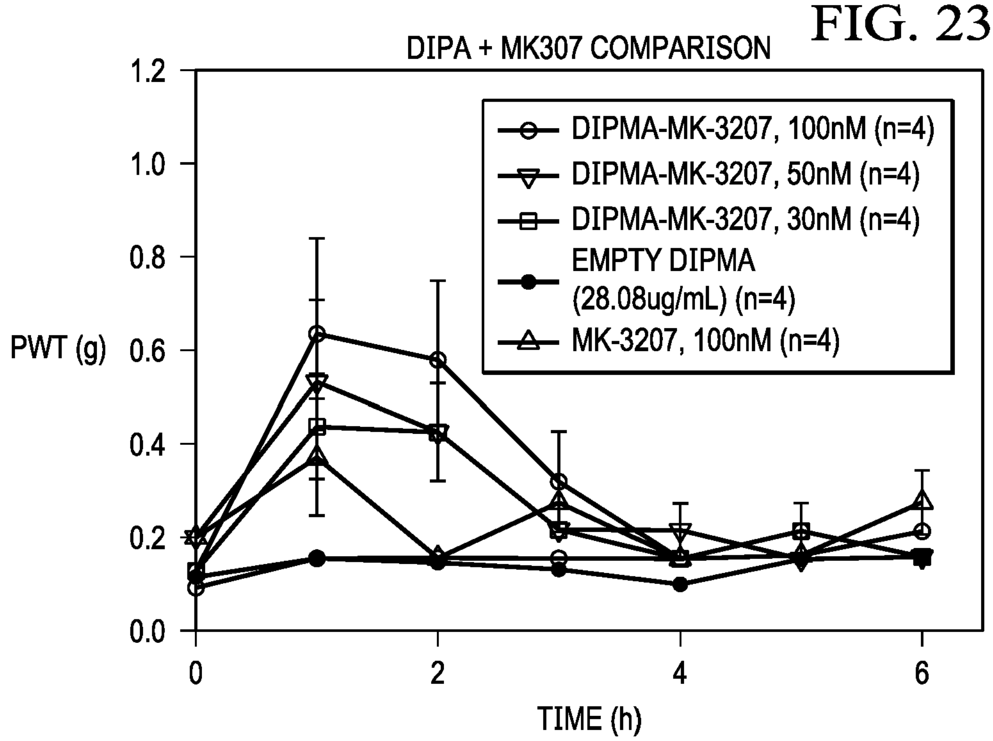

FIG. 24

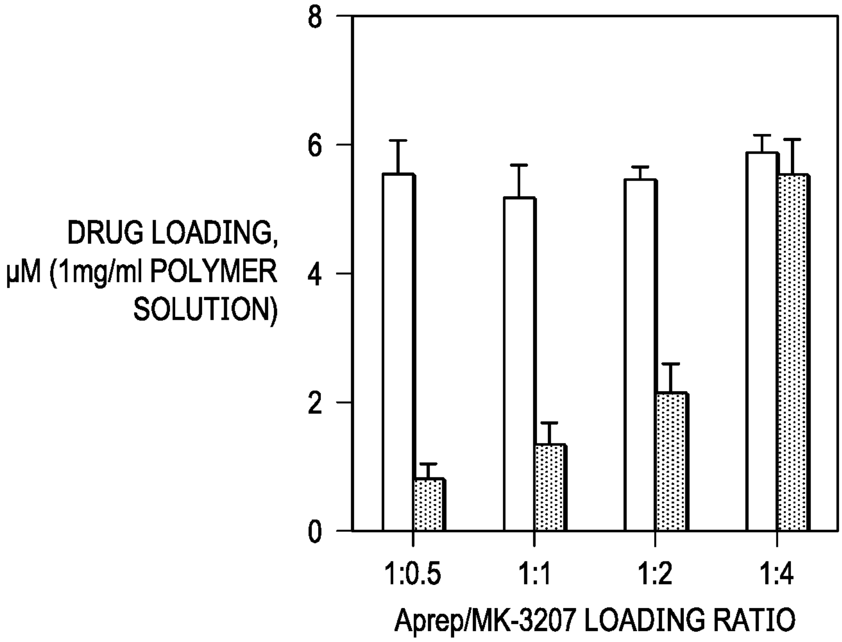
8
6
4
2
0
DRUG LOADING,
µM (1mg/ml POLYMER
SOLUTION)
1:0.5
1:1
1:2
1:4
Aprep/MK-3207 LOADING RATIO

FIG. 25

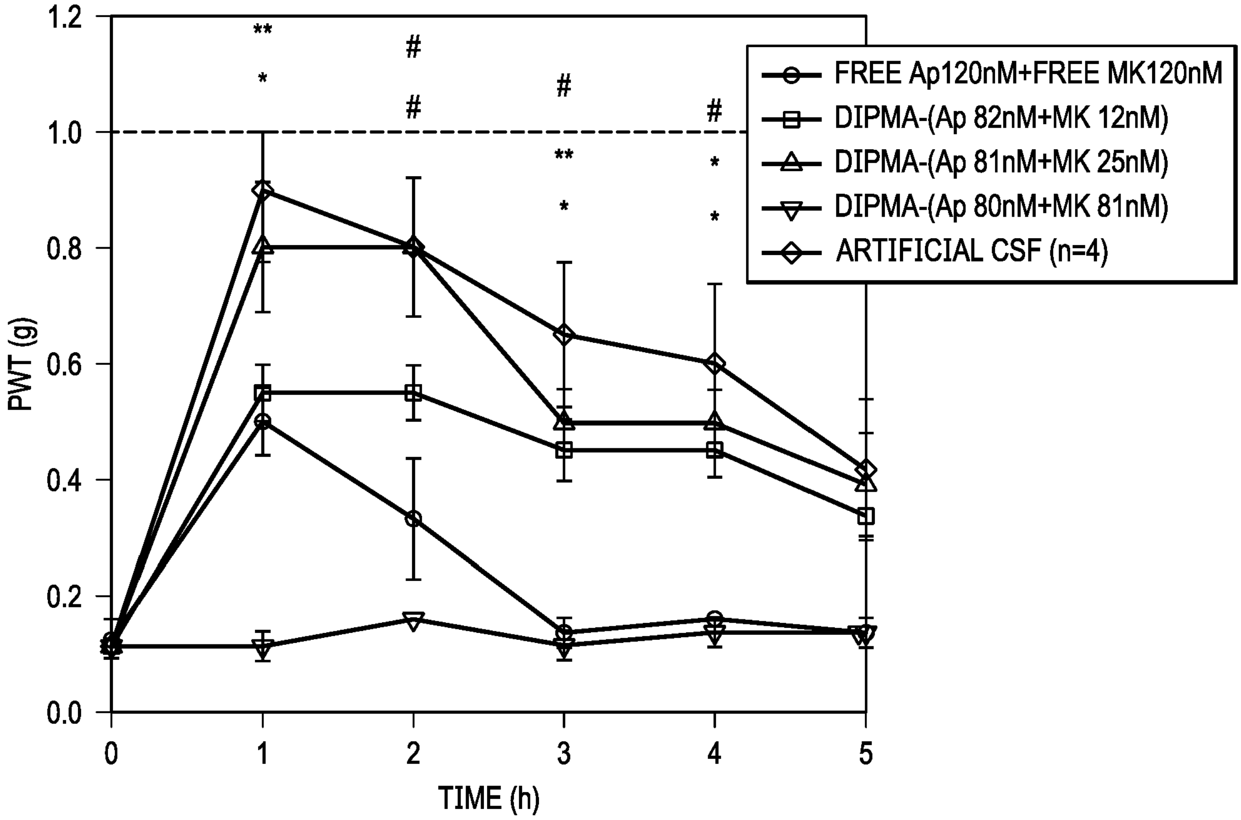
1.2
1.0
0.8
0.6
0.4
0.2
0.0
PWT (g)
0
1
2
3
4
5
TIME (h)
**
*
#
#
#
**
*
#
*
*
FREE Ap120nM+FREE MK120nM
DIPMA-(Ap 82nM+MK 12nM)
DIPMA-(Ap 81nM+MK 25nM)
DIPMA-(Ap 80nM+MK 81nM)
ARTIFICIAL CSF (n=4)

FIG. 26

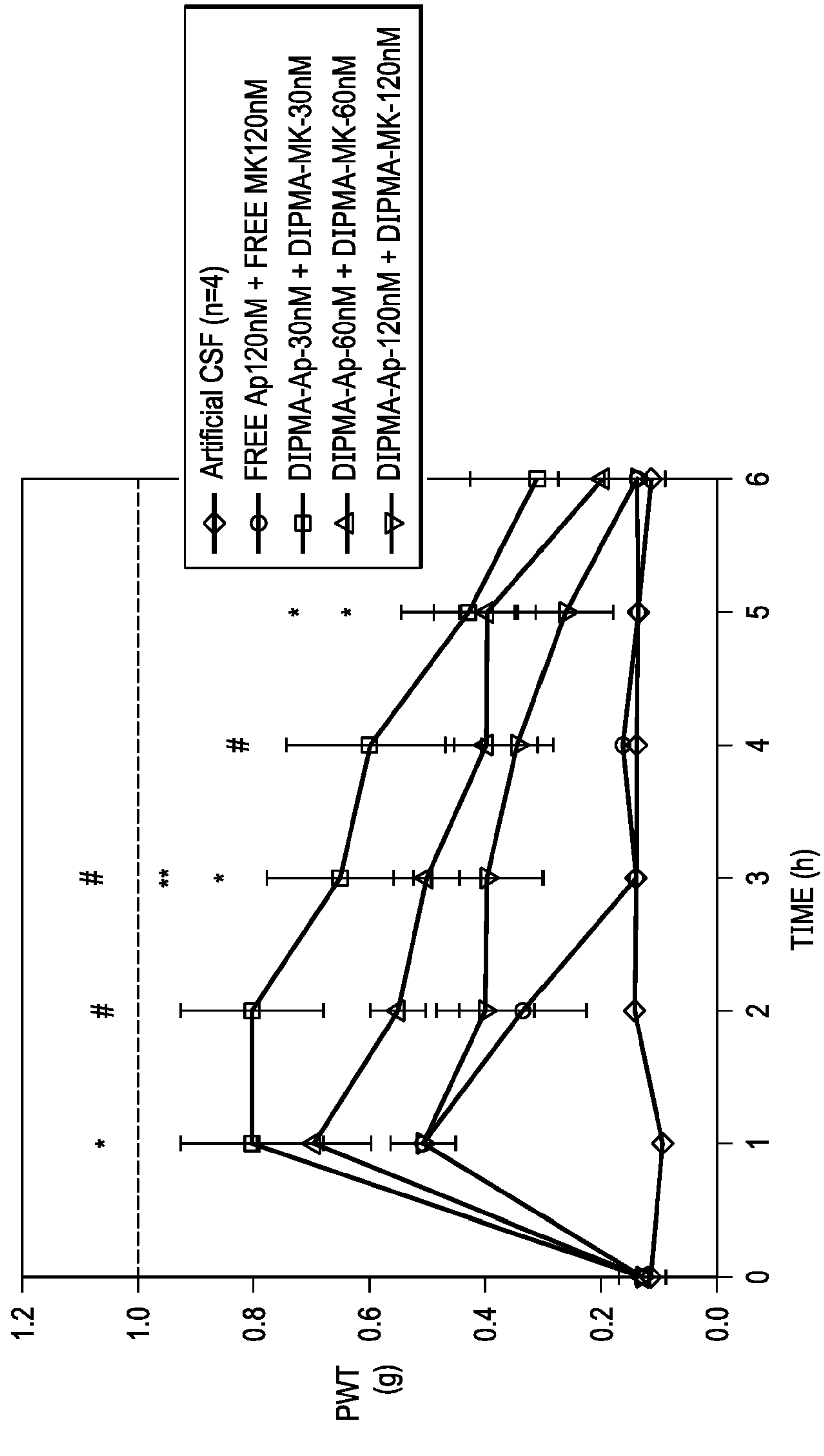
Artificial CSF (n=4)
FREE Ap120nM + FREE MK120nM
DIPMA-Ap-30nM + DIPMA-MK-30nM
DIPMA-Ap-60nM + DIPMA-MK-60nM
DIPMA-Ap-120nM + DIPMA-MK-120nM
PWT (g)
TIME (h)
1.2
1.0
0.8
0.6
0.4
0.2
0.0
0
1
2
3
4
5
6
*
#
#
**
*
#
*
*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- AU 2018903997
- WO 2016025747 A1

### Non-patent literature cited in the description


- **AUDET, M. ; BOUVIER, M.** *Nat Chem Biol,* 2008, vol. 4, 397-403
- **SATO, P. Y. et al.** *Physiological reviews,* 2015, vol. 95, 377-404
- **MURPHY, J. E. et al.** *Proc Natl Acad Sci USA,* 2009, vol. 106, 17615-17622
- **DEFEA, K. A. et al.** *Proc Natl Acad Sci USA,* 2000, vol. 97, 11086-11091
- **DEFEA, K. A. et al.** *J Cell Biol,* 2000, vol. 148, 1267-1281
- **DAAKA Y et al.** *J Biol Chem,* 1998, vol. 273, 685-688
- **LUTTERALL L. M. et al.** *Science,* 1999, vol. 283, 655-661
- **THOMSEN ARB et al.** *Trends in Pharmacological Sciences,* 01 September 2018, vol. 39 (10), 879-891
- **WEIWEI G et al.** *Molecular Pharmaceutics,* 06 December 2010, vol. 7 (6), 1913-1920
- **KEJIN Z et al.** *Angewandte Chemie International Edition,* 14 April 2011, vol. 50 (27), 6109-6114
- **XIN M et al.** *International Journal of Molecular Sciences,* 10 July 2018, vol. 19 (7), 2006
- **YARWOOD et al.** *Proc. Natl. Acad. Sci. USA.,* vol. 114 (46), 12309-12314
- **JENSEN, D.D. et al.** *Sci. Transl. Med.,* 2017, vol. 31 (9), 392
- **SAKURADA, T. et al.** *Neuropharmacology,* 1992, vol. 31 (12), 1279-85
- **STORY, G.M. et al.** *Cell,* 2003, vol. 112 (6), 819-29
- **KANE, P.M.** *Microbiol. Mol. Biol. Rev.,* 2006, vol. 70, 177-91
- **GREENLEE, R.Z.** Polymer Handbook. Wiley, 1989, II/53
- **MOAD G. ; RIZZARDO, E ; THANG S.** *H. Polymer,* 2008, vol. 49, 1079-1131
- **JENSEN, D.D. et al.** Neurokinin 1 receptor signaling in endosomes mediates sustained nociception and is a viable therapeutic target for prolonged pain relief. *Sci. Transl. Med.,* 2017, vol. 31 (9), 392
- **EVA SANTOS-NOGUEIRA et al.** *J Neurotrauma.,* 2012, vol. 29 (5), 898-904
- *Proc. Natl. Acad. Sci. USA.,* vol. 114 (46), 12309-12314